# FASCICULE DE BREVET EUROPEEN

(11) **EP 1 047 387 B1**
(45) Date de publication et mention de la délivrance du brevet: **08.09.2004**
(21) Numéro de dépôt: 99934800.6
(22) Date de dépôt: 28.07.1999
(51) Int. Cl.: A61K 7/13

(54) **COMPOSITION DE TEINTURE POUR FIBRES KERATINIQUES AVEC UN COLORANT DIRECT CATIONIQUE ET UN SEL D'AMMONIUM QUATERNAIRE**
FÄRBEMITTEL FÜR KERATINFASERN, DAS EIN KATIONISCHER FARBSTOFFE UND QUATERNARE AMMONIUM-SALZE ENTHÄLT
DYEING COMPOSITION FOR KERATINOUS FIBRES WITH DIRECT CATIONIC COLOURING AGENT AND A QUATERNARY AMMONIUM SALT

(30) Priorité: 19.08.1998 FR 9810547
(43) Date de publication de la demande: 02.11.2000
(73) Titulaire: L'OREAL, 75008 Paris (FR)
(72) Inventeur: RONDEAU, Christine, F-78500 Sartrouville (FR)
(74) Mandataire: Miszputen, Laurent
(86) Numéro de dépôt international: PCT/FR1999/001865
(87) Numéro de publication internationale: WO 2000/010517

(56) Documents cités:
- EP-A- 0 312 343
- EP-A- 0 705 597
- EP-A- 0 819 422
- EP-A- 0 850 637
- EP-A- 0 850 638
- WO-A-95/01772
- WO-A-95/15144
- WO-A-99/20234
- WO-A-99/20235
- FR-A- 2 140 205
- FR-A- 2 189 006
- FR-A- 2 282 860
- FR-A- 2 285 851
- US-A- 4 168 144

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins un sel d'ammonium quaternaire.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, Il arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes sur le plan de la puissance, de l'homogénéité de la couleur répartie le long de la fibre, on dit alors que la coloration est trop sélective, et sur le plan de la tenacité, en terme de résistance aux diverses agressions que peuvent subir les cheveux (lumière, intempéries,shampooings).

Or, après d'importantes recherches menées sur la question, la demanderesse vient maintenant de découvrir qu'il est possible d'obtenir de nouvelles compositions pour la teinture des fibres kératiniques capables de conduire à des colorations puissantes et peu sélectives et résistant bien néanmoins aux diverses agressions que peuvent subir les cheveux, en associant au moins un sel d'ammonium quaternaire à au moins un colorant direct cationique connu de l'art antérieur et de formules respectivement définies ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, **(i)**au moins un colorant direct cationique dont la structure répond aux formules (I) à (IV) définies ci-après, caractérisée par le fait qu'elle contient en outre **(ii)**au moins un sel d'ammonium quaternaire.
**(i)** Le colorant direct cationique utilisable selon la présente invention est un composé choisi parmi ceux de formules (I), (II), (III), (III'), (IV) suivantes :

   **a) les composés de formule (I) suivante :** 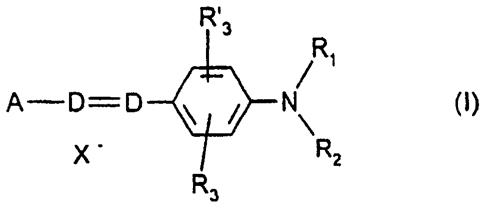 dans laquelle :
      D représente un atome d'azote ou le groupement -CH,
      R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
      R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
      X⁻ représente un anion de préférence choisi parmi le chlorure, le méthyl sulfate et l'acétate,
      A représente un groupement choisi par les structures A1 à A18 suivantes : 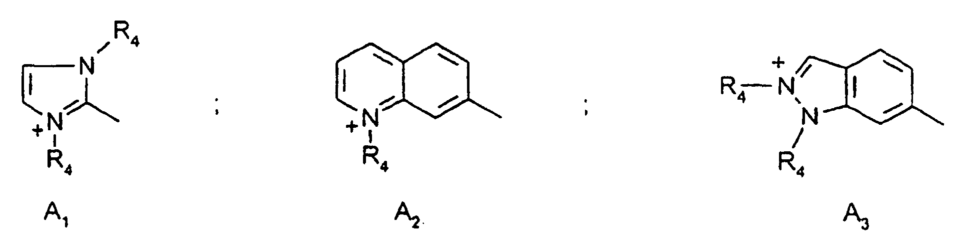 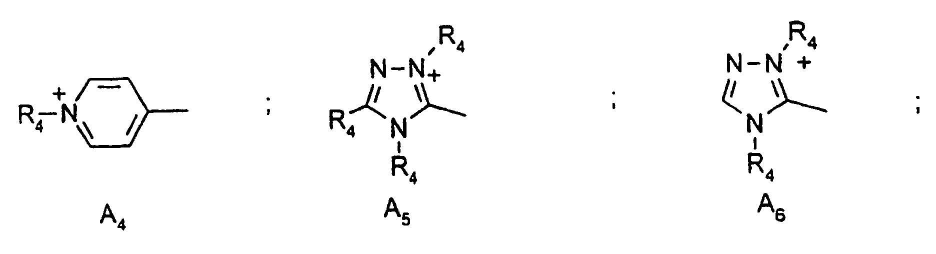 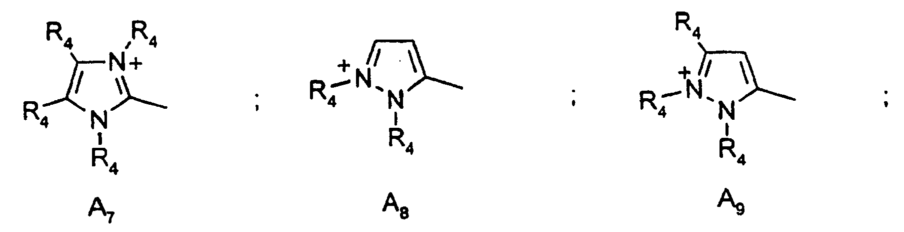 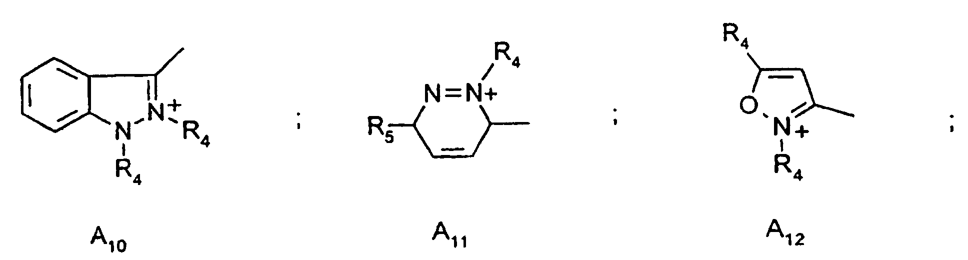 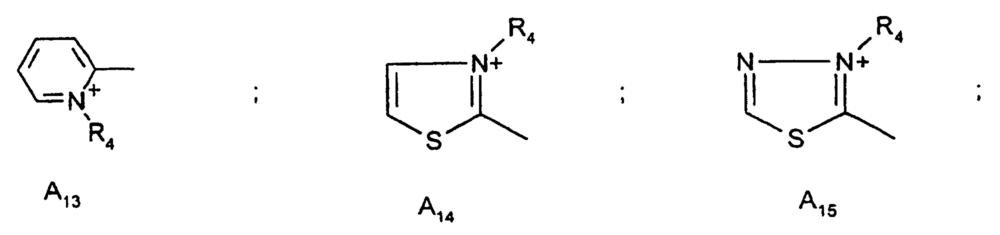 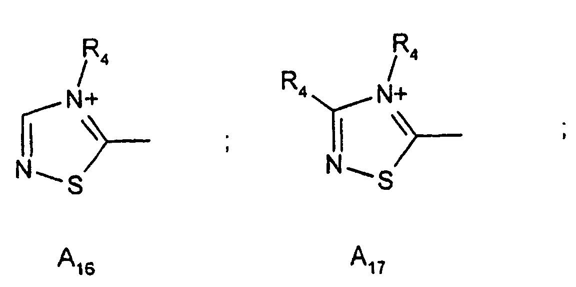 et 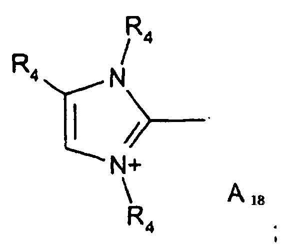 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
   **b) les composés de formule (II) suivante :** 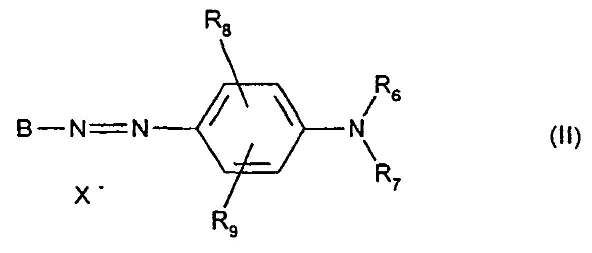 dans laquelle :
      R₆ représente un atome d'hydrogène ou un radical alkyle en C₁-C₄,
      R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
      R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
      X⁻ représente un anion de préférence choisi parmi le chlorure, le méthyl sulfate et l'acétate,
      B représente un groupement choisi par les structures B1 à B6 suivantes : 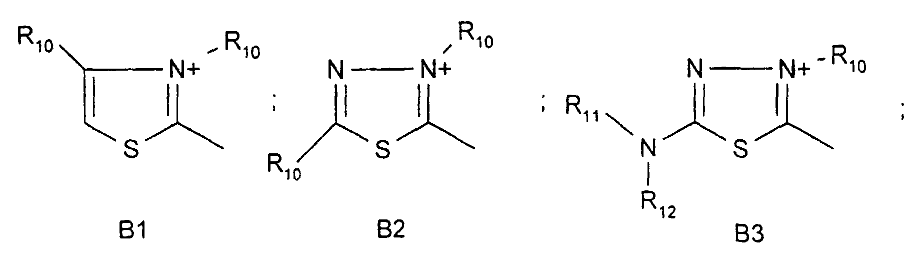 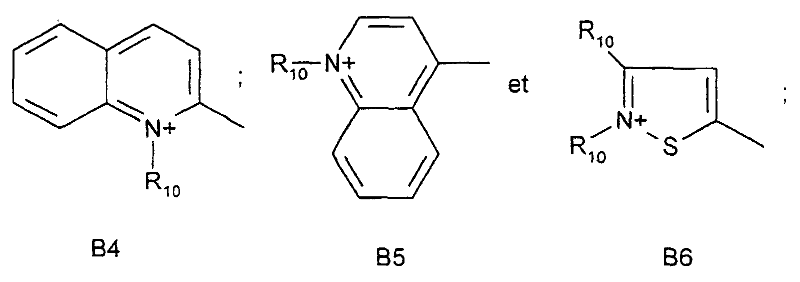 dans lesquelles R₁₀ représente un radical alkyle en C₁-C₄, R₁₁, et R₁₂, identiques ou différents, représentent un atome d'hydrogène ou un radical alkyle en C₁-C₄ ;
   **c) les composés de formules (III) et (III') suivantes :** 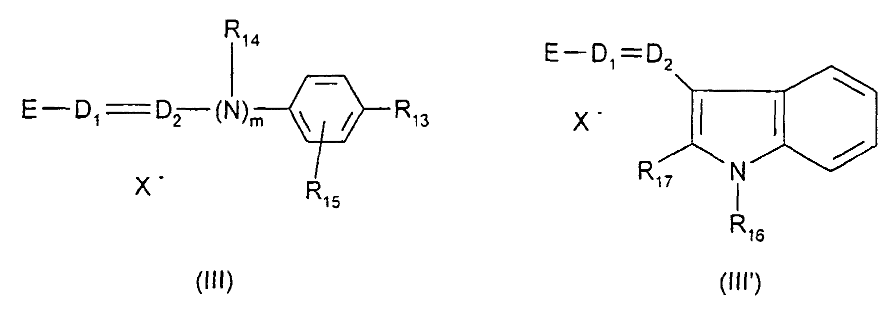 dans lesquelles :
      R₁₃ représente un atome d'hydrogène, un radical alcoxy en C₁-C₄, un atome d'halogène tel que le brome, le chlore, l'iode ou le fluor ou un radical amino,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      R₁₆ et R₁₇, identiques ou différents, représentent un atome d'hydrogène ou un radical alkyle en C₁-C₄,
      D₁ et D₂, identiques ou différents, représentent un atome d'azote ou le groupement -CH,
      m=0 ou 1,
      étant entendu que lorsque R₁₃ représente un groupement amino non substitué, alors D₁ et D₂ représentent simultanément un groupement -CH et m = 0,
      X⁻ représente un anion de préférence choisi parmi le chlorure, le méthyl sulfate et l'acétate,
      E représente un groupement choisi par les structures E1 à E8 suivantes : 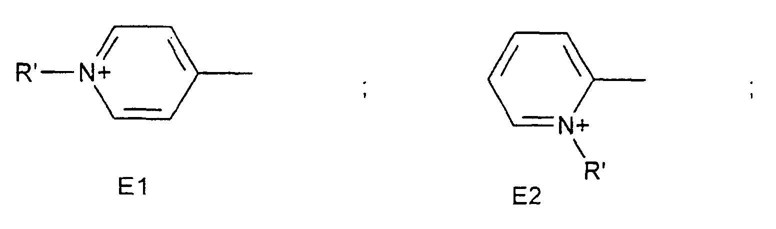 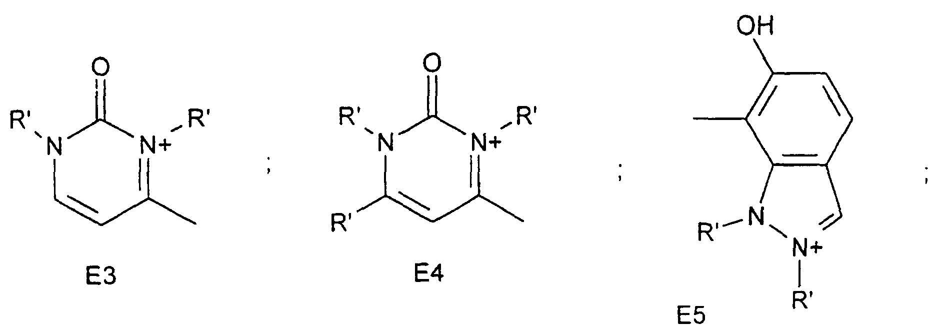 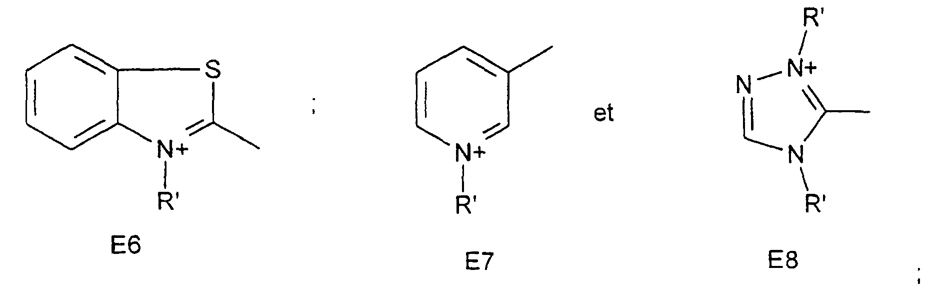 dans lesquelles R' représente un radical alkyle en C₁-C₄ ;
      lorsque m = 0 et que D₁ représente un atome d'azote, alors E peut également désigner un groupement de structure E9 suivante : 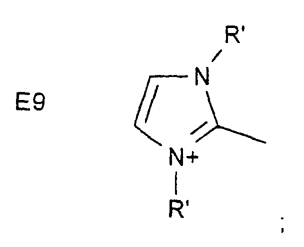 dans laquelle R' représente un radical alkyle en C₁-C₄.
   **d) les composés de formule (IV) suivante :**

      G―N=N―J (IV)
   dans laquelle :
   **le symbole G** représente un groupement choisi parmi les structures G₁ à G₃ suivantes : 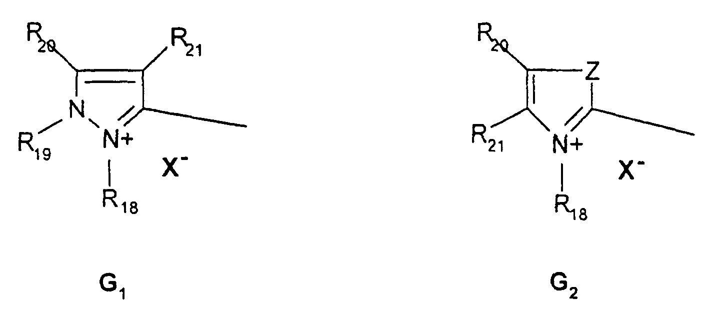 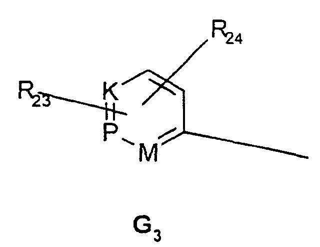 structures G₁ à G₃ dans lesquelles,
   R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
   R₁₉ désigne un radical alkyle en C₁-C₄ ou un radical phényle;
   R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄, alcoxy en C₁-C₄, ou NO₂;
   R₂₀ peut désigner en outre un atome d'hydrogène;
   Z désigne un atome d'oxygène, de soufre ou un groupement -NR₁₉;
   M représente un groupement -CH, -CR (R désignant alkyle en C₁-C₄), ou -NR₂₂(X⁻)ᵣ;
   K représente un groupement -CH, -CR (R désignant alkyle en C₁-C₄), ou -NR₂₂(X⁻)r;
   P représente un groupement -CH, -CR (R désignant alkyle en C₁-C₄), ou -NR₂₂(X⁻)ᵣ; r désigne zéro ou 1;
   R₂₂ représente un atome O⁻, un radical alcoxy en C₁-C₄, ou un radical alkyle en C₁-C₄;
   R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂:
   X⁻ représente un anion de préférence choisi parmi le chlorure, l'iodure, le méthyl sulfate, l'éthyl sulfate, l'acétate et le perchlorate;
   sous réserve que,
   si R₂₂ désigne O⁻, alors r désigne zéro;
   si K ou P ou M désignent -N-alkyle C₁-C₄ X⁻ , alors R₂₃ ou R₂₄ est différent d'un atome d'hydrogène;
   si K désigne -NR₂₂(X⁻)ᵣ, alors M= P= -CH, -CR;
   si M désigne -NR₂₂(X⁻)ᵣ, alors K= P= -CH, -CR;
   si P désigne -NR₂₂(X⁻)ᵣ, alors K= M et désignent -CH ou -CR;
   si Z désigne un atome de soufre avec R₂₁ désignant alkyle en C₁-C₄, alors R₂₀ est différent d'un atome d'hydrogène;
   si Z désigne -NR₁₉ avec R₁₉ désignant alkyle en C₁-C₄, alors au moins l'un des radicaux R₁₈, R₂₀ ou R₂₁ du groupement de structure G₂ est différent d'un radical alkyle en C₁-C₄;
   **le symbole J** représente :
   **-(a)** un groupement de structure J₁ suivante : 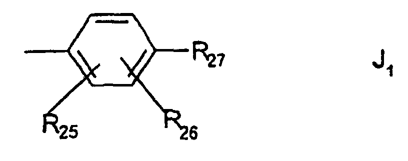 structure J₁ dans laquelle,
      R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
      R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre;
      R₂₇ représente un atome d'hydrogène, un radical -OH, un radical -NHR₂₈, un radical -NR₂₉R₃₀;
      R₂₈ représente un atome d'hydrogène, un radical alkyle en C₁-C₄, un radical monohydroxyalkyle en C₁-C₄, polyhydroxyalkyle en C₂-C₄, un radical phényle;
      R₂₉ et R₃₀, identiques ou différents, représentent un radical alkyle en C₁-C₄ , un radical monohydroxyalkyle en C₁-C₄, polyhydroxyalkyle en C₂-C₄;
   **-(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 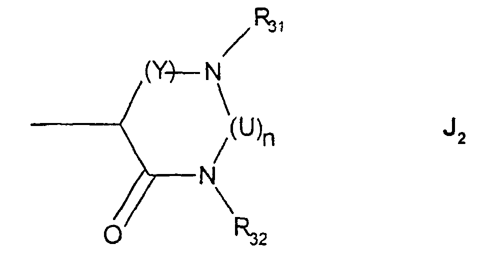 structure J₂ dans laquelle,
      R₃₁ et R₃₂, identiques ou différents, représentent un atome d'hydrogène, un radical alkyle en C₁-C₄, un radical phényle;
      Y désigne le radical -CO- ou le radical 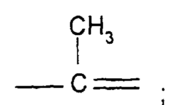 n = 0 ou 1, avec, lorsque n désigne 1, U désigne le radical -CO- .

   Dans les structures (I) à (IV) définies ci-dessus le groupement alkyle ou alcoxy en C₁-C₄ désigne de préférence méthyle, éthyle, butyle, méthoxy, éthoxy.
   Les colorants directs cationiques de formules (I), (II), (III) et (III') utilisables dans les compositions tinctoriales conformes à l'invention, sont des composés connus et sont décrits par exemple dans les demandes de brevets WO 95/01772, WO 95/15144 et EP-A-0 714 954. Ceux de formule (IV) utilisables dans les compositions tinctoriales conformes à l'invention, sont des composés connus et sont décrits par exemple dans les demandes de brevets FR-2189006, FR-2285851 et FR-2140205 et ses certificats d'addition.
   Parmi les colorants directs cationiques de formule (I) utilisables dans les compositions tinctoriales conformes à l'invention, on peut plus particulièrement citer les composés répondant aux structures (I1) à (I54) suivantes : 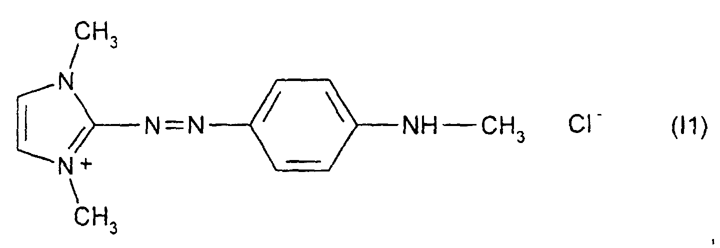 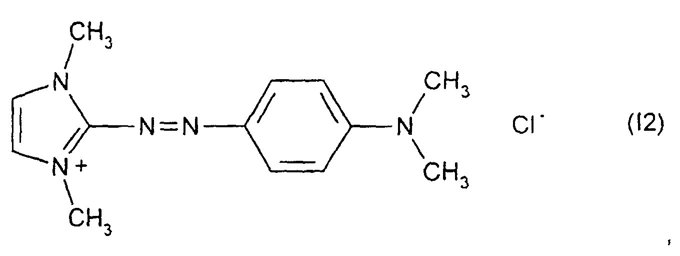 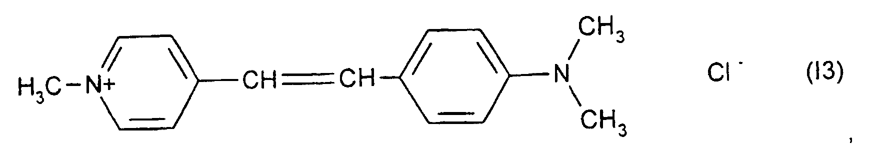 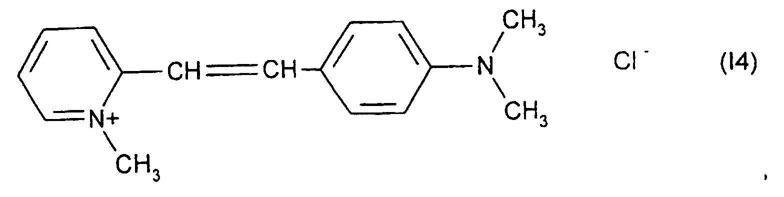 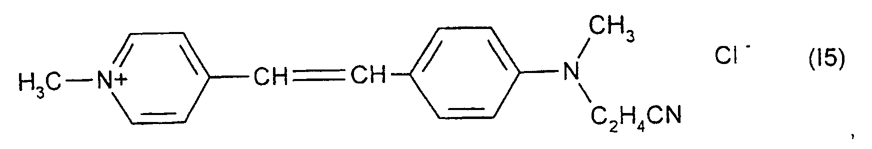 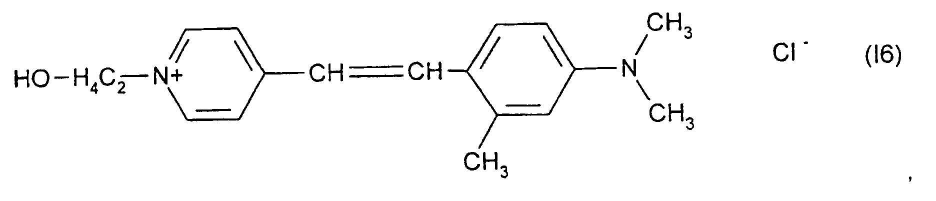 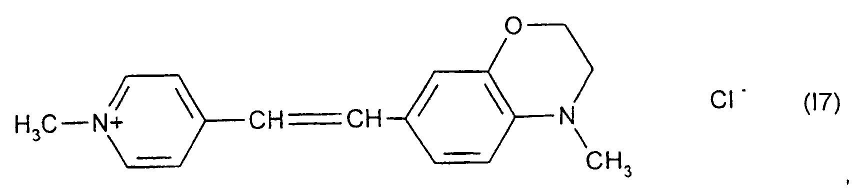 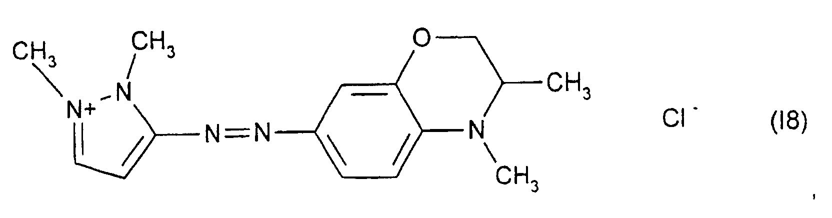 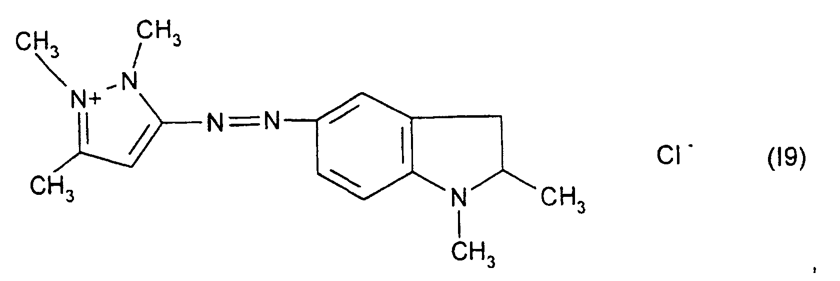 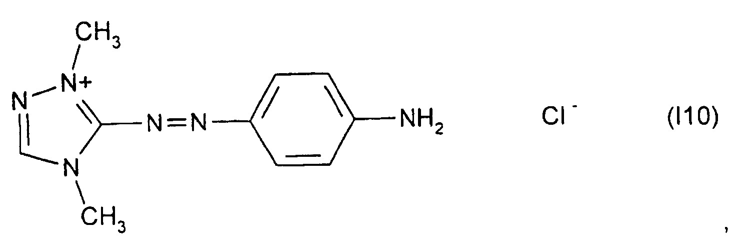 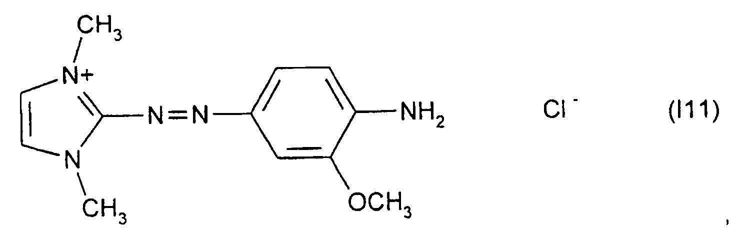 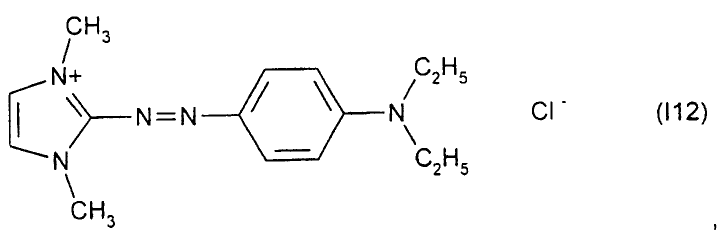 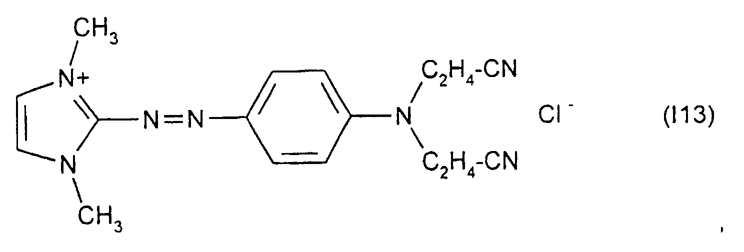 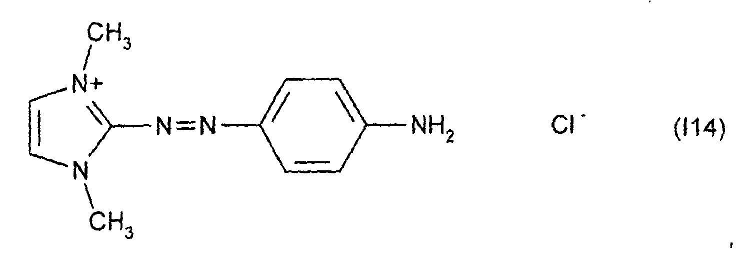 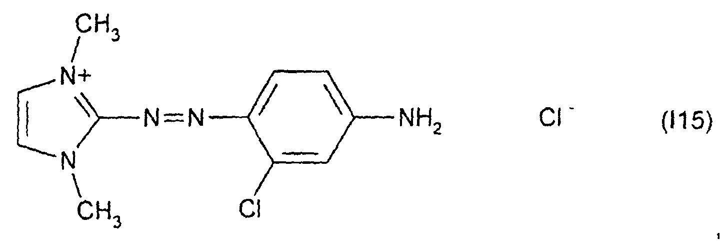 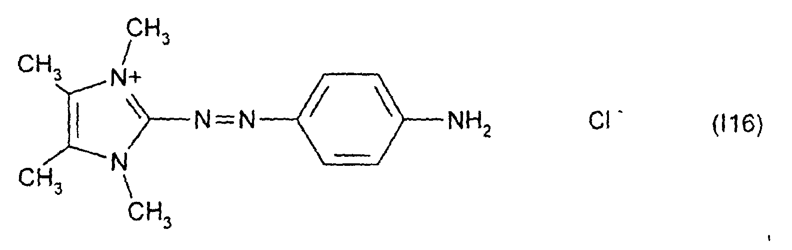 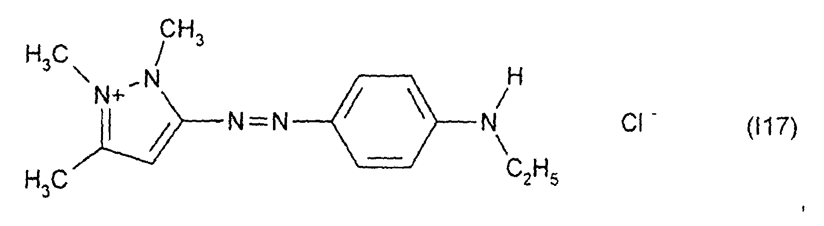 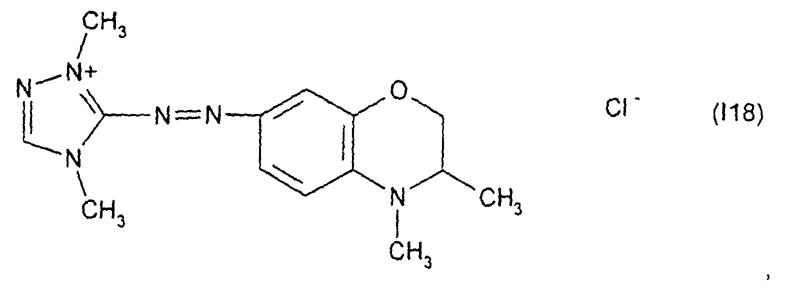 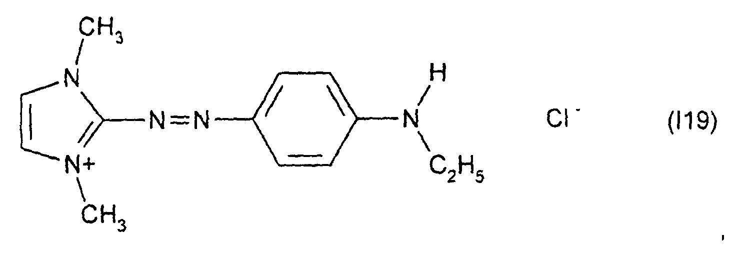 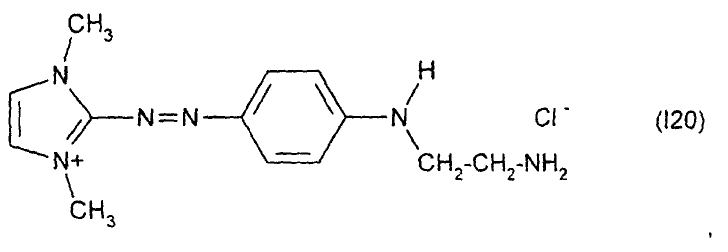 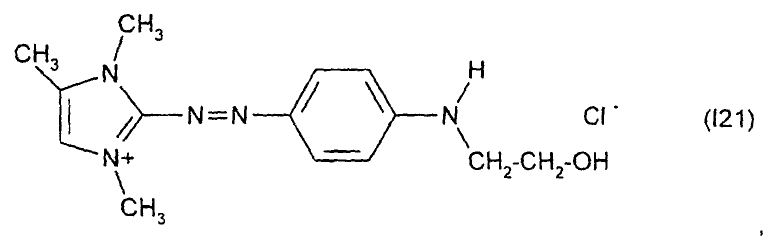 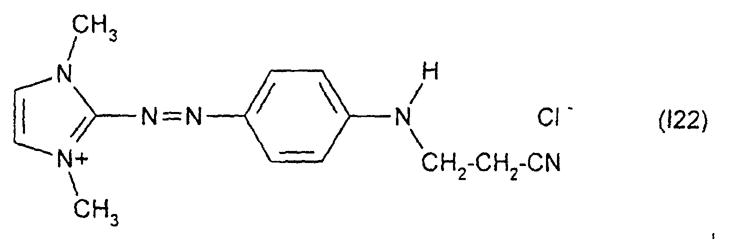 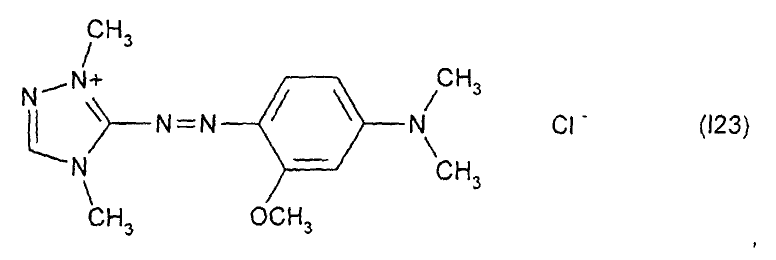 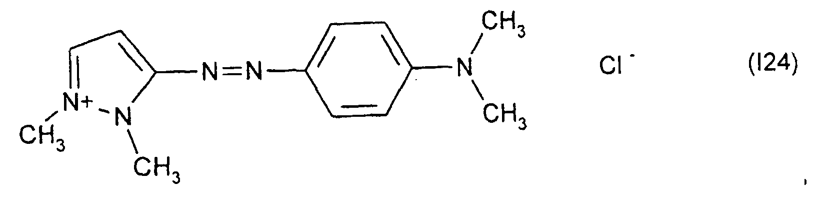 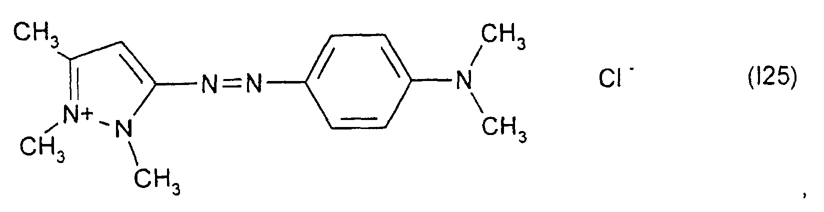 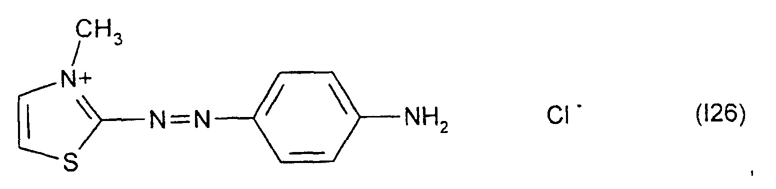 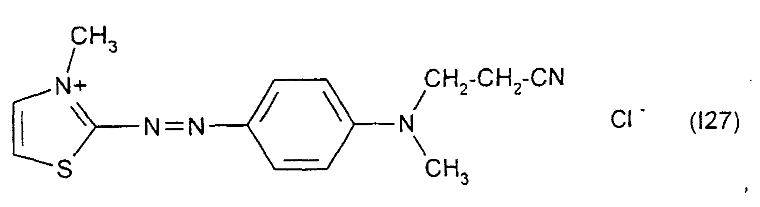 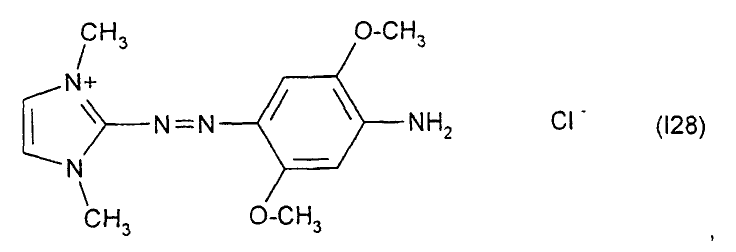 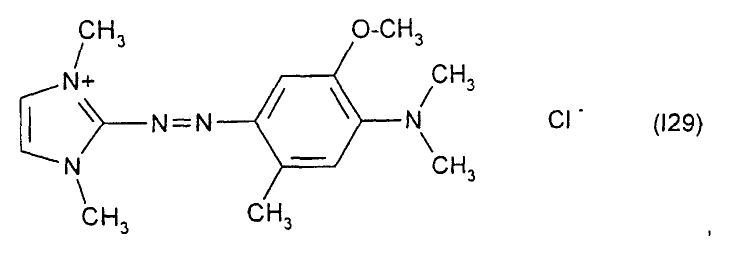 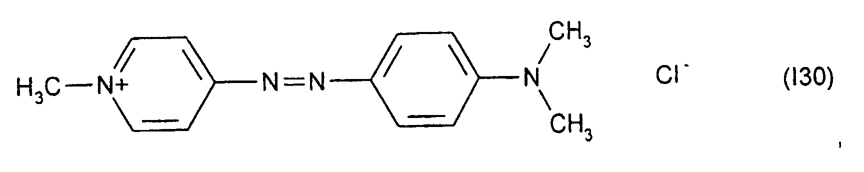 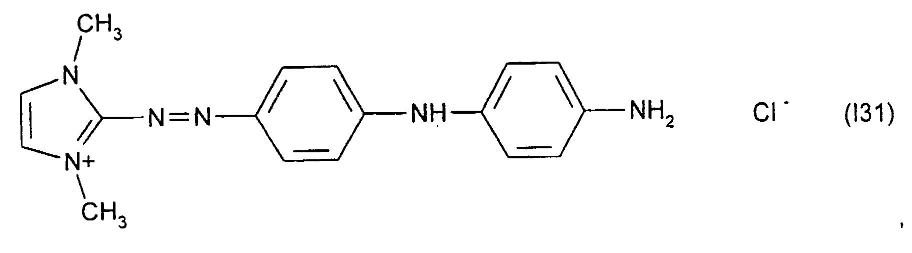 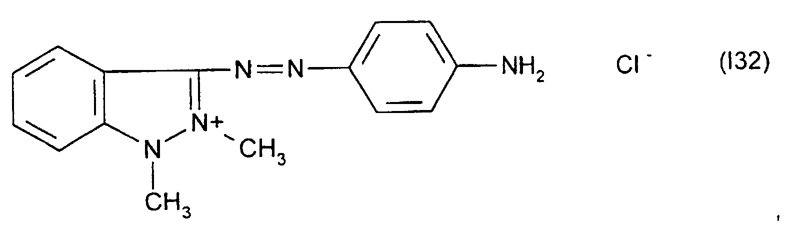 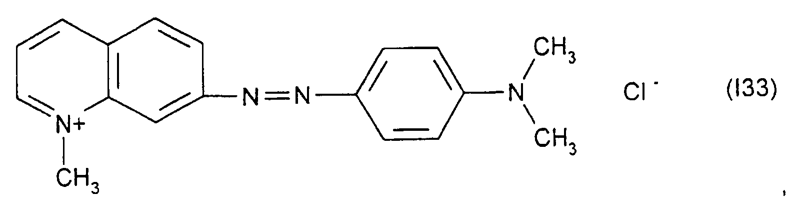 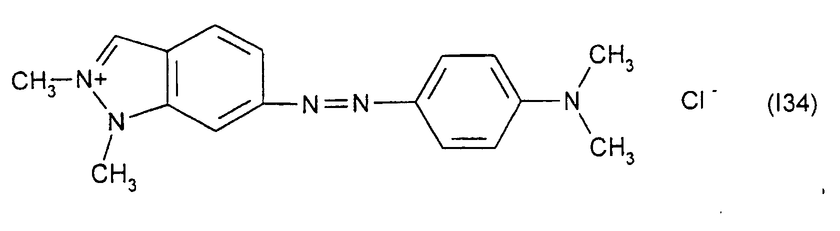 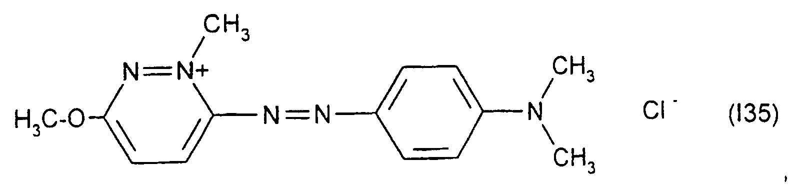 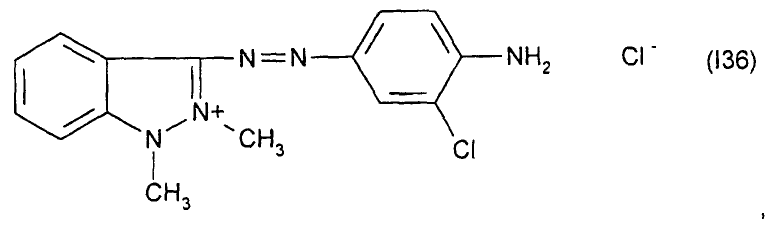 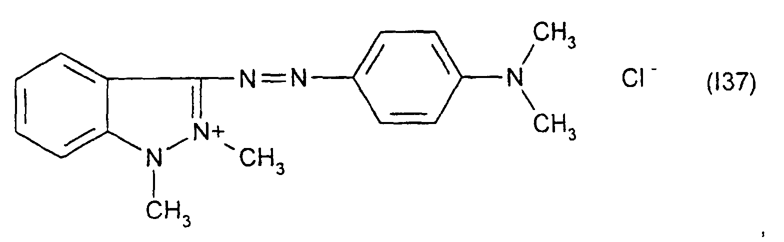 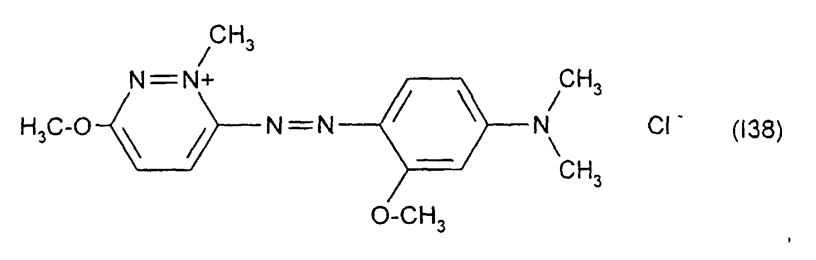 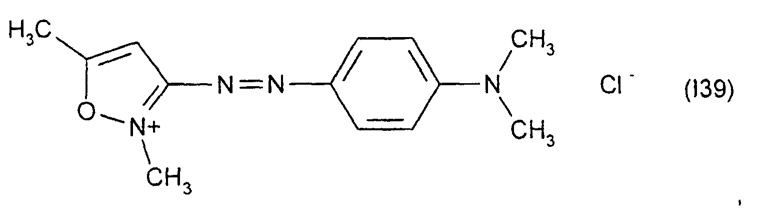 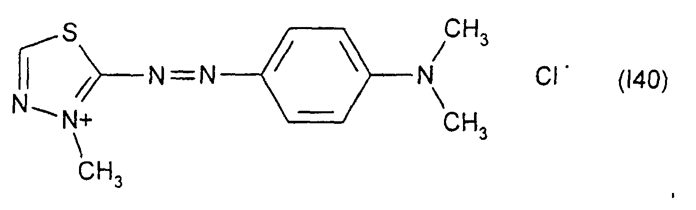 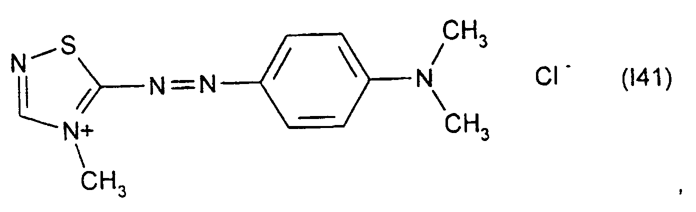 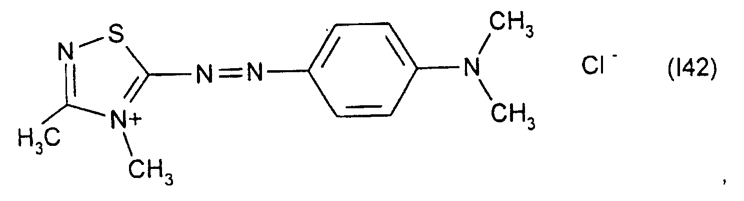 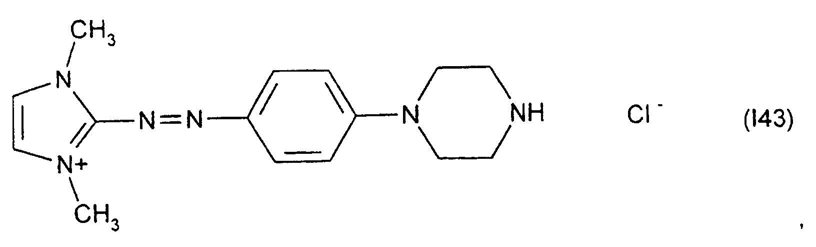 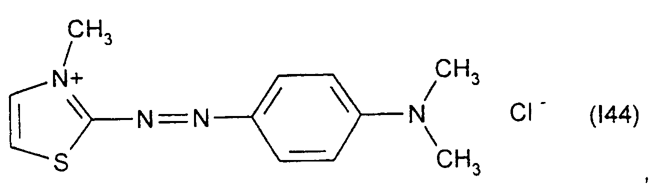 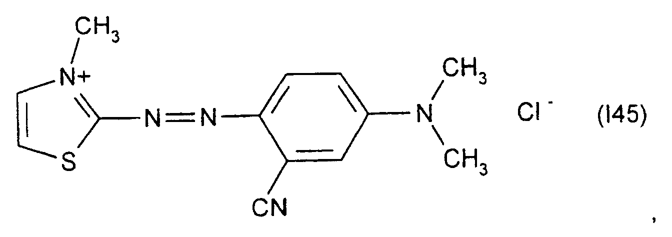 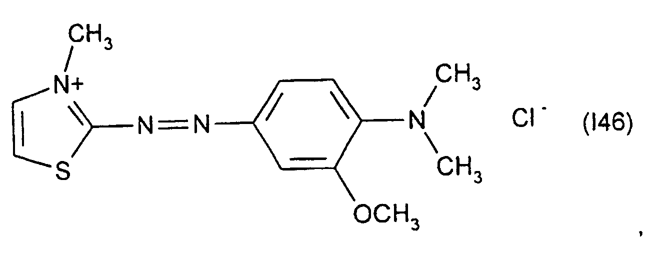 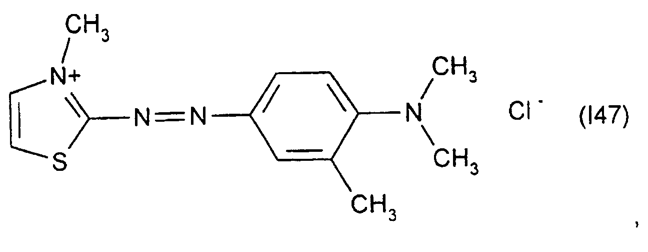 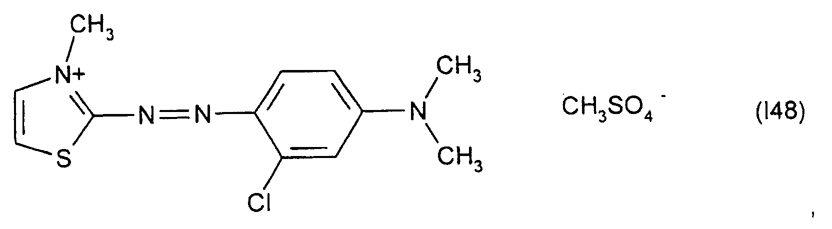 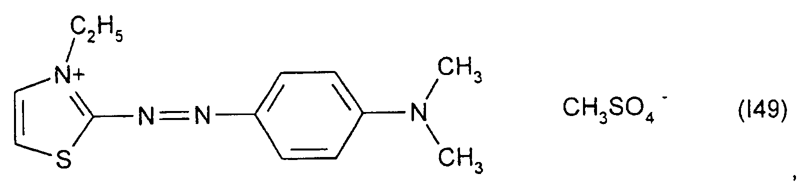 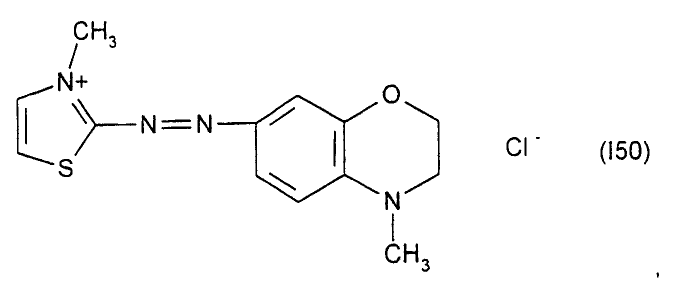 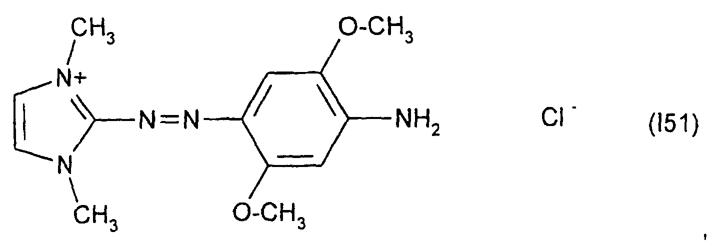 et 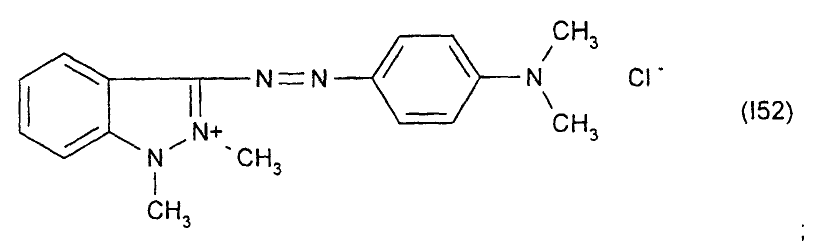 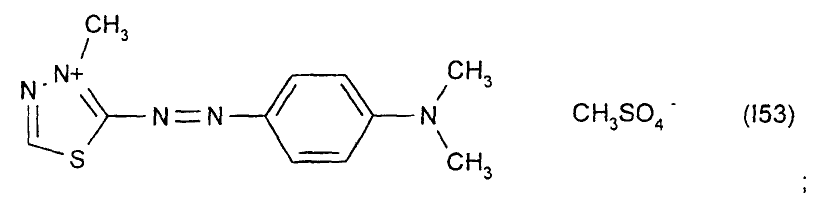 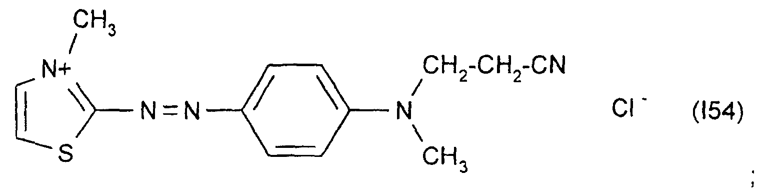
   Parmi les composés de structures (I1) à (I54) décrits ci-dessus, on préfère tout particulièrement les composés répondant aux structures (I1), (I2), (I14) et (I31).
   Parmi les colorants directs cationiques de formule (II) utilisables dans les compositions tinctoriales conformes à l'invention, on peut plus particulièrement citer les composés répondant aux structures (II1) à (II9) suivantes : 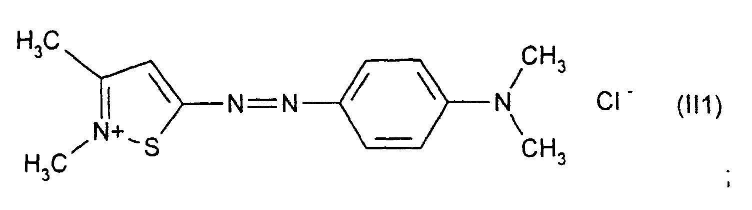 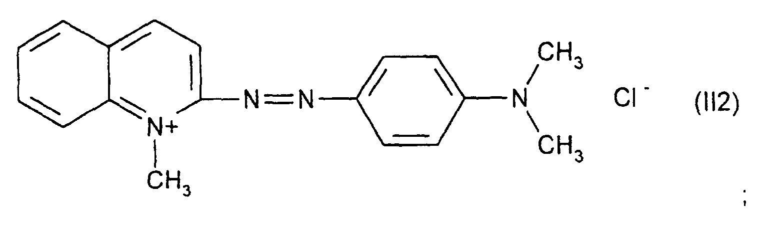 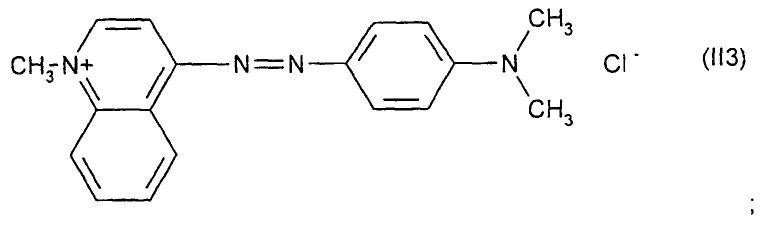 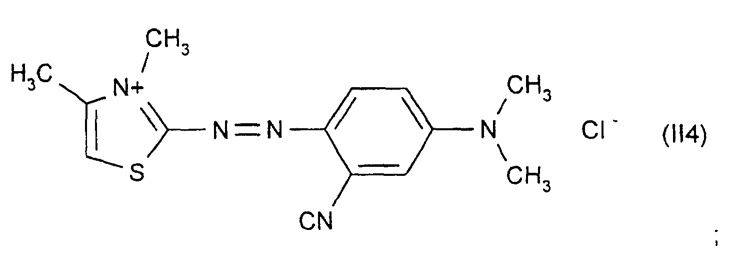 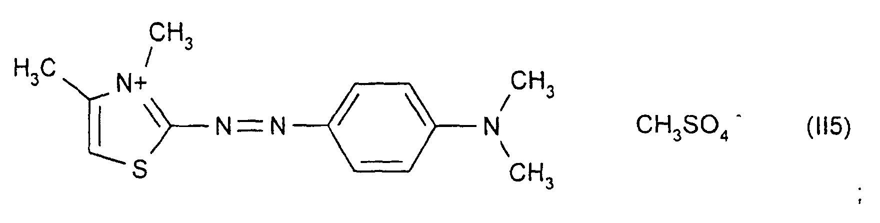 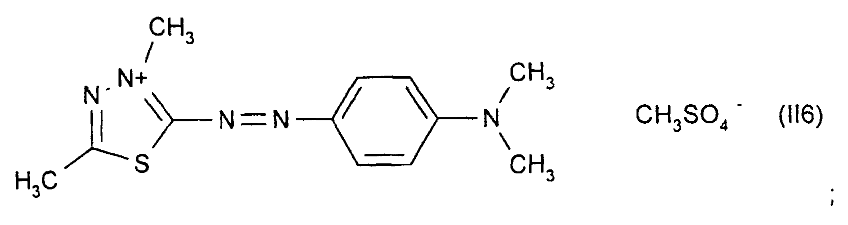 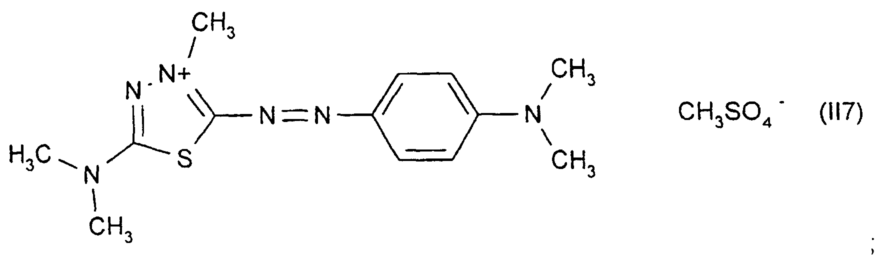 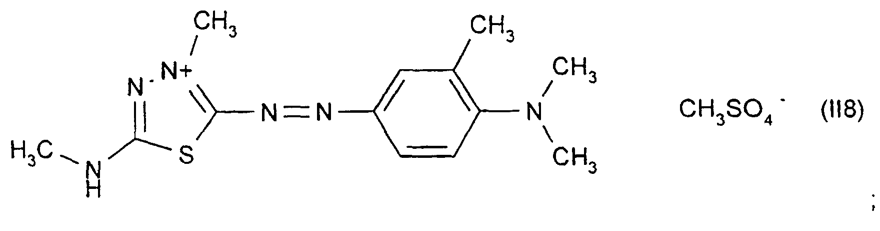 et 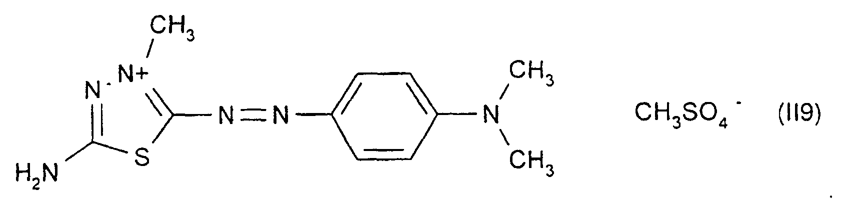
   Parmi les colorants directs cationiques de formule (III), utilisables dans les compositions tinctoriales conformes à l'invention, on peut plus particulièrement citer les composés répondant aux structures (III1) à (III18) suivantes : 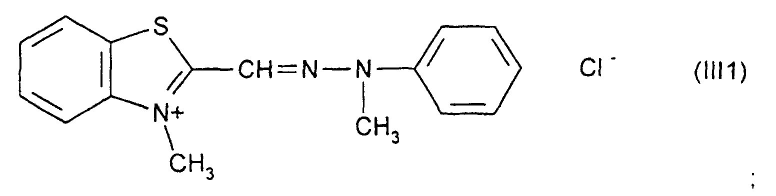 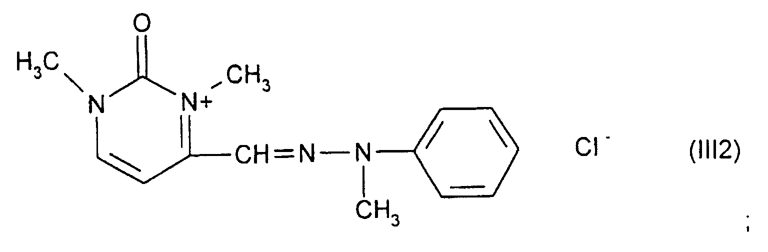 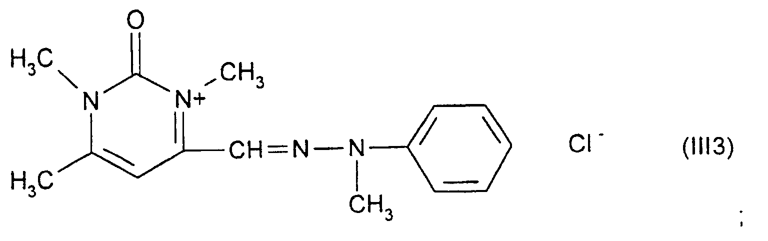 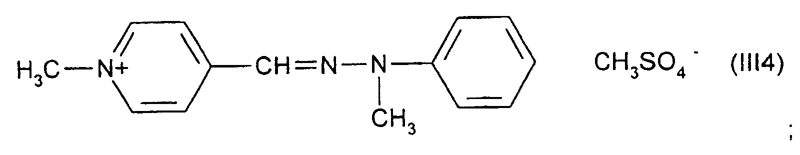 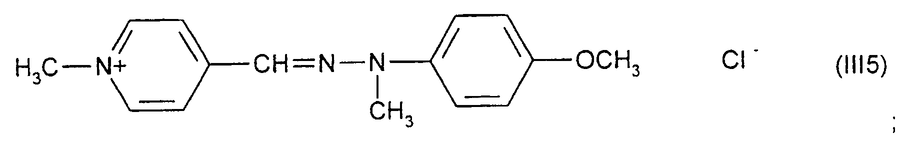 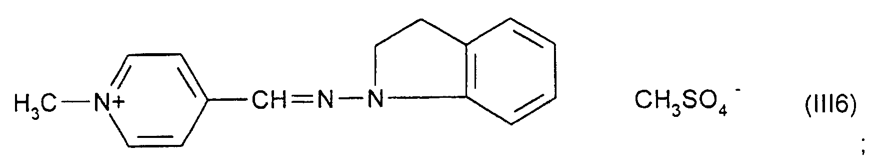 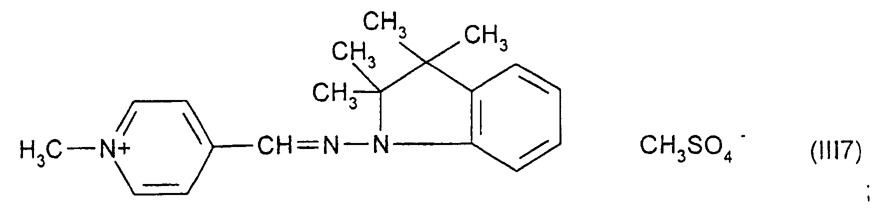 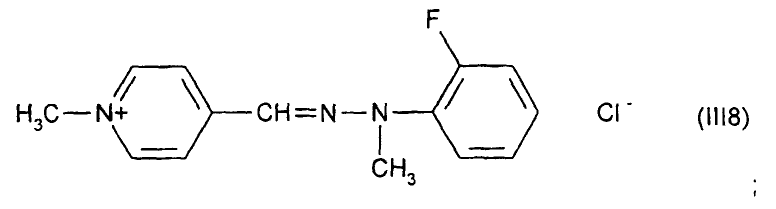 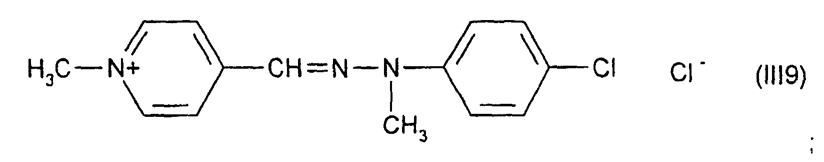 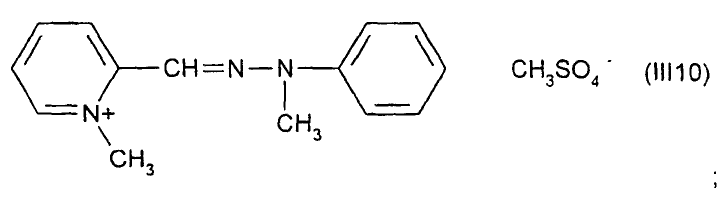 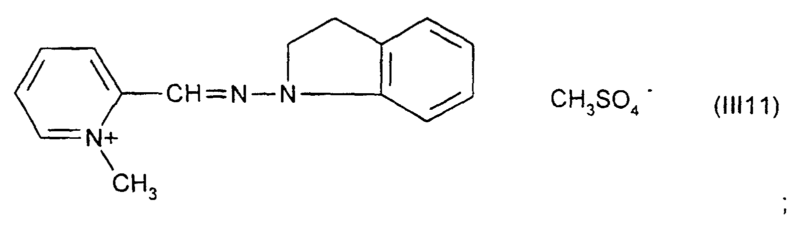 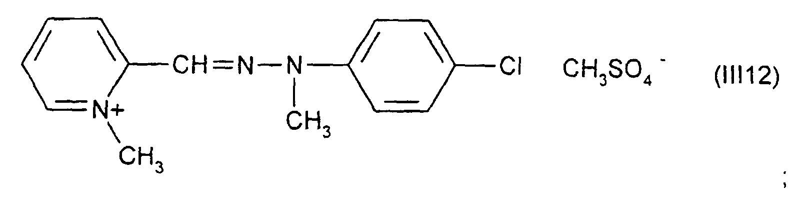 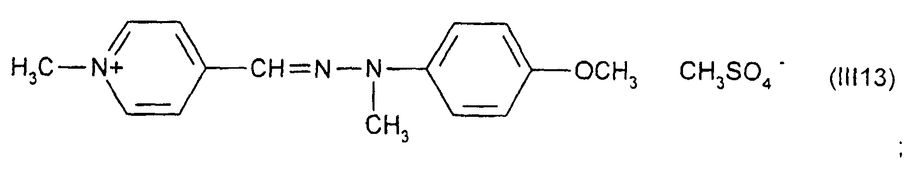 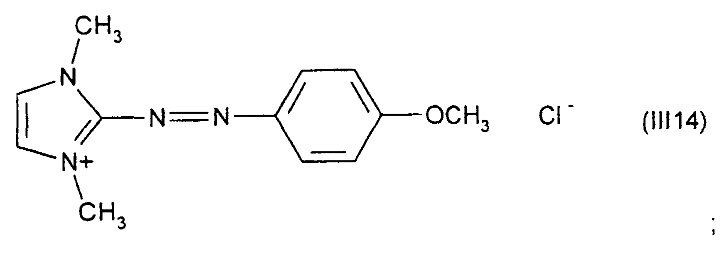 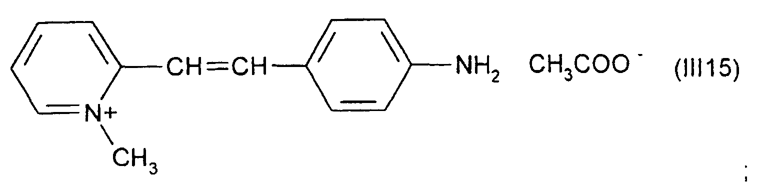 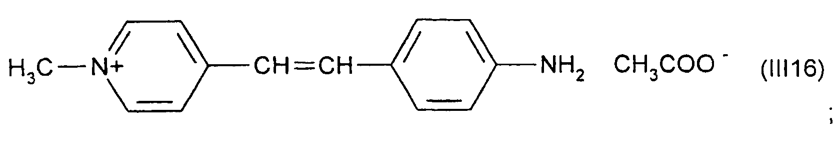 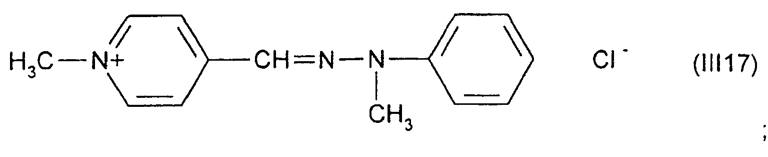 et 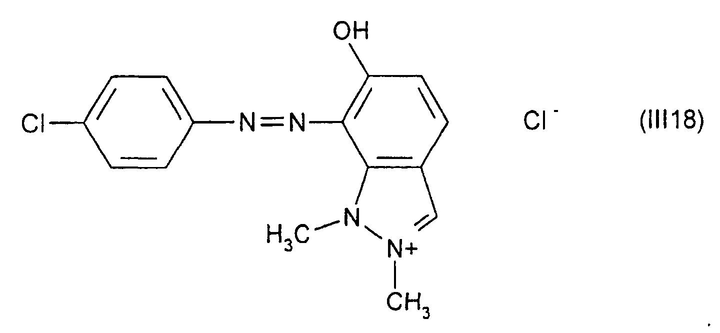
   Parmi les composés particuliers de structures (III1) à (III18) décrits ci-dessus, on préfère tout particulièrement les composés répondant aux structures (III4), (III5) et (III13).
   Parmi les colorants directs cationiques de formule (III'), utilisables dans les compositions tinctoriales conformes à l'invention, on peut plus particulièrement citer les composés répondant aux structures (III'1) à (III'3) suivantes : 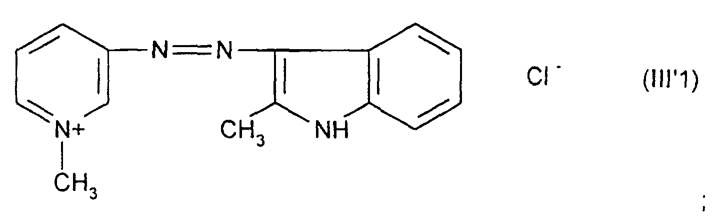 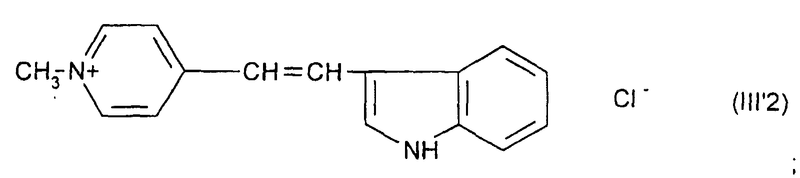 et 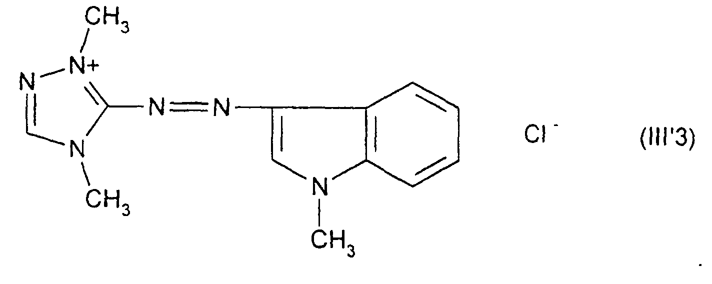
   Parmi les colorants directs cationiques de formule (IV) utilisables dans les compositions tinctoriales conformes à l'invention, on peut citer plus particulièrement les composés de structures (IV)₁ à (IV)₇₇ suivantes : 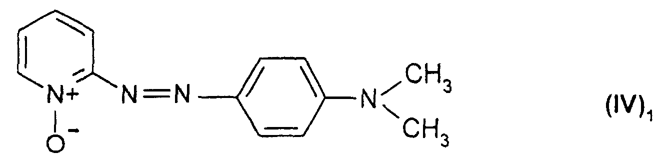 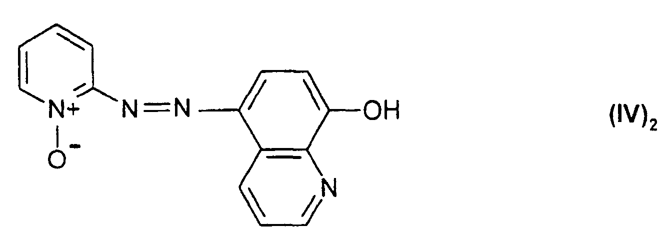 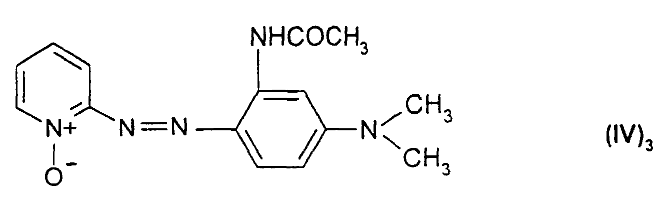 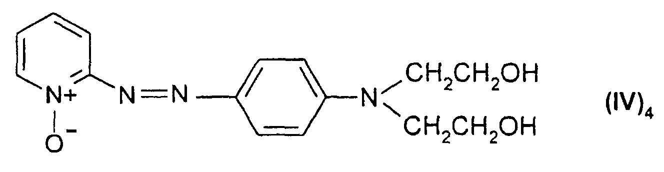 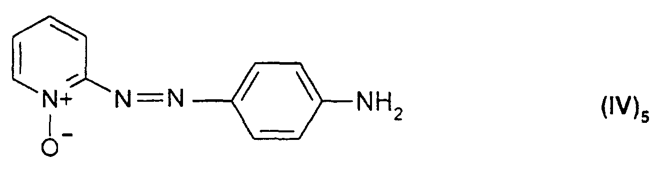 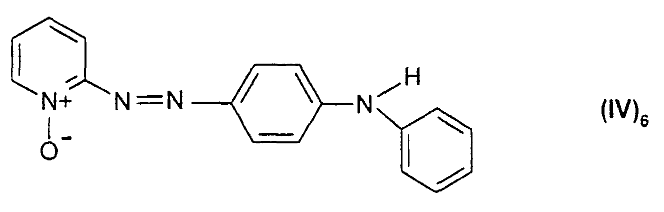 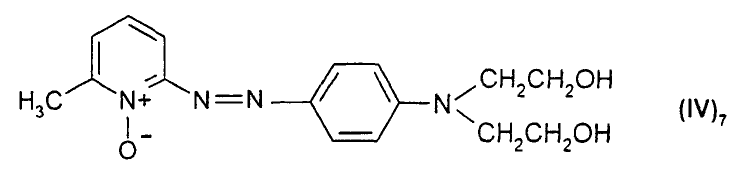 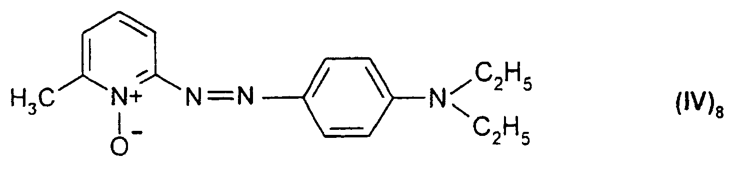 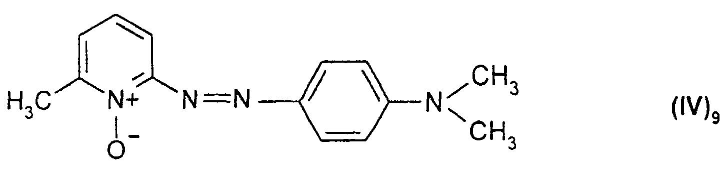 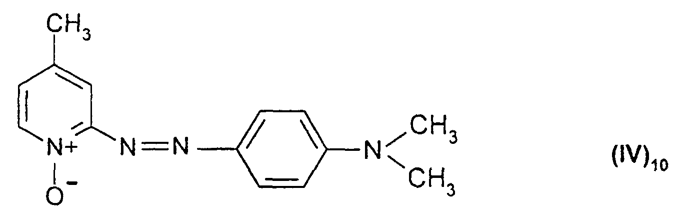 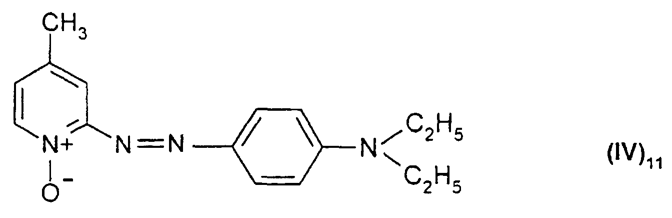 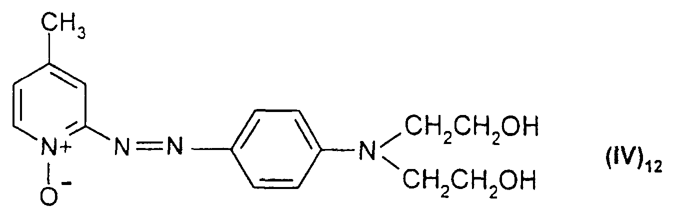 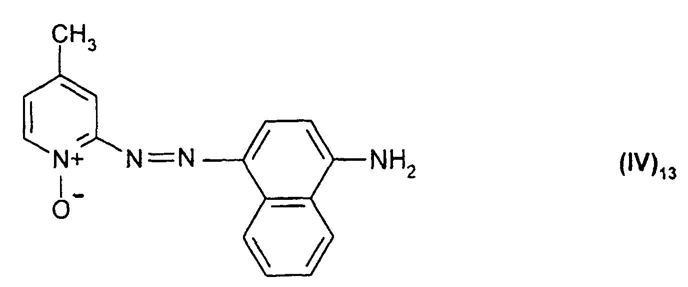 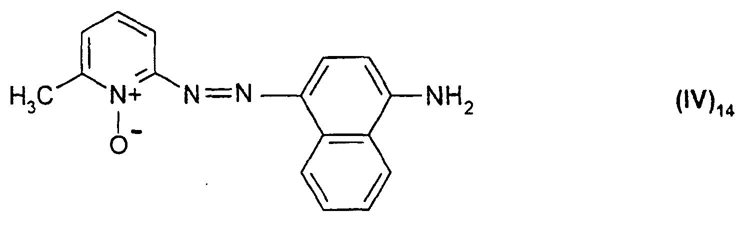 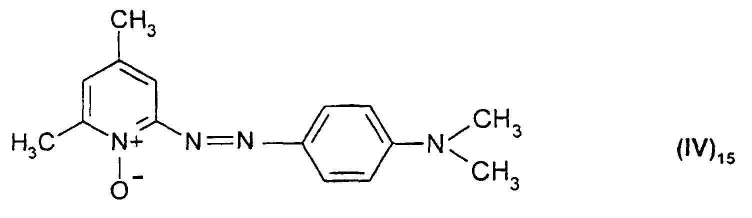 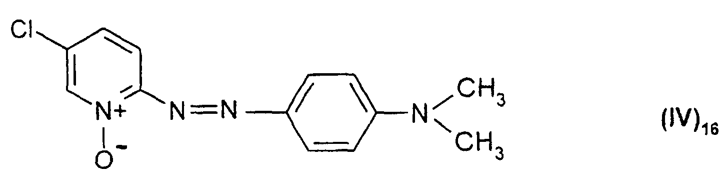 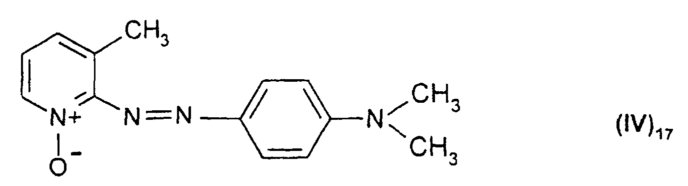 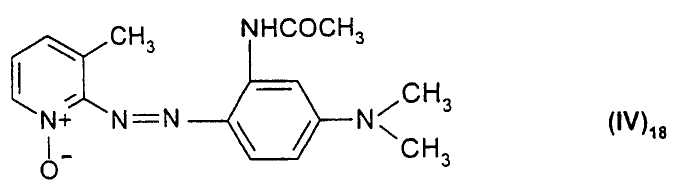 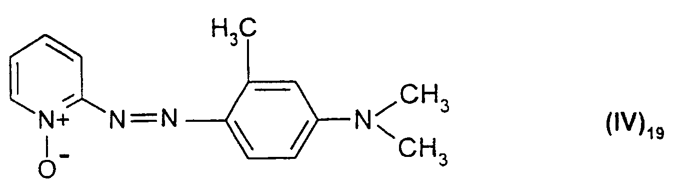 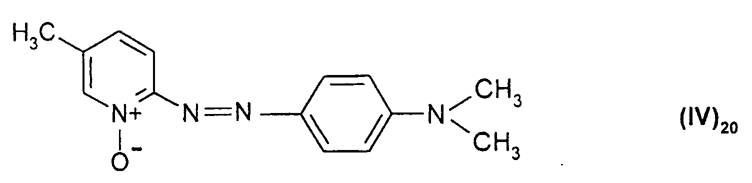 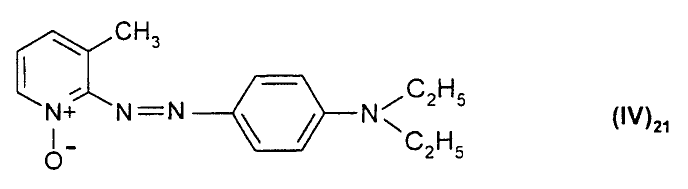 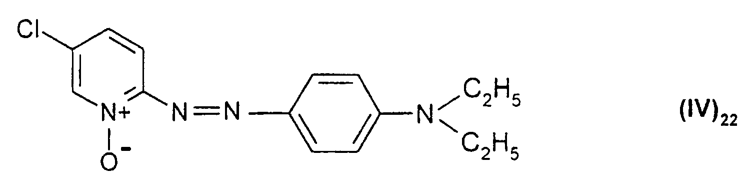 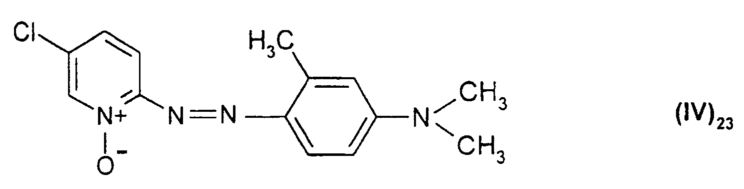 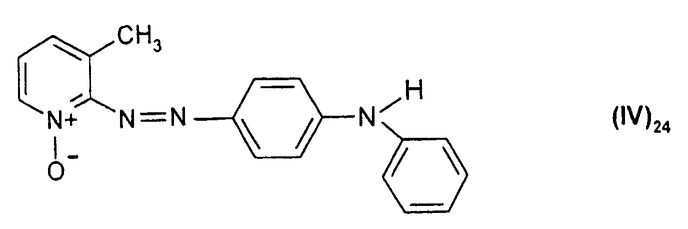 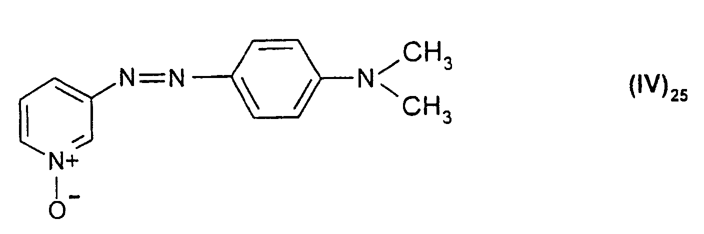 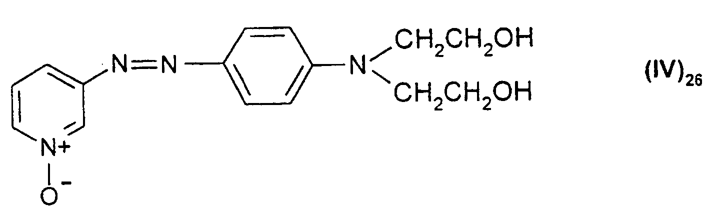 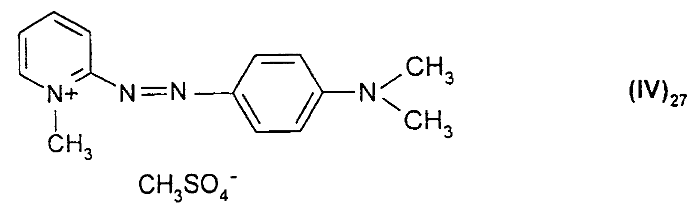 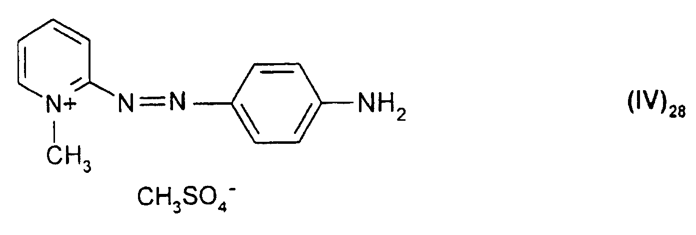 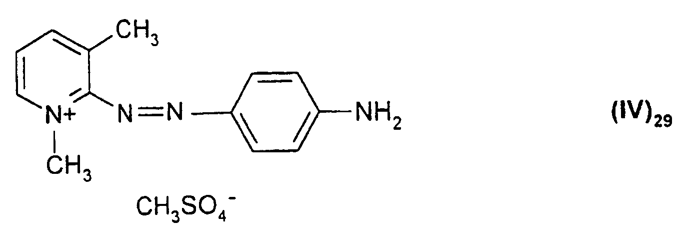 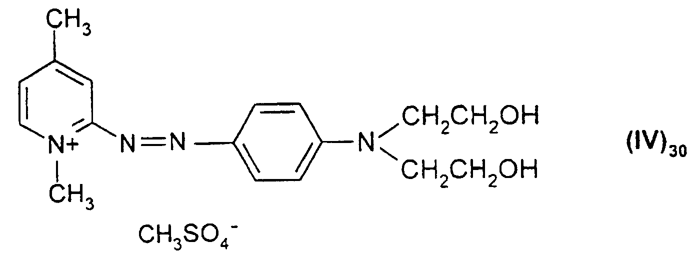 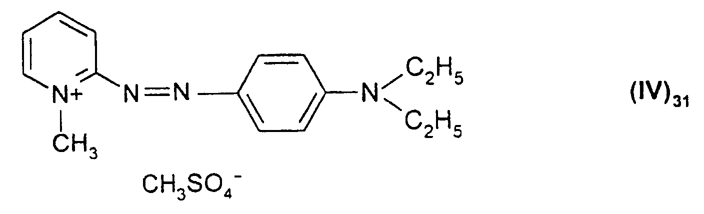 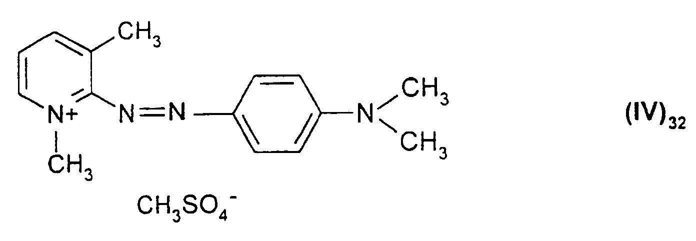 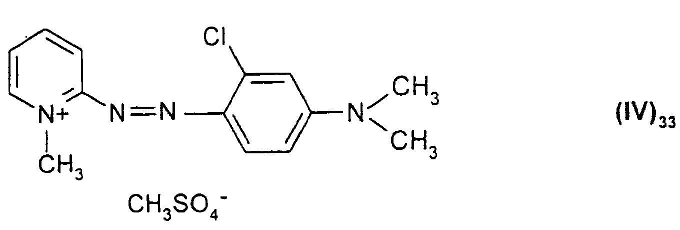 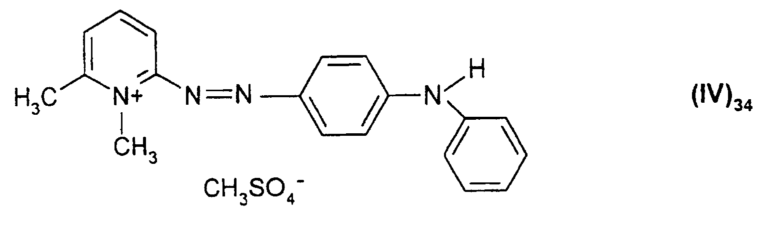 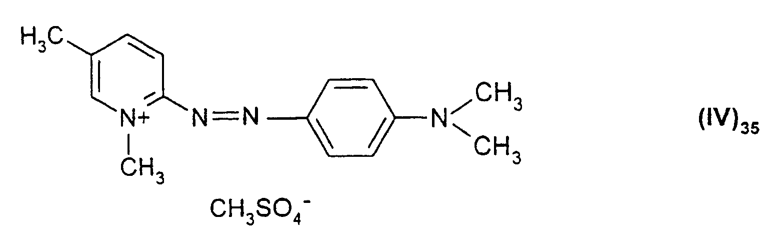 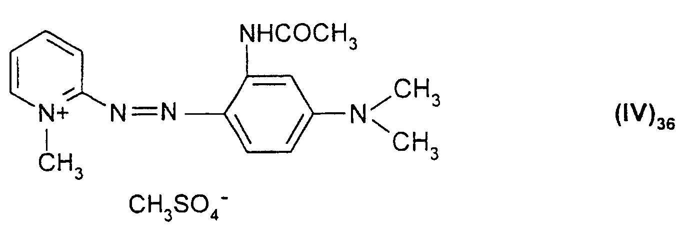 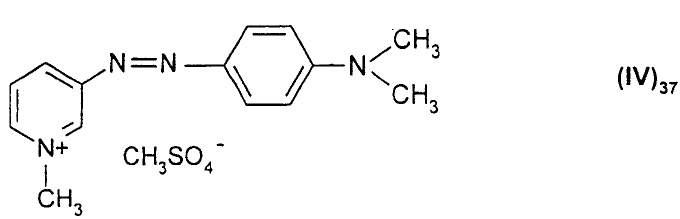 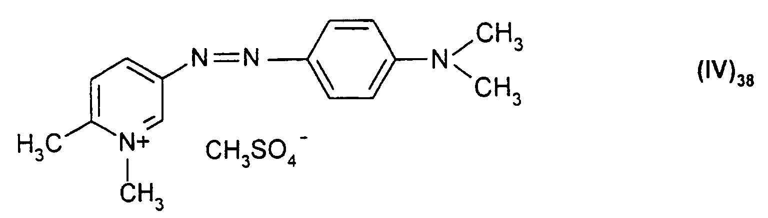 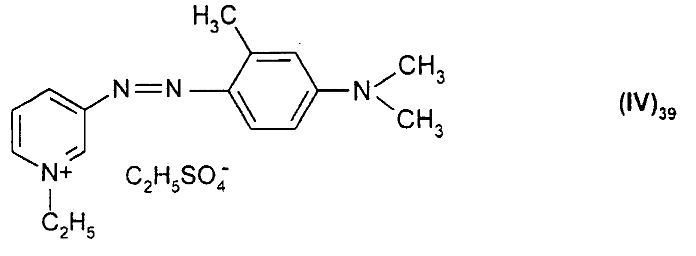 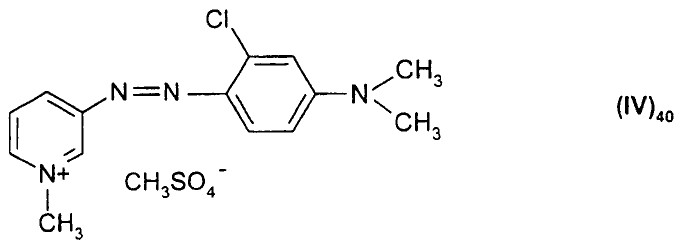 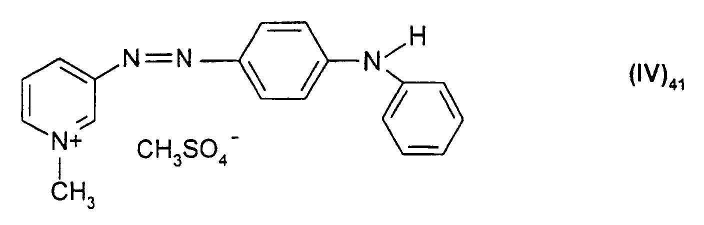 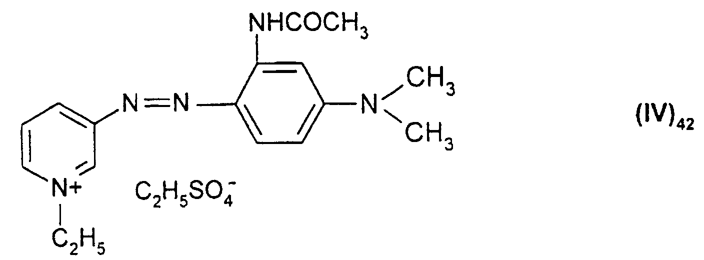 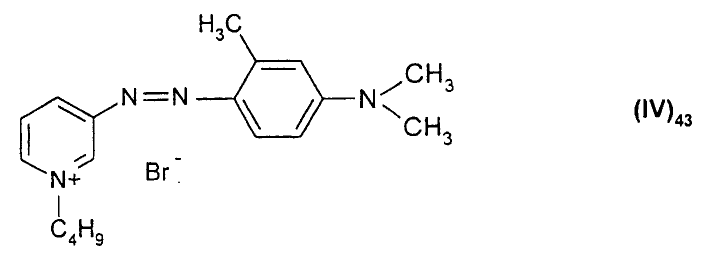 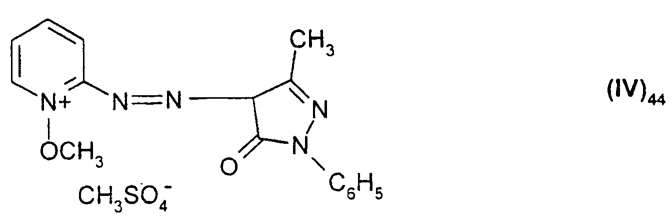 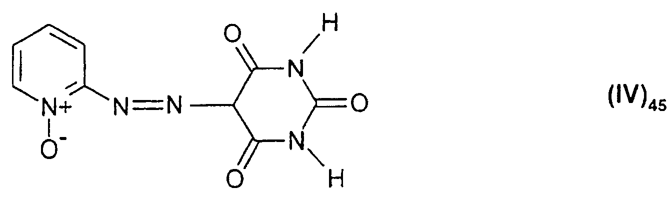 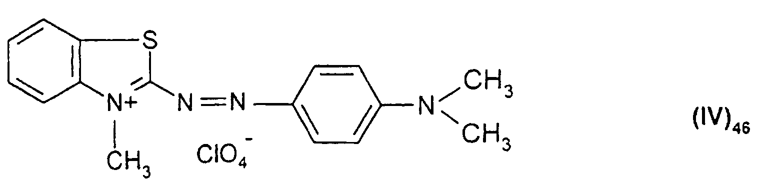 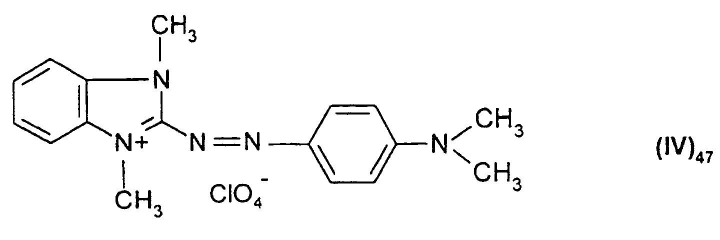 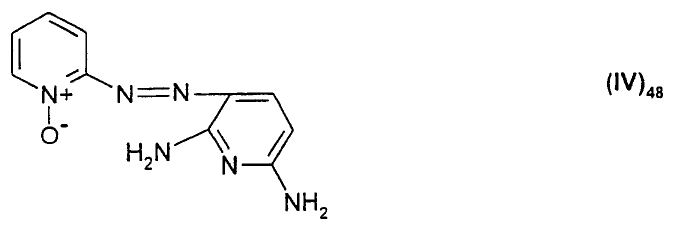 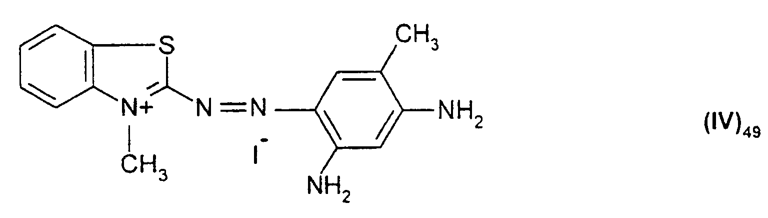 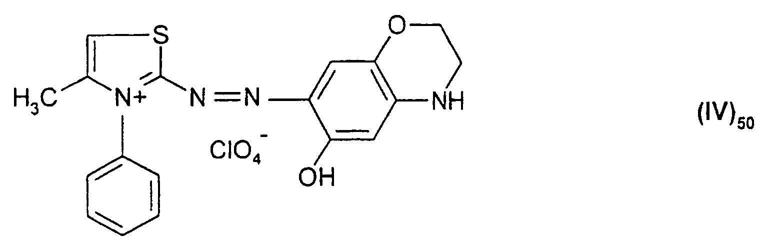 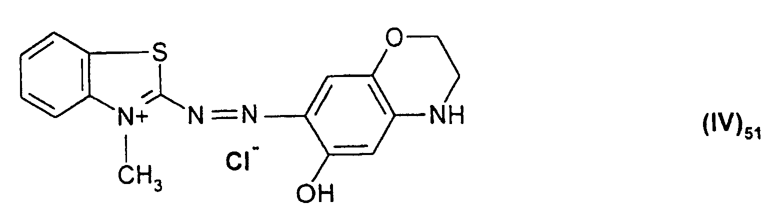 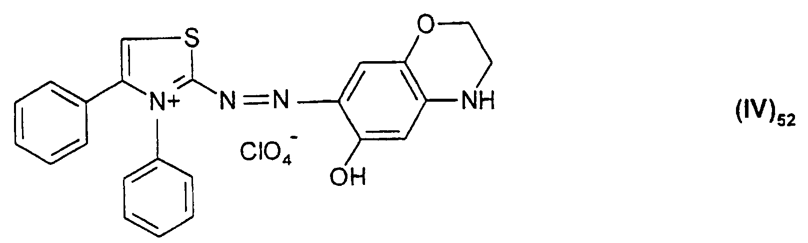 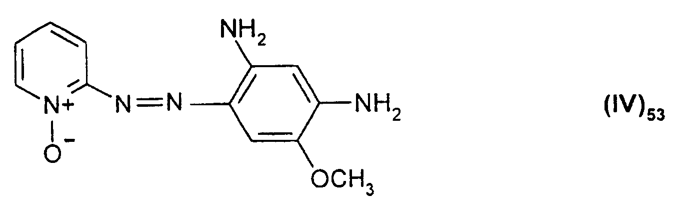 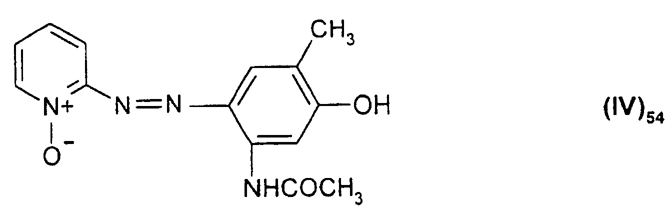 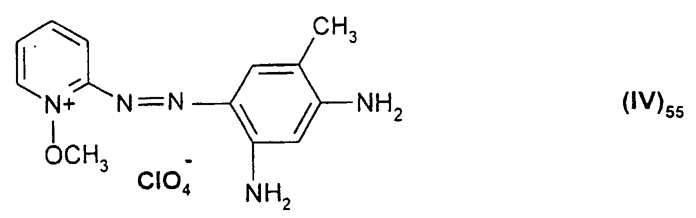 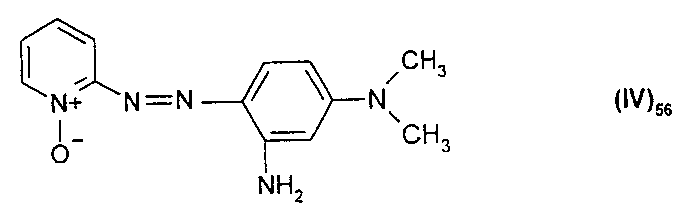 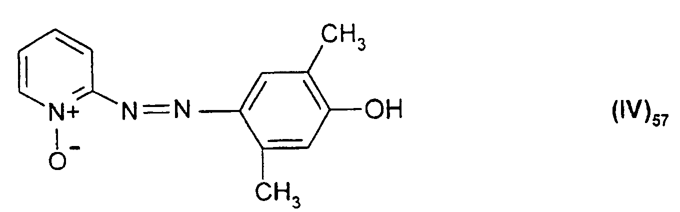 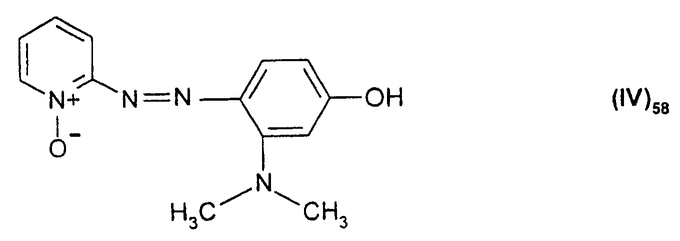 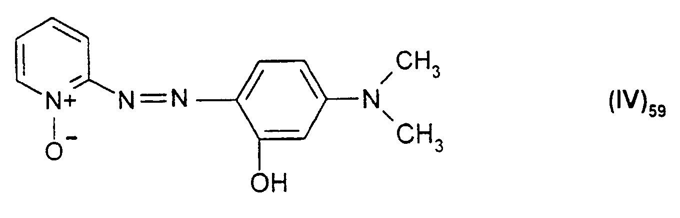 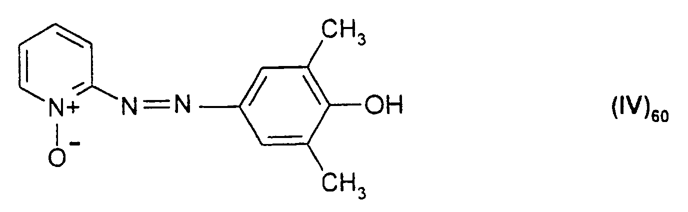 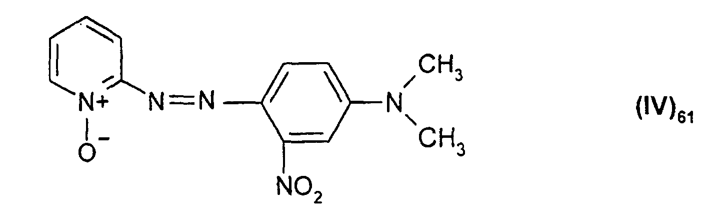 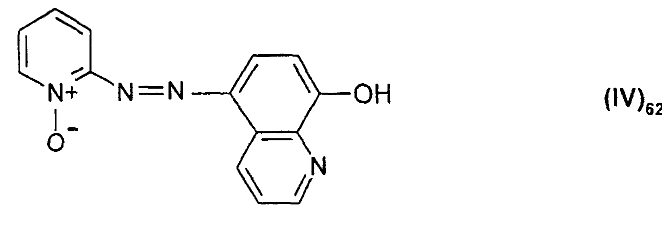 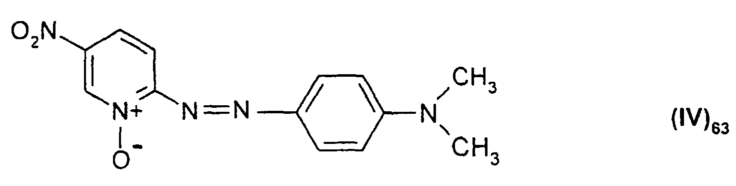 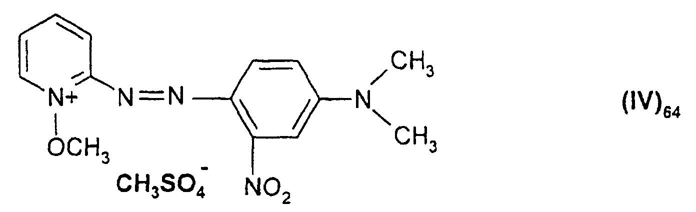 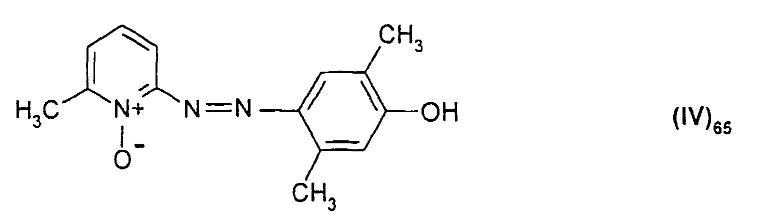 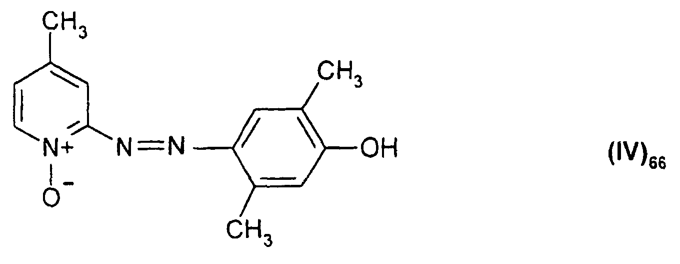 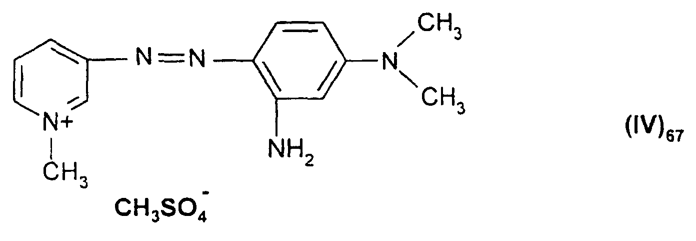 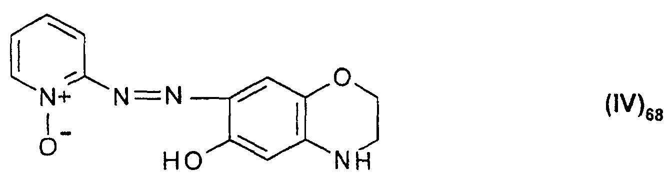 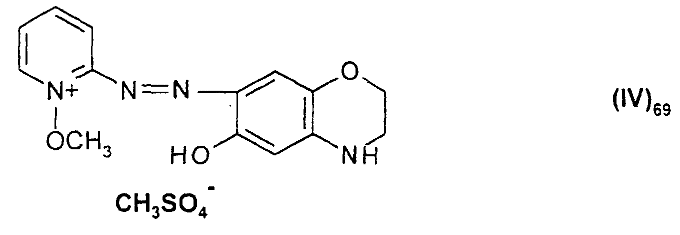 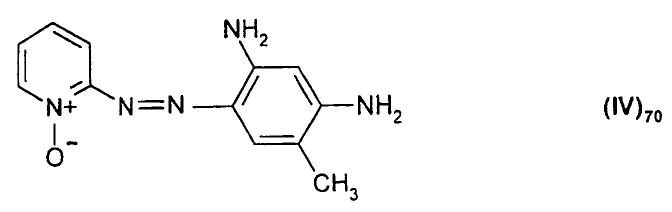 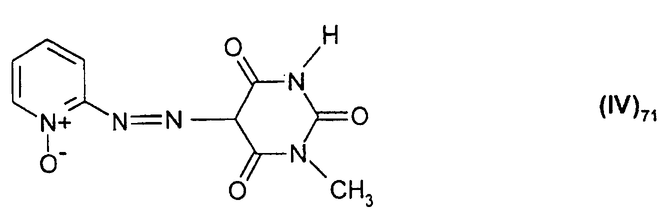 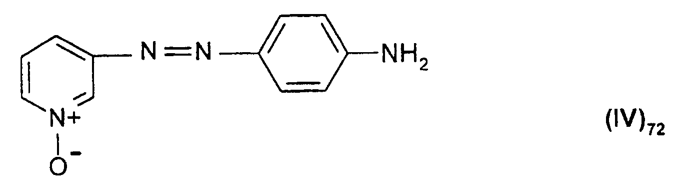 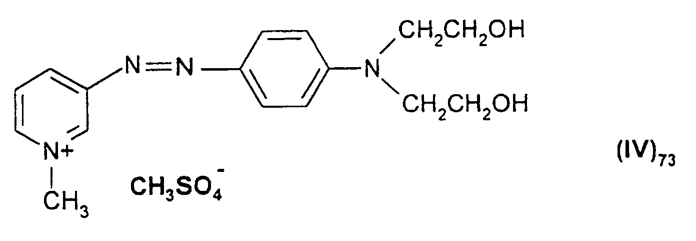 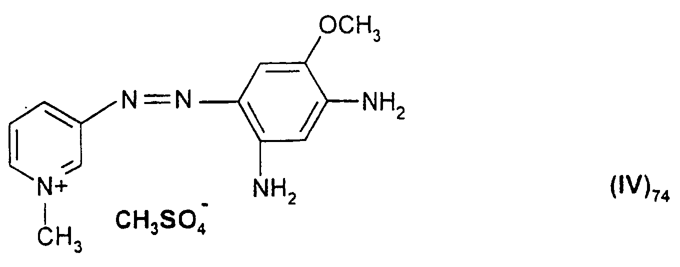 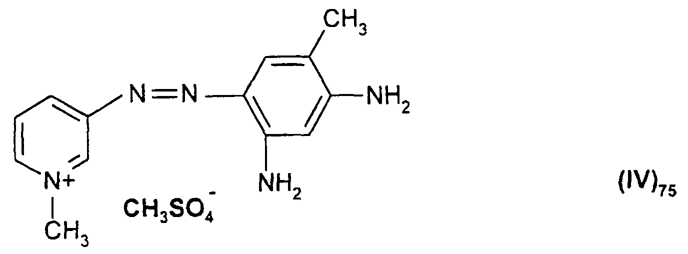 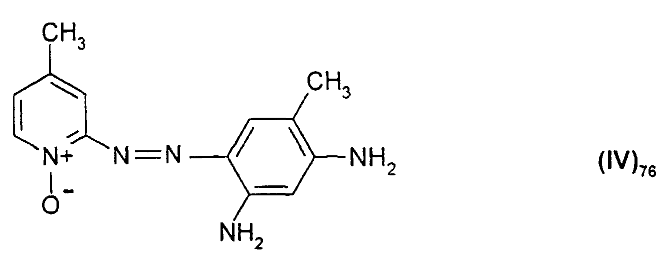 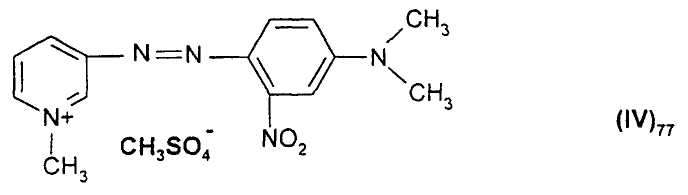
   Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.
**(ii)** Les sels d'ammonium quaternaire utilisables selon la présente invention sont choisis dans le groupe constitué par :
   **(ii)**_{**1**} - ceux de formule (V) suivante : 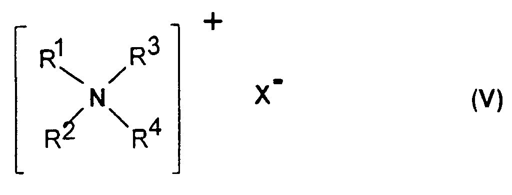 dans laquelle,
      les radicaux R¹ à R⁴, identiques ou différents, désignent un radical hydrocarboné aliphatique saturé ou insaturé, linéaire ou ramifié, comportant de 1 à environ 30 atomes de carbone, ou un radical alcoxy, alcoxycarbonylalkyle, polyoxyalkylène, alkylamido, alkylamidoalkyle, hydroxyalkyle, aromatique, aryle, alkylaryle, comportant environ de 12 à environ 30 atomes de carbone, avec au moins un radical parmi R¹ R² R³ R⁴ désignant un radical comportant de 8 à 30 atomes de carbone;
      X⁻ est un anion choisi dans le groupe des halogénures, phosphates, acétates, lactates, alkylsulfates;
      Parmi eux, on peut citer par exemple, (a)les sels de dialkyldiméthylammonium ou d'alkyltriméthylammonium dans lesquels le radical alkyle comporte environ de 12 à environ 22 atomes de carbone, tels que les chlorures de distéaryldiméthylammonium, de cétyltriméthylammonium, de béhényltriméthylammonium, (b)les sels de dialkyl(C₁-C₂)alkyl(C₁₂-C₂₂)hydroxyalkyl(C₁-C₂)ammonium tels que le chlorure d'oléocétylhydroxyéthylammonium, ou encore (c)le chlorure de stéaramidopropyldiméthyl (myristyl acétate) ammonium de formule : 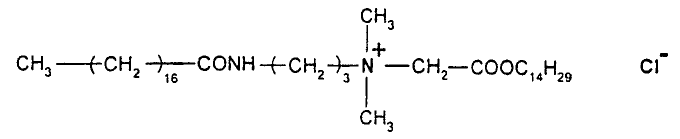 vendu sous la marque CERAPHYL 70 par la société VAN DYK.
   **(ii)**_{**2**} - les sels de l'imidazolium de formule (VI) suivante : 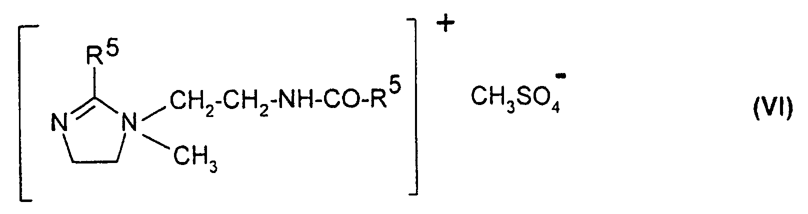 dans laquelle,
      R⁵ est choisi parmi les radicaux alcényle et/ou alkyles comportant de 13 à 31 atomes carbone et dérivés des acides gras du suif, tel que le produit vendu sous la marque "REWOQUAT W 7500" par la société REWO;
   **(ii)**_{**3**} - les sels de diammonium quaternaire de formule (VII) suivante : 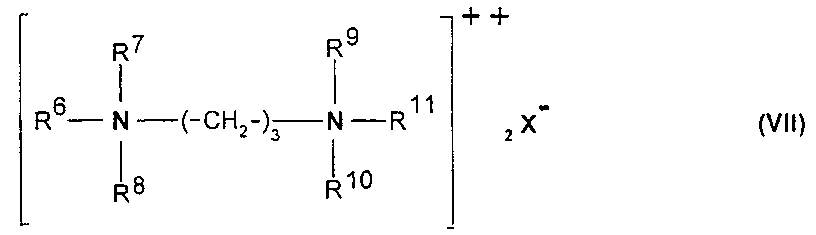 dans laquelle,
      R⁶ désigne un radical aliphatique comportant environ de 16 à 30 atomes de carbone, R⁷, R⁸, R⁹, R¹⁰, et R¹¹ sont choisis parmi l'hydrogène ou un radical alkyle comportant de 1 à 4 atomes de carbone, et X⁻ est un anion choisi dans le groupe des halogénures, acétates, phosphates et sulfates. De tels sels de diammonium quaternaire comprennent notamment le dichlorure de propanesuif diammonium.

Selon la présente invention, on préfère les sels d'ammonium quaternaire de formule (V) dans laquelle R¹ à R⁴, identiques ou différents, désignent des radicaux alkyles ou hydroxyalkyles comportant environ de 12 à environ 22 atomes de carbone, et en particulier, le chlorure de béhényltriméthylammonium, le chlorure de cétyltriméthylammonium et le chlorure d'oléocétyldiméthylhydroxyéthylammonium.

Le ou les sels d'ammonium quaternaire (ii) utilisés selon l'invention, représentent de préférence de 0,01 à 10% en poids environ du poids total de la composition tinctoriale et encore plus préférentiellement de 0,05 à 5% en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VIII) suivante : 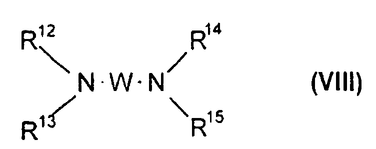 dans laquelle W est un reste propylène éventuellement substitué par un groupement hydroxyle ou un radical alkyle en C₁-C₆ ; R¹², R¹³, R¹⁴ et R¹⁵, identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques (i) définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques (i) une ou plusieurs bases d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques. Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique (i) et du sel d'ammonium quaternaire (ii) ainsi que des bases d'oxydation, un ou plusieurs coupleurs de façon à modifier ou à enrichir en reflets les nuances obtenues en mettant en oeuvre le ou les colorants direct(s) cationique(s) (i) et la ou les bases d'oxydation.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant le sel d'ammonium quaternaire.

Lorsque l'association du colorant direct cationique (i) et du sel d'ammonium quaternaire (ii) selon l'invention est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante, alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydo-réductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique (i) tel que défini précédemment et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le sel d'ammonium quaternaire (ii) tel que défini précédemment.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique (i) tel que défini précédemment et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le sel d'ammonium quaternaire tel que défini précédemment.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) ou (B2) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 à 3 :

On a préparé les trois compositions de teinture directe réunies dans le tableau suivant :
*(toutes teneurs exprimées en grammes)*

| **EXEMPLES N°→** | **1** | **2** | **3** |
|---|---|---|---|
| Colorant direct cationique de formule (I1) | 0,20 | | |
| Colorant direct cationique de formule(I14) | | 0,20 | |
| Colorant direct cationique de formule (IV)₂₇ | | | 0,10 |
| Chlorure d'oléocétyldiméthylhydroxyéthylammonium | 2,0 MA* | | |
| Chlorure de béhényltriméthylammonium | | 2,0 MA* | |
| Chlorure de cétyltriméthylammonium | | | 2,0 MA* |
| Ethanol | 10 | 10 | 10 |
| 2-amino-2-méthyl-1-propanol qs | pH 9 | pH 9 | pH 9 |
| Eau déminéralisée qsp | 100 | 100 | 100 |
| MA* désigne Matière Active | | | |

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| **Exemples** | **Nuances obtenues** |
|---|---|
| 1 | Rouge puissant |
| 2 | Orangé puissant |
| 3 | Pourpre puissant |

## Revendications

1. Composition pour la teinture des fibres kératiniques et en particulier des fibres kératiniques humaines telles que les cheveux, renfermant dans un milieu approprié pour la teinture, (i) au moins un colorant direct cationique choisi parmi les composés de formules (I), (II), (III), (III'), (IV) suivantes :
**a) les composés de formule (I) suivante :** 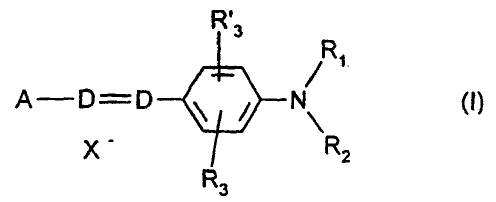 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X⁻ représente un anion de préférence choisi parmi le chlorure, le méthyl sulfate et l'acétate,
A représente un groupement choisi par les structures A1 à A18 suivantes : 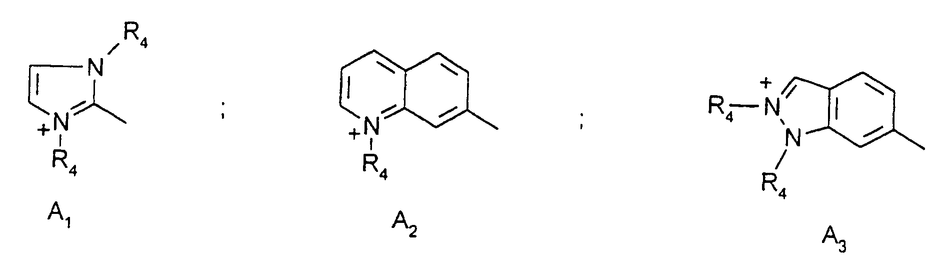 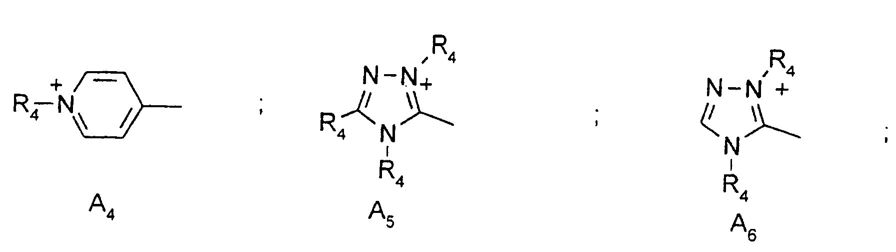 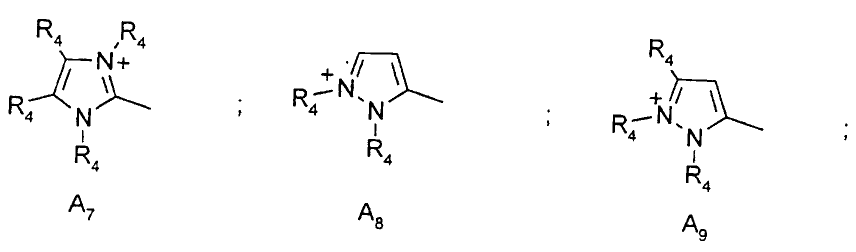 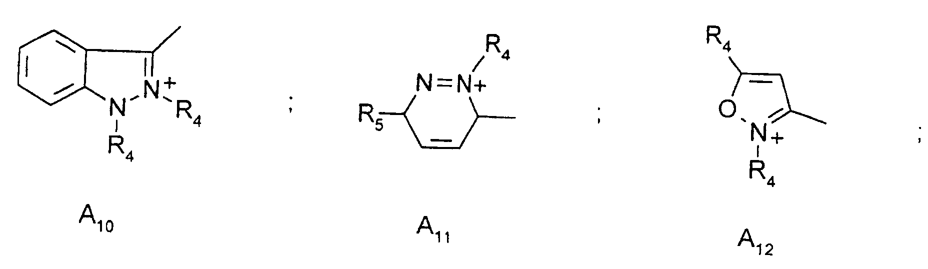 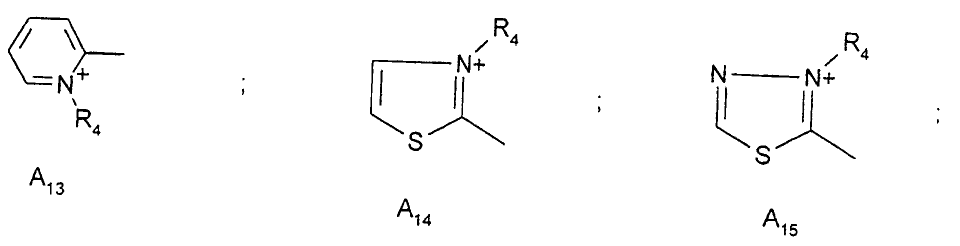 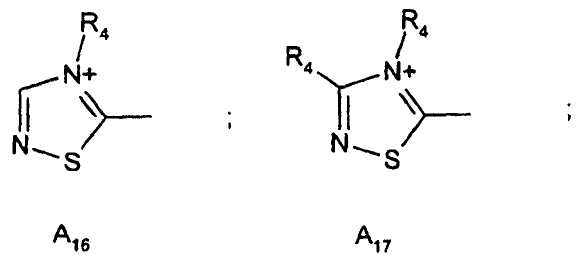 et 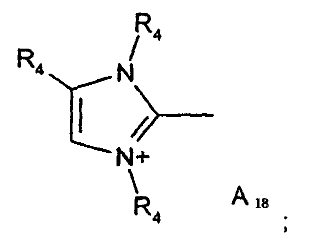 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) les composés de formule (II) suivante :** 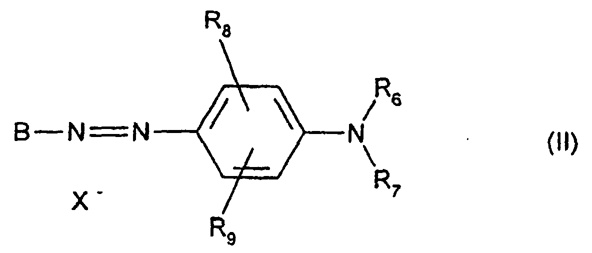 dans laquelle :
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à B6 suivantes : 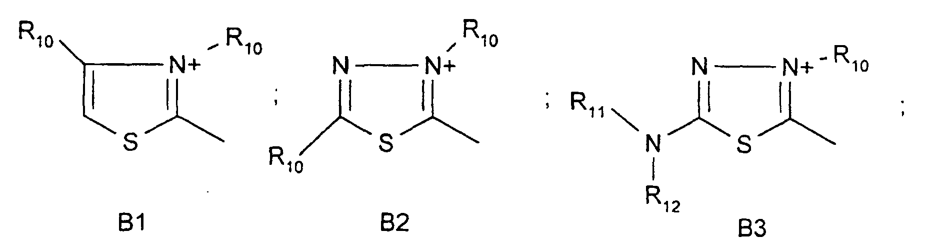 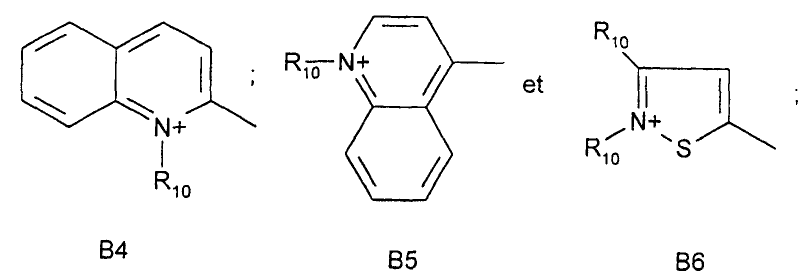 dans lesquelles R₁₀ représente un radical alkyle en C₁-C₄, R₁₁, et R₁₂, identiques ou différents, représentent un atome d'hydrogène ou un radical alkyle en C₁-C₄ ;
**c) les composés de formules (III) et (III') suivantes :** 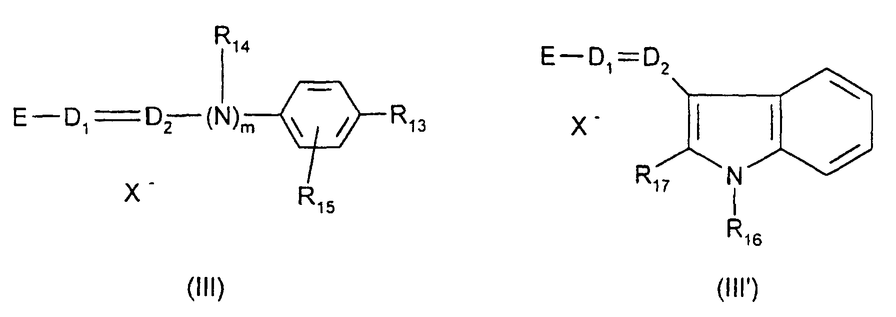 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH,
m = 0 ou 1,
étant entendu que lorsque R₁₃ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes : 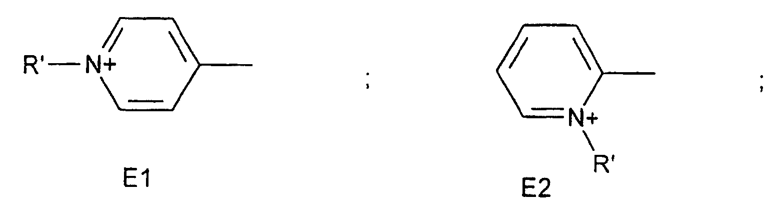 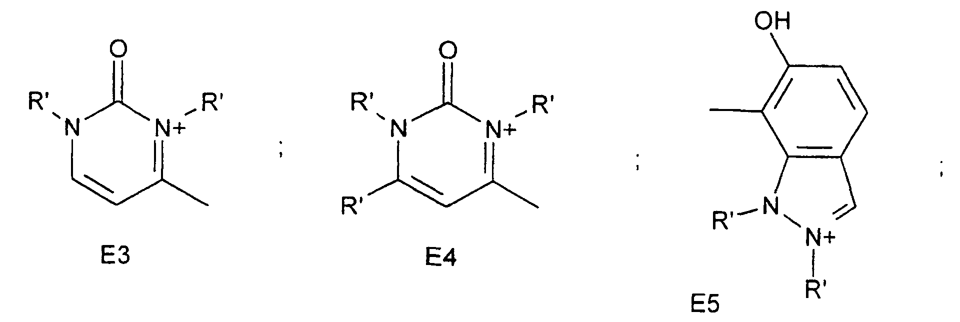 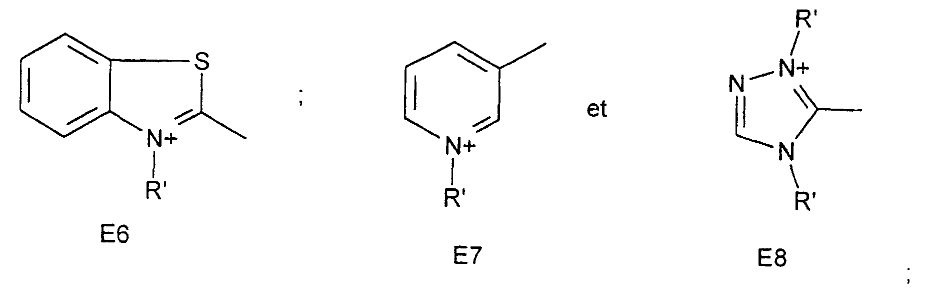 dans lesquelles R' représente un radical alkyle en C₁-C₄ ;
lorsque m = 0 et que D₁ représente un atome d'azote, alors E peut également désigner un groupement de structure E9 suivante : 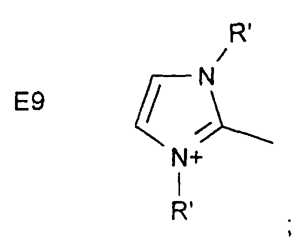 dans laquelle R' représente un radical alkyle en C₁-C₄.
**d) les composés de formule (IV) suivante :**
G―N=N―J (IV)
dans laquelle :
**le symbole G** représente un groupement choisi parmi les structures G₁ à G₃ suivantes : 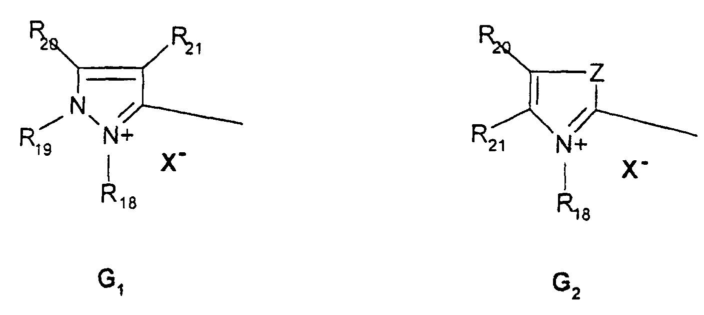 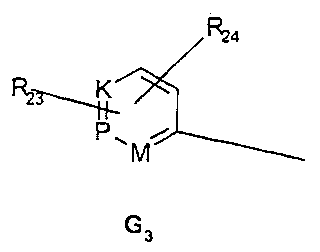 structures G₁ à G₃ dans lesquelles,
R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₁₉ désigne un radical alkyle en C₁-C₄ ou un radical phényle;
R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄, alcoxy en C₁-C₄, ou NO₂;
R₂₀ peut désigner en outre un atome d'hydrogène;
Z désigne un atome d'oxygène, de soufre ou un groupement -NR₁₉;
M représente un groupement -CH, -CR (R désignant alkyle en C₁-C₄), ou -NR₂₂(X⁻)ᵣ;
K représente un groupement -CH, -CR (R désignant alkyle en C₁-C₄), ou -NR₂₂(X⁻)ᵣ;
P représente un groupement -CH, -CR (R désignant alkyle en C₁-C₄), ou -NR₂₂(X⁻)ᵣ; r désigne zéro ou 1;
R₂₂ représente un atome O⁻, un radical alcoxy en C₁-C₄, ou un radical alkyle en C₁-C₄;
R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
X⁻ représente un anion de préférence choisi parmi le chlorure, l'iodure, le méthyl sulfate, l'éthyl sulfate, l'acétate et le perchlorate;
sous réserve que,
si R₂₂ désigne O⁻, alors r désigne zéro;
si K ou P ou M désignent -N-alkyle C₁-C₄ X⁻, alors R₂₃ ou R₂₄ est différent d'un atome d'hydrogène;
si K désigne -NR₂₂(X⁻)ᵣ, alors M= P= -CH, -CR;
si M désigne -NR₂₂(X⁻)ᵣ, alors K= P= -CH, -CR;
si P désigne -NR₂₂(X⁻)ᵣ, alors K= M et désignent -CH ou -CR;
si Z désigne un atome de soufre avec R₂₁ désignant alkyle en C₁-C₄, alors R₂₀ est différent d'un atome d'hydrogène;
si Z désigne -NR₁₉ avec R₁₉ désignant alkyle en C₁-C₄, alors au moins l'un des radicaux R₁₈, R₂₀ ou R₂₁ de G₂ est différent d'un radical alkyle en C₁-C₄;
**le symbole J représente :**
- **(a)** un groupement de structure J₁ suivante : 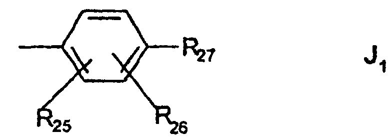 structure J₁ dans laquelle,
R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec
R₂₆ un cycle à 5 ou 6 chaînons contenant ou non un ou plusieurs hétéroatomes choisis parmi l'azote, l'oxygène ou le soufre;
R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre;
R₂₇ représente un atome d'hydrogène, un radical -OH, un radical -NHR₂₈, un radical -NR₂₉R₃₀;
R₂₈ représente un atome d'hydrogène, un radical alkyle en C₁-C₄, un radical monohydroxyalkyle en C₁-C₄, polyhydroxyalkyle en C₂-C₄, un radical phényle;
R₂₉ et R₃₀, identiques ou différents, représentent un radical alkyle en C₁-C₄, un radical monohydroxyalkyle en C₁-C₄, polyhydroxyalkyle en C₂-C₄;
- **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 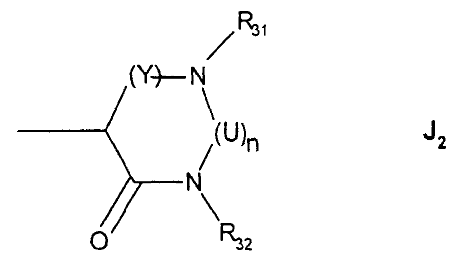 structure J₂ dans laquelle,
R₃₁ et R₃₂, identiques ou différents, représentent un atome d'hydrogène , un radical alkyle en C₁-C₄, un radical phényle;
Y désigne le radical -CO- ou le radical 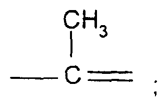 n = 0 ou 1, avec, lorsque n désigne 1, U désigne le radical -CO- .
ladite composition étant **caractérisée par le fait qu'**elle contient en outre
**(ii)** au moins un sel d'ammonium quaternaire choisi dans le groupe comprenant :
**(ii)**_{**1**} - ceux de formule (V) suivante : 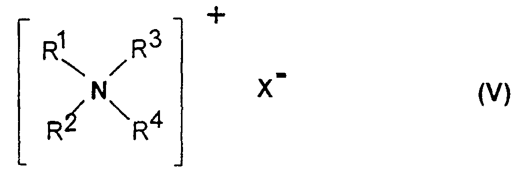 dans laquelle,
les radicaux R¹ à R⁴, identiques ou différents, désignent un radical hydrocarboné aliphatique saturé ou insaturé, linéaire ou ramifié, comportant de 1 à environ 30 atomes de carbone, ou un radical alcoxy, alcoxycarbonylalkyle, polyoxyalkylène, alkylamido, alkylamidoalkyle, hydroxyalkyle, aromatique, aryle, alkylaryle, comportant de 12 à environ 30 atomes de carbone, avec au moins un radical parmi R¹ R² R³ R⁴ désignant un radical comportant de 8 à 30 atomes de carbone;
X⁻ est un anion choisi dans le groupe des halogénures, phosphates, acétates, lactates, alkylsulfates;
**(ii)**_{**2**} - les sels de l'imidazolium de formule (VI) suivante : 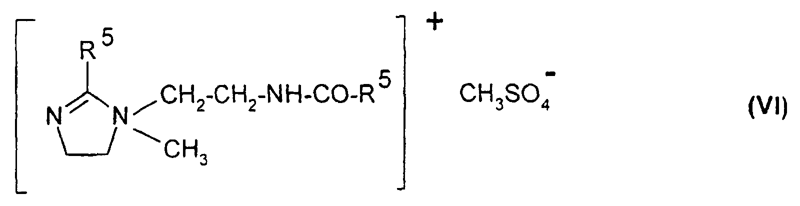 dans laquelle,
R⁵ est choisi parmi les radicaux alcényle et/ou alkyles comportant de 13 à 31 atomes carbone et dérivés des acides gras du suif.
**(ii)**_{**3**} - les sels de diammonium quaternaire de formule (VII) suivante : 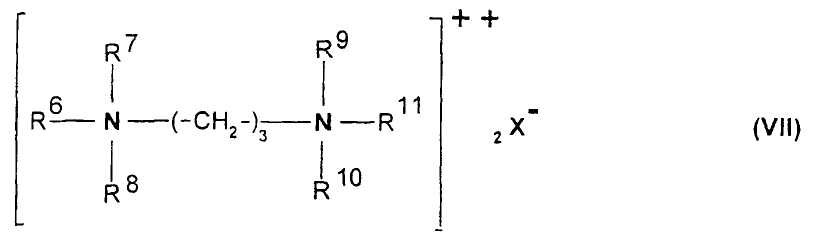 dans laquelle,
R⁶ désigne un radical aliphatique comportant de 16 à 30 atomes de carbone, R⁷, R⁸, R9, R¹⁰, et R¹¹ sont choisis parmi l'hydrogène ou un radical alkyle comportant de 1 à 4 atomes de carbone, et X⁻ est un anion choisi dans le groupe des halogénures, acétates, phosphates et sulfates.

2. Composition selon la revendication 1, **caractérisée par le fait que** les colorants directs cationiques de formule (I) sont choisis parmi les composés répondant aux structures (I1) à (I54) suivantes : 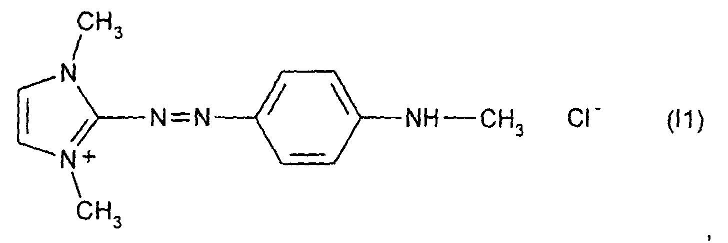 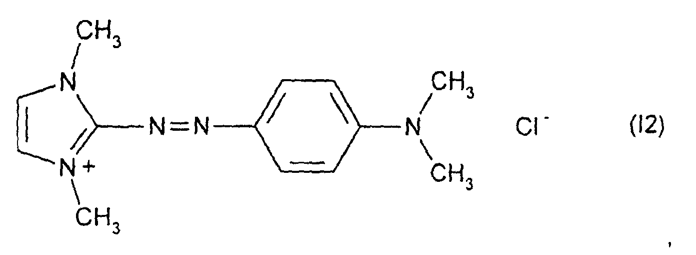 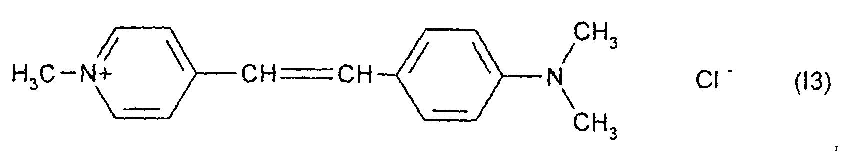 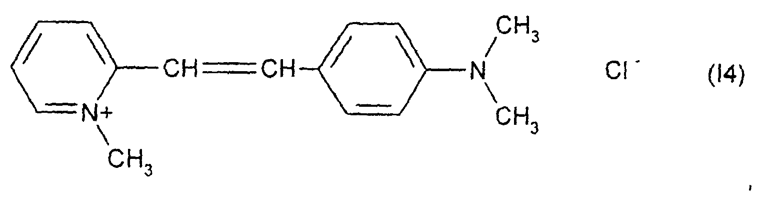 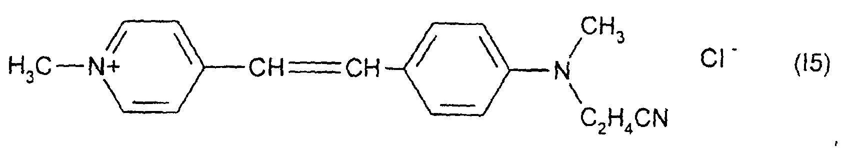 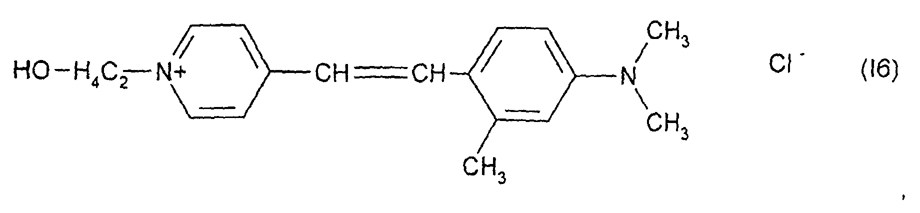 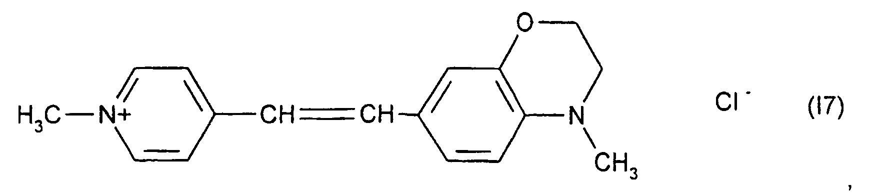 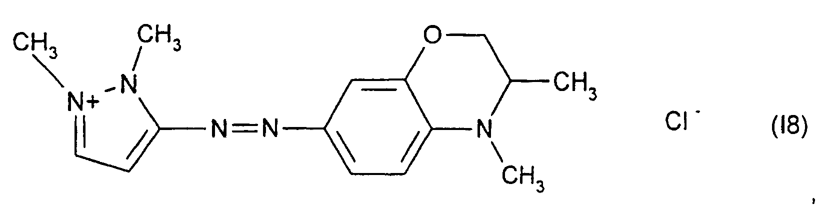 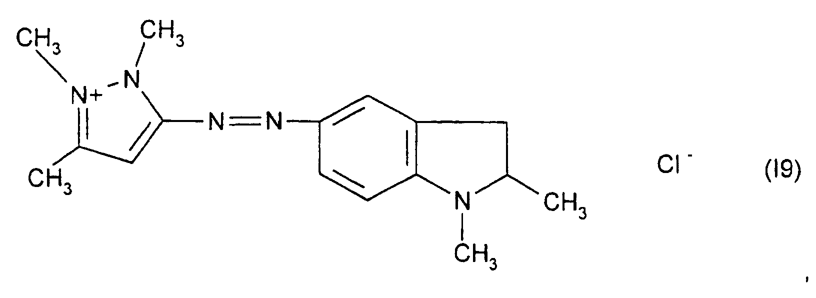 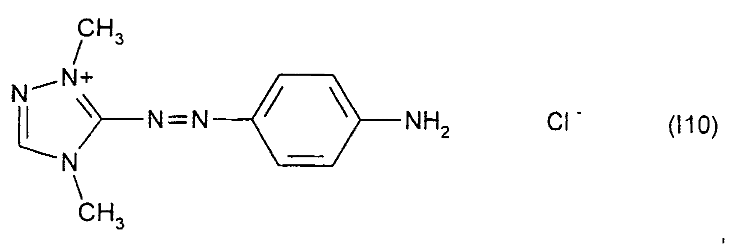 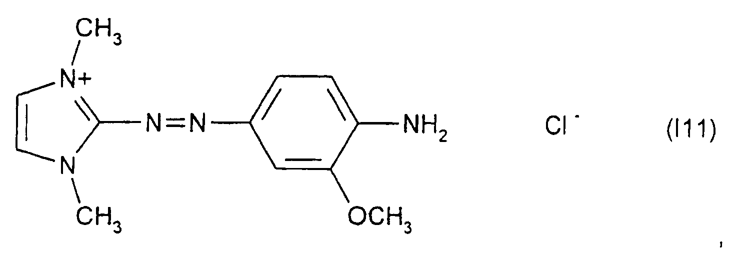 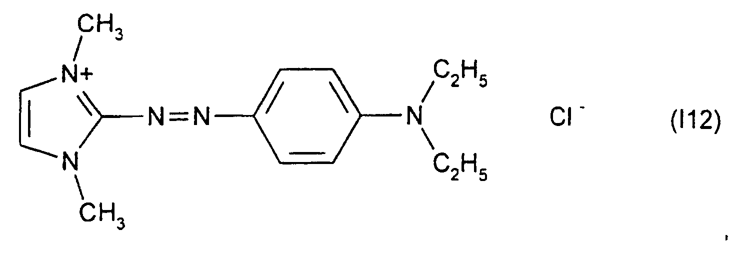 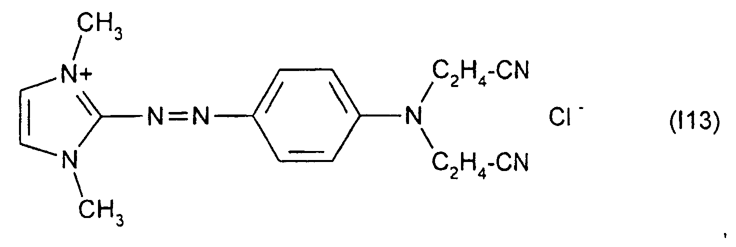 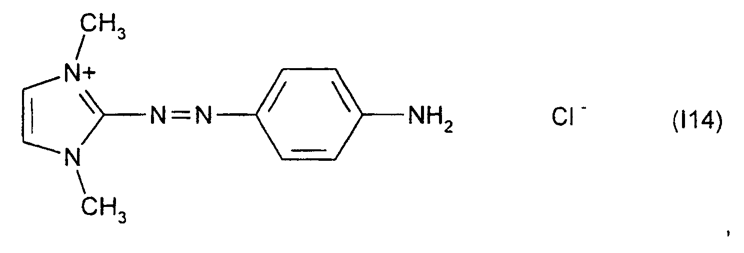 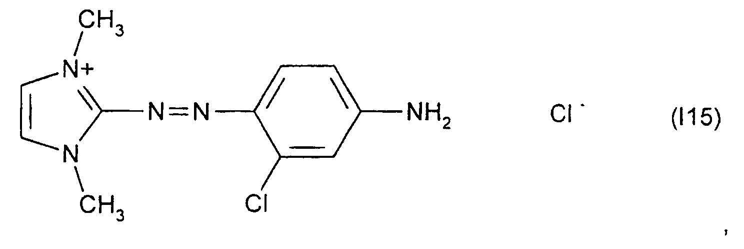 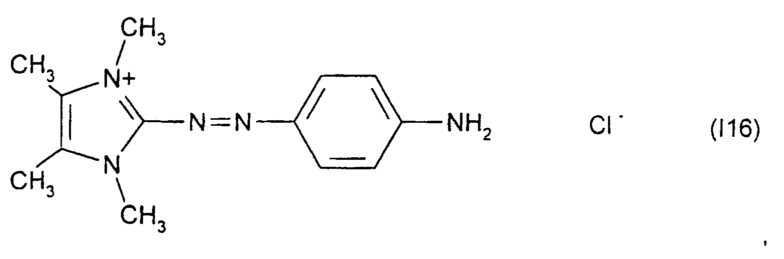 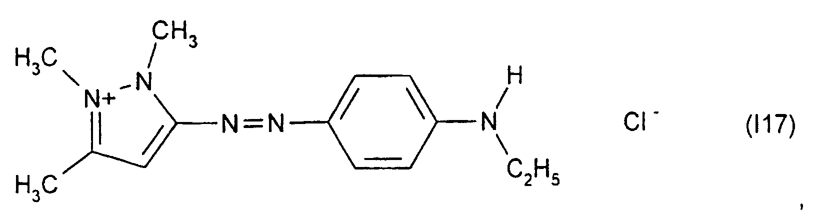 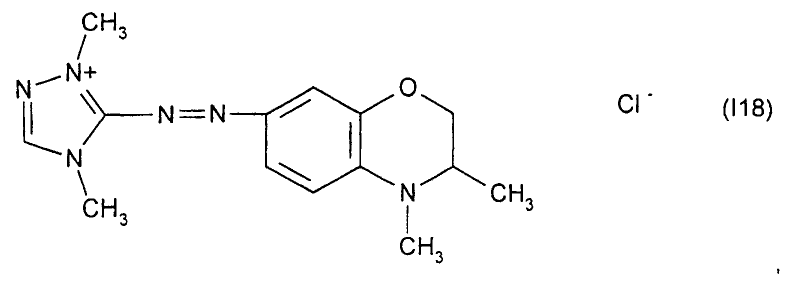 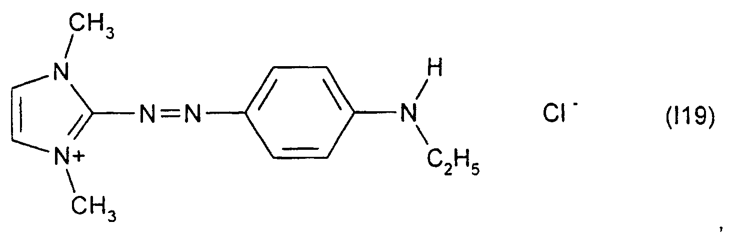 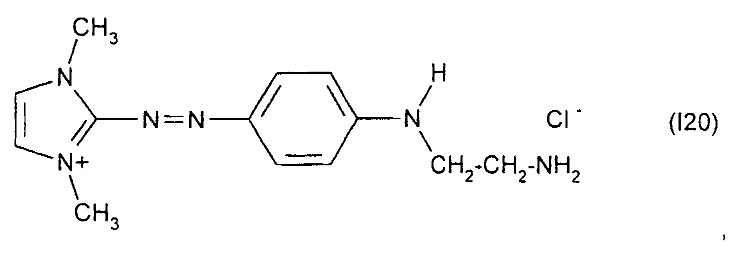 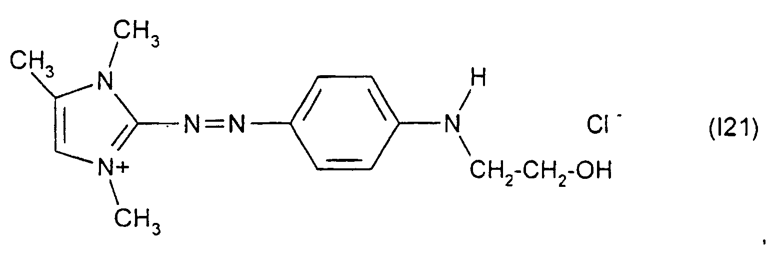 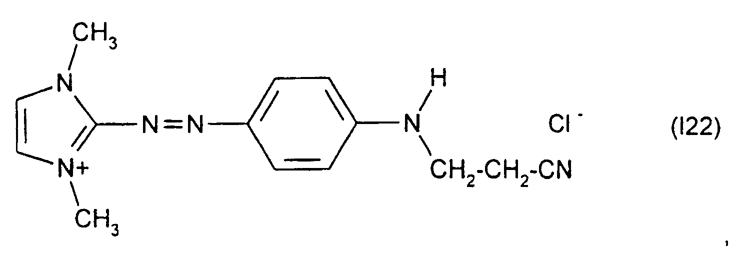 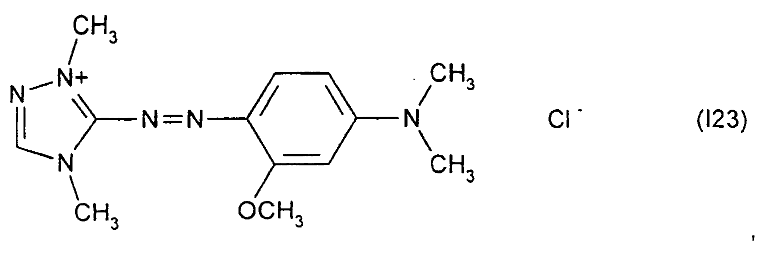 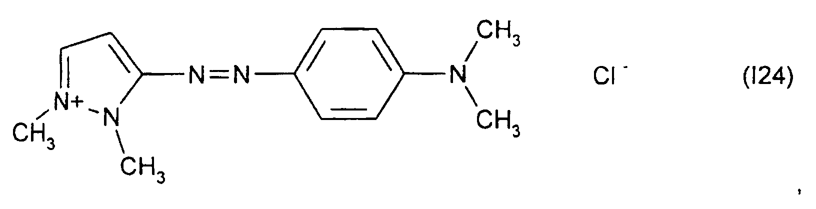 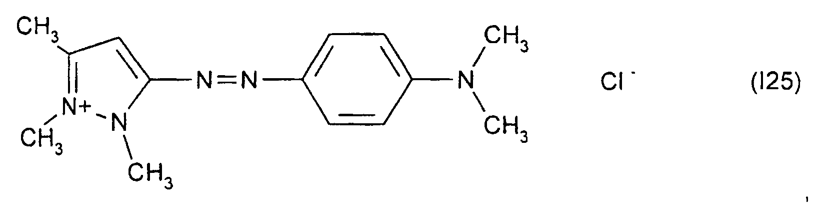 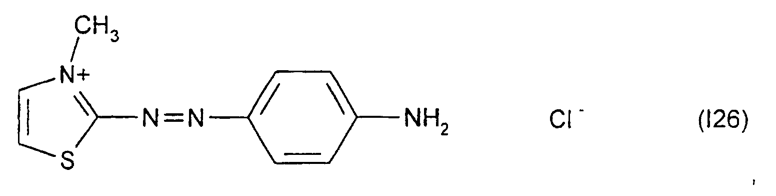 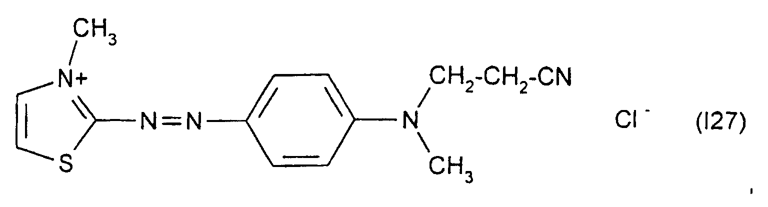 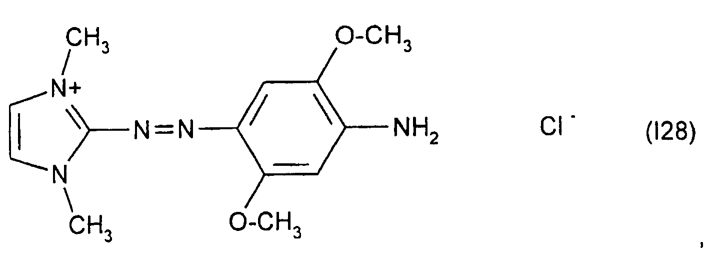 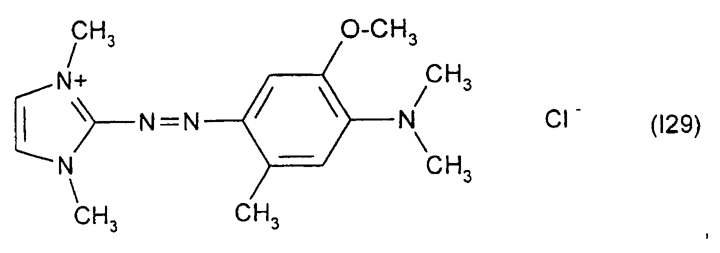 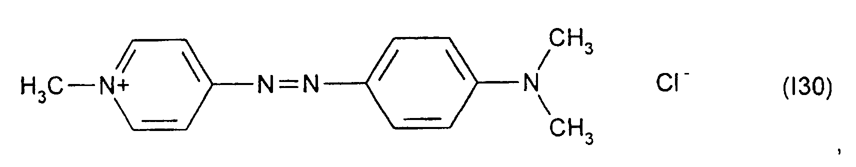 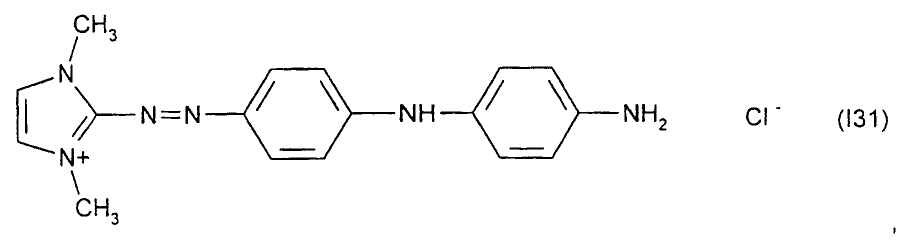 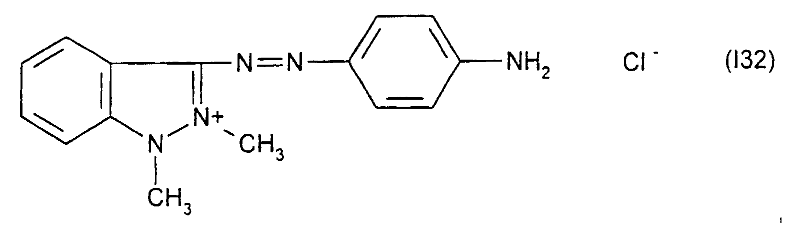 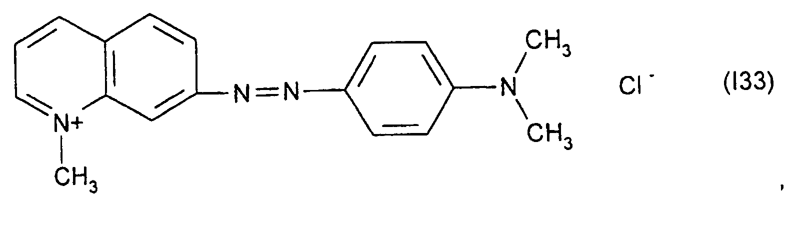 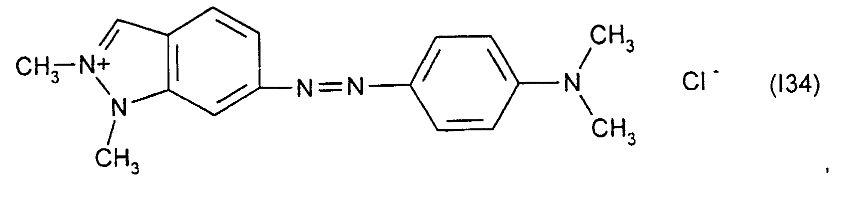 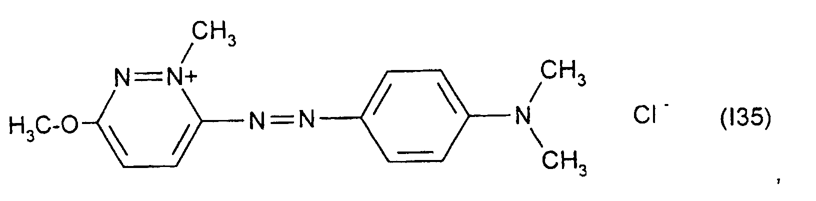 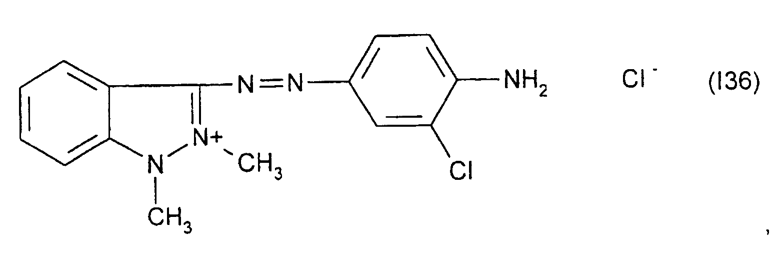 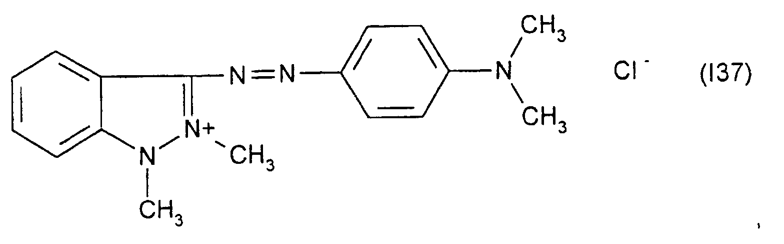 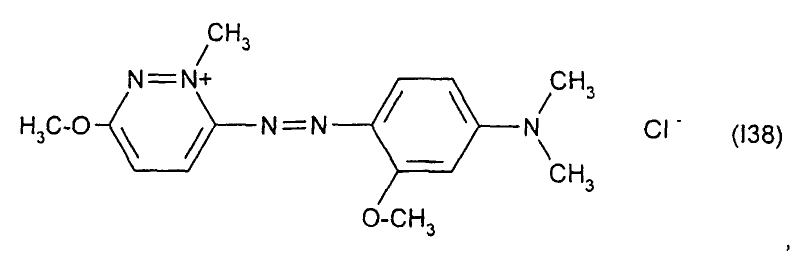 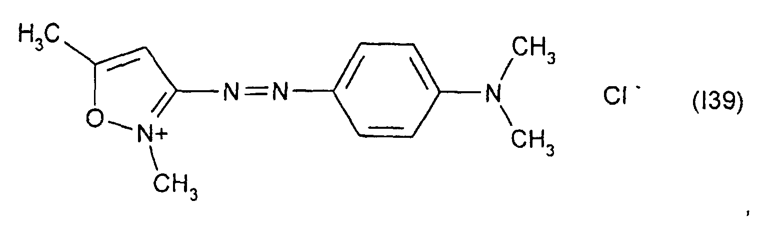 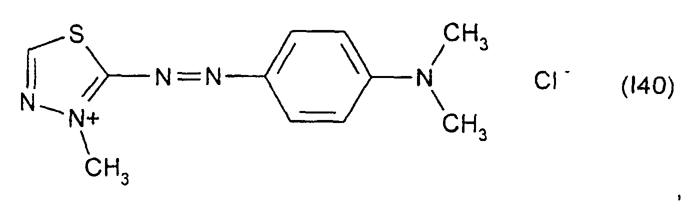 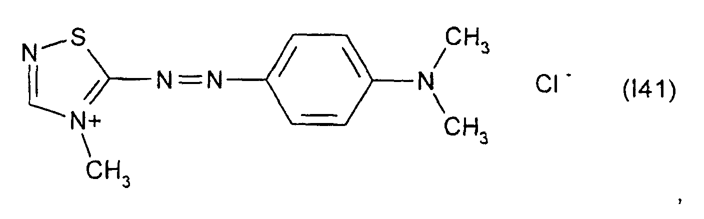 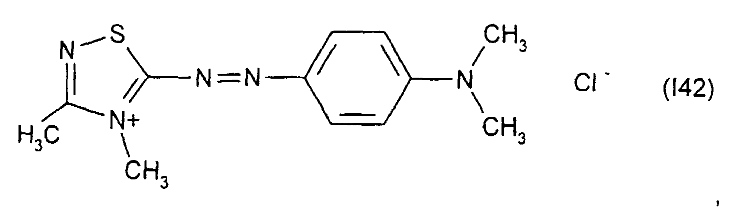 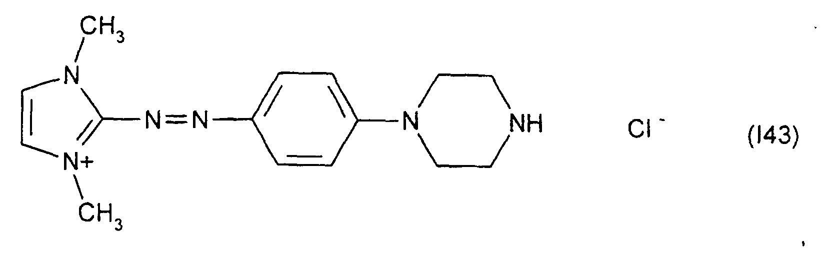 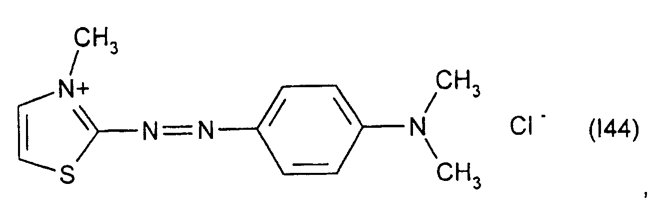 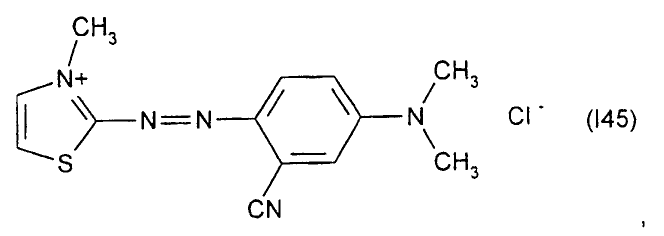 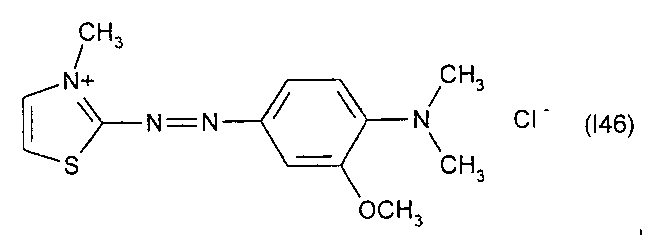 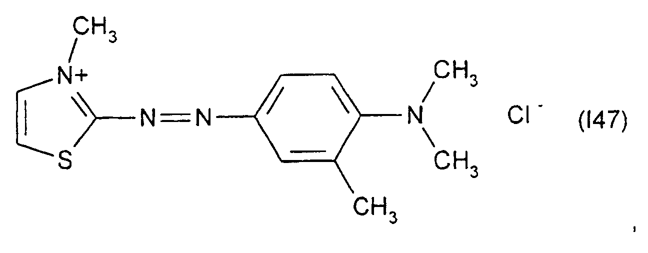 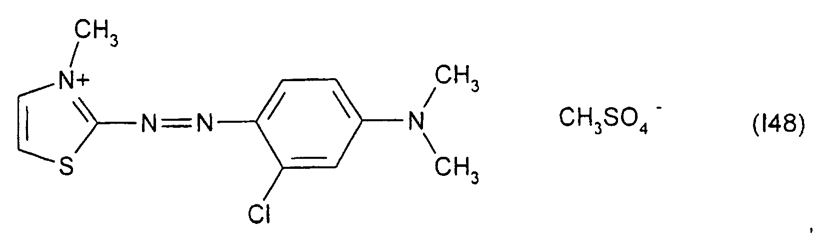 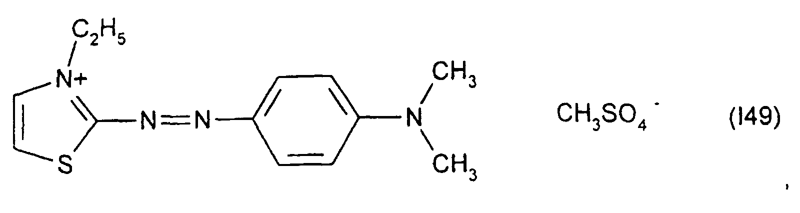 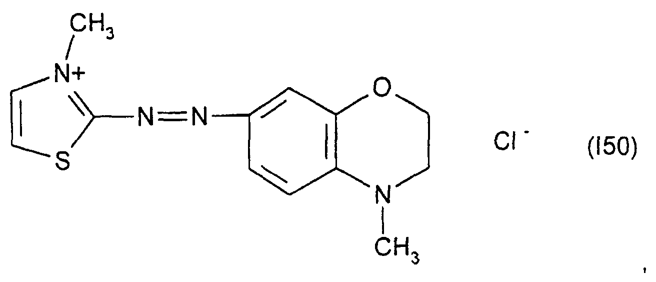 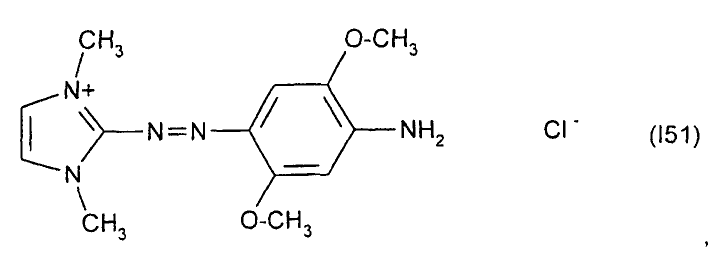 et

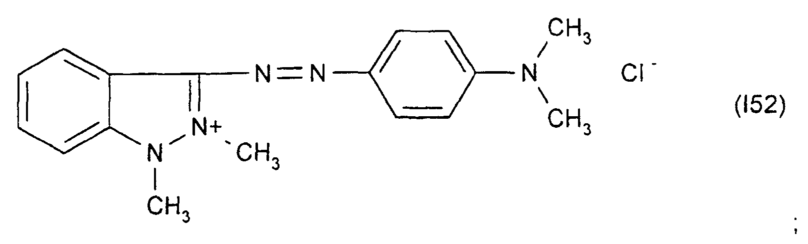

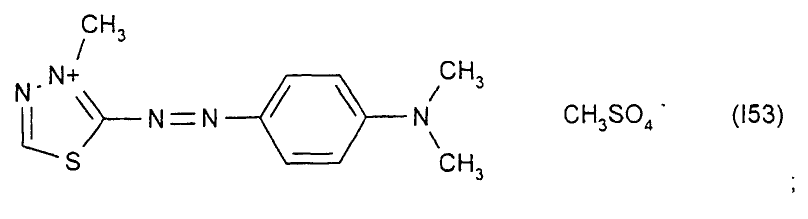

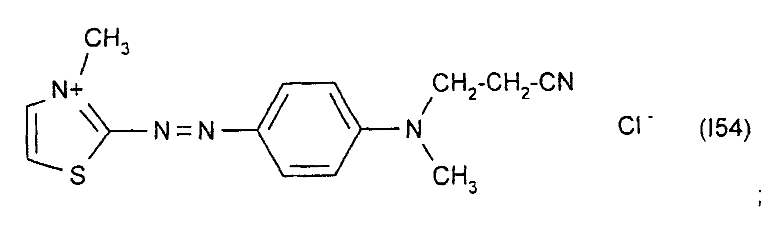

3. Composition selon la revendication 2, **caractérisée par le fait que** les colorants directs cationiques répondent aux structures (I1), (I2), (I14), et (I31).

4. Composition selon la revendication 1, **caractérisée par le fait que** les colorants directs cationiques de formule (II) sont choisis parmi les composés répondant aux structures (II1) à (II9) suivantes : 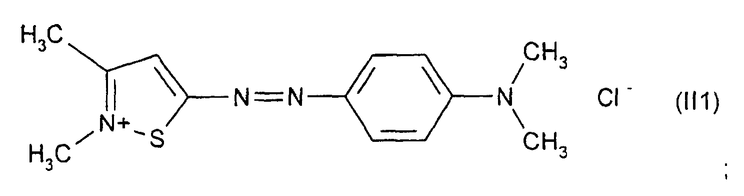 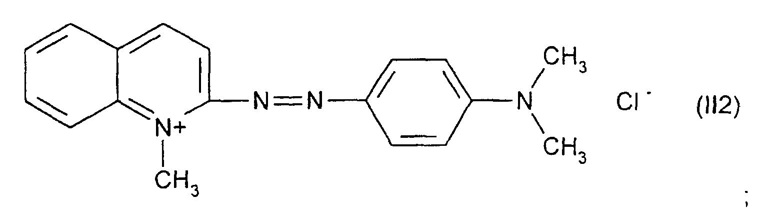 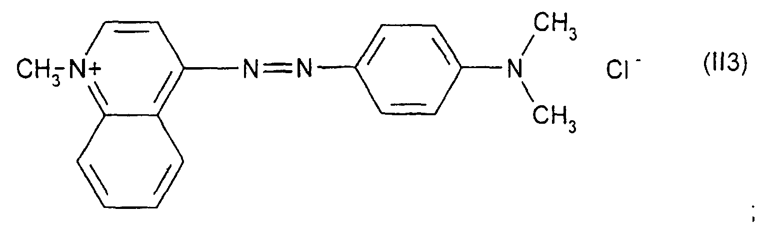 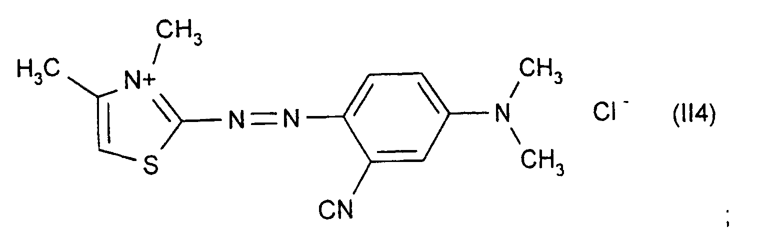 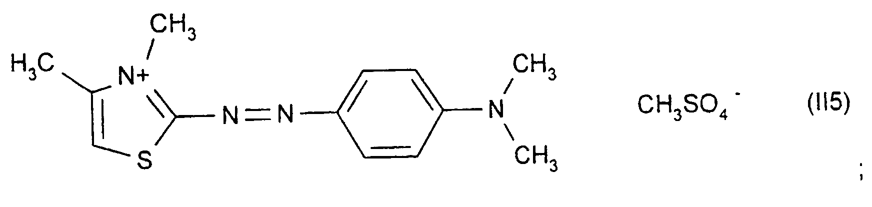 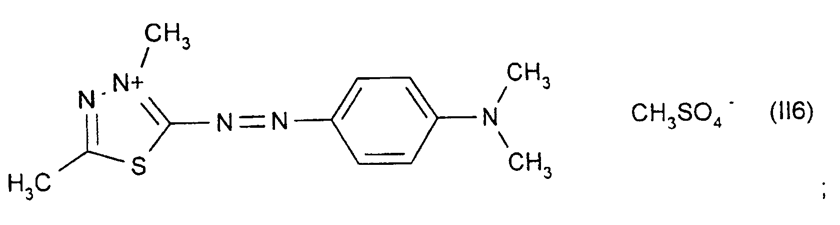 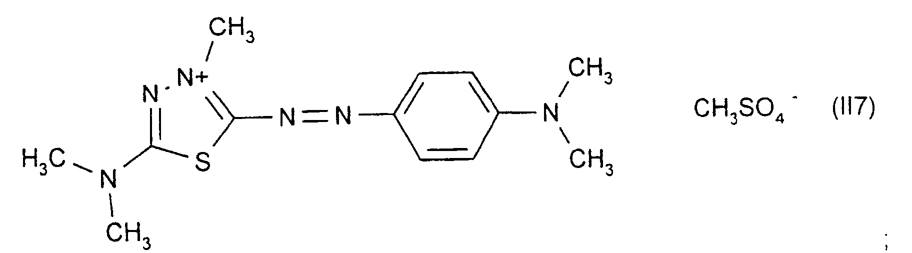 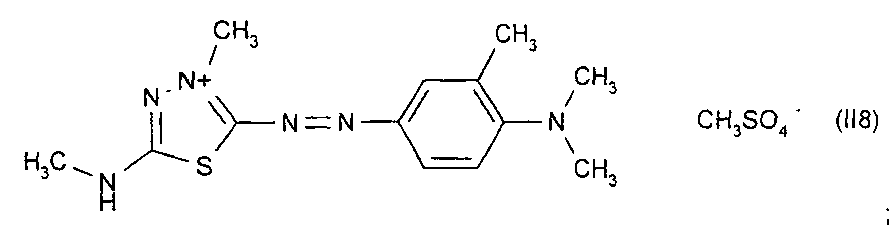 et

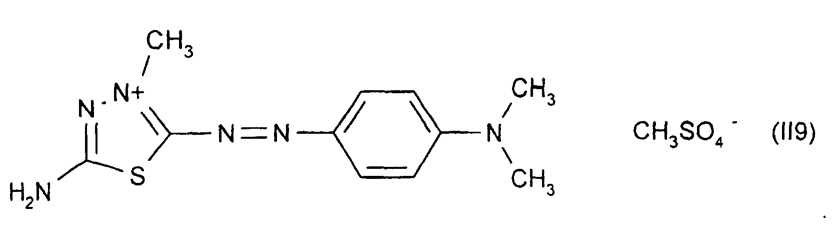

5. Composition selon la revendication 1, **caractérisée par le fait que** les colorants directs cationiques de formule (III) sont choisis parmi les composés répondant aux structures (III1) à (III18) suivantes : 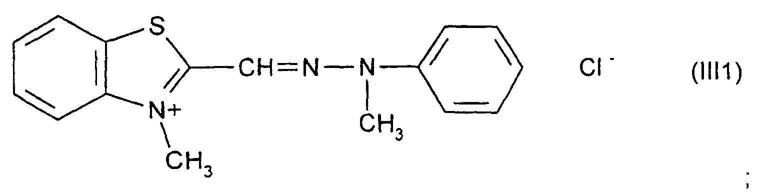 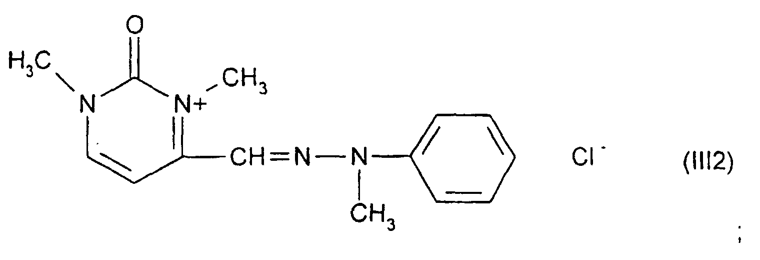 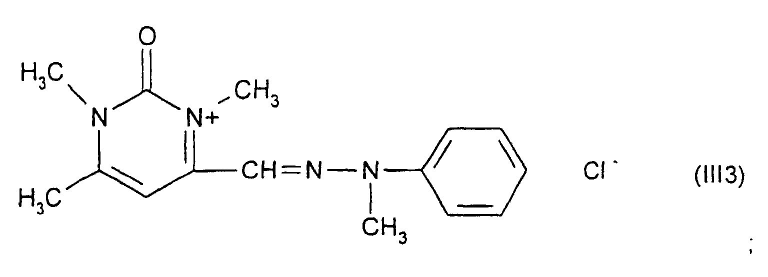 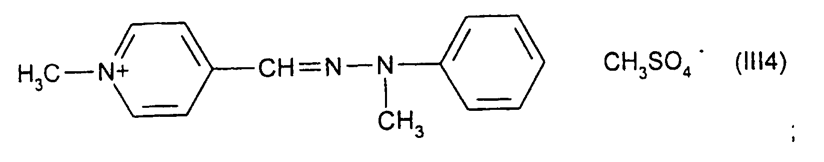 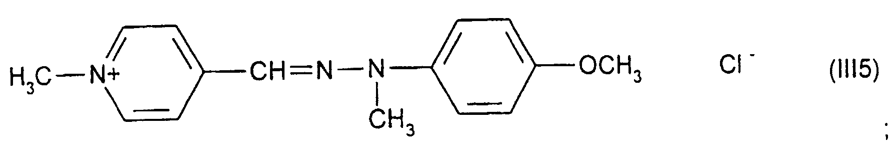 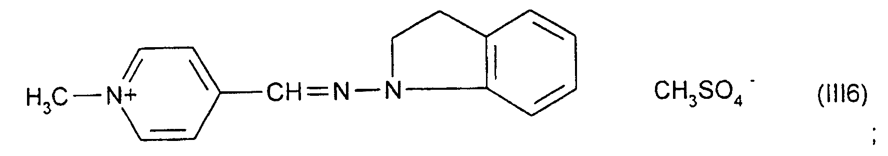 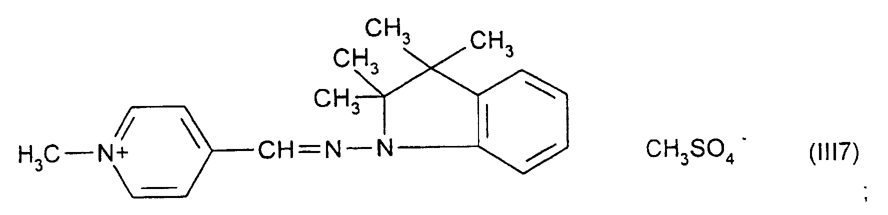 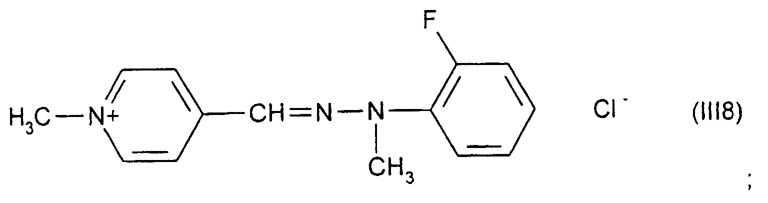 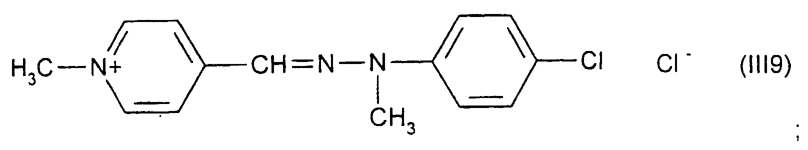 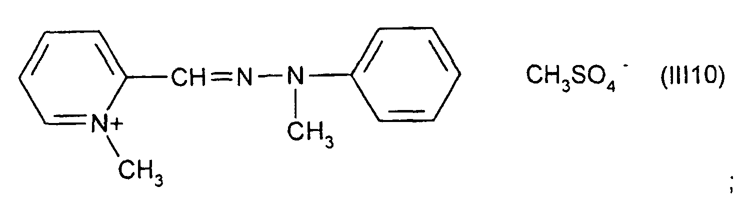 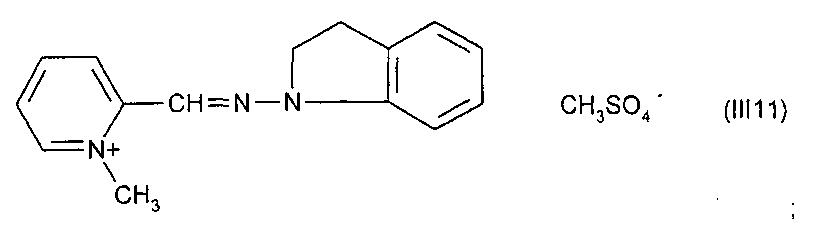 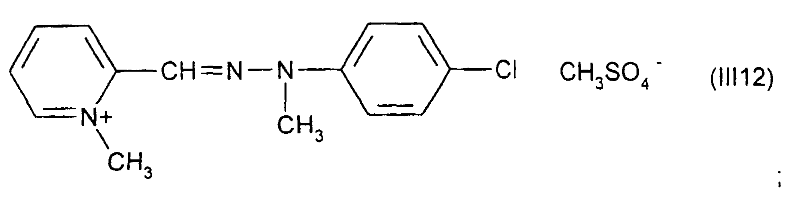 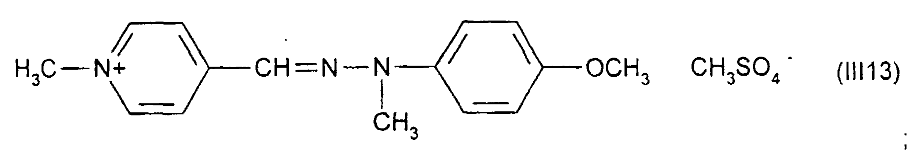 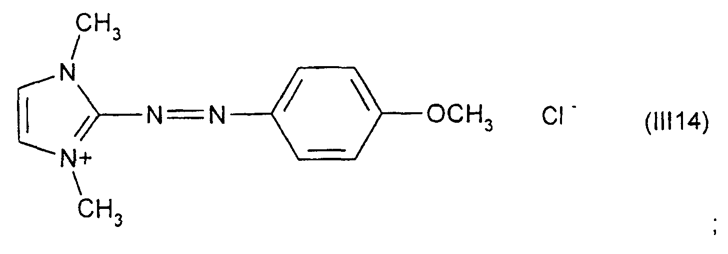 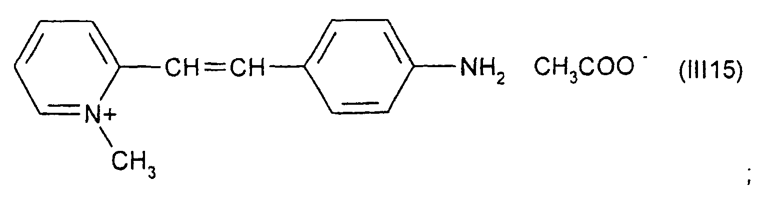 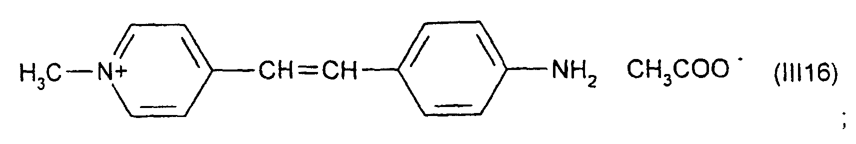 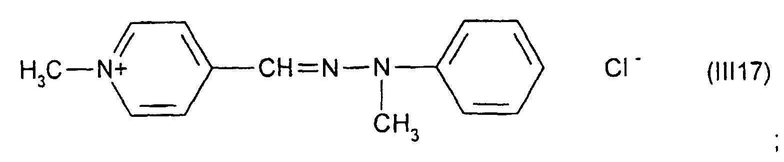 et

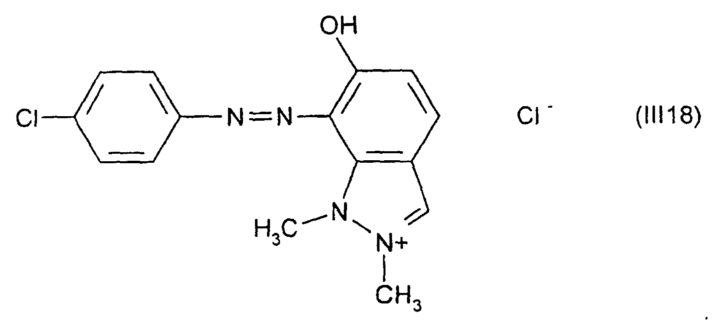

6. Composition selon la revendication 5, **caractérisée par le fait que** les colorants directs cationiques de formule (III) sont choisis parmi les composés répondant aux structures (III4), (III5) et (III13).

7. Composition selon la revendication 1, **caractérisée par le fait que** les colorants directs cationiques de formule (III') sont choisis parmi les composés répondant aux structures (III'1) à (III'3) suivantes : 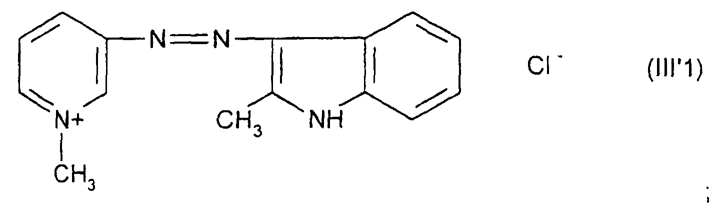 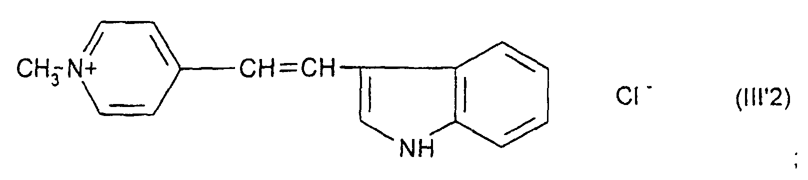 et

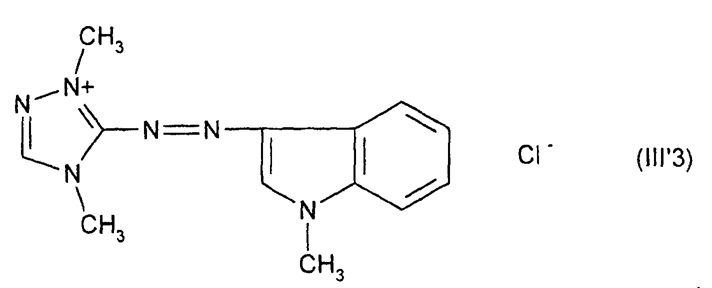

8. Composition selon la revendication 1, **caractérisée par le fait que** les colorants directs cationiques de formule (IV) sont choisis parmi les composés répondant aux structures (IV₁) à (IV₇₇) suivantes :

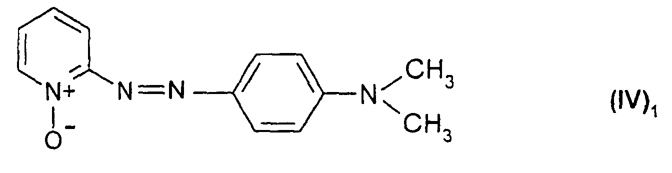

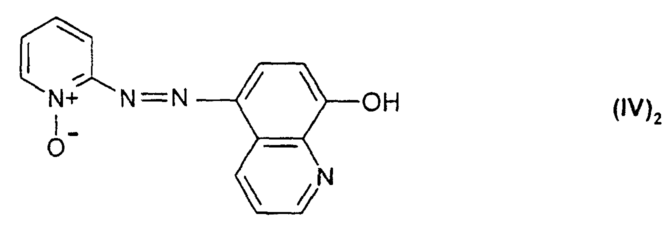

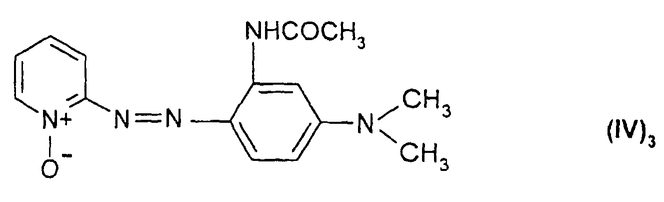

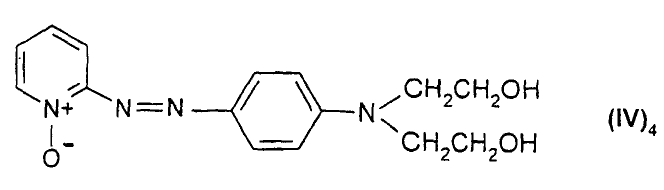

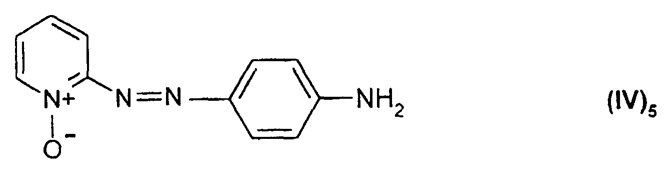

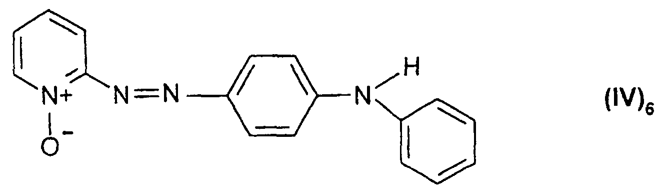

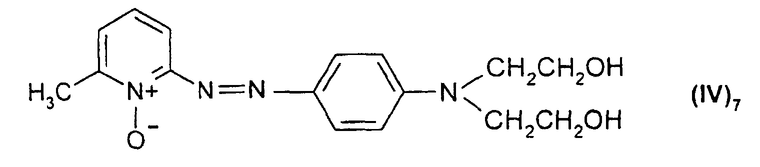

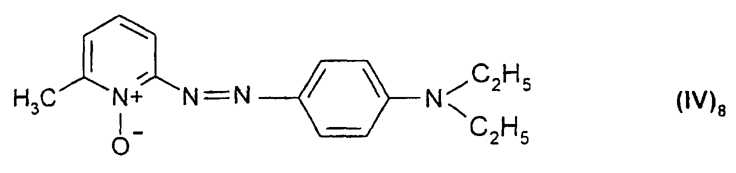

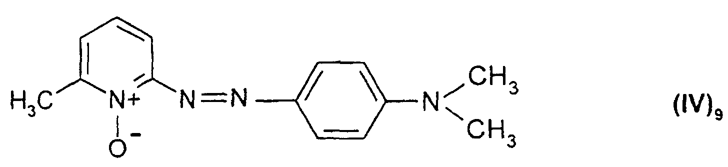

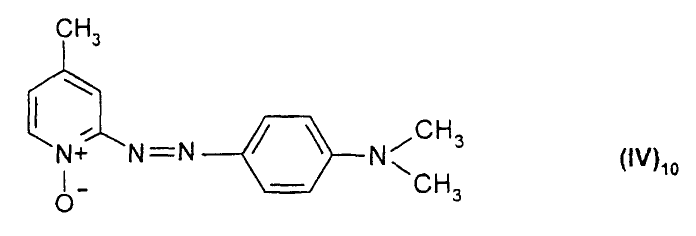

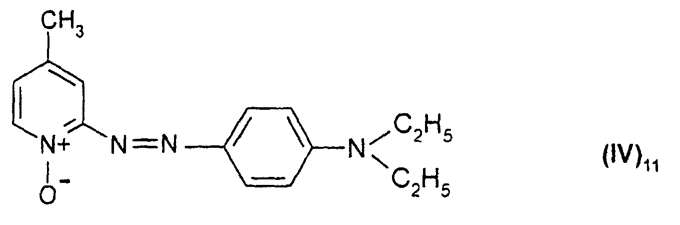

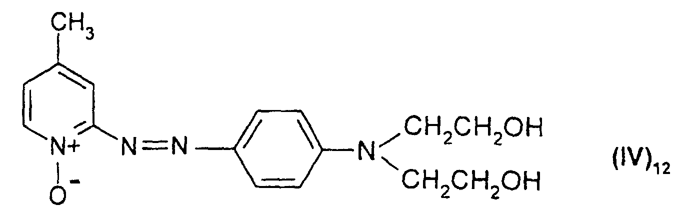

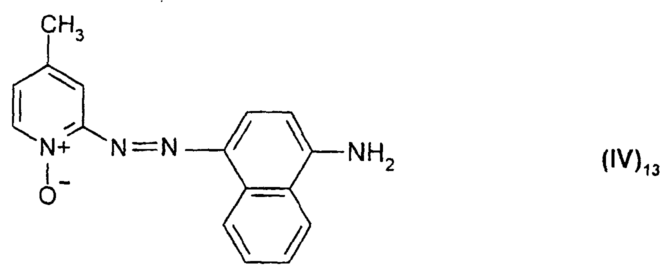

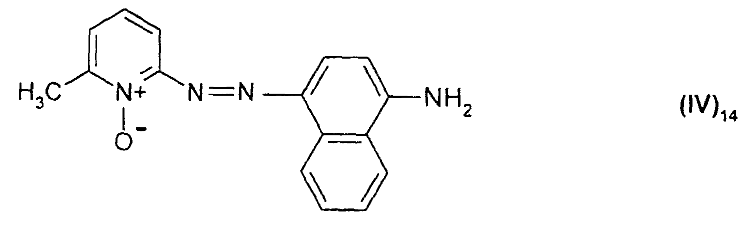

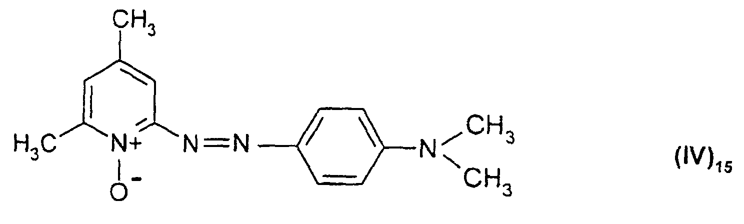

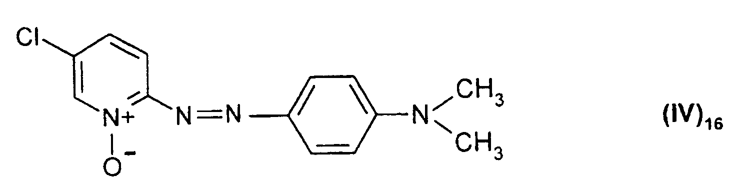

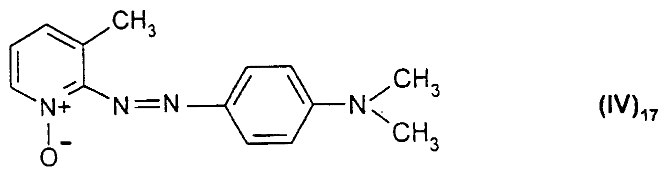

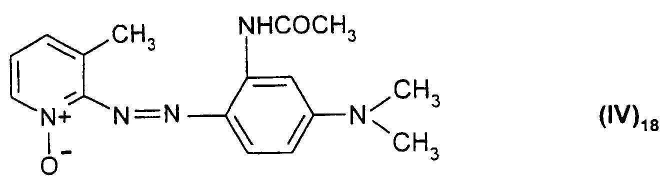

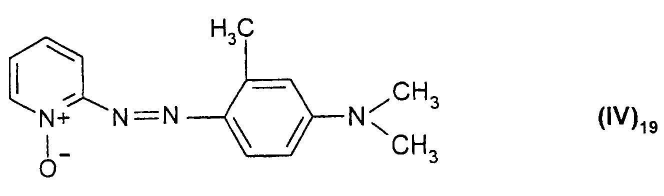

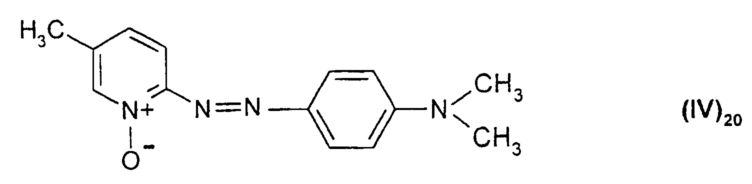

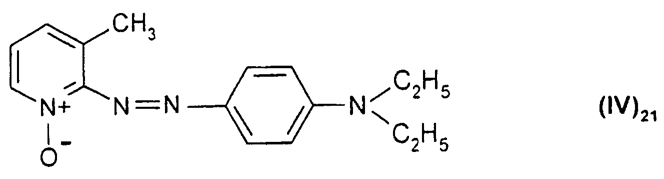

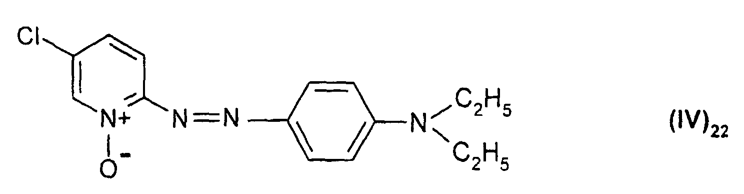

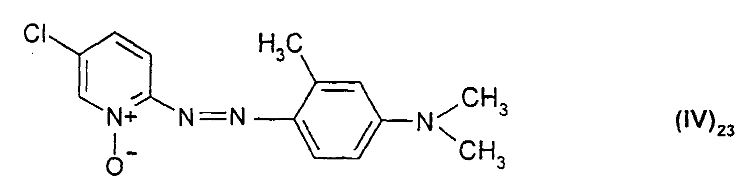

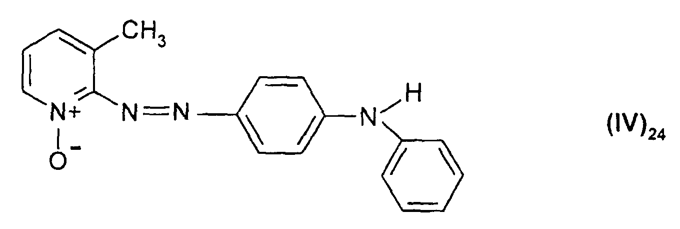

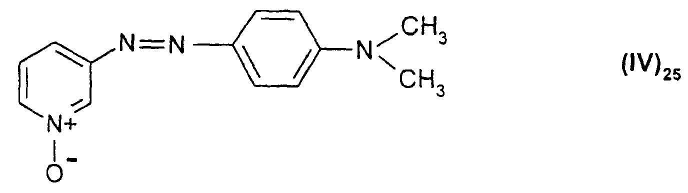

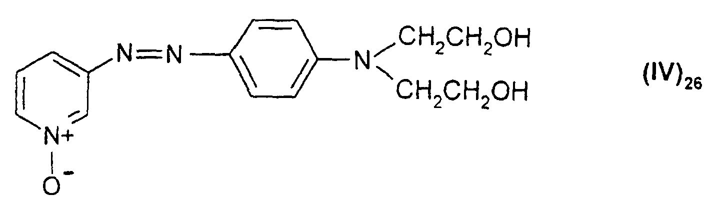

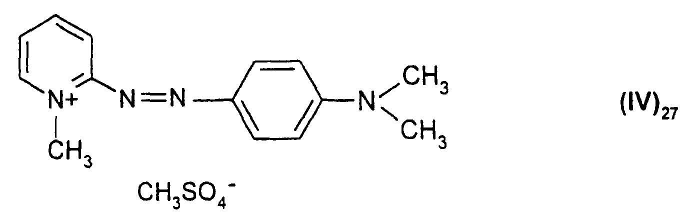

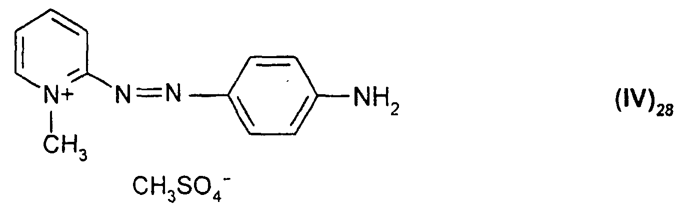

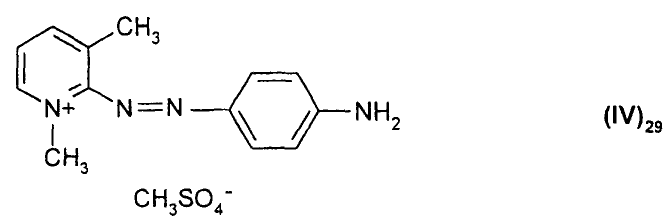

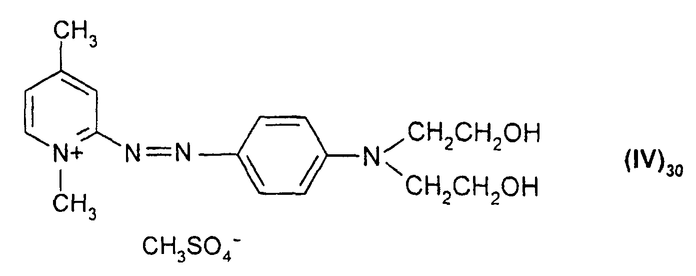

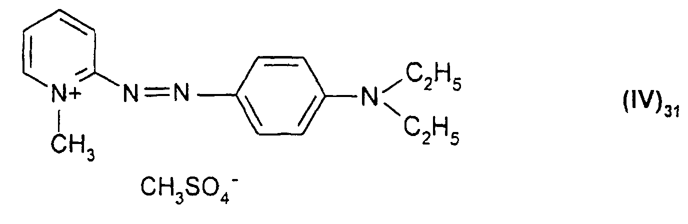

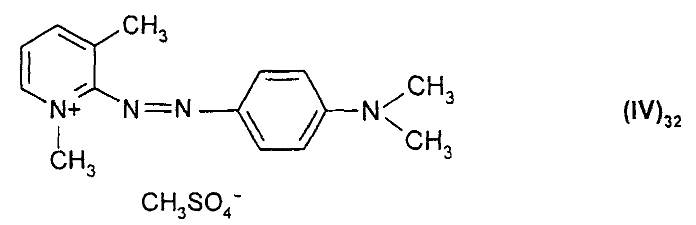

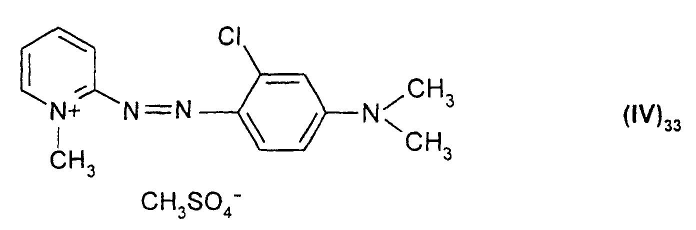

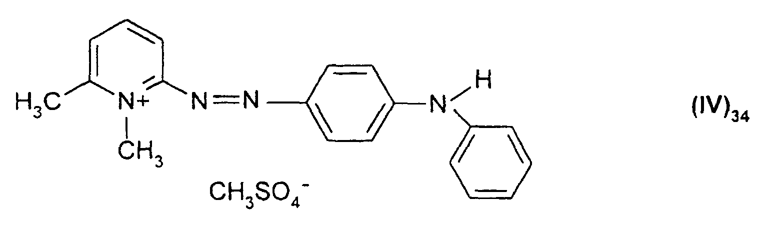

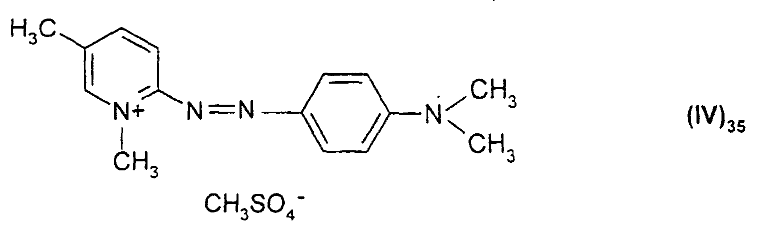

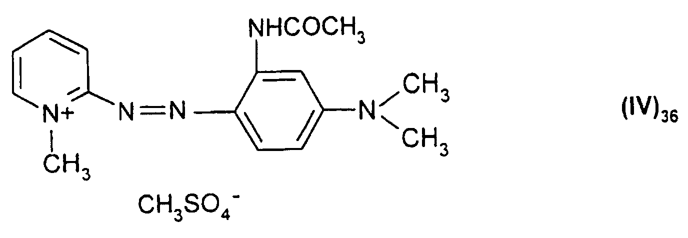

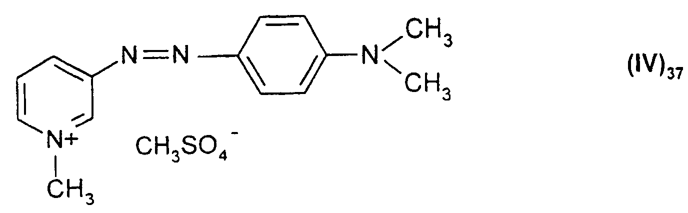

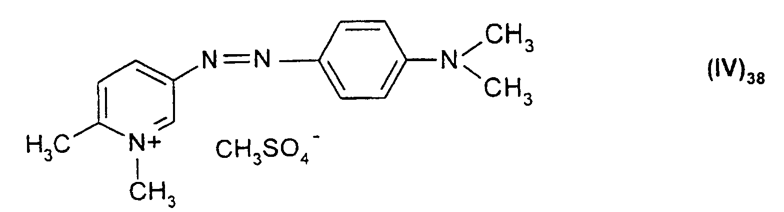

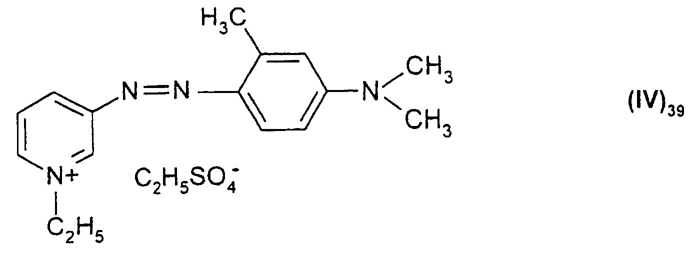

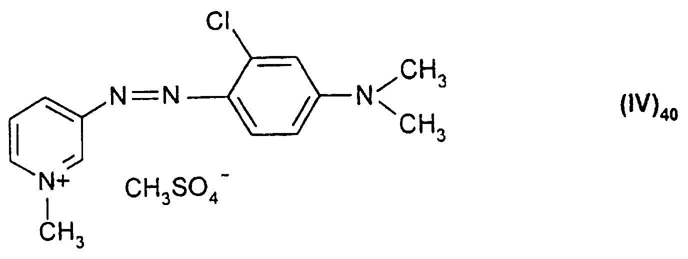

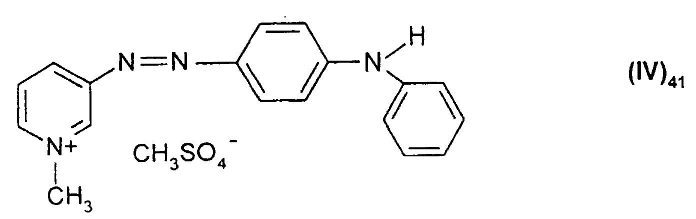

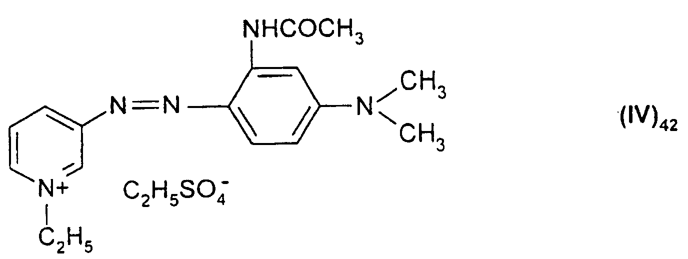

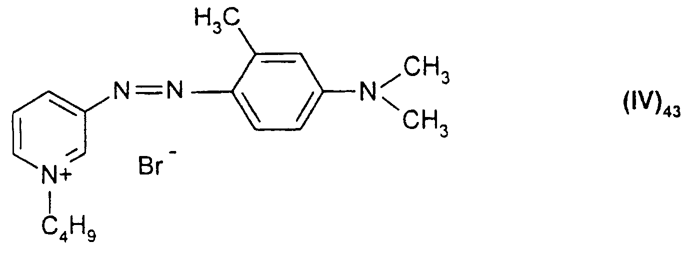

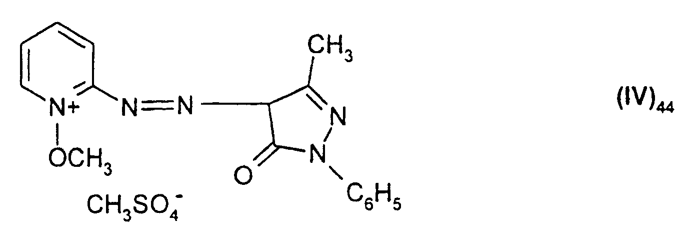

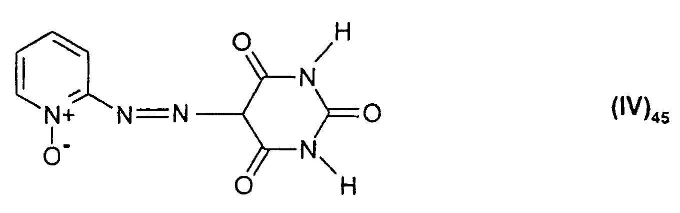

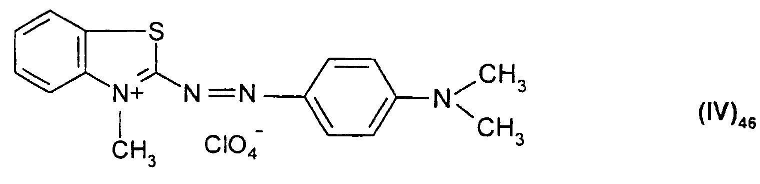

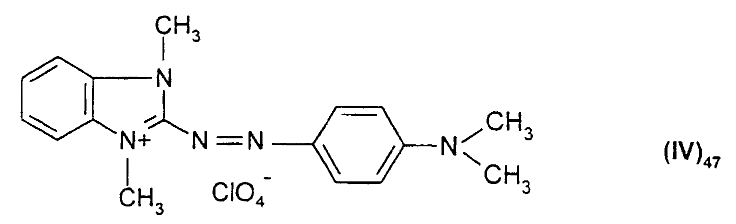

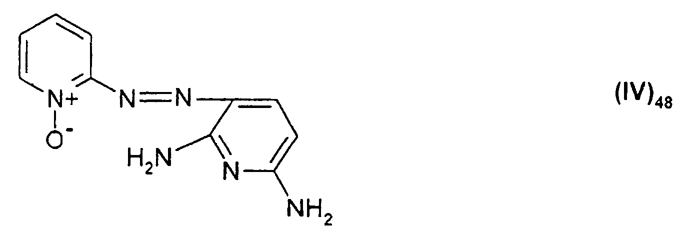

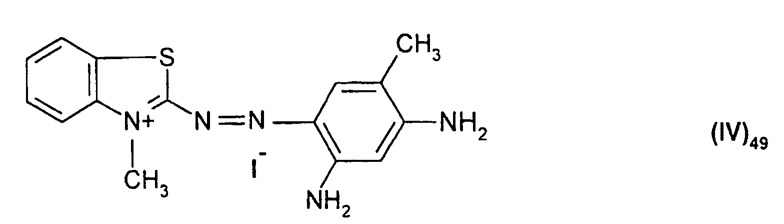

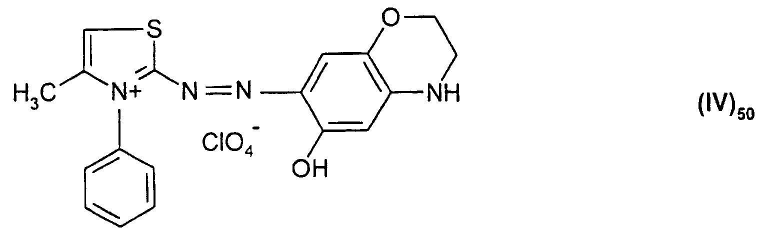

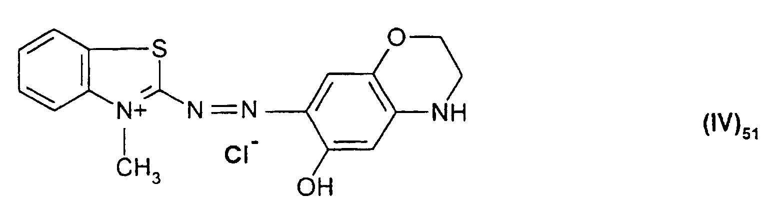

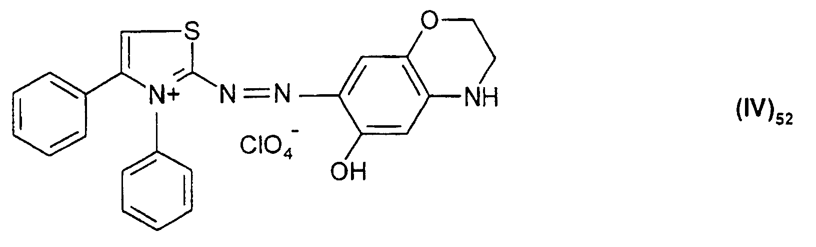

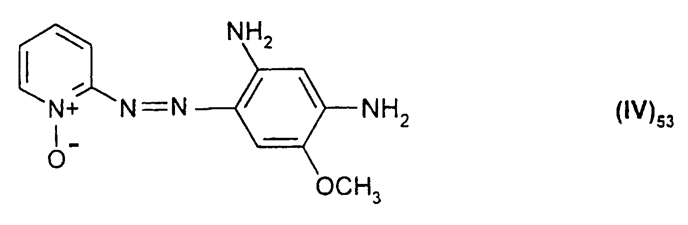

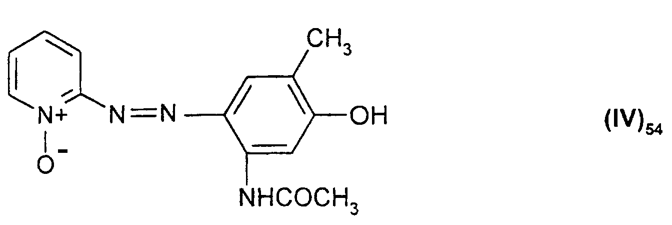

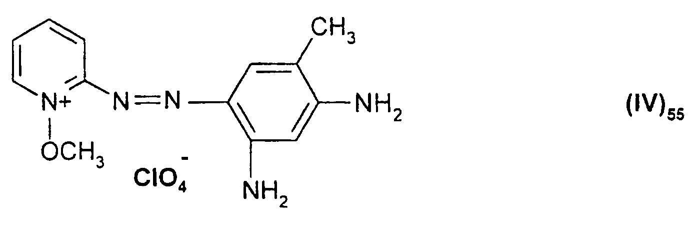

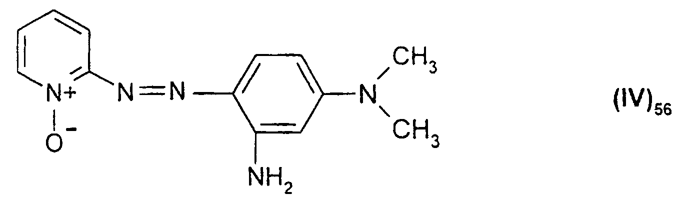

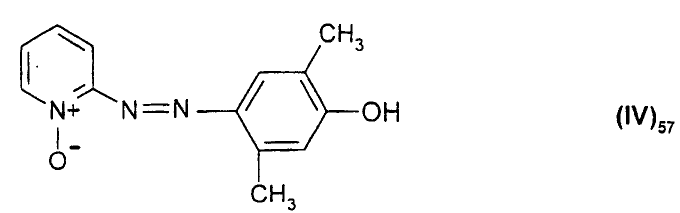

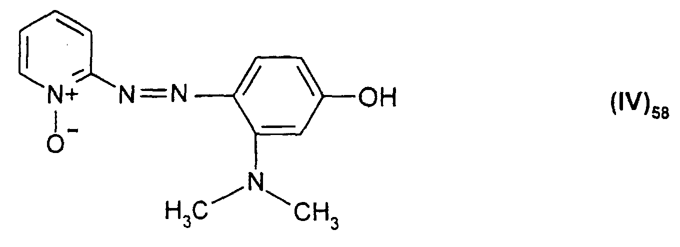

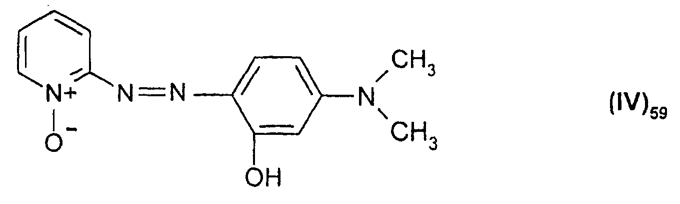

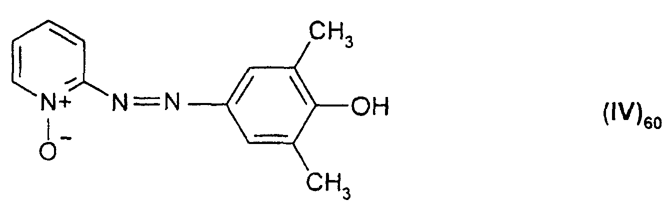

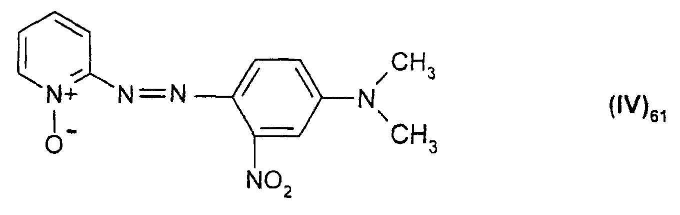

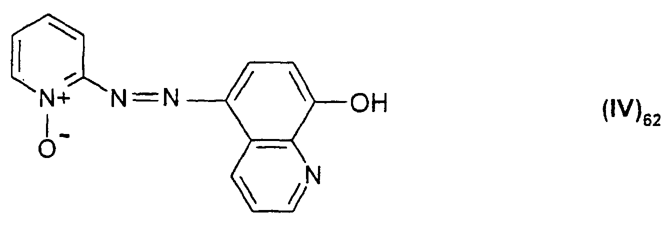

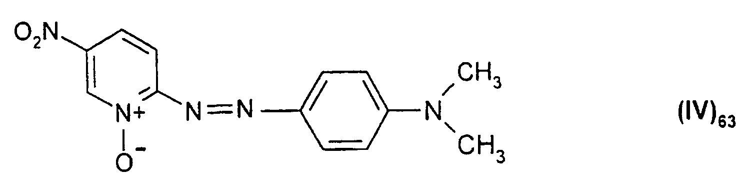

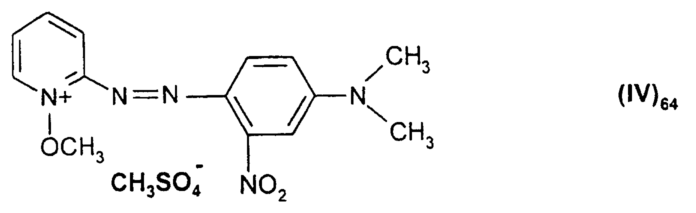

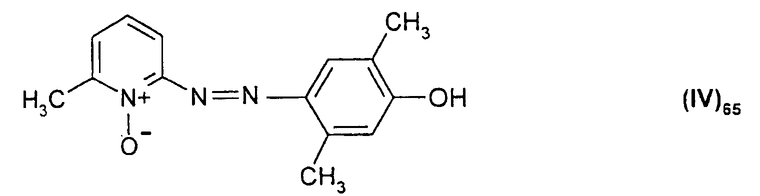

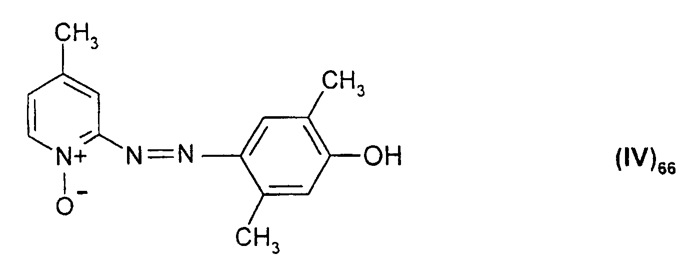

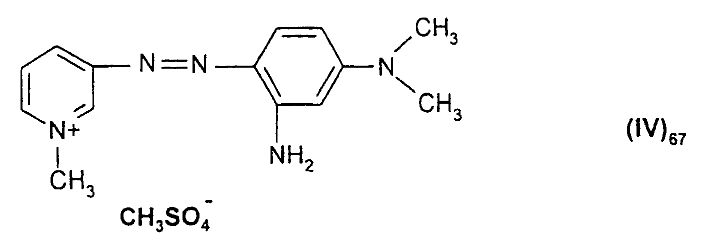

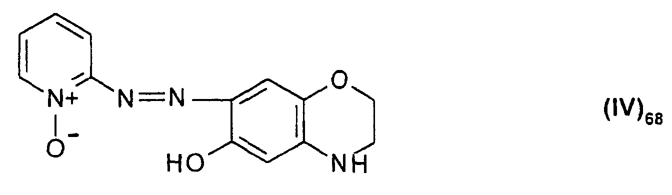

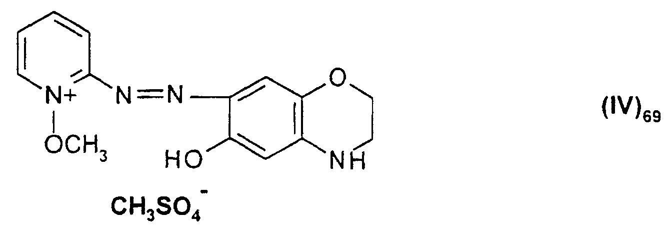

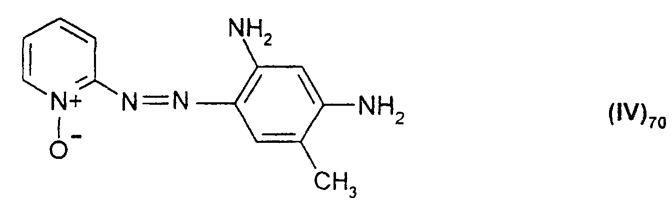

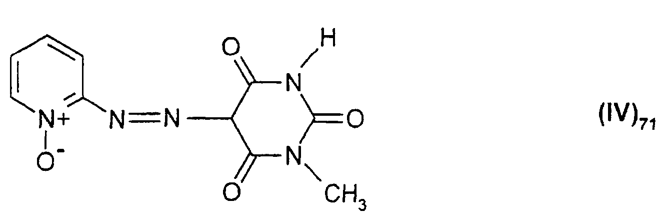

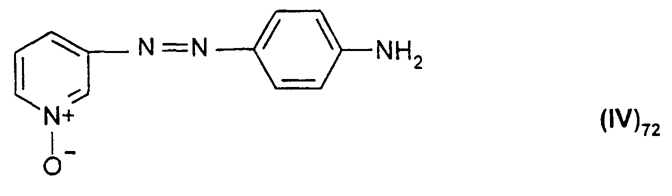

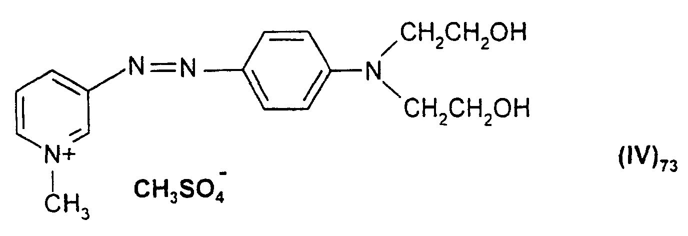

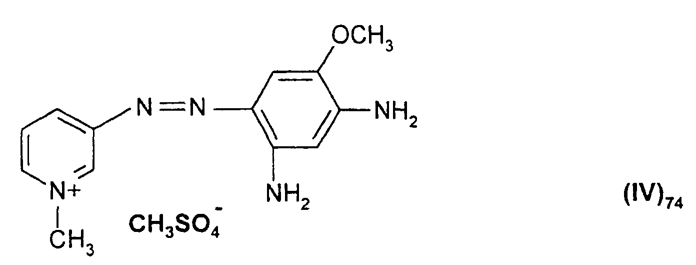

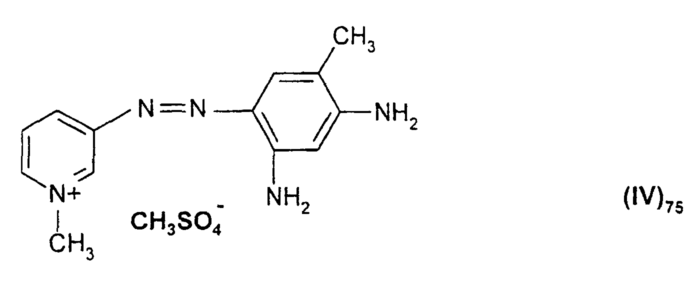

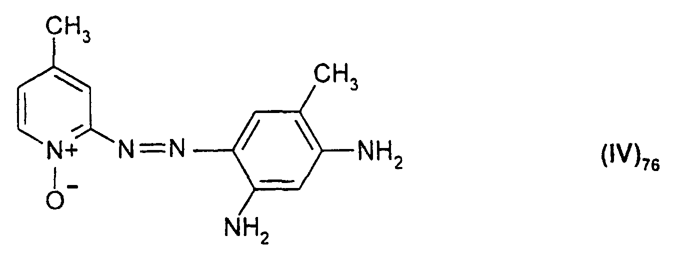

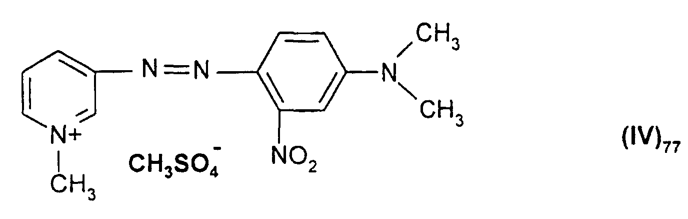

9. Composition selon l'une quelconque des revendications précédentes, **caractérisée par le fait que** le ou les colorants directs cationiques de formules (I), (II), (III), (III') ou (IV) représentent de 0,001 à 10 % en poids du poids total de la composition.

10. Composition selon la revendication 9, **caractérisée par le fait que** le ou les colorants directs cationiques de formules (I), (II), (III) (III') ou (IV) représentent de 0,005 à 5 % en poids du poids total de la composition.

11. Composition selon l'une quelconque des revendications précédentes, **caractérisée par le fait que** le sel d'ammonium quaternaire (ii) de formule (V) est un sel de dialkyldiméthylammonium ou d'alkyltriméthylammonium dans lesquels le radical alkyle comporte de 12 à 22 atomes de carbone.

12. Composition selon la revendication 11, **caractérisée par le fait qu'**il s'agit du chlorure de distéaryldiméthylammonium, du chlorure de cétyltriméthylammonium, du chlorure de béhényltriméthylammonium.

13. Composition selon l'une quelconque des revendications précédentes, **caractérisée par le fait que** le sel d'ammonium quaternaire (ii) de formule (V) est un sel de dialkyl(C₁-C₂)alkyl(C₁₂-C₂₂)hydroxyalkyl(C₁-C₂)ammonium.

14. Composition selon la revendication 13, **caractérisée par le fait qu'**il s'agit du chlorure d'oléocétylhydroxyéthylammonium.

15. Composition selon l'une quelconque des revendications précédentes, **caractérisée par le fait que** le sel d'ammonium quaternaire (ii) de formule (V) est le chlorure de stéaramidopropyldiméthyl (myristyl acétate) ammonium de formule:

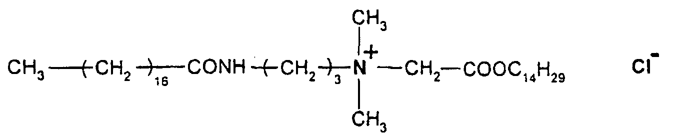

16. Composition selon l'une quelconque des revendications précédentes, **caractérisée par le fait que** le ou les sels d'ammonium quaternaire (ii) représentent de 0,01 à 10% en poids du poids total de la composition tinctoriale.

17. Composition selon la revendication 16, **caractérisée par le fait que** le ou les sels d'ammonium quaternaire représentent de 0,05 à 5% en poids du poids total de la composition tinctoriale.

18. Composition selon l'une quelconque des revendications précédentes, **caractérisée par le fait que** le milieu approprié pour la teinture (ou support) est constitué par de l'eau ou par un mélange d'eau et d'au moins un solvant organique.

19. Composition selon l'une quelconque des revendications précédentes, **caractérisée par le fait qu'**elle présente un pH compris entre 2 et 11, et de préférence entre 5 et 10.

20. Composition selon l'une quelconque des revendications précédentes, **caractérisée par le fait qu'**elle est destinée à la teinture d'oxydation et qu'elle contient une ou plusieurs bases d'oxydation choisie parmi les paraphénylènediamines, les bis-phénylalkylènediamines, les para-aminophénols, les ortho-aminophénols et les bases hétérocycliques.

21. Composition selon la revendication 20, **caractérisée par le fait que** la ou les bases d'oxydation représentent 0,0005 à 12 % en poids du poids total de la composition tinctoriale.

22. Composition selon la revendication 21, **caractérisée par le fait que** la ou les bases d'oxydation représentent 0,005 à 6 % en poids du poids total de la composition tinctoriale.

23. Composition selon l'une quelconque des revendications 20 à 22, **caractérisée par le fait qu'**elle renferme un ou plusieurs coupleurs choisis parmi les métaphénylènediamines, les méta-aminophénols, les métadiphénols et les coupleurs hétérocycliques.

24. Composition selon la revendication 23, **caractérisée par le fait que** le ou les coupleurs représentent de 0,0001 à 10 % en poids du poids total de la composition tinctoriale.

25. Composition selon la revendication 24, **caractérisée par le fait que** le ou les coupleurs représentent de 0,005 à 5 % en poids du poids total de la composition tinctoriale.

26. Composition selon l'une quelconque des revendications précédentes, **caractérisée par le fait qu'**elle est destinée à la teinture directe éclaircissante ou la teinture d'oxydation et qu'elle renferme alors au moins un agent oxydant.

27. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 26, pendant un temps suffisant pour développer la coloration désirée, après quoi on rince, on lave éventuellement au shampooing, on rince à nouveau et on sèche.

28. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 26, pendant un temps suffisant pour développer la coloration désirée, sans rinçage final.

29. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique (i) tel que défini dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le sel d'ammonium quaternaire (ii) tel que défini dans les revendications précédentes.

30. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique (i) tel que défini dans les revendications précédentes et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le sel d'ammonium quaternaire (ii) tel que défini dans les revendications précédentes.

31. Dispositif à plusieurs compartiments ou "kit" de teinture à plusieurs compartiments, **caractérisé par le fait qu'**un premier compartiment renferme la composition (A1) ou (A2) telle que définie à la revendication 29 ou 30 et un second compartiment renferme la composition (B1) ou (B2) telle que définie à la revendication 29 ou 30.

## Patentansprüche

1. Zusammensetzung zum Färben von Keratinfasern und insbesondere menschlichen Keratinfasern, wie dem Haar, die in einem zum Färben geeigneten Medium enthält: (i) mindestens einen kationischen Direktfarbstoff, der unter den Verbindungen der folgenden Formeln (I), (II), (III), (III') oder (IV) ausgewählt ist:
(a) Verbindungen der folgenden Formel (I): 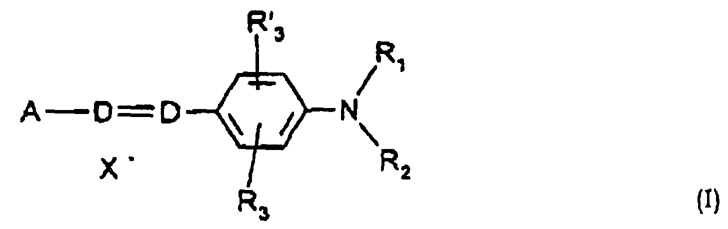 wobei in der Formel (I) bedeuten:
D ein Stickstoffatorn oder die Gruppe -CH,
R₁ und R₂, die gleich oder verschieden sind, ein Wasserstoffatom; eine C₁₋₄-Alkylgruppe, die mit einer Gruppe -CN, -OH oder -NH₂ substituiert sein kann oder mit einem Kohlenstoffatom des Benzolrings einen gegebenenfalls sauerstoff- oder stickstoffhaltigen Heterocyclus bilden kann, der mit einer oder mehreren C₁₋₄-Alkylgruppen substituiert sein kann; oder eine 4'-Aminophenylgruppe,
R₃ und R'₃, die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder Acetyloxy,
X ein Anion, das vorzugsweise unter Chlorid, Methylsulfat und Acetat ausgewählt ist,
A eine Gruppe, die unter den folgenden Strukturen A1 bis A18 ausgewählt ist: 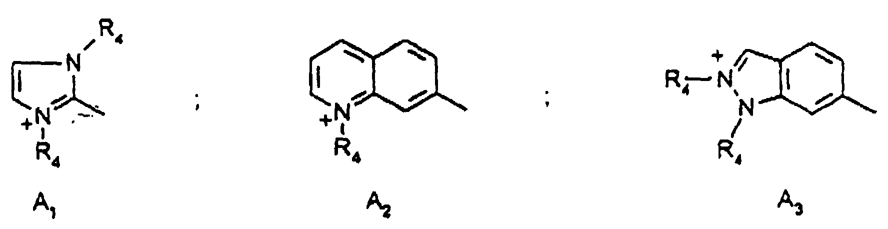 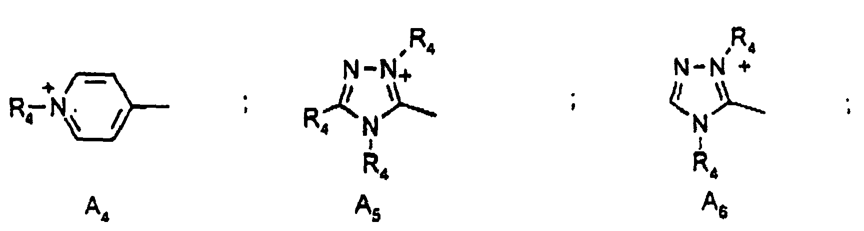 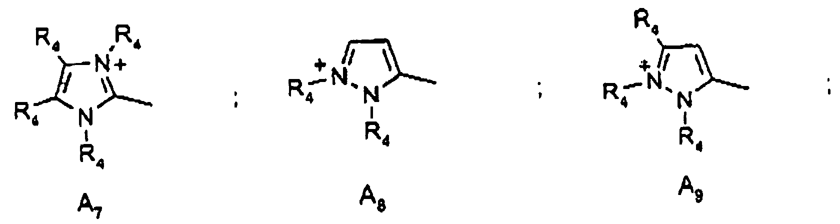 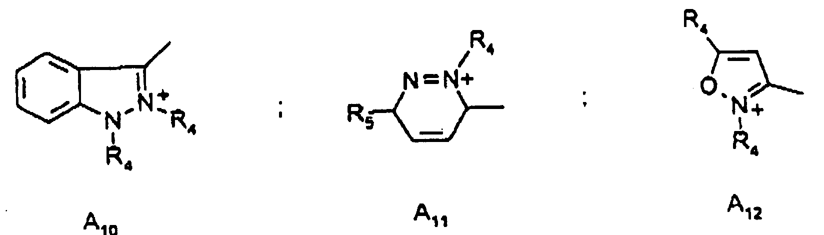 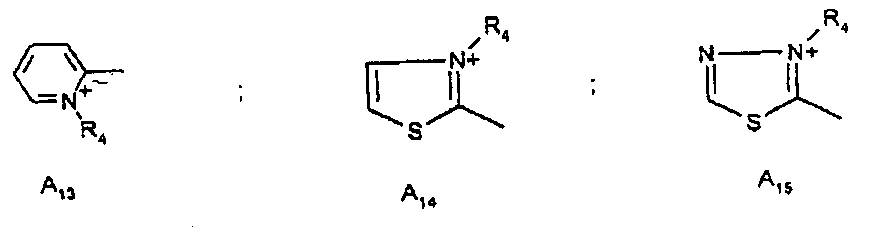 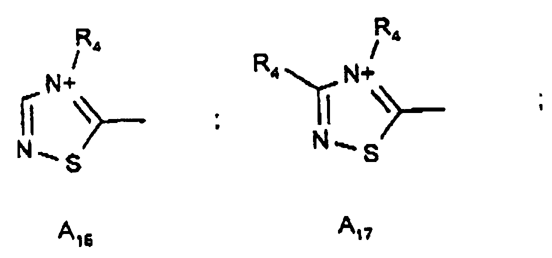 und 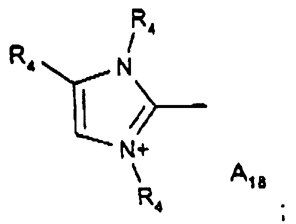 worin die Gruppe R₄ eine C₁₋₄-Alkylgruppe bedeutet, die mit einer Hydroxygruppe substituiert sein kann, und R₅ eine C₁₋₄-Alkoxygruppe ist, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist;
(b) Verbindungen der folgenden Formel (II): 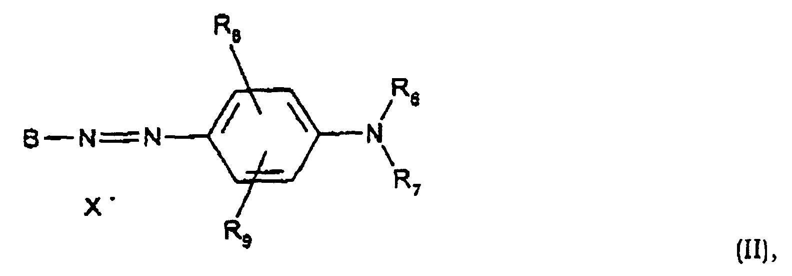 wobei in der Formel (II) bedeuten:
R₆ ein Wasserstoffatom oder eine C₁₋₄-Alkylgruppe,
R₇ ein Wasserstoffatom, eine Alkylgruppe, die mit der Gruppe -CN oder einer Aminogruppe substituiert sein kann, oder 4'-Aminophenyl oder R₇ bildet mit R₆ einen gegebenenfalls sauerstoffhaltigen und/oder stickstoffhaltigen Heterocyclus, der mit einer C₁₋₄-Alkylgruppe substituiert sein kann,
R₈ und R₉, die gleich oder verschieden sind, ein Wasserstoffatom, ein Halogenatom, wie Brom, Chlor, Iod oder Fluor, eine C₁₋₄-Alkylgruppe, eine C₁₋₄-Alkoxygruppe oder die Gruppe -CN,
X ein Anion, das vorzugsweise unter Chlorid, Methylsulfat und Acetat ausgewählt ist,
B eine Gruppe, die unter den folgenden Strukturen B1 bis B6 ausgewählt ist: 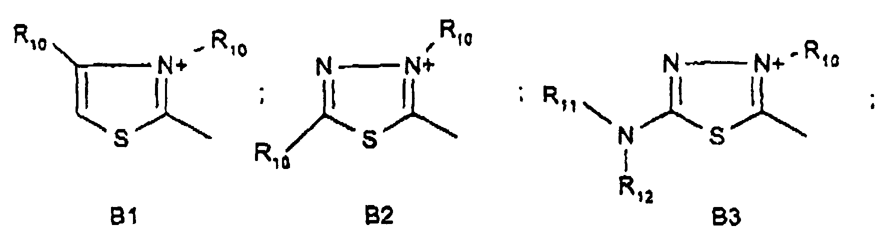 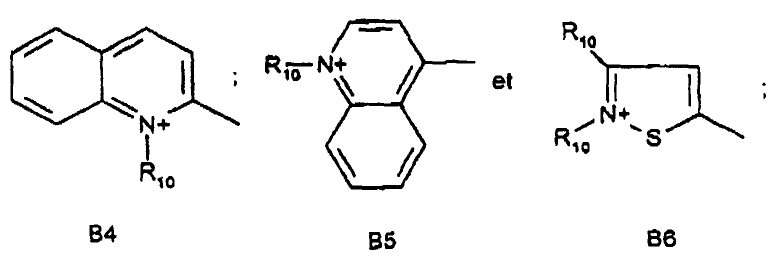 worin die Gruppe R₁₀ eine C₁₋₄-Alkylgruppe bedeutet und die Gruppen R₁₁ und R₁₂, die gleich oder verschieden sind, Wasserstoff oder C₁₋₄-Alkyl bedeuten;
(c) Verbindungen der folgenden Formel (III) und (III'): 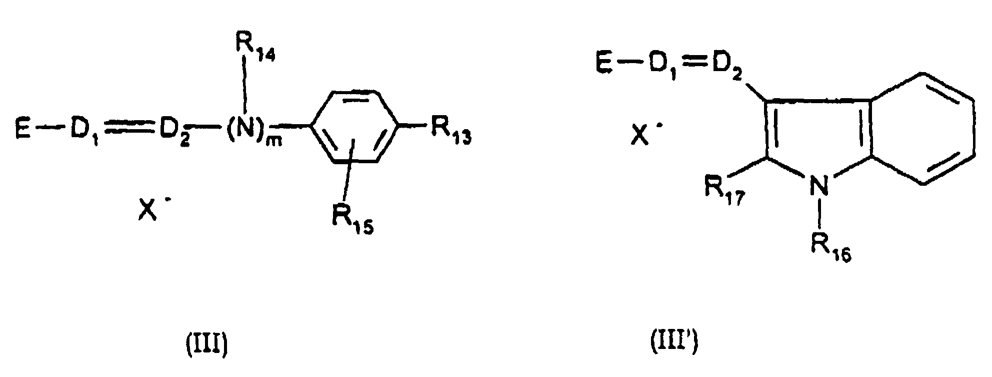 wobei in den Formeln (III) und (III') bedeuten:
R₁₃ ein Wasserstoffatom, eine C₁₋₄-Alkoxygruppe, ein Halogenatom, wie Brom, Chlor, Iod oder Fluor, oder eine Aminogruppe,
R₁₄ ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig und/ oder mit einer oder mehreren C₁₋₄-Alkylgruppen substituiert ist;
R₁₅ ein Wasserstoffatom oder ein Halogenatom, wie Brom, Chlor, Iod oder Fluor,
R₁₆ und R₁₇, die identisch oder voneinander verschieden sind, Wasserstoff oder C₁₋₄-Alkyl,
D₁ und D₂, die gleich oder verschieden sind, ein Stickstoffatom oder die Gruppe -CH,
m = 0 oder 1,
mit der Maßgabe, dass die Gruppen D₁ und D₂ gleichzeitig -CH bedeuten und m = 0, wenn R₁₃ eine unsubstituierte Aminogruppe ist,
X⁻ ein Anion, das vorzugsweise unter Chlorid, Methylsulfat und Acetat ausgewählt ist,
E eine Gruppe, die unter den folgenden Strukturen E1 bis E8 ausgewählt ist: 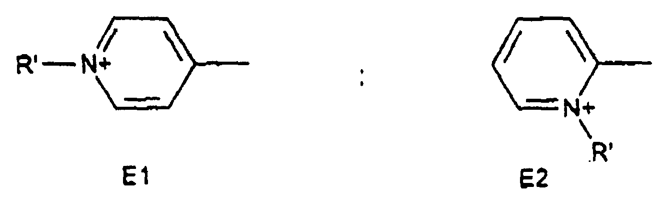 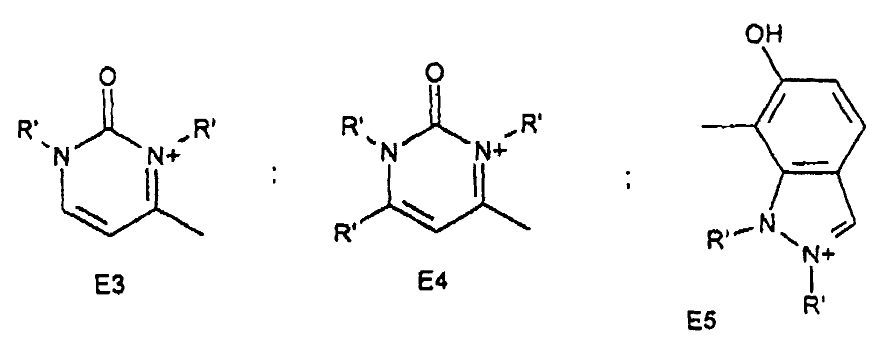 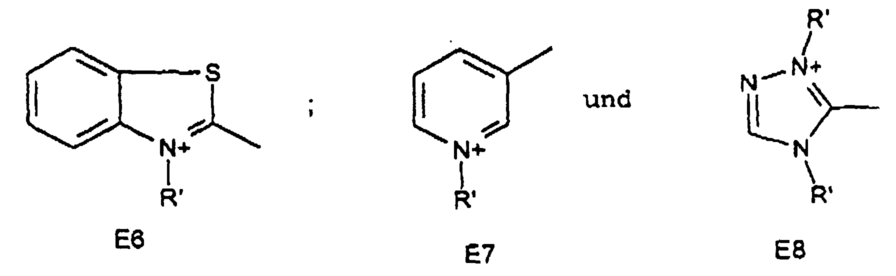 wobei R' eine C₁₋₄-Alkylgruppe bedeutet;
wenn m = 0 und D₁ ein Stickstoffatom bedeutet, kann die Gruppe E auch eine Gruppe der folgenden Struktur E9 sein: 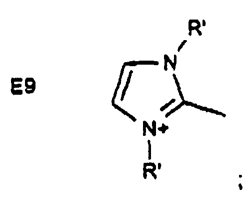 wobei R' eine C₁₋₄-Alkylgruppe bedeutet;
d) Verbindungen der folgenden Formel (IV):
G―N=N―J (IV),
worin bedeuten:
das Symbol G eine Gruppe, die unter den folgenden Strukturen G₁ bis G₃ ausgewählt ist: 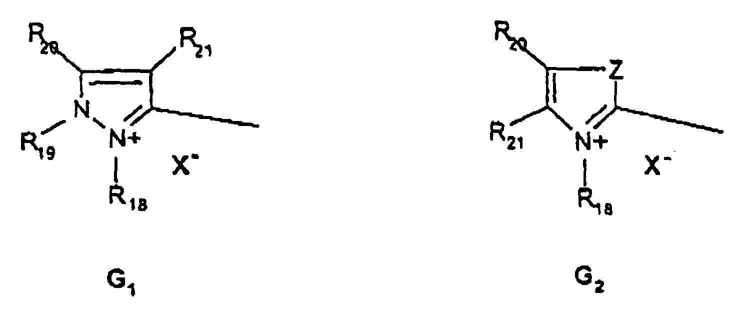 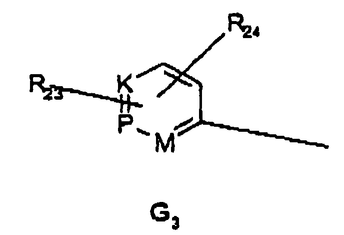 wobei in den Strukturen G₁ bis G₃ bedeuten:
R₁₈ C₁₋₄-Alkyl oder eine Phenylgruppe, die mit einer C₁₋₄-Alkylgruppe oder einem Halogenatorn substituiert sein kann, das unter Chlor, Brom, Iod und Fluor ausgewählt ist;
R₁₉ C₁₋₄-Alkyl oder Phenyl;
R₂₀ und R₂₁, die gleich oder verschieden sind, C₁₋₄-Alkyl, Phenyl oder R₂₀ und R₂₁ bilden in G₁ gemeinsam einen Benzolring, der mit einer oder mehreren Gruppen C₁₋₄-Alkyl, C₁₋₄-Alkoxy oder NO₂ substituiert ist, oder bilden in G₂ gemeinsam einen Benzolring, der gegebenenfalls mit einer oder mehreren Gruppen C₁₋₄-Alkyl, C₁₋₄-Alkoxy oder NO₂ substituiert ist;
wobei R₂₀ außerdern ein Wasserstoffatom bedeuten kann;
Z ein Sauerstoffatom, ein Schwefelatom oder eine Gruppe -NR₁₉;
M -CH, -CR (wobei R eine C₁₋₄-Alkylgruppe ist), oder -NR₂₂(X⁻)ᵣ;
K -CH, -CR (wobei R eine C₁₋₄-Alkylgruppe bedeutet) oder -NR₂₂(X⁻)ᵣ;
P eine Gruppe -CH, -CR (wobei R eine C₁₋₄-Alkylgruppe bedeutet) oder -NR₂₂(X⁻)ᵣ; wobei r Null oder 1 ist;
R₂₂ ein Atom O⁻, C₁₋₄-Alkoxy oder C₁₋₄-Alkyl;
R₂₃ und R₂₄, die gleich oder verschieden sind, ein Wasserstoffatom, ein Halogenatom, das unter Chlor, Brom, Iod und Fluor ausgewählt ist, eine C₁₋₄-Alkylgruppe, eine C₁₋₄-Alkoxygruppe oder die Gruppe -NO₂;
X⁻ ein Anion, das vorzugsweise unter Chlorid, Iodid, Methylsulfat, Ethylsulfat, Acetat und Perchlorat ausgewählt ist;
mit der Maßgabe, dass
wenn R₂₂ O⁻ bedeutet, r Null ist;
wenn K oder P oder M -N(C₁₋₄)alkyl X⁻ bedeutet, R₂₃ oder R₂₄ von Wasserstoff verschieden ist;
wenn K -NR₂₂(X⁻)ᵣ bedeutet, M = P = -CH, -CR;
wenn M -NR₂₂(X⁻)ᵣ bedeutet, K = P = -CH, -CR;
wenn P -NR₂₂(X⁻)ᵣ bedeutet, K = M = -CH oder -CR bedeutet; wenn Z ein Schwefelatom und R₂₁ C₁₋₄-Alkyl ist, R₂₀ von Wasserstoff verschieden ist;
wenn Z -NR₁₉ und R₁₉ C₁₋₄-Alkyl bedeutet, mindestens eine der Gruppen R₁₈, R₂₀ oder R₂₁ der Struktur G₂ von einer C₁₋₄-Alkylgruppe verschieden ist;
das Symbol J:
- (a) eine Gruppe der folgenden Struktur J₁: 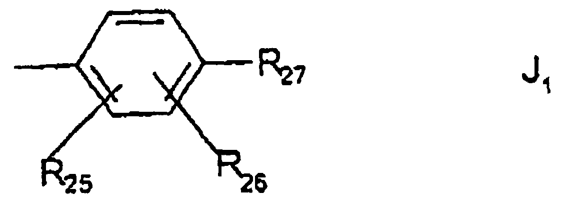 wobei in der Struktur J₁ bedeuten:
R₂₅ ein Wasserstoffatom, ein Halogenatom, das unter Chlor, Brom, Iod und Fluor ausgewählt ist, C₁₋₄-Alkyl, C₁₋₄-Alkoxy, -OH, -NO₂, -NHR₂₈, -NR₂₉R₃₀, -NHCOalkyl (C₁₋₄) oder R₂₅ bildet mit R₂₆ einen 5- oder 6-gliedrigen Ring, der gegebenenfalls ein oder mehrere Heteroatome enthält, die unter Stickstoff, Sauerstoff oder Schwefel ausgewählt sind;
R₂₆ ein Wasserstoffatom, ein Halogenatom, das unter Chlor, Brom, Iod und Fluor ausgewählt ist, C₁₋₄-Alkyl, C₁₋₄-Alkoxy oder R₂₆ bildet mit R₂₇ oder R₂₈ einen 5- oder 6-gliedrigen Ring, der gegebenenfalls ein oder mehrere Heteroatome enthält, die unter Stickstoff, Sauerstoff oder Schwefel ausgewählt sind;
R₂₇ Wasserstoff, -OH, -NHR₂₈ oder -NR₂₉R₃₀;
R₂₈ Wasserstoff, C₁₋₄-Alkyl, C₁₋₄-Monohydroxyalkyl, C₂₋₄-Polyhydroxyalkyl oder Phenyl;
R₂₉ und R₃₀, die gleich oder verschieden sind, C₁₋₄-Alkyl, C₁₋₄-Monohydroxyalkyl oder C₂₋₄-Polyhydroxyalkyl;
- (b) eine stickstoffhaltige, 5- oder 6-gliedrige heterocyclische Gruppe, die weitere Heteroatome und/oder carbonylhaltige Gruppen enthalten und mit einer oder mehreren Gruppen C₁₋₄-Alkyl, Amino oder Phenyl substituiert sein kann, insbesondere eine Gruppe der folgenden Struktur J₂: 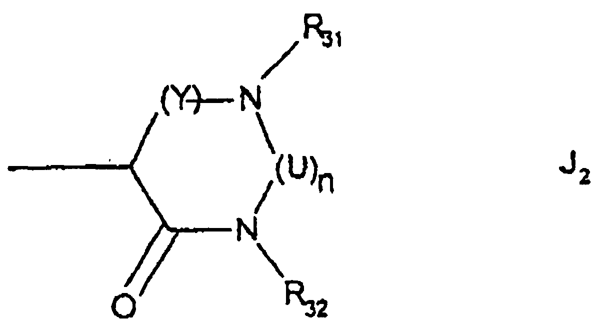 wobei in der Struktur J₂ bedeuten:
R₃₁ und R₃₂, die gleich oder verschieden sind, Wasserstoff, C₁₋₄-Alkyl oder Phenyl;
Y die Gruppe -CO- oder die Gruppe 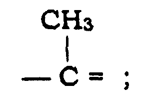
n = 0 oder 1, wobei U die Gruppe -CO- bedeutet, wenn n 1 ist,
wobei die Zusammensetzung **dadurch gekennzeichnet ist, dass** sie ferner enthält:
(ii) ein quartäres Ammoniumsalz, das unter den folgenden Verbindungen ausgewählt ist:
(ii)₁ Salzen der folgenden allgemeinen Formel (V): 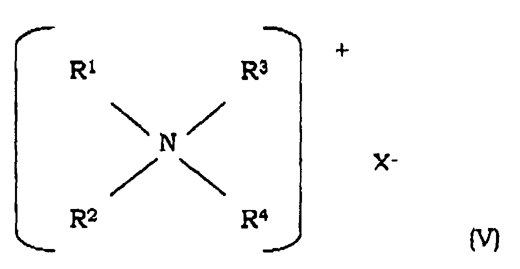 worin die Gruppen R¹ bis R⁴, die identisch oder voneinander verschieden sein können, eine geradkettige oder verzweigte, gesättigte oder ungesättigte aliphatische Gruppe auf Kohlenwasserstoffbasis mit etwa 1 bis 30 Kohlenstoffatomen oder Alkoxy, Alkoxycarbonylalkyl, Polyoxyalkylen, Alkylamido, Alkylamidoalkyl, Hydroxyalkyl, eine aromatische Gruppe, Aryl, Alkylaryl mit etwa 12 bis etwa 30 Kohlenstoffatornen bedeuten, wobei mindestens eine der Gruppen R¹, R², R³ oder R⁴ eine Gruppe mit 8 bis 30 Kohlenstoffatomen bedeutet;
X⁻ ist ein Anion, das unter den Halogeniden, Phosphaten, Acetaten, Lactaten und Alkylsulfaten ausgewählt ist;
(ii)₂ - Imidazoliumsalzen der folgenden Formel (VI): 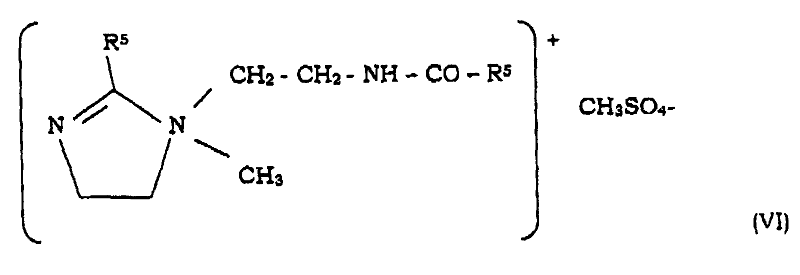 worin die Gruppe R⁵ eine Alkenyl- und/oder Alkylgruppe mit 13 bis 31 Kohlenstoffatomen bedeutet, die von Talgfettsäuren abgeleitet sind;
(ii)₃ - Quartären Diammoniumsalzen der folgenden Formel (VIIs): 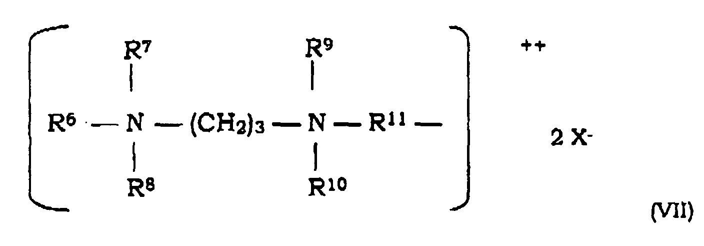 worin die Gruppe R⁶ eine aliphatische Gruppe mit etwa 16 bis 30 Kohlenstoffatomen bedeutet; R⁷, R⁸, R⁹, R¹⁰ und R¹¹ sind unter Wasserstoff oder einer Alkylgruppe mit 1 bis 4 Kohlenstoffatomen ausgewählt; und X⁻ ist ein Anion, das unter den Halogeniden, Acetaten, Phosphaten und Sulfaten ausgewählt ist.

2. Zusammensetzung nach Anspruch 1, **dadurch gekennzeichnet, dass** die kationischen Direktfarbstoffe der Formel (I) unter den Verbindungen der folgenden Strukturen (I1) bis (I54) ausgewählt sind:

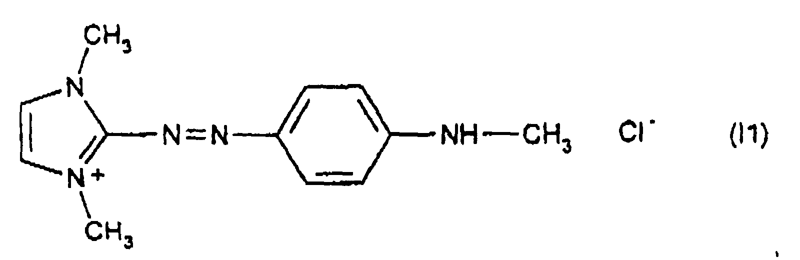

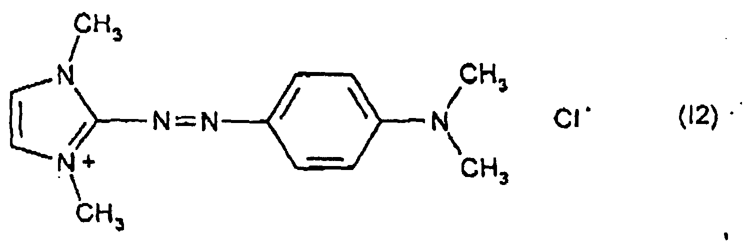

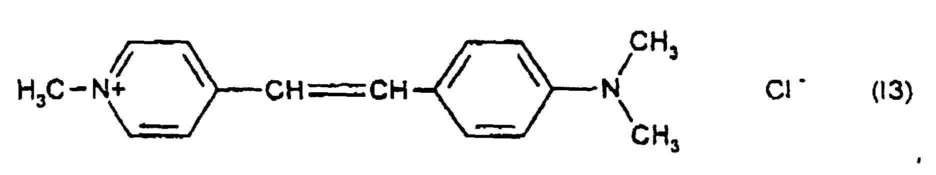

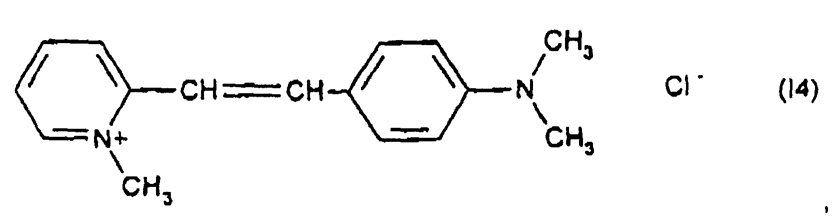

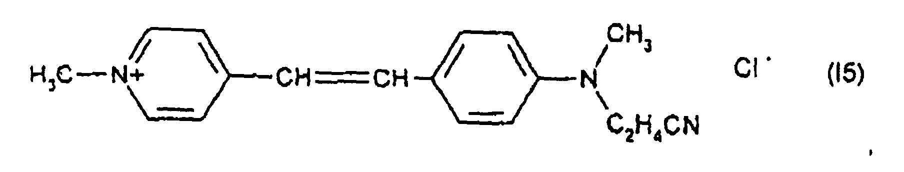

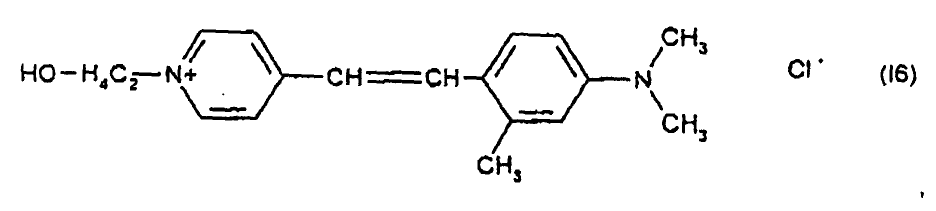

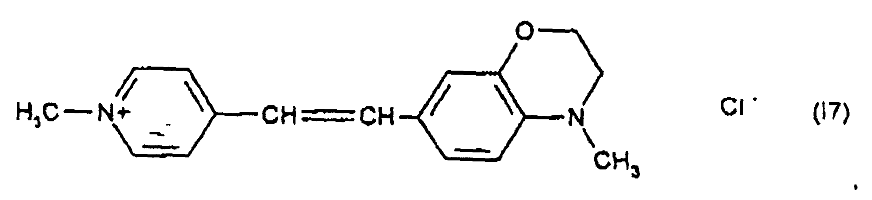

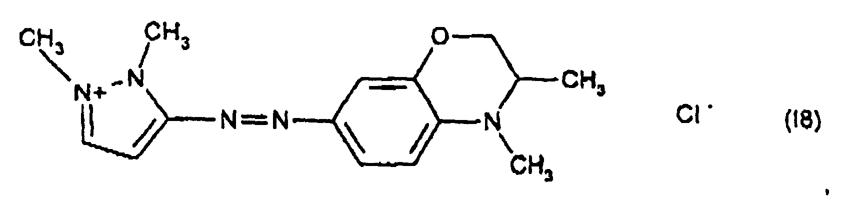

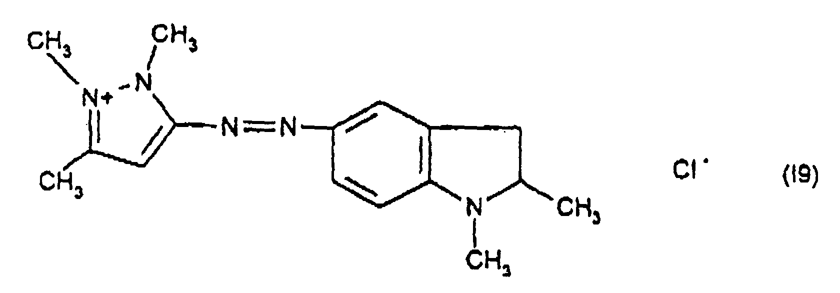

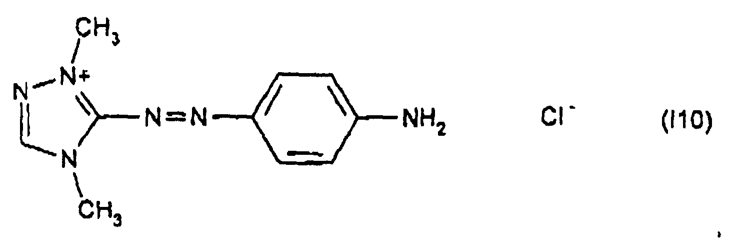

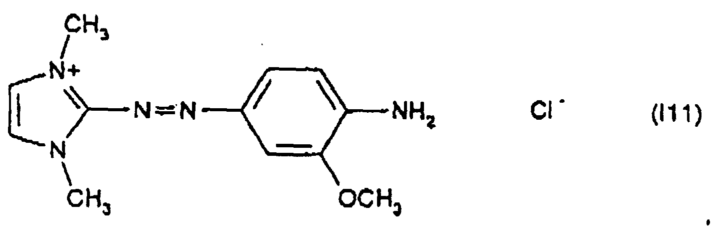

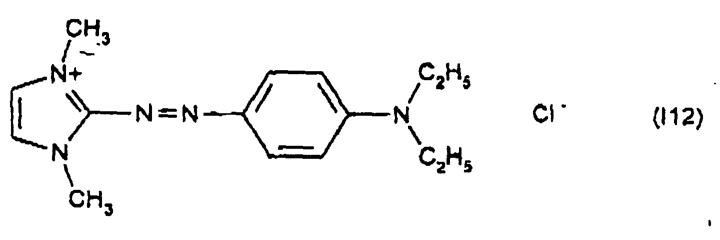

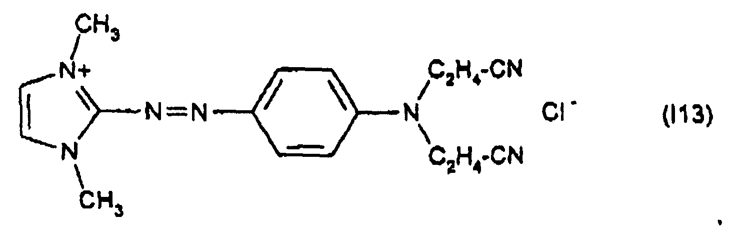

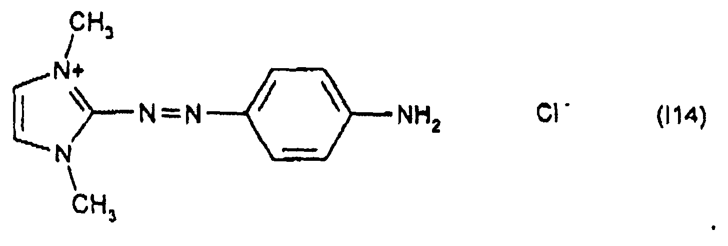

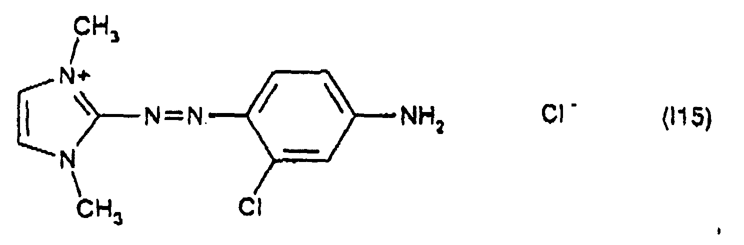

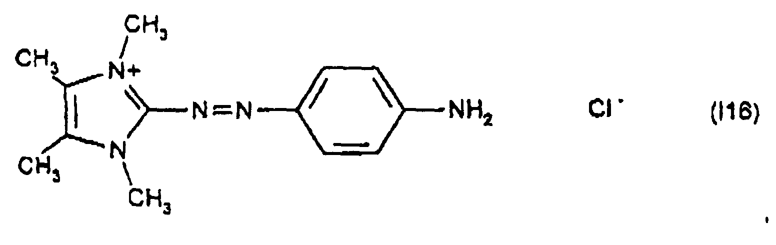

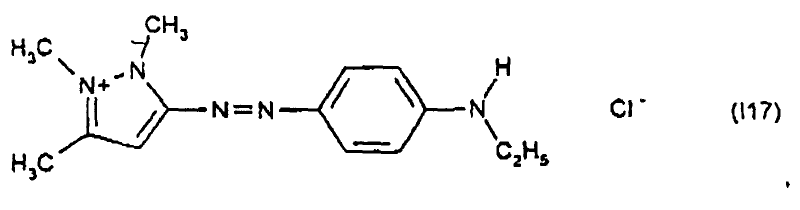

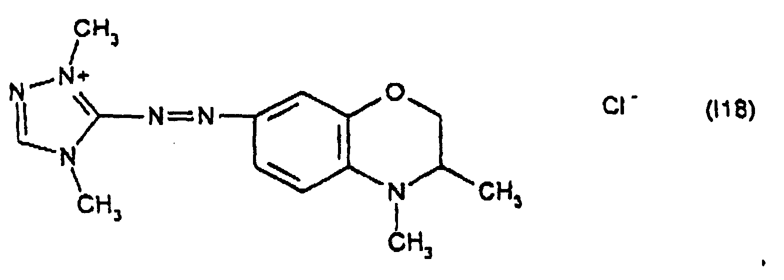

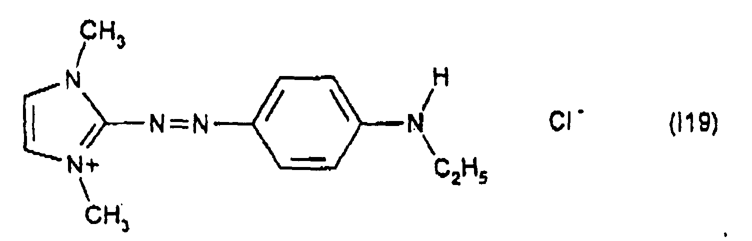

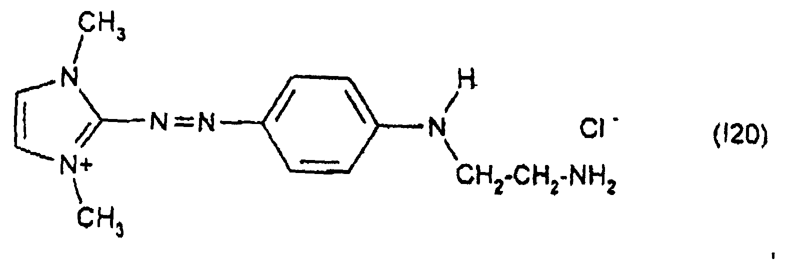

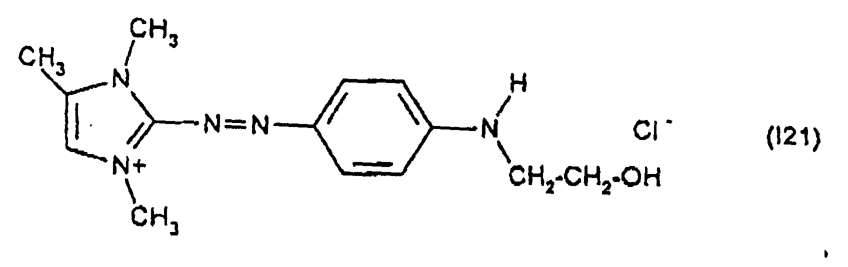

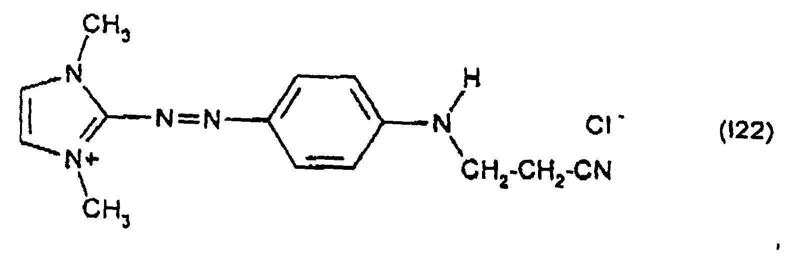

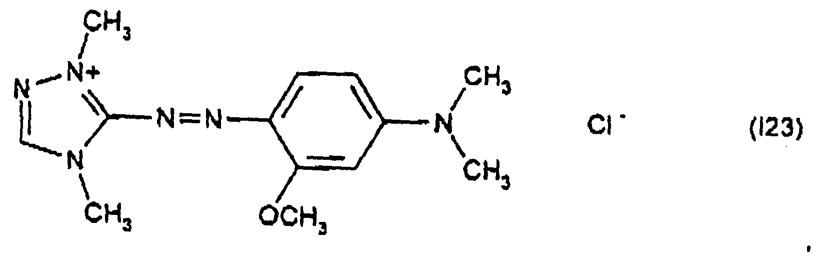

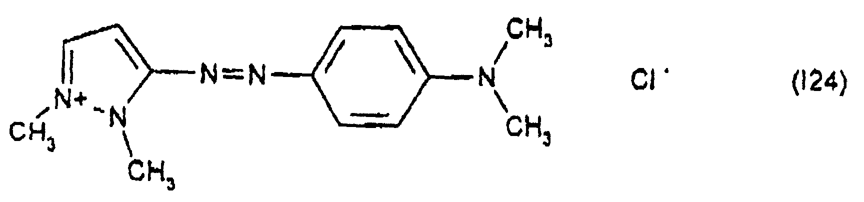

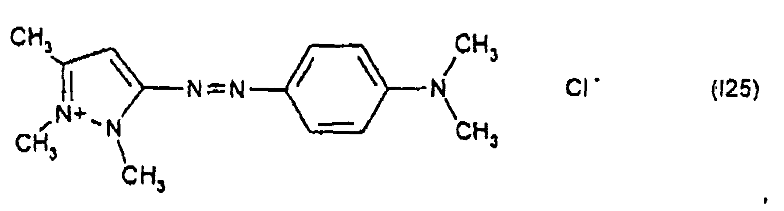

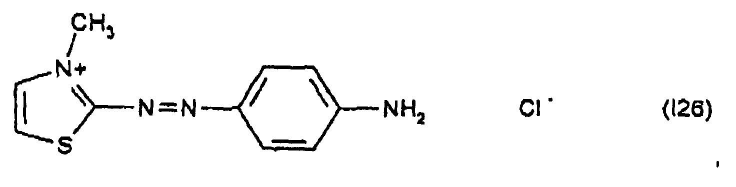

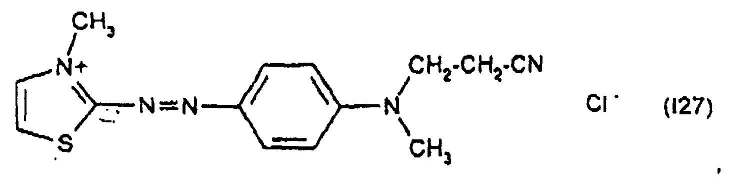

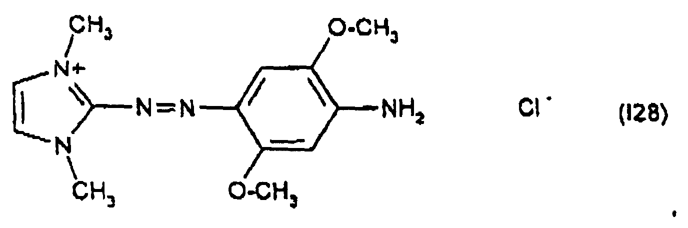

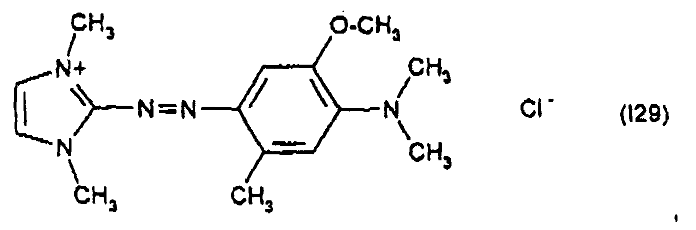

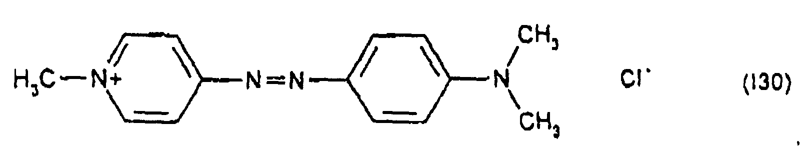

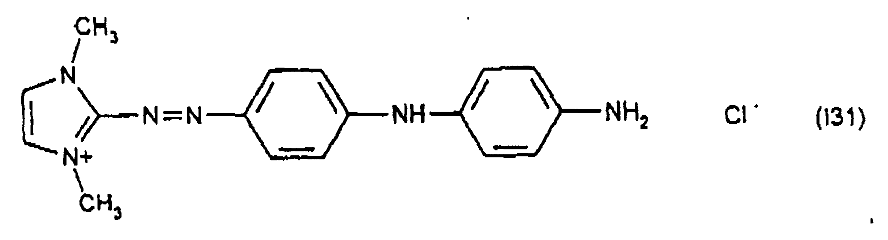

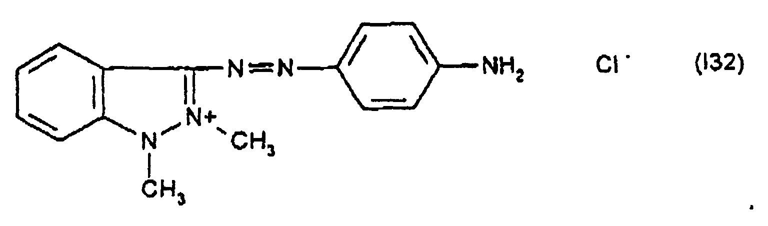

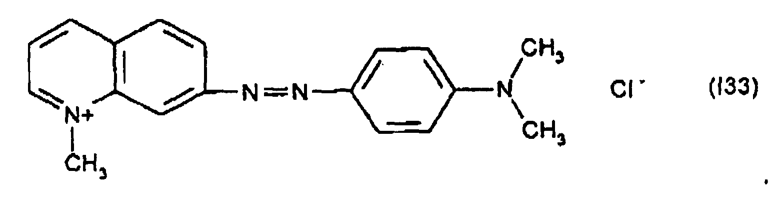

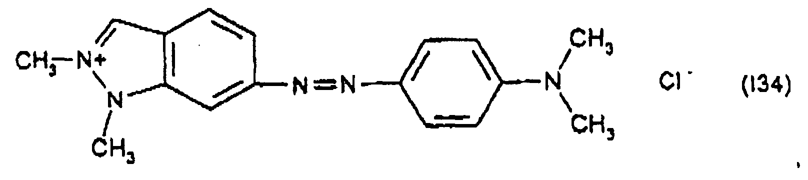

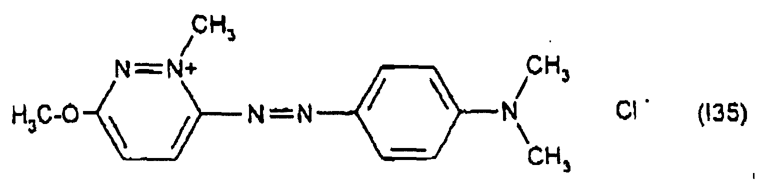

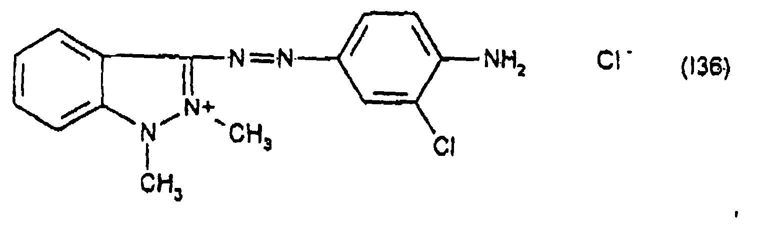

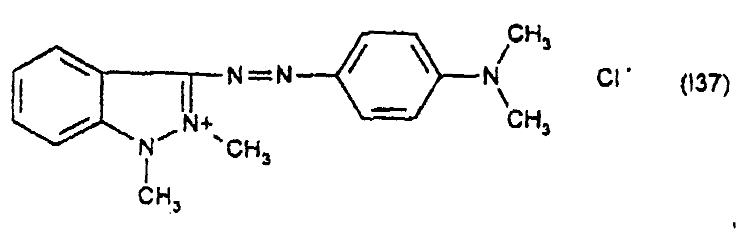

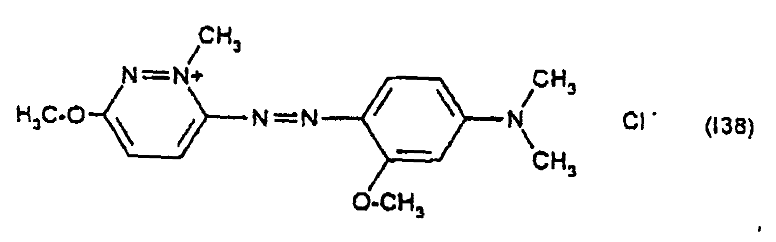

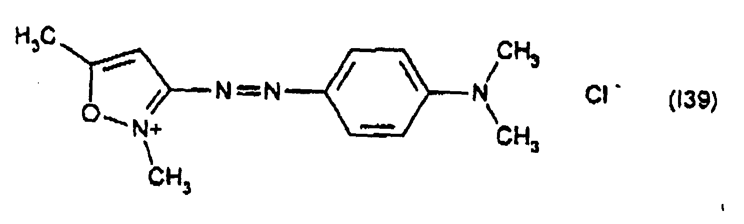

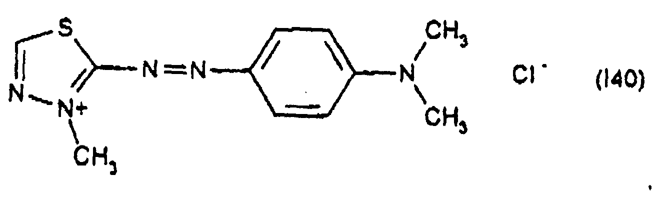

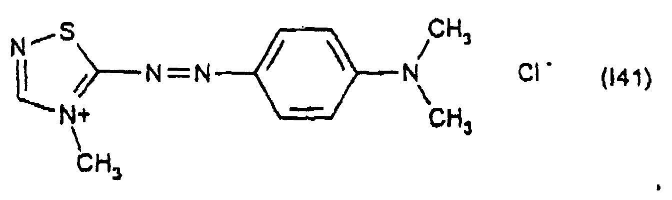

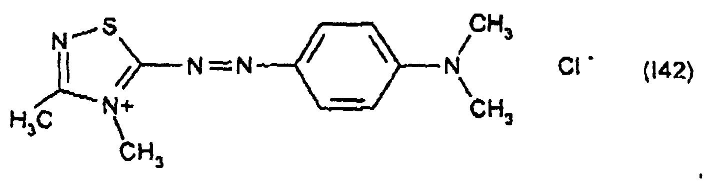

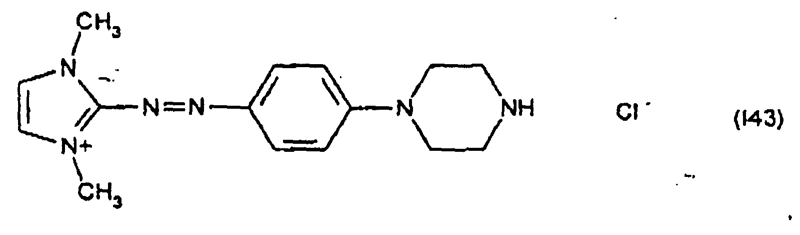

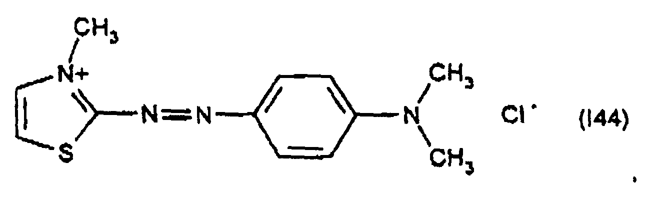

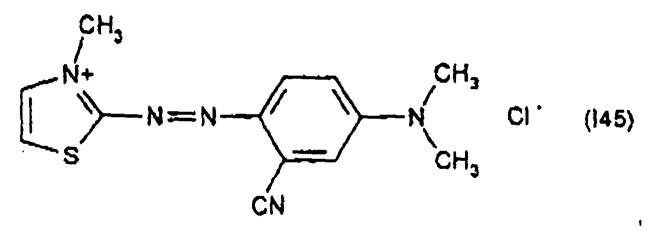

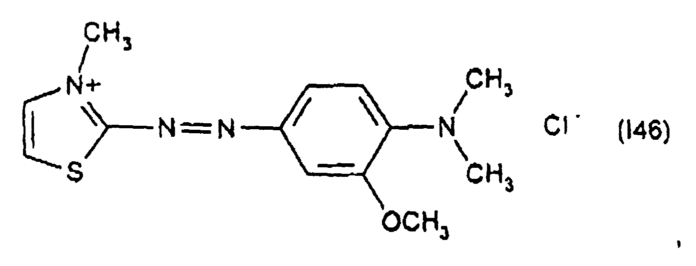

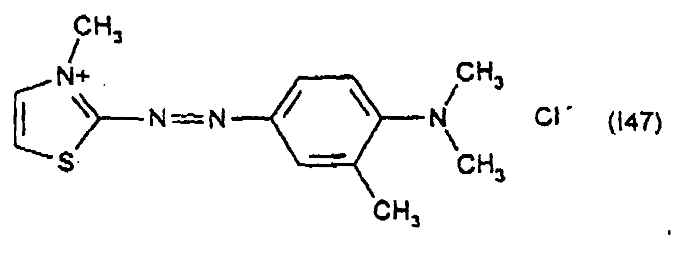

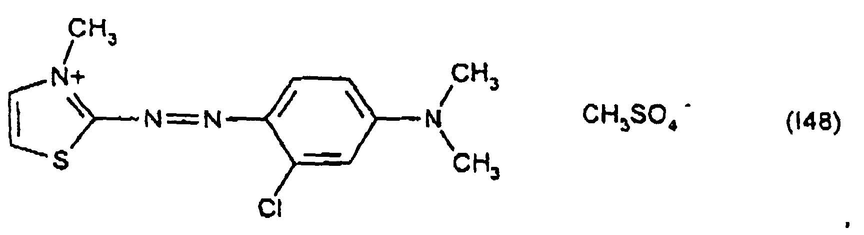

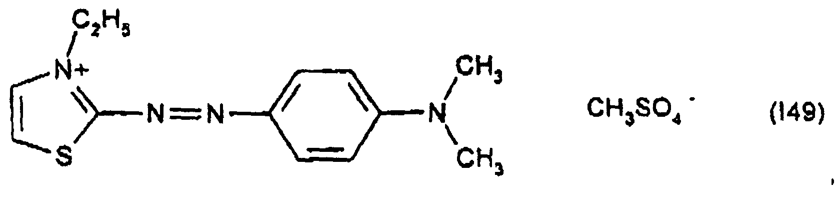

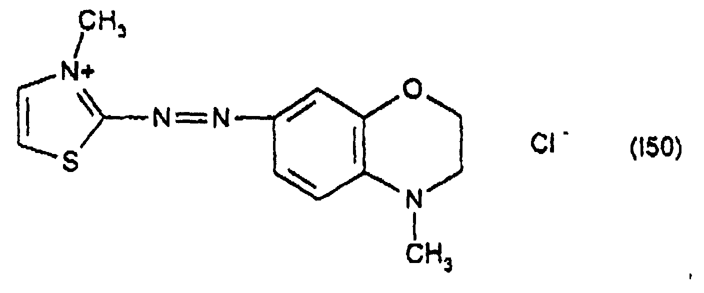

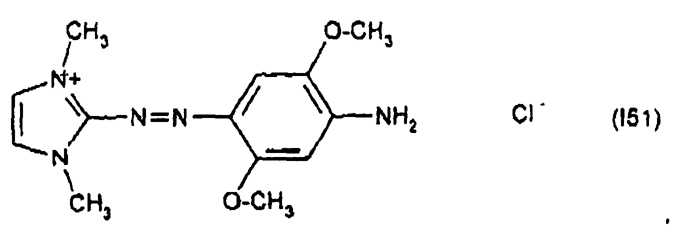

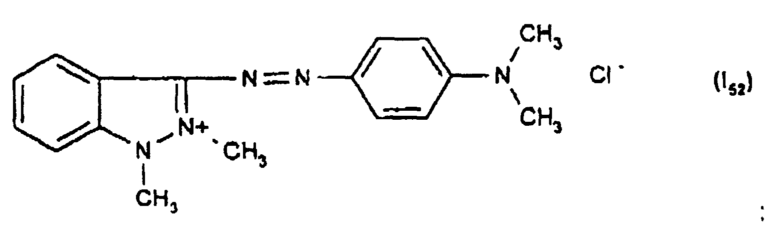

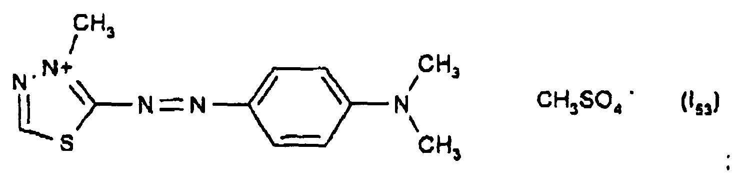

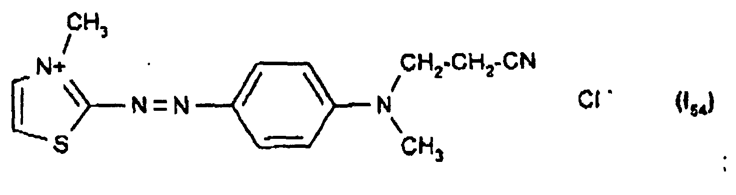

3. Zusammensetzung nach Anspruch 2, **dadurch gekennzeichnet, dass** die kationischen Direktfarbstoffe den Strukturen (I1), (I2), (I14) und (I31) entsprechen.

4. Zusammensetzung nach Anspruch 1, **dadurch gekennzeichnet, dass** die kationischen Direktfarbstoffe der Formel (II) unter den Verbindungen der folgenden Strukturen (II1) bis (II19) ausgewählt sind: 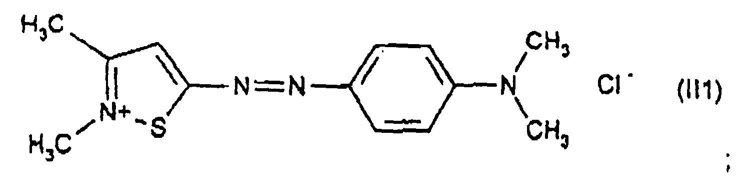 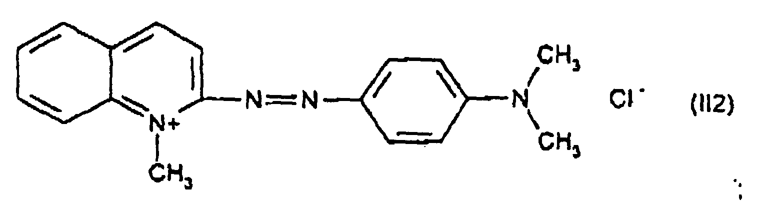 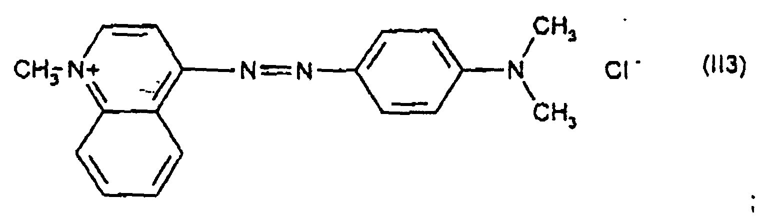 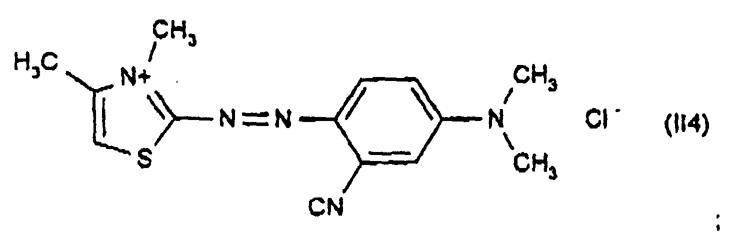 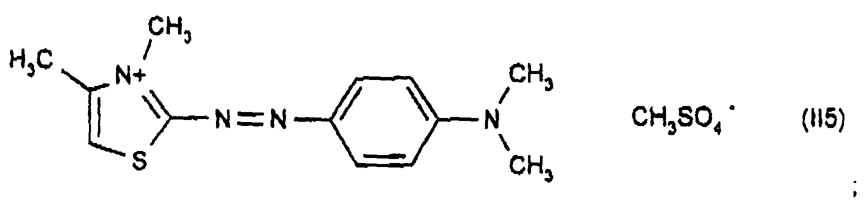 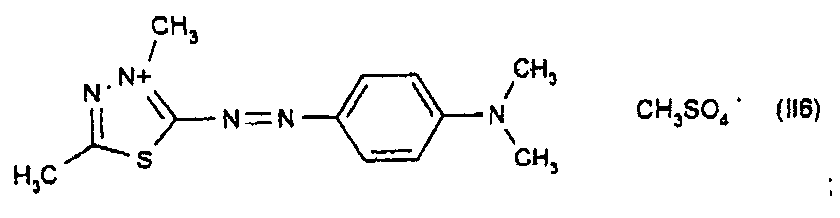 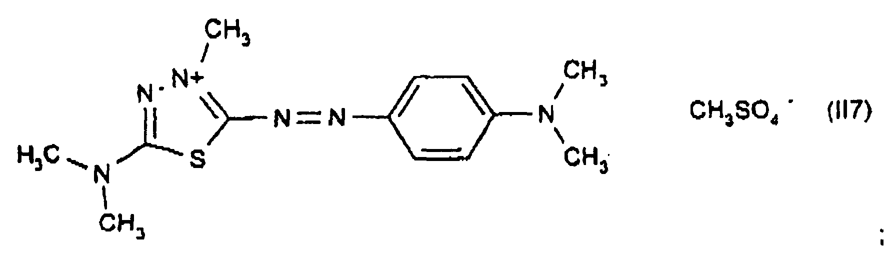 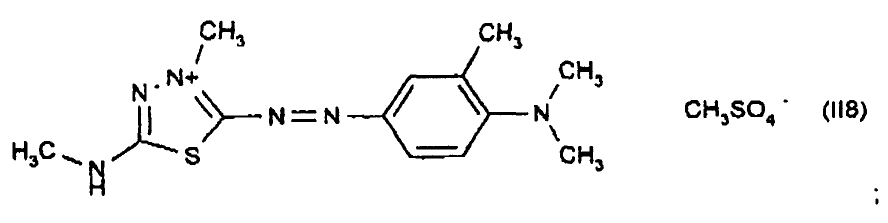 und

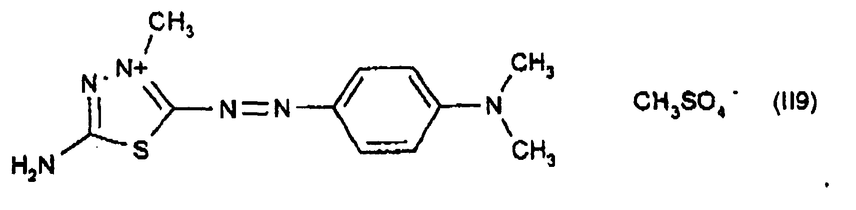

5. Zusammensetzung nach Anspruch 1, **dadurch gekennzeichnet, dass** die kationischen Direktfarbstoffe der Formel (III) unter den Verbindungen der folgenden Strukturen (III1) bis (III18) ausgewählt sind: 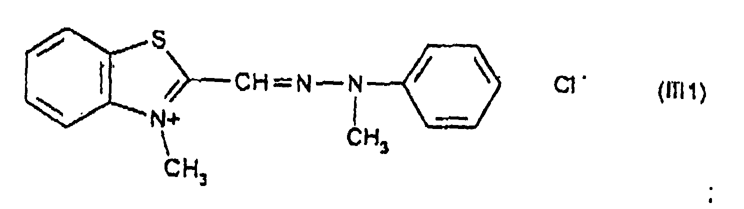 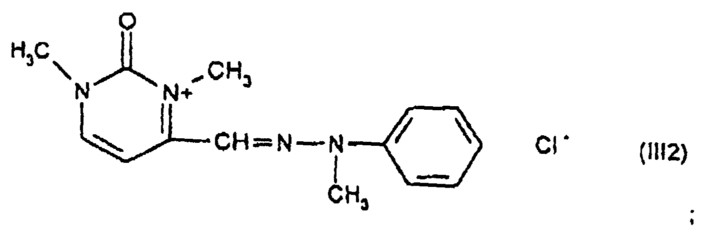 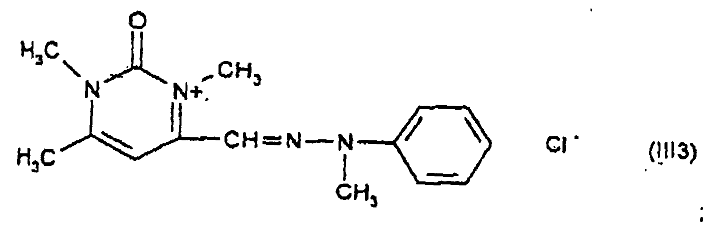 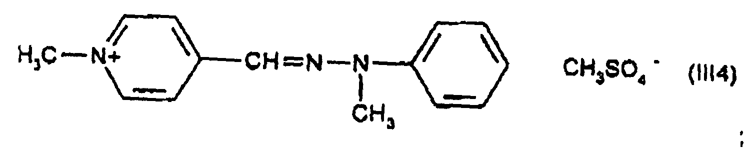 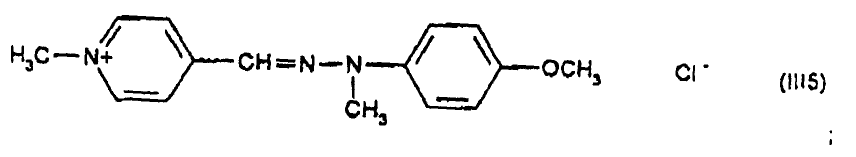 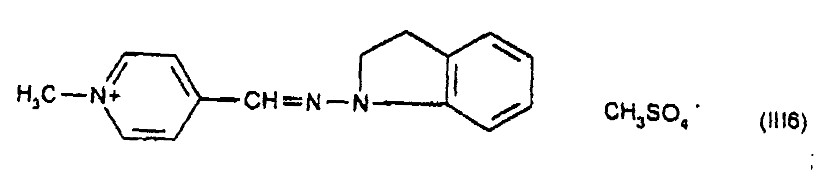 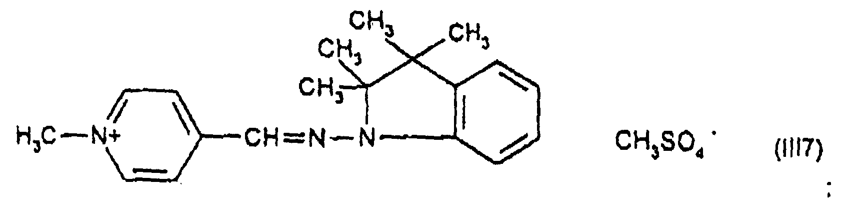 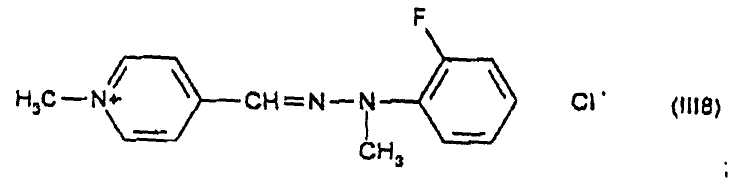 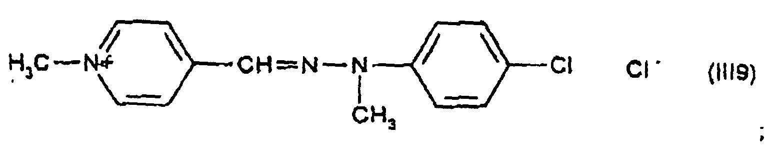 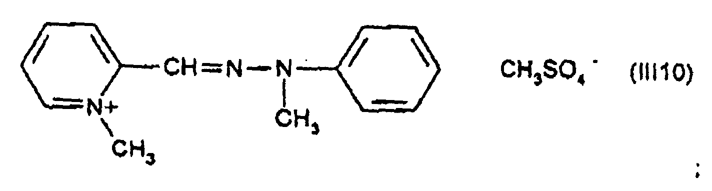 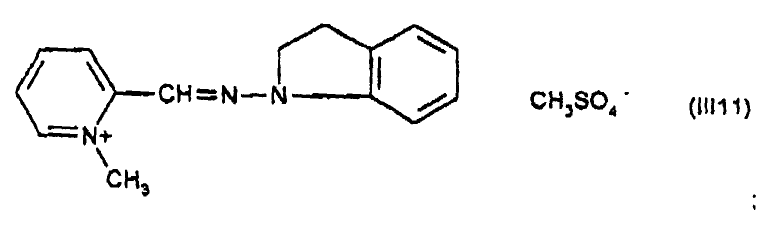 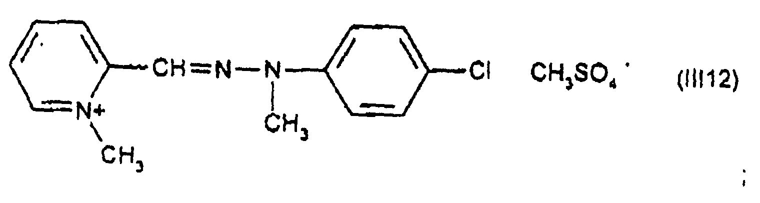 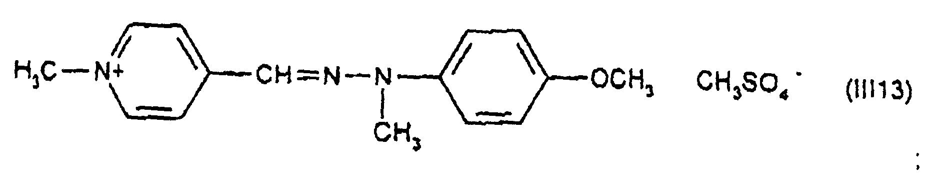 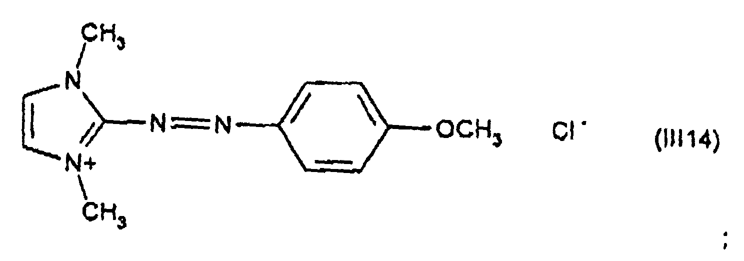 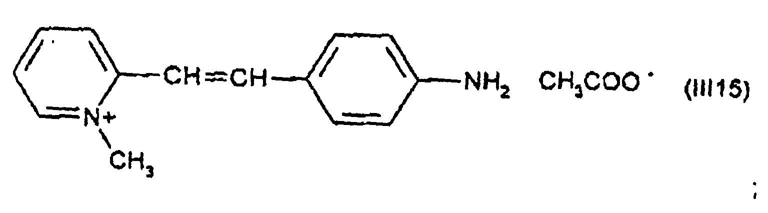 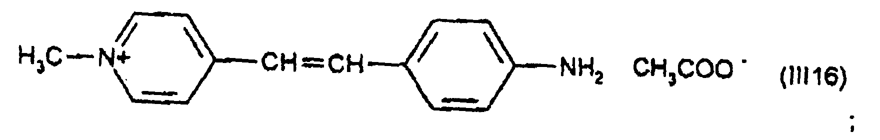 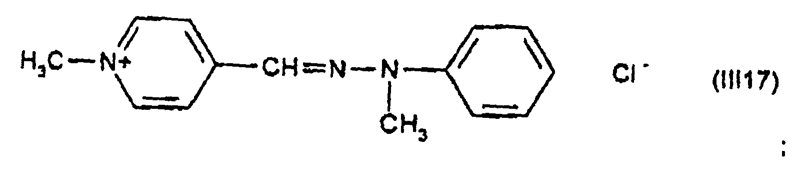 und

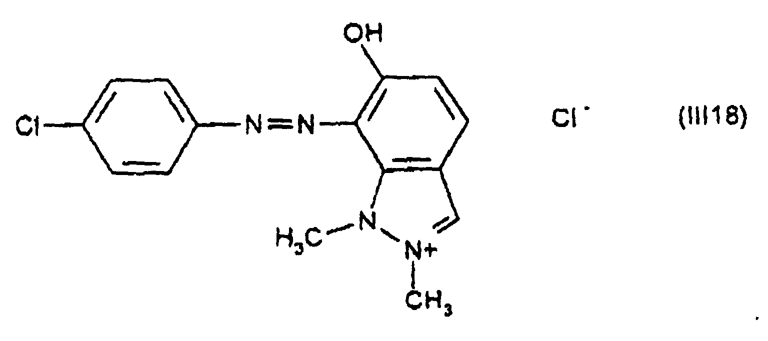

6. Zusammensetzung nach Anspruch 5, **dadurch gekennzeichnet, dass** die kationischen Direktfarbstoffe der Formel (III) den Strukturen (III4), (III5) und (III13) entsprechen.

7. Zusammensetzung nach Anspruch 1, **dadurch gekennzeichnet, dass** die kationischen Direktfarbstoffe der Formel (III') unter den Verbindungen der folgenden Strukturen (III'1) bis (III'3) ausgewählt sind: 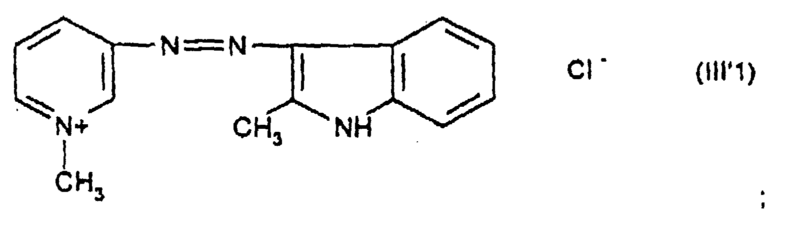 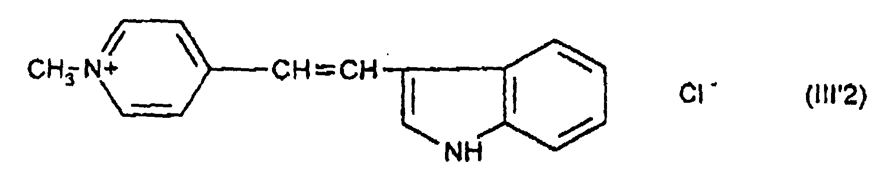 und

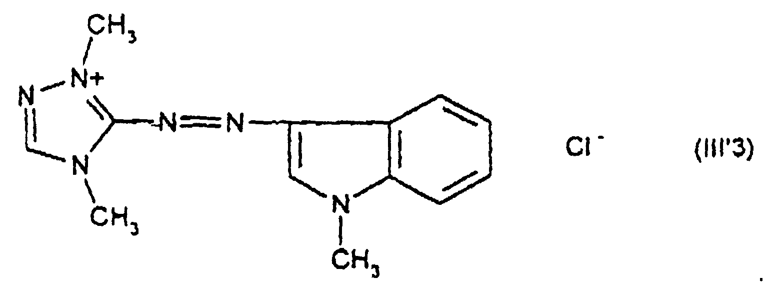

8. Zusammensetzung Anspruch 1, **dadurch gekennzeichnet, dass** die kationischen Direktfarbstoffe der Formel (IV) unter den Verbindungen der folgenden Strukturen (IV₁) bis (IV₇₇) ausgewählt sind:

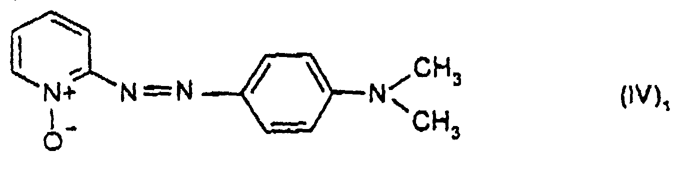

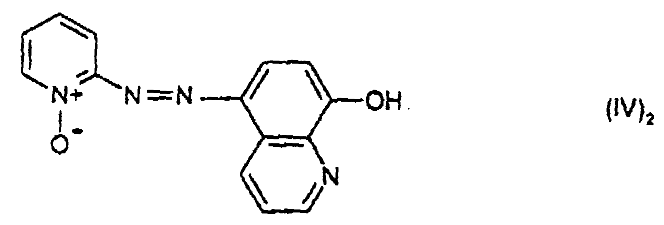

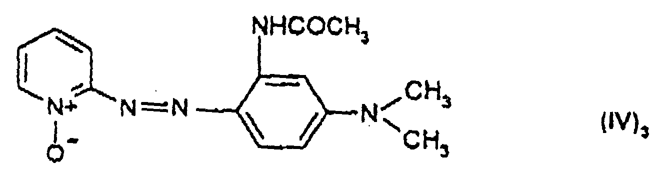

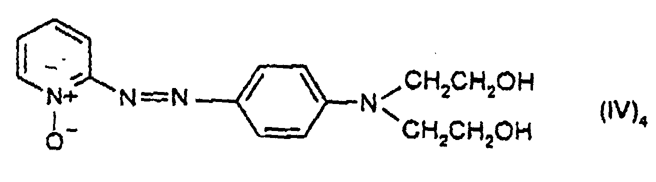

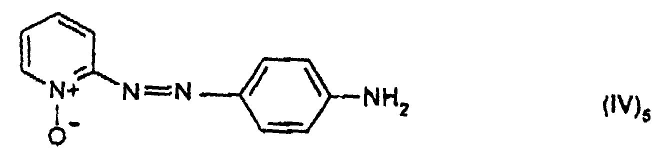

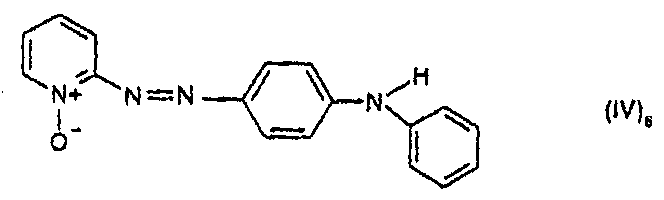

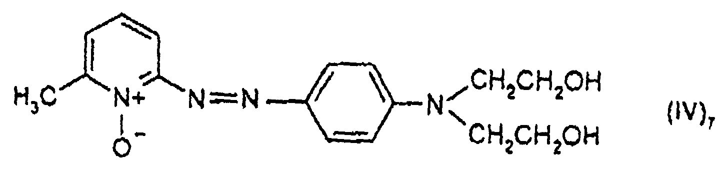

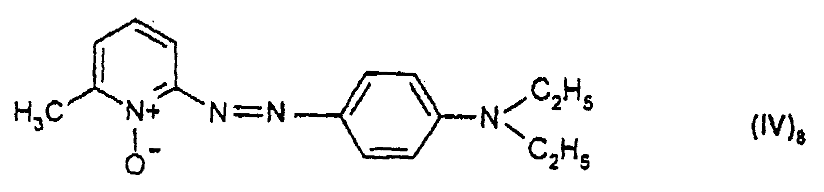

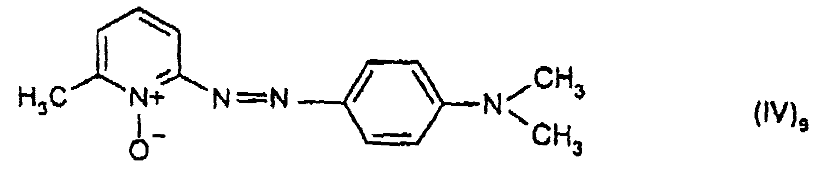

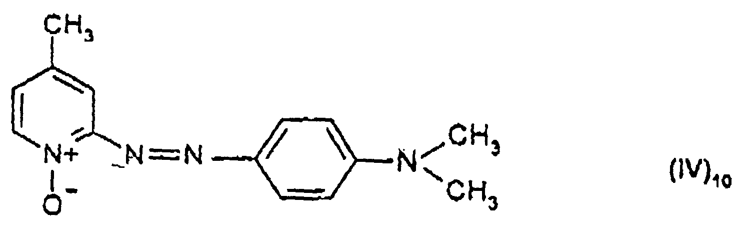

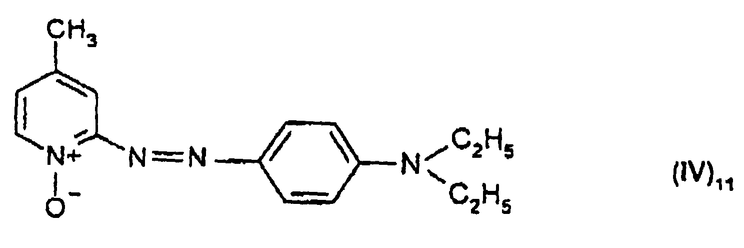

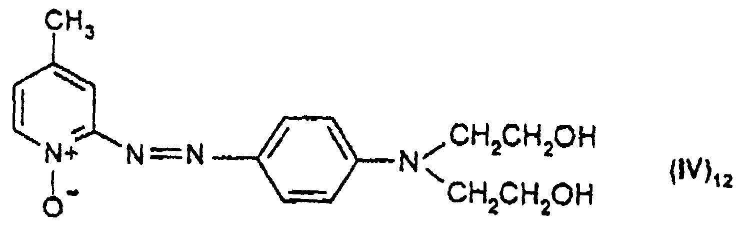

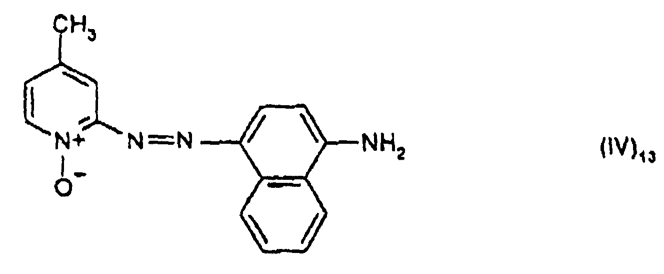

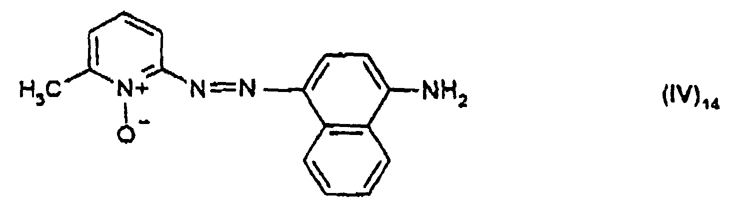

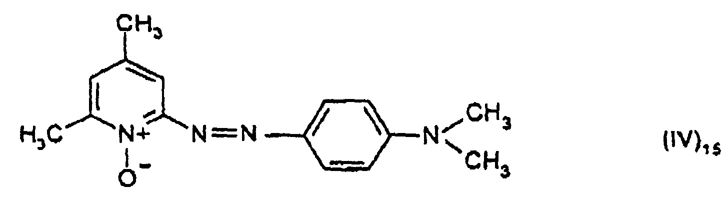

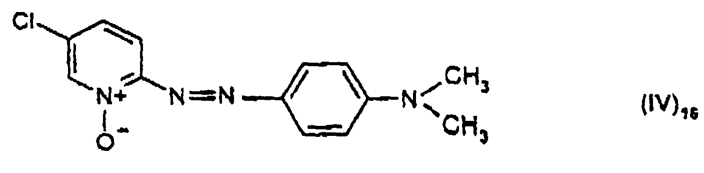

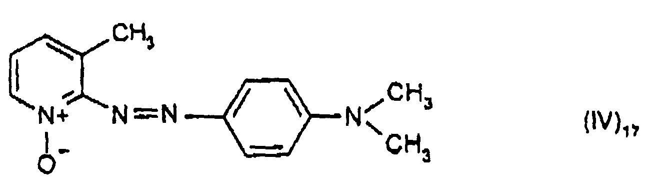

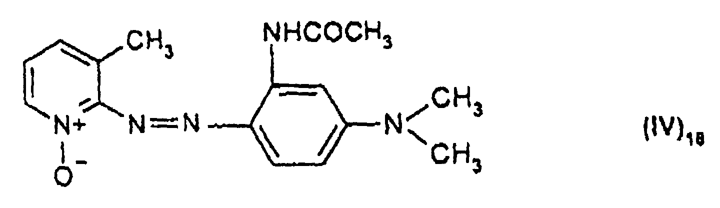

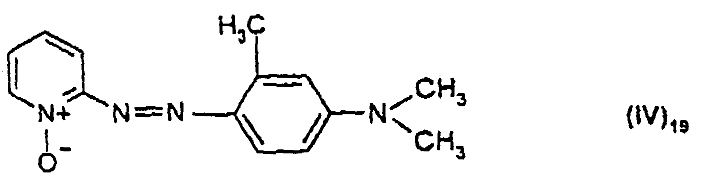

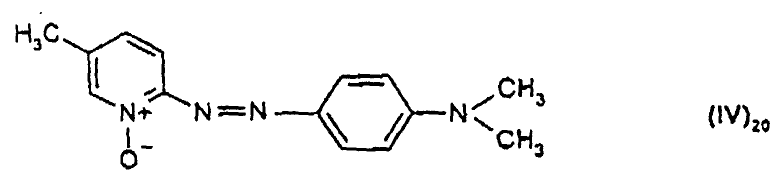

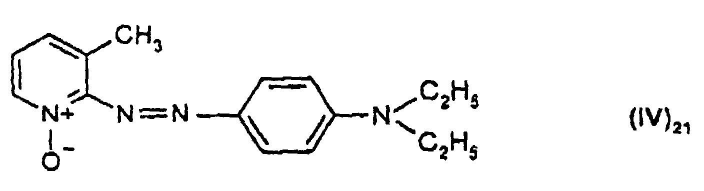

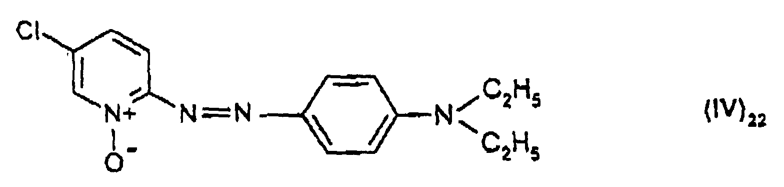

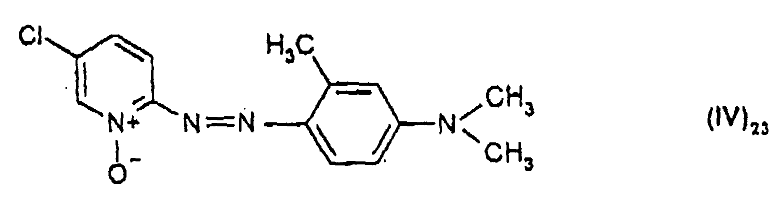

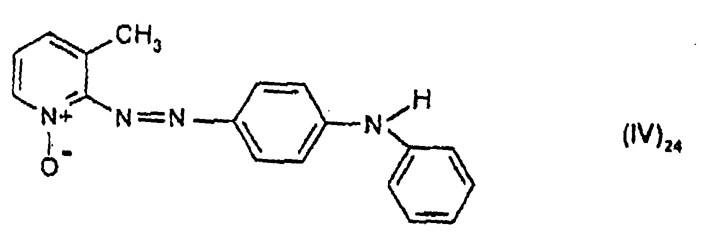

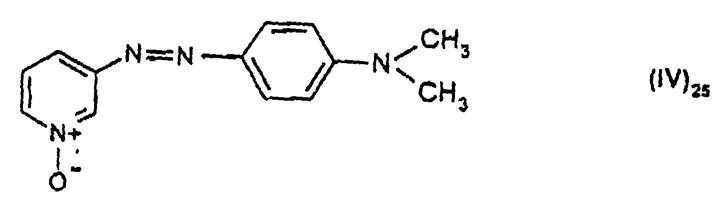

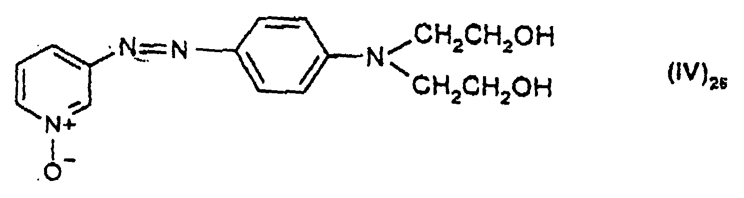

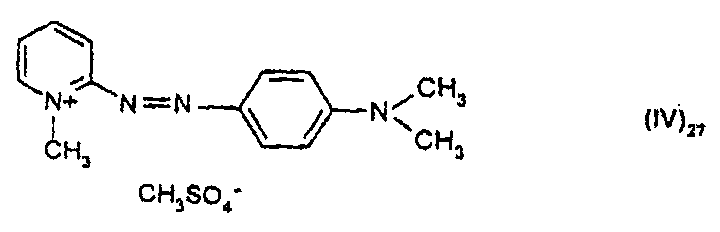

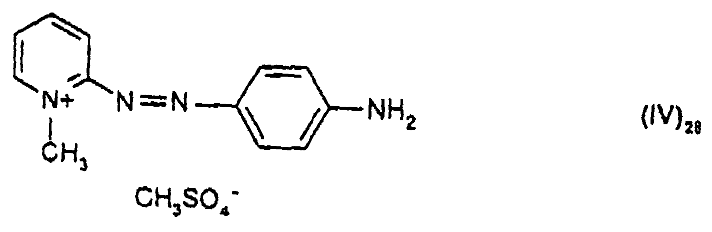

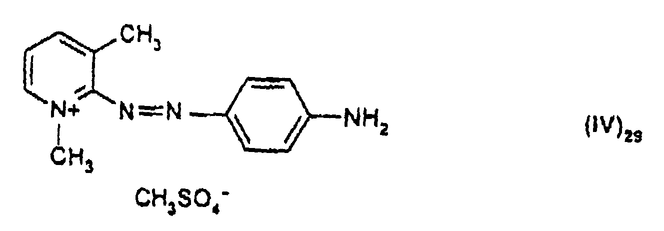

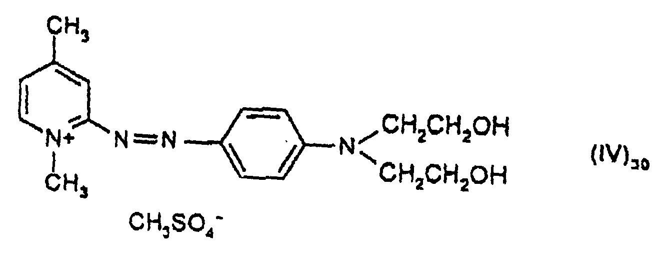

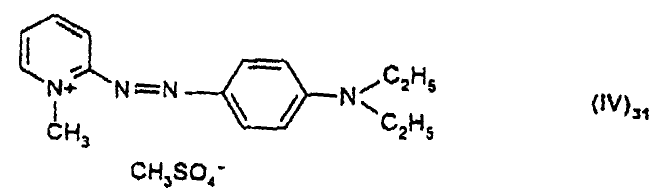

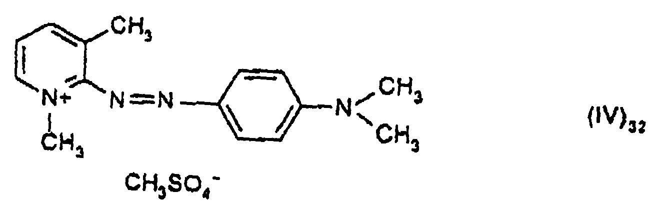

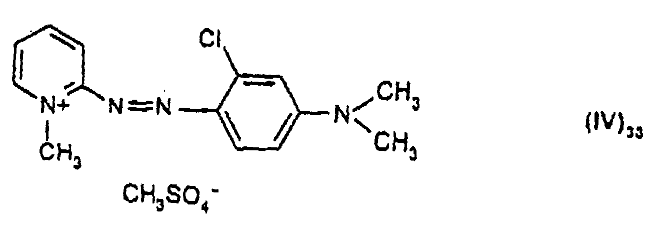

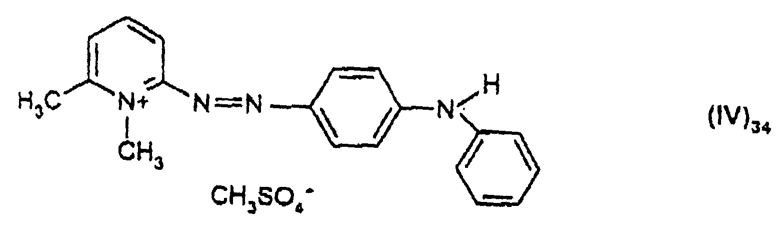

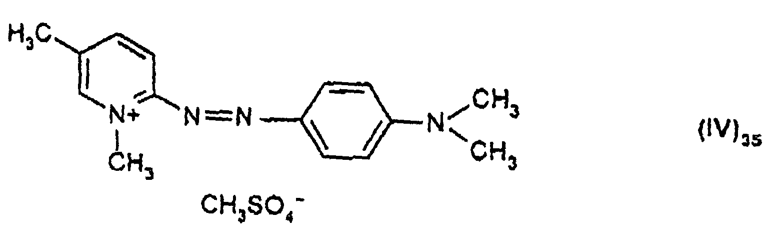

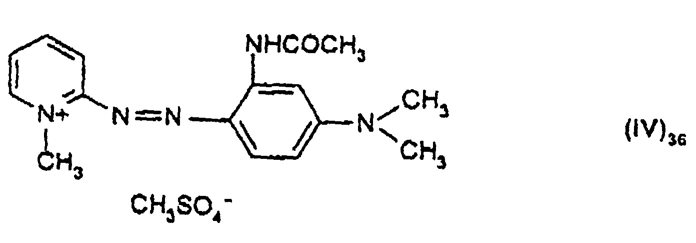

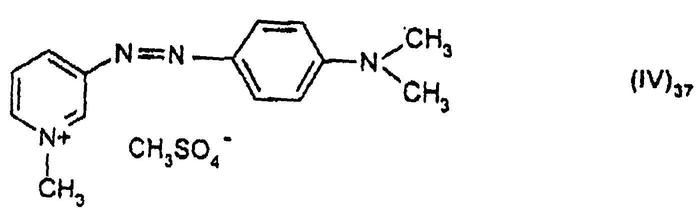

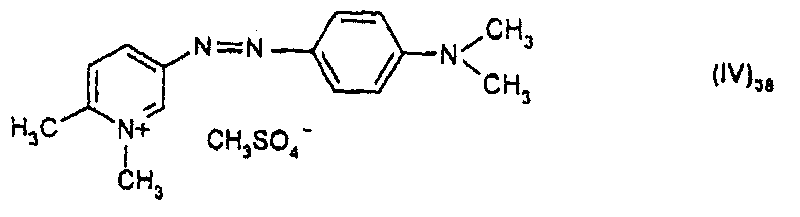

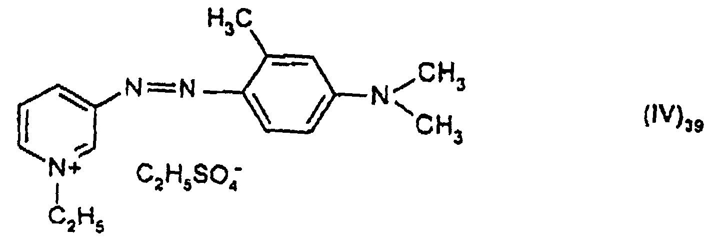

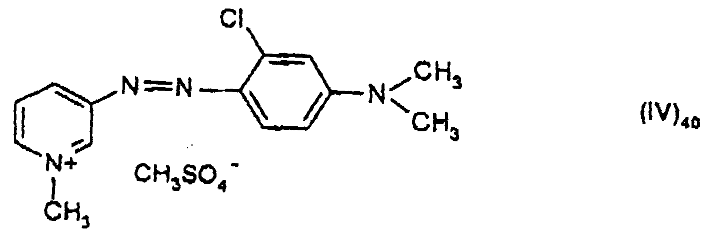

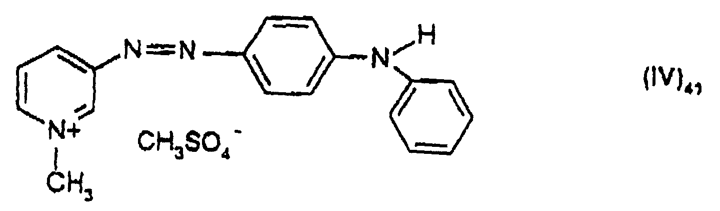

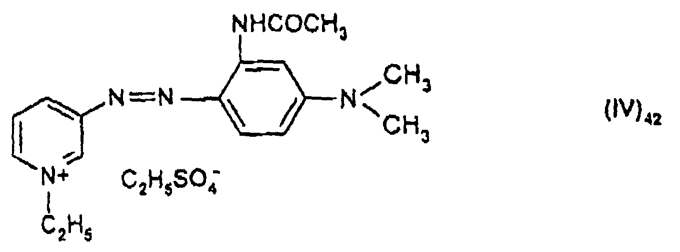

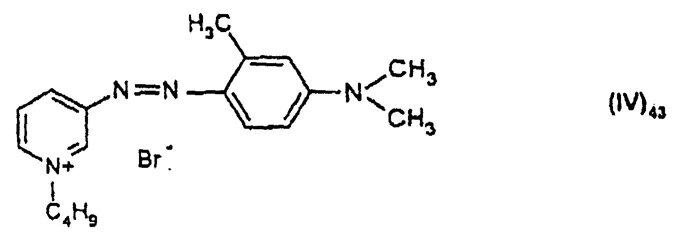

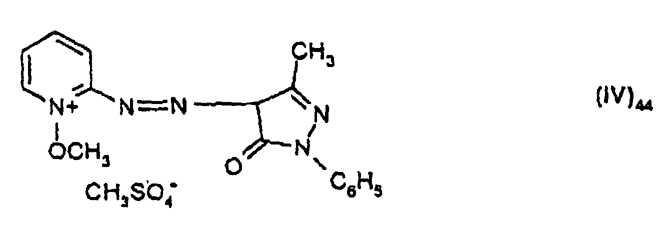

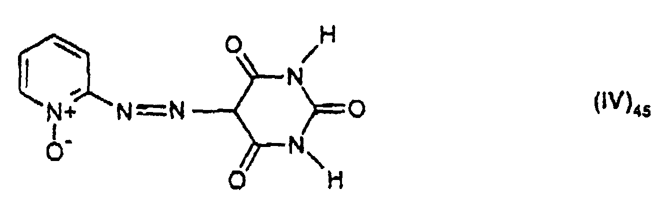

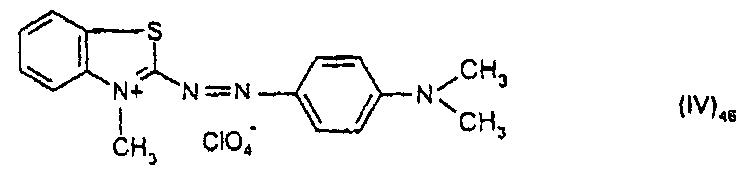

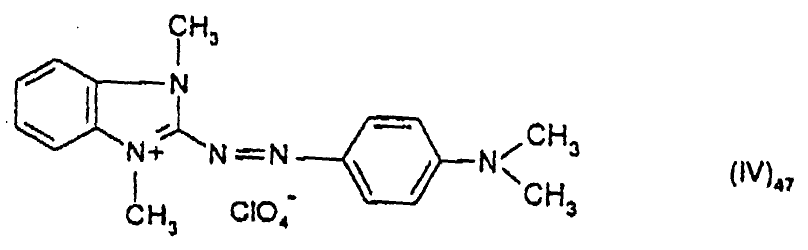

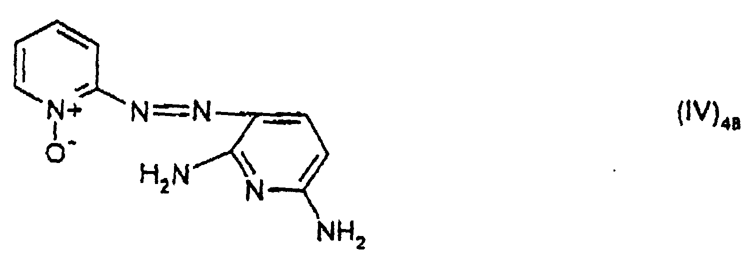

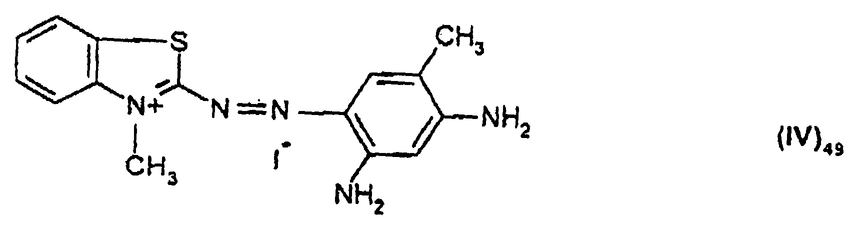

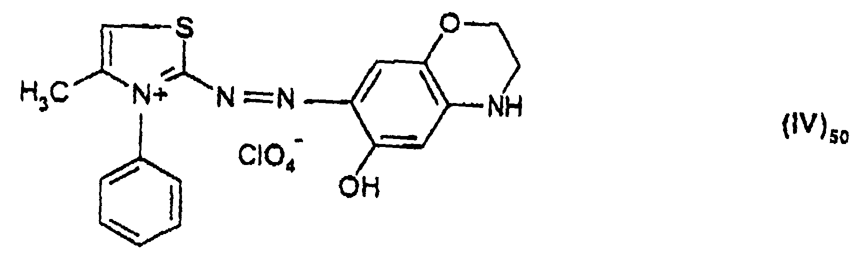

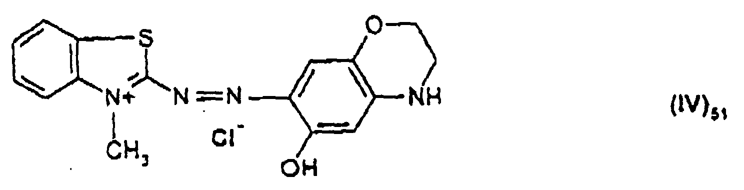

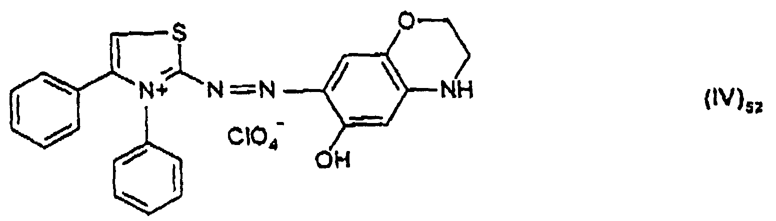

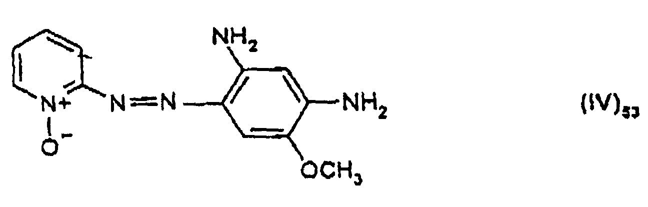

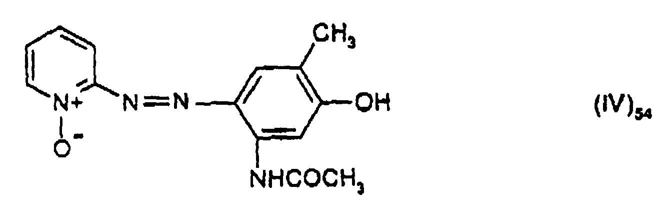

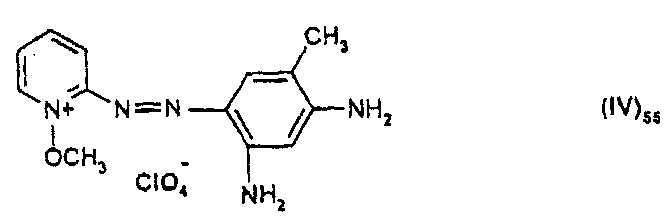

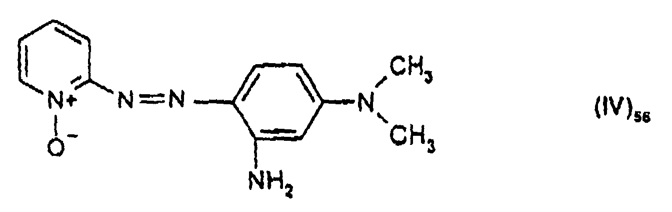

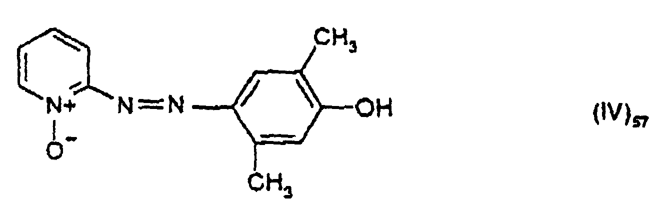

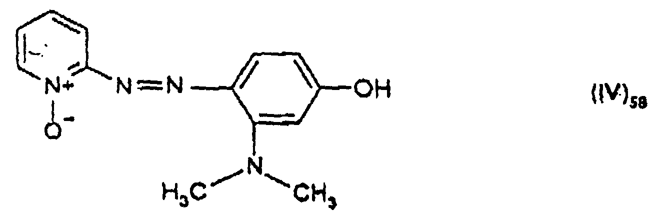

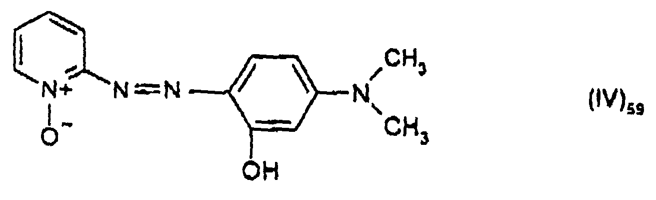

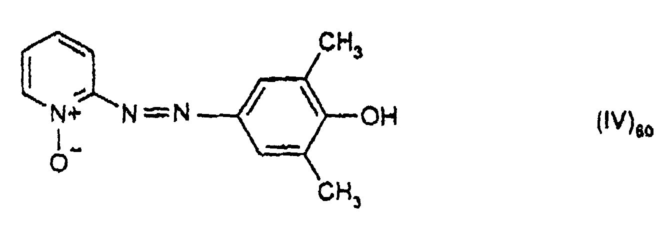

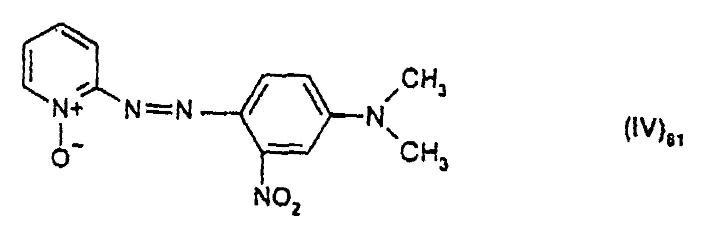

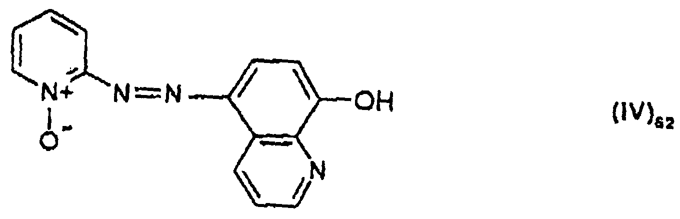

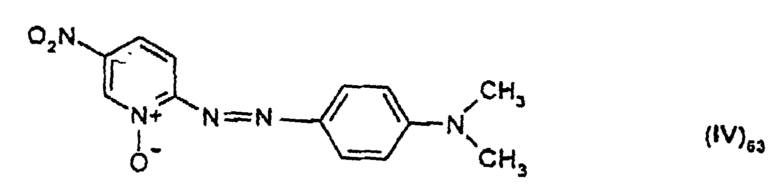

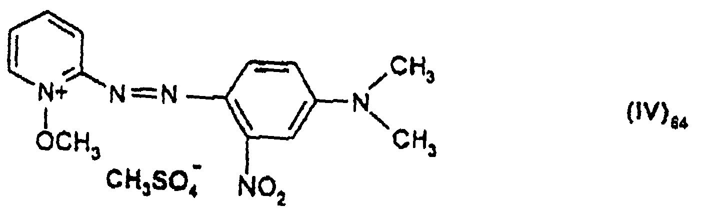

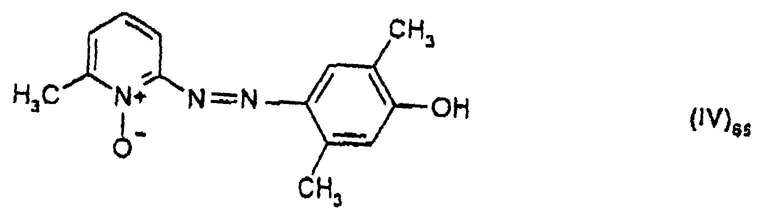

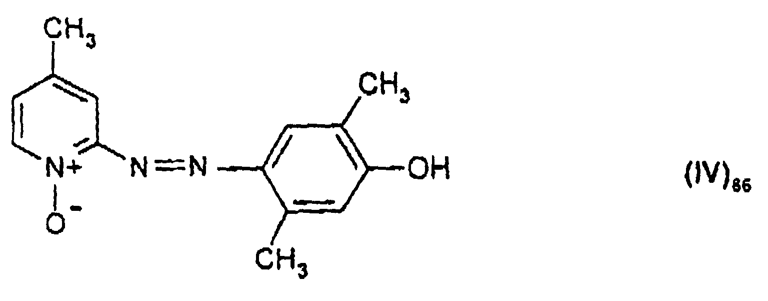

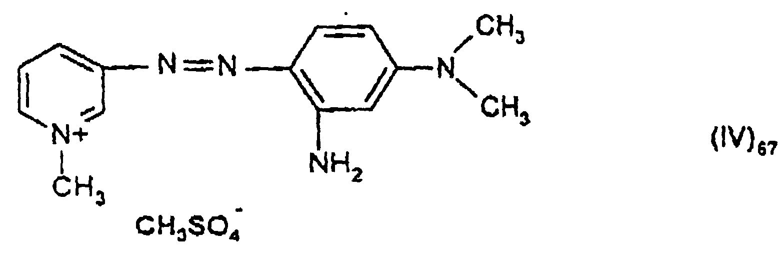

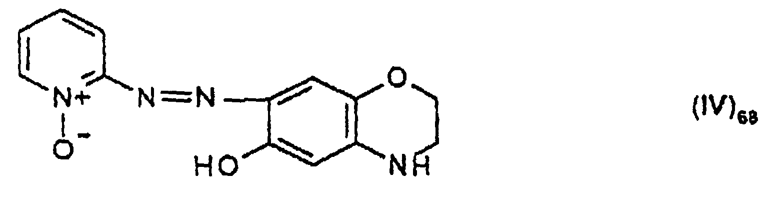

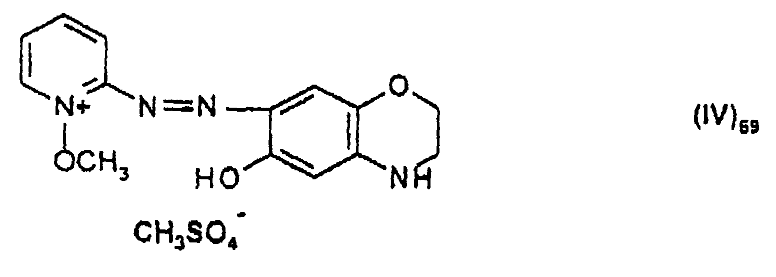

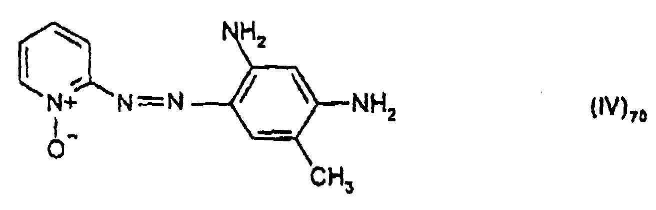

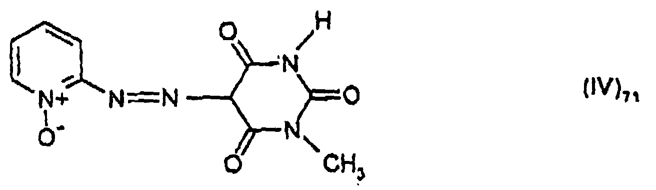

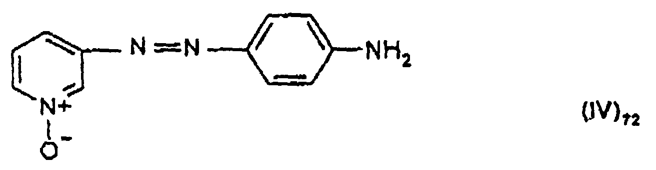

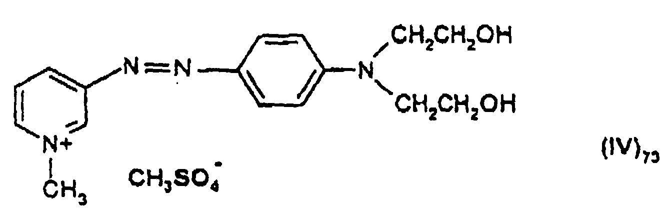

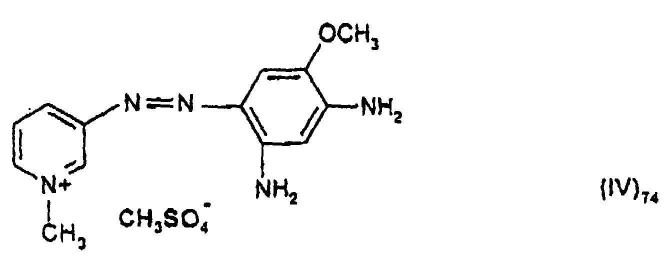

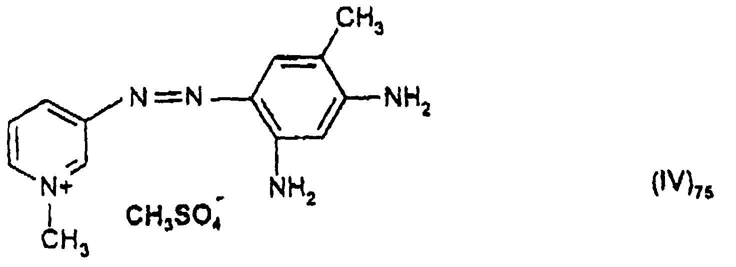

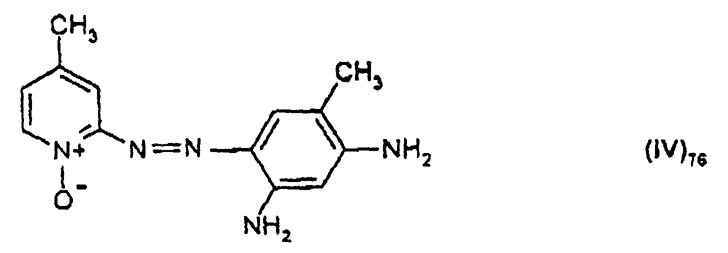

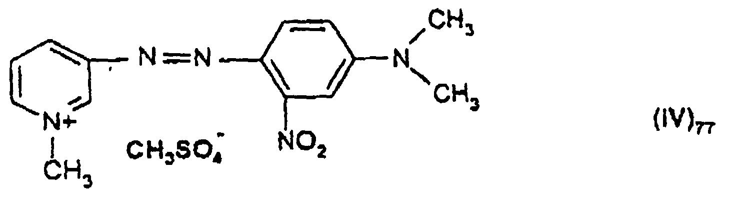

9. Zusammensetzung nach einem der vorhergehenden Ansprüche, **dadurch gekennzeichnet, dass** der oder die kationische(n) Direktfarbstoff(e) der Formeln (I), (II), (III), (III') oder (IV) 0,001 bis 10 Gew.-% des Gesamtgewichts der Zusammensetzung ausmachen.

10. Zusammensetzung nach Anspruch 9, **dadurch gekennzeichnet, dass** der oder die kationische(n) Direktfarbstoff(e) der Formeln (I), (II), (III), (III') oder (IV) 0,005 bis 5 Gew.-% des Gesamtgewichts der Zusammensetzung ausmachen.

11. Zusammensetzung nach einem der vorhergehenden Ansprüche, **dadurch gekennzeichnet, dass** das quartäre Ammoniumsalz (ii) der Formel (V) ein Dialkyldimethylammoniumsalz oder ein Alkyltrimethylammoniumsalz ist, wobei deren Alkylgruppe 12 bis 22 Kohlenstoffatome aufweist.

12. Zusammensetzung nach Anspruch 11, **dadurch gekennzeichnet, dass** es sich um das Distearyldimethylammoniumchlorid, Cetyltrimethylammoniumchlorid oder Behenyltrimethylammoniumchlorid handelt

13. Zusammensetzung nach einem der vorhergehenden Ansprüche, **dadurch gekennzeichnet, dass** das quartäre Ammoniumsalz (ii) der Formel (V) ein Dialkyl(C₁₋₂)alkyl(C₁₂₋₂₂)hydroxyalkyl(C₁₋₂)-ammoniumsalz ist.

14. Zusammensetzung nach Anspruch 13, **dadurch gekennzeichnet, dass** es sich um das Oleocetylhydroxyethylammoniumchlorid handelt.

15. Zusammensetzung nach einem der vorhergehenden Ansprüche, **dadurch gekennzeichnet, dass** das quartäre Ammoniumsalz (ii) der Formel (V) das Stearamidopropyldimethyl(myristylacetat)-ammoniumchlorid der folgenden Formel ist:

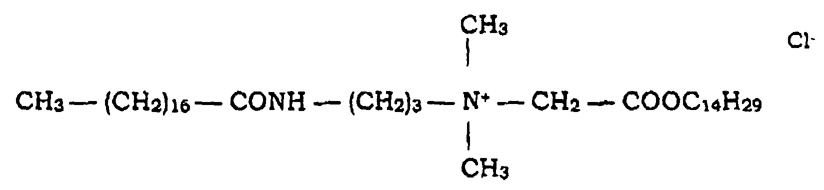

16. Zusammensetzung nach einem der vorhergehenden Ansprüche, **dadurch gekennzeichnet, dass** das oder die quartäre(n) Ammoniumsalz(e) der Formel (V) 0,01 bis 10 Gew.-% des Gesamtgewichts der Farbmittelzusammensetzung ausmachen.

17. Zusammensetzung nach Anspruch 16, **dadurch gekennzeichnet, dass** das oder die quartäre(n) Ammoniumsalz(e) der Formel (V) 0,05 bis 5 Gew.-% des Gesamtgewichts der Farbmittelzusammensetzung ausmachen.

18. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

19. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

20. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

21. Zusammensetzung nach Anspruch 20, **dadurch gekennzeichnet, dass** die Oxidationsbase(n) 0,0005 bis 12 Gew.-% des Gesamtgewichts der Farbmittelzusammensetzung ausmachen.

22. Zusammensetzung nach Anspruch 21, **dadurch gekennzeichnet, dass** die Oxidationsbase(n) 0,005 bis 6 Gew.-% des Gesamtgewichts der Farbmittelzusammensetzung ausmachen.

23. Zusammensetzung nach einem der Ansprüche 20 bis 22, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

24. Zusammensetzung nach Anspruch 23, **dadurch gekennzeichnet, dass** der oder die Kuppler 0,0001 bis 10 Gew.-% des Gesamtgewichts der Farbmittelzusammensetzung ausmachen.

25. Zusammensetzung nach Anspruch 24, **dadurch gekennzeichnet, dass** der oder die Kuppler 0,005 bis 5 Gew.-% des Gesamtgewichts der Farbmittelzusammensetzung ausmachen.

26. Zusammensetzung nach einem der vorhergehenden Ansprüche, **dadurch gekennzeichnet, dass** sie zum oxidativen Färben oder für die aufhellende Direktfärbung vorgesehen ist und mindestens ein Oxidationsmittel enthält.

27. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 26 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

28. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 26 während einer Zeitspanne, die zur Bildung der gewünschten Färbung ausreichend ist, aufgebracht wird, ohne dass nochmals gespült wird.

29. Verfahren zum Färben von Keratinfasern und insbesondere menschlichen Keratinfasern, wie dem Haar, **dadurch gekennzeichnet, dass** es einen vorbereitenden Schritt umfasst, der darin besteht, einerseits eine Zusammensetzung (A1), die in einem zum Färben geeigneten Medium mindestens einen in den vorhergehenden Ansprüchen definierten Direktfarbstoff (i) und mindestens eine Oxidationsbase enthält, und andererseits eine Zusammensetzung (B1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A1) oder die Zusammensetzung (B1) das in den vorhergehenden Ansprüchen definierte quartäre Ammoniumsalz (ii) enthält.

30. Verfahren zum Färben von Keratinfasern und insbesondere menschlichen Keratinfasern, wie dem Haar, **dadurch gekennzeichnet, dass** es einen vorbereitenden Schritt umfasst, der darin besteht, einerseits eine Zusammensetzung (A2), die in einem zum Färben geeigneten Medium mindestens einen in den vorhergehenden Ansprüchen definierten Direktfarbstoff (i) enthält, und andererseits eine Zusammensetzung (B2) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A2) oder die Zusammensetzung (B2) das in den vorhergehenden Ansprüchen definierte quartäre Ammoniumsalz (ii) enthält.

31. Vorrichtung mit mehreren Abteilungen oder "Kit" zum Färben mit mehreren Abteilungen, **dadurch gekennzeichnet, dass** eine erste Abteilung die Zusammensetzung (A1) oder (A2) nach Anspruch 29 oder 30 und eine andere Abteilung die Zusammensetzung (B1) oder (B2) nach Anspruch 29 oder 30 enthält.

## Claims

1. Composition for dyeing keratinous fibres and in particular human keratinous fibres such as hair, containing in an appropriate dyeing medium, **(i)** at least one cationic direct dye chosen from the compounds of the following formulae (I), (II), (III), (III'), (IV):
**a) the compounds of the following formula (I):** 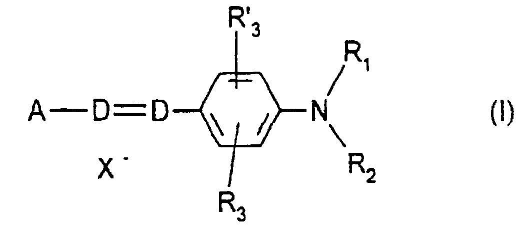 in which:
D represents a nitrogen atom or the -CH group,
R₁ and R₂, which are identical or different, represent a hydrogen atom; a C₁-C₄ alkyl radical which may be substituted with a -CN, -OH or -NH₂ radical or form with a carbon atom of the benzene ring an optionally oxygen-containing or nitrogen-containing heterocycle which may be substituted with one or more C₁-C₄ alkyl radicals; a 4'-aminophenyl radical,
R₃ and R'₃, which are identical or different, represent a hydrogen or halogen atom chosen from chlorine, bromine, iodine and fluorine, a cyano, C₁-C₄ alkyl, C₁-C₄ alkoxy or acetyloxy radical,
X⁻ represents an anion which is preferably chosen from chloride, methylsulphate and acetate,
A represents a group chosen from the following structures A₁ to A₁₈: 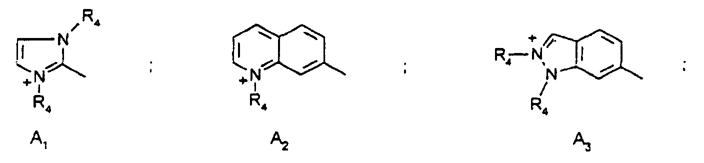 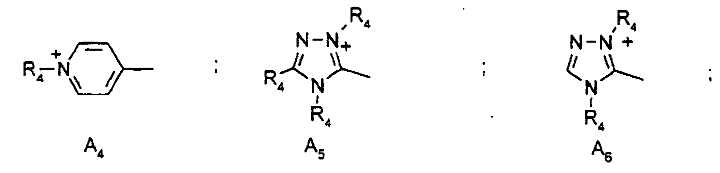 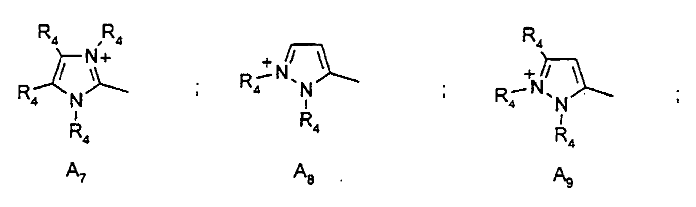 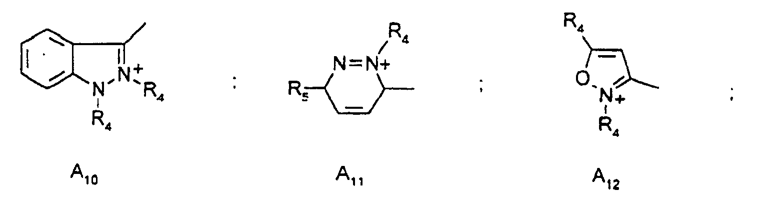 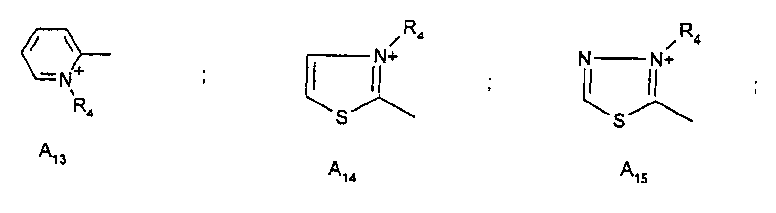 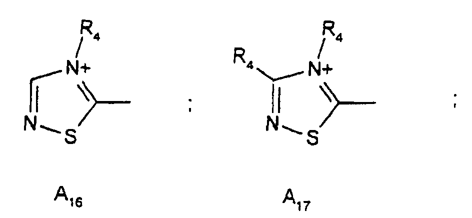 and 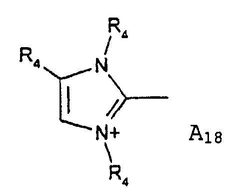 in which R₄ represents a C₁-C₄ alkyl radical which may be substituted with a hydroxyl radical and R₅ represents a C₁-C₄ alkoxy radical, with the proviso that when D represents -CH, A represents A₄ or A₁₃ and R₃ is different from an alkoxy radical, then R₁ and R₂ do not simultaneously denote a hydrogen atom;
**b) the compounds of the following formula (II):** 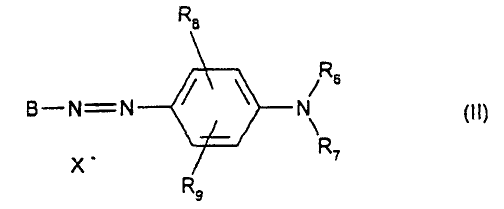 in which:
R₆ represents a hydrogen atom or a C₁-C₄ alkyl radical,
R₇ represents a hydrogen atom, an alkyl radical which may be substituted with a -CN radical or with an amino group, a 4'-aminophenyl radical or forms with R₆ an optionally oxygen-containing and/or nitrogen-containing heterocycle which may be substituted with a C₁-C₄ alkyl radical,
R₈ and R₉ , which are identical or different, represent a hydrogen atom, a halogen atom such as bromine, chlorine, iodine or fluorine, a C₁-C₄ alkyl or C₁-C₄ alkoxy radical, a -CN radical,
X⁻ represents an anion which is preferably chosen from chloride, methylsulphate and acetate,
B represents a group chosen from the following structures B1 to B6: 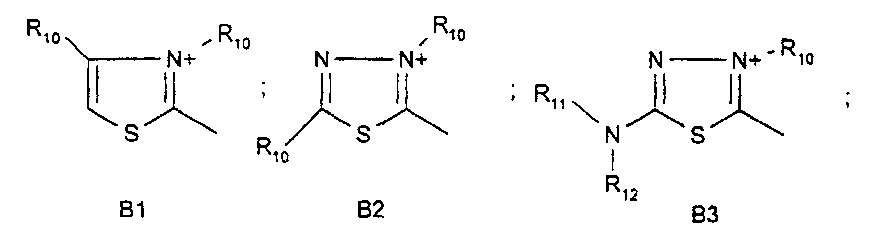 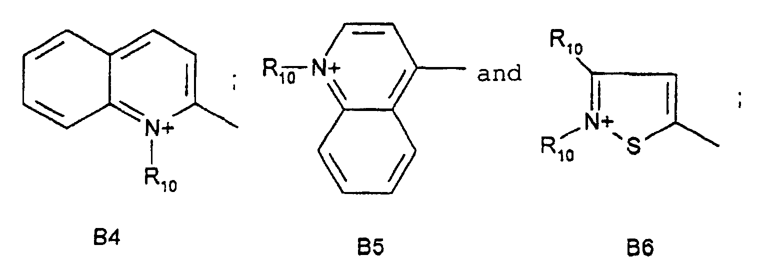 in which R₁₀ represents a C₁-C₄ alkyl radical, R₁₁ and R₁₂, which are identical or different, represent a hydrogen atom or a C₁-C₄ alkyl radical;
**c) the compounds of the following formulae (III) and (III'):** 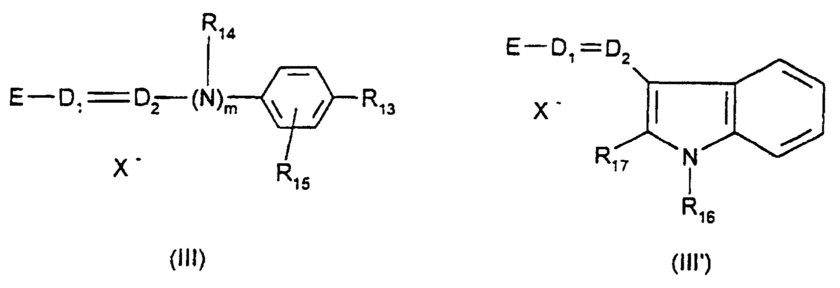 in which:
R₁₃ represents a hydrogen atom, a C₁-C₄ alkoxy radical, a halogen atom such as bromine, chlorine, iodine or fluorine or an amino radical,
R₁₄ represents a hydrogen atom, a C₁-C₄ alkyl radical or forms with a carbon atom of the benzene ring a heterocycle which is optionally oxygen-containing and/or substituted with one or more C₁-C₄ alkyl groups,
R₁₅ represents a hydrogen or halogen atom such as bromine, chlorine, iodine of fluorine,
R₁₆ and R₁₇, which are identical or different, represent a hydrogen atom or a C₁-C₄ alkyl radical,
D₁ and D₂, which are identical or different, represent a nitrogen atom or the -CH group,
m = 0 or 1,
it being understood that when R₁₃ represents an unsubstituted amino group, then D₁ and D₂ simultaneously represent a -CH group and m = 0,
X⁻ represents an anion which is preferably chosen from chloride, methylsulphate and acetate,
E represents a group chosen from the following structures E1 to E8: 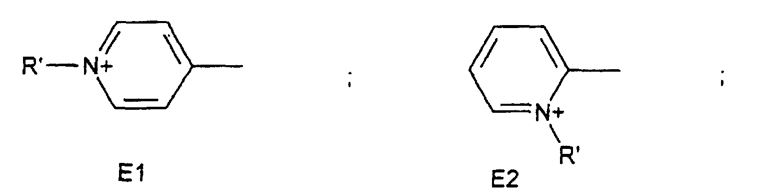 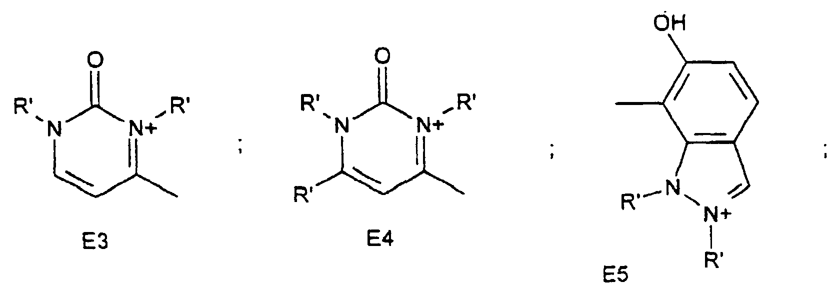 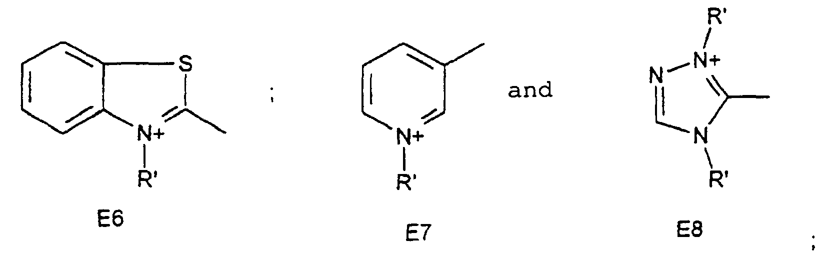 in which R' represents a C₁-C₄ alkyl radical;
when m = 0 and D₁ represents a nitrogen atom, then E may also denote a group having the following structure E9: 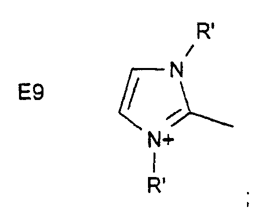 in which R' represents a C₁-C₄ alkyl radical,
**d) the compounds of the following formula (IV):**
G―N=N―J (IV)
in which:
**the symbol G** represents a group chosen from the following structures G₁ to G₃: 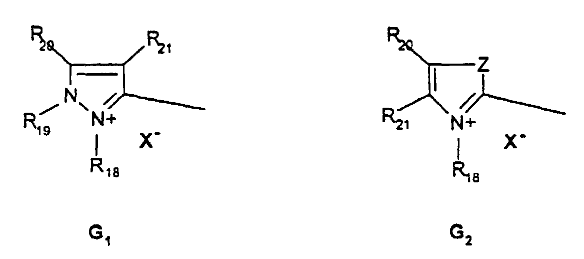 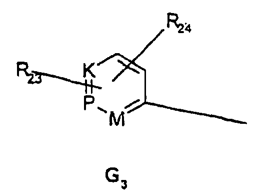 in which structures G₁ to G₃,
R₁₈ denotes a C₁-C₄ alkyl radical, a phenyl radical which may be substituted with a C₁-C₄ alkyl radical or a halogen atom chosen from chlorine, bromine, iodine and fluorine;
R₁₉ denotes a C₁-C₄ alkyl radical or a phenyl radical;
R₂₀ and R₂₁, which are identical or different, represent a C₁-C₄ alkyl radical, a phenyl radical, or form together in G₁ a benzene ring which is substituted with one or more C₁-C₄ alkyl, C₁-C₄ alkoxy or NO₂ radicals, or form together in G₂ a benzene ring which is optionally substituted with one or more C₁-C₄ alkyl, C₁-C₄ alkoxy or NO₂ radicals;
R₂₀ may denote, in addition, a hydrogen atom;
Z denotes an oxygen or sulphur atom or an -NR₁₉ group;
M represents a group -CH, -CR (R denoting C₁-C₄ alkyl), or -NR₂₂(X⁻)ᵣ;
K represents a group -CH, -CR (R denoting C₁-C₄ alkyl), or -NR₂₂(X⁻)ᵣ;
P represents a group -CH, -CR (R denoting C₁-C₄ alkyl), or -NR₂₂(X⁻)ᵣ; r denotes zero or 1;
R₂₂ represents an O⁻ atom, a C₁-C₄ alkoxy radical or a C₁-C₄ alkyl radical;
R₂₃ and R₂₄, which are identical or different, represent a hydrogen or halogen atom chosen from chlorine, bromine, iodine and fluorine, a C₁-C₄ alkyl radical, a C₁-C₄ alkoxy radical or an -NO₂ radical;
X⁻ represents an anion which is preferably chosen from chloride, iodide, methylsulphate, ethylsulphate, acetate and perchlorate;
with the proviso that
if R₂₂ denotes O⁻, then r denotes zero;
if K or P or M denote -N-(C₁-C₄ alkyl)X⁻, then R₂₃ or R₂₄ is different from a hydrogen atom;
if K denotes -NR₂₂(X⁻)_{r'}, then M = P = -CH, -CR;
if M denotes -NR₂₂(X⁻)ᵣ, then K = P = -CH, -CR;
if P denotes -NR₂₂(X⁻)ᵣ, then K = M and denote -CH or -CR;
if Z denotes a sulphur atom with R₂₁ denoting C₁-C₄ alkyl, then R₂₀ is different from a hydrogen atom;
if Z denotes -NR₁₉ with R₁₉ denoting C₁-C₄ alkyl, then at least one of the R₁₈, R₂₀ or R₂₁ radicals of G₂ is different from a C₁-C₄ alkyl radical;
**the symbol J** represents:
- **(a)** a group having the following structure J₁: 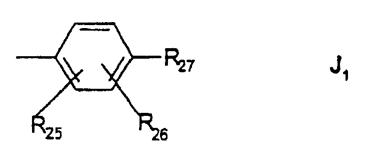 in which structure J₁,
R₂₅ represents a hydrogen atom, a halogen atom chosen from chlorine, bromine, iodine and fluorine, a C₁-C₄ alkyl radical, a C₁-C₄ alkoxy radical, a radical -OH, -NO₂, -NHR₂₈, -NR₂₉R₃₀, -NHCO(C₁-C₄alkyl), or forms with R₂₆ a 5- or 6-membered ring containing or otherwise one or more heteroatoms chosen from nitrogen, oxygen or sulphur;
R₂₆ represents a hydrogen atom, a halogen atom chosen from chlorine, bromine, iodine and fluorine, a C₁-C₄ alkyl or C₁-C₄ alkoxy radical, or forms with R₂₇ or R₂₈ a 5- or 6-membered ring containing or otherwise one or more heteroatoms chosen from nitrogen, oxygen or sulphur;
R₂₇ represents a hydrogen atom, an -OH radical, an -NHR₂₈ radical, an -NR₂₉R₃₀ radical;
R₂₈ represents a hydrogen atom, a C₁-C₄ alkyl radical, a C₁-C₄ monohydroxyalkyl radical, a C₂-C₄ polyhydroxyalkyl radical, a phenyl radical;
R₂₉ and R₃₀, which are identical or different, represent a C₁-C₄ alkyl radical, a C₁-C₄ monohydroxyalkyl radical, a C₂-C₄ polyhydroxyalkyl radical;
- **(b)** a 5- or 6- membered nitrogen-containing heterocycle group which is capable of containing other heteroatoms and/or carbonyl-containing groups and which may be substituted with one or more C₁-C₄ alkyl, amino or phenyl radicals,
and in particular a group having the following structure J₂: 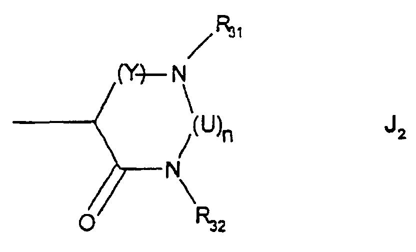 in which structure J₂,
R₃₁ and R₃₂, which are identical or different, represent a hydrogen atom, a C₁-C₄ alkyl radical, a phenyl radical;
Y denotes the -CO- radical or the radical 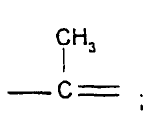 n = 0 or 1, with, when n denotes 1, U denotes the -CO- radical.
the said composition being **characterized in that** it contains, in addition,
**(ii)** at least one quaternary ammonium salt chosen from the group comprising:
**(ii)**_{**1**} - those of the following formula (V): 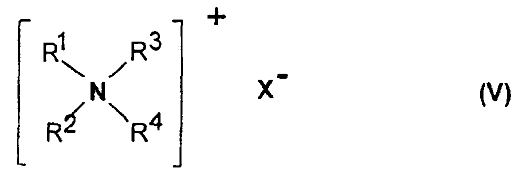 in which,
the radicals R¹ to R⁴, which are identical or different, denote a saturated or unsaturated, linear or branched, aliphatic hydrocarbon radical comprising from 1 to about 30 carbon atoms, or an alkoxy, alkoxycarbonylalkyl, polyoxyalkylene, alkylamido, alkylamidoalkyl, hydroxyalkyl, aromatic, aryl or alkylaryl radical comprising from 12 to about 30 carbon atoms, with at least one radical among R¹, R², R³ and R⁴ denoting a radical comprising from 8 to 30 carbon atoms;
X⁻ is an anion chosen from the group comprising halides, phosphates, acetates, lactates and alkyl sulphates;
**(ii)**_{**2**} - the imidazolium salts of the following formula (VI): 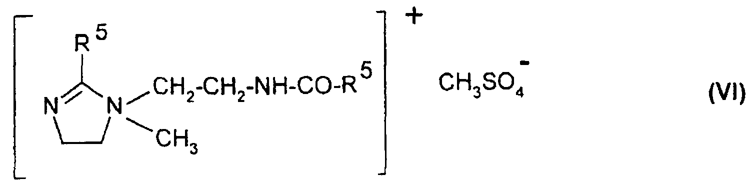 in which,
R⁵ is chosen from the alkenyl and/or alkyl radicals comprising from 13 to 31 carbon atoms and derived from tallow fatty acids.
**(ii)**_{**3**} - the quaternary diammonium salts of the following formula (VII): 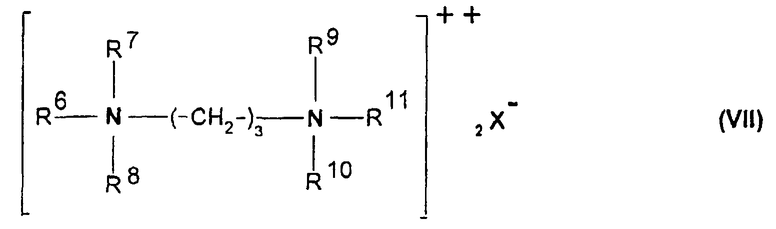 in which,
R⁶ denotes an aliphatic radical comprising from 16 to 30 carbon atoms, R⁷, R⁸, R⁹, R¹⁰ and R¹¹ are chosen from hydrogen or an alkyl radical comprising from 1 to 4 carbon atoms, and X⁻ is an anion chosen from the group comprising halides, acetates, phosphates and sulphates.

2. Composition according to Claim 1, **characterized in that** the cationic direct dyes of formula (I) are chosen from the compounds corresponding to the following structures (I1) to (I54): 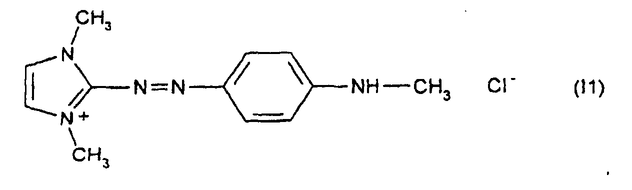 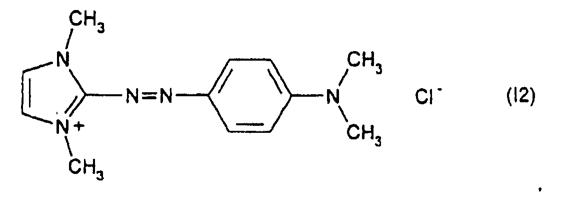 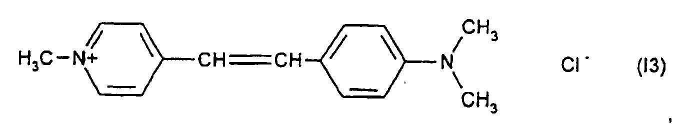 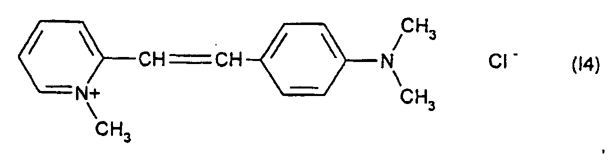 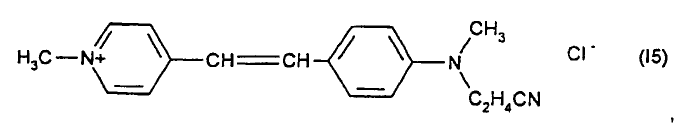 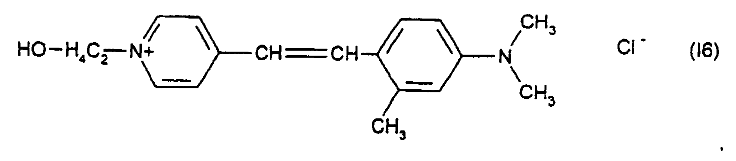 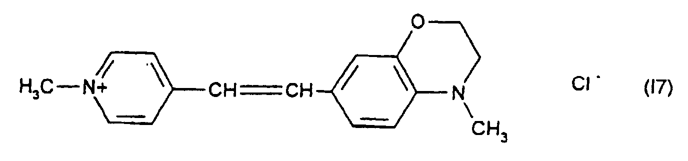 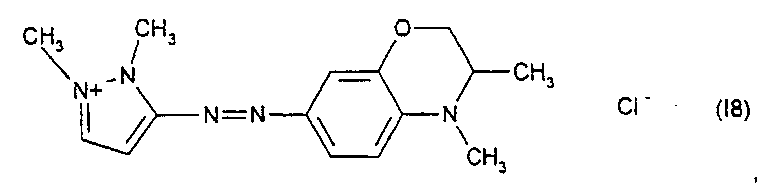 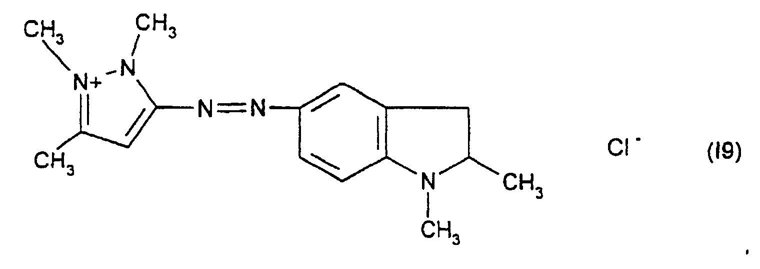 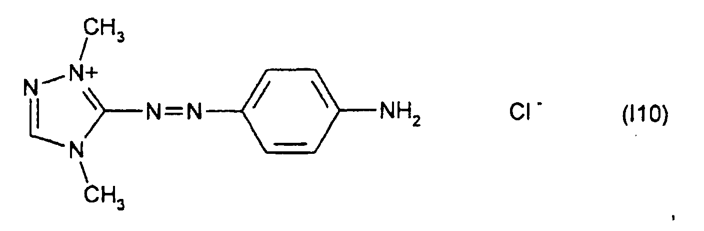 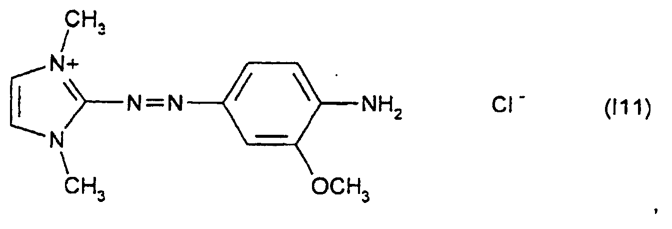 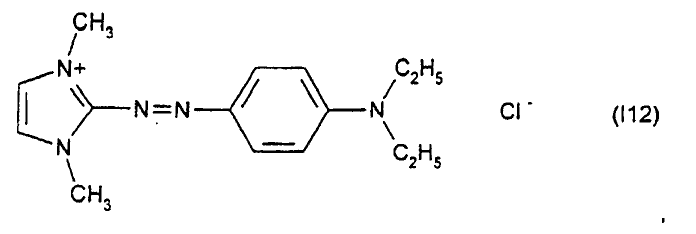 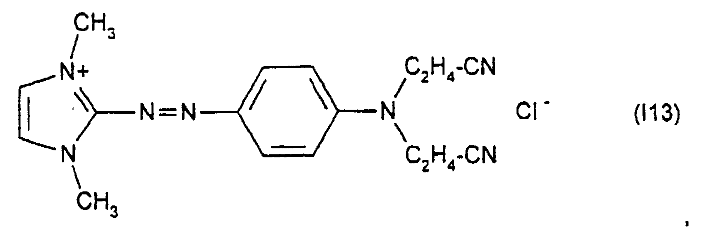 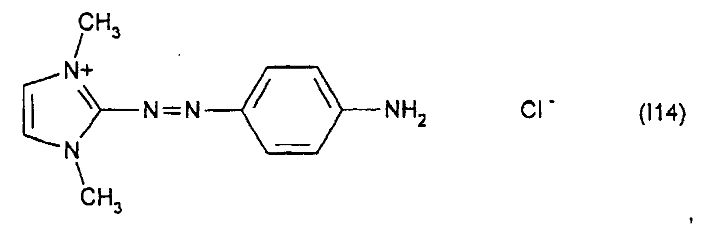 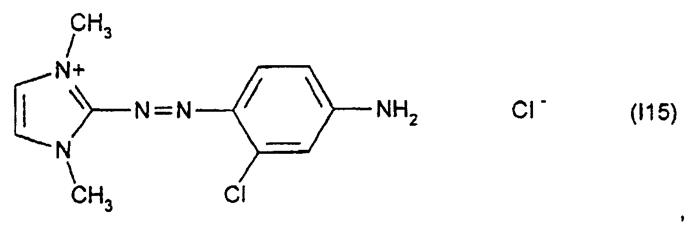 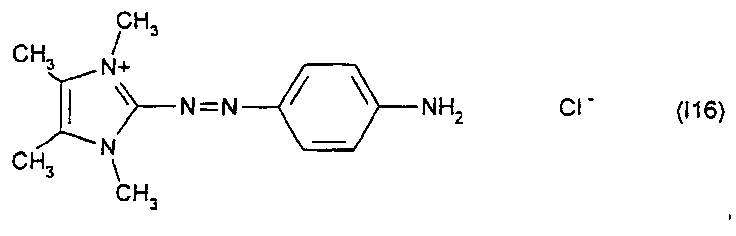 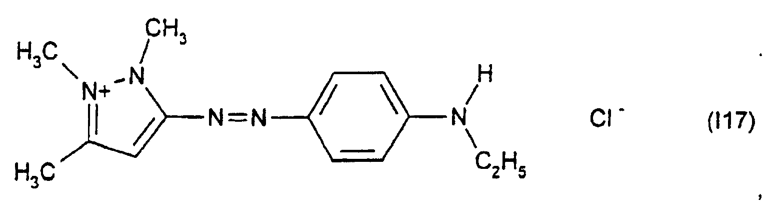 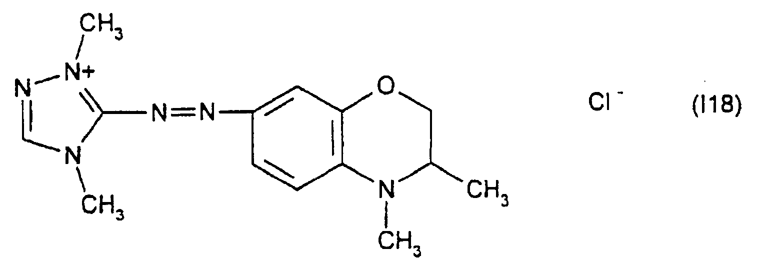 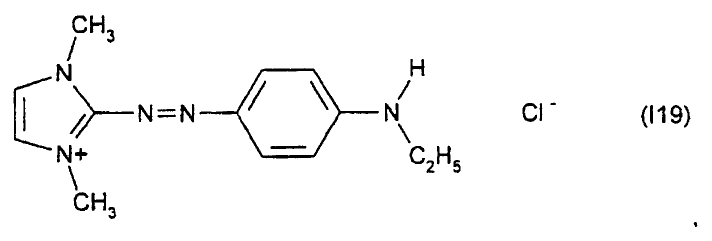 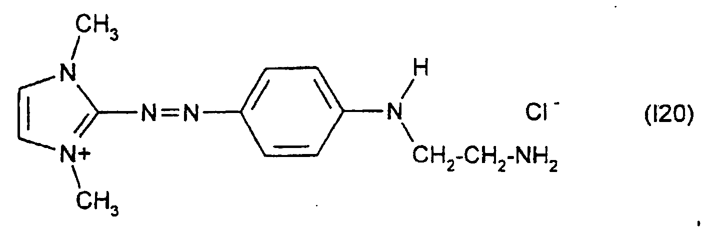 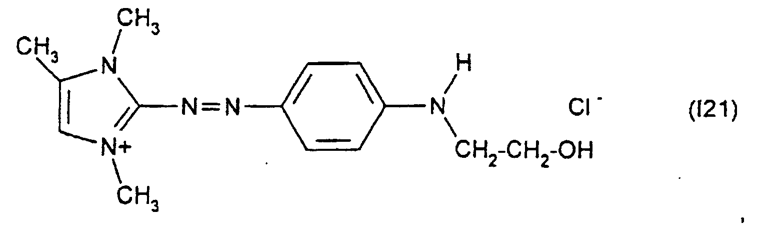 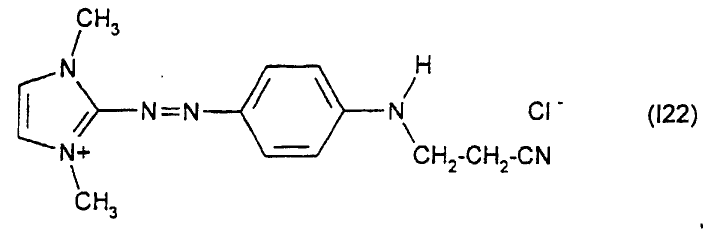 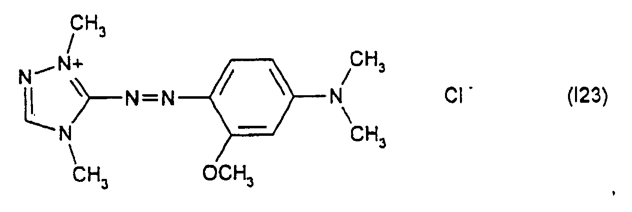 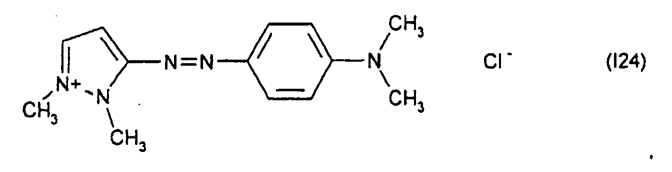 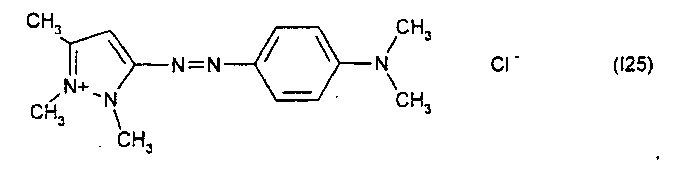 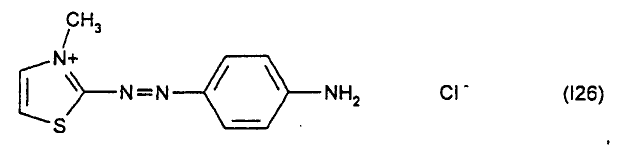 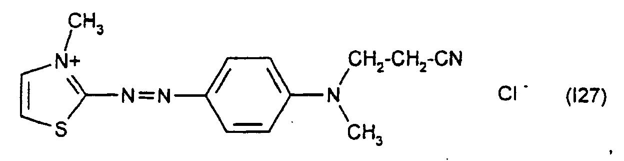 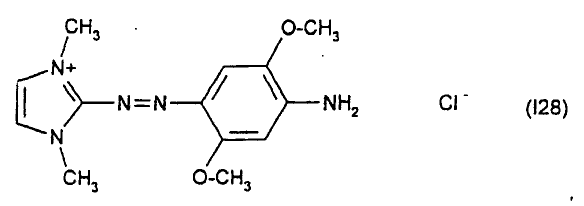 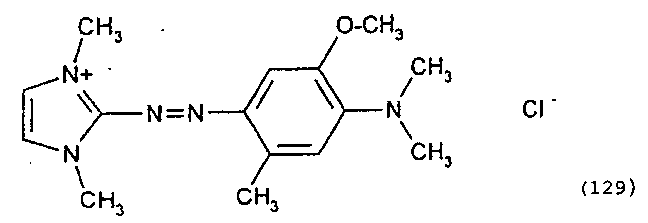 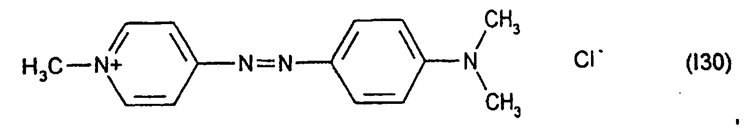 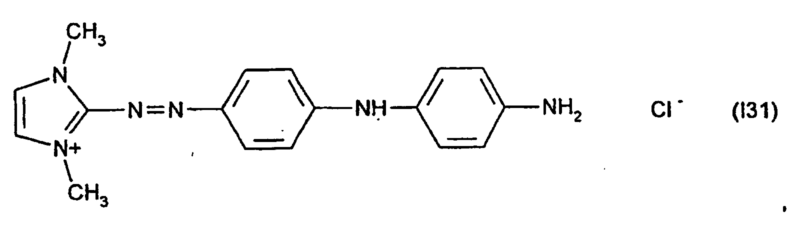 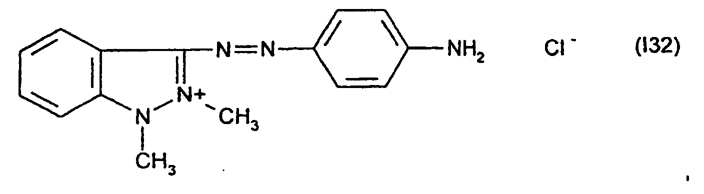 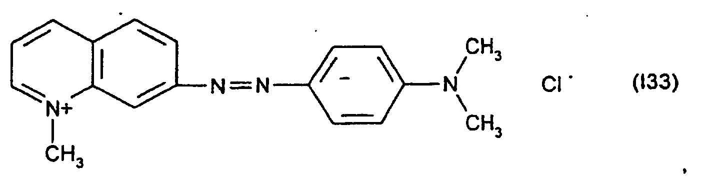 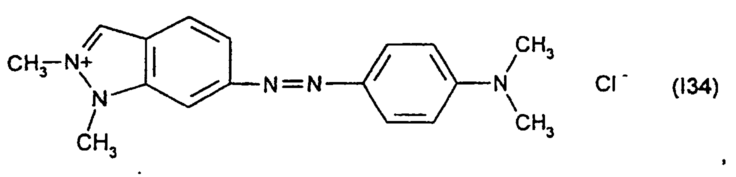 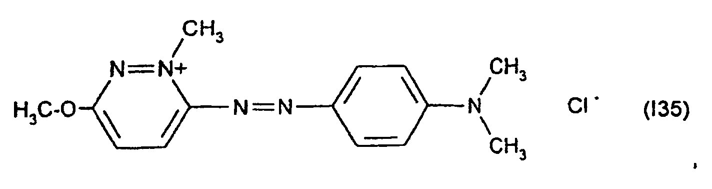 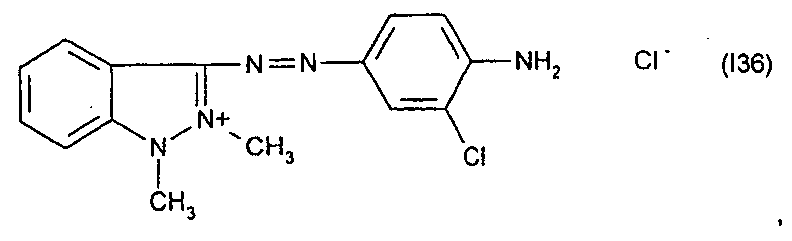 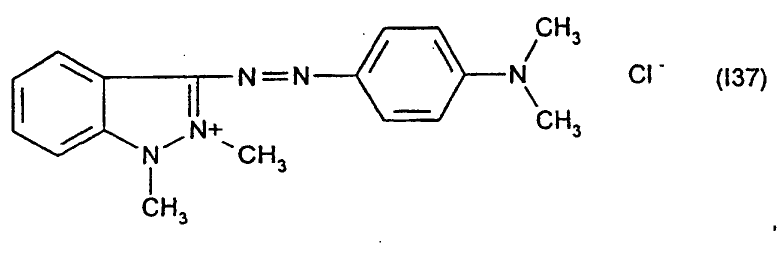 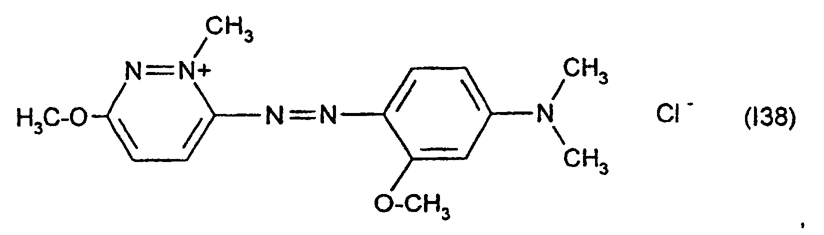 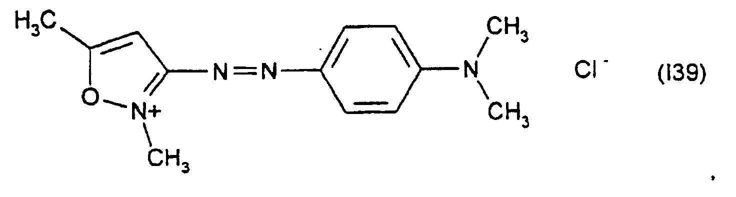 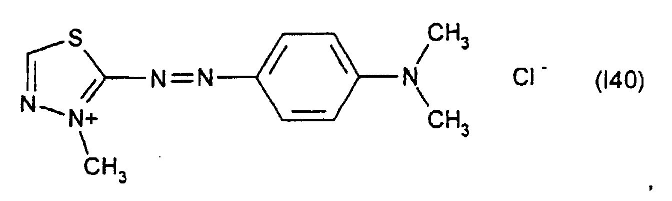 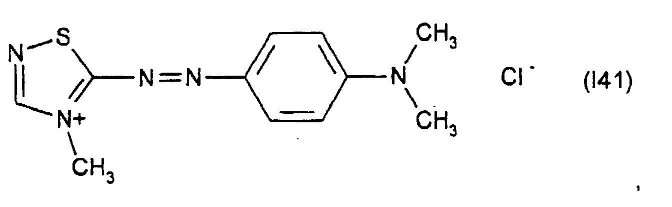 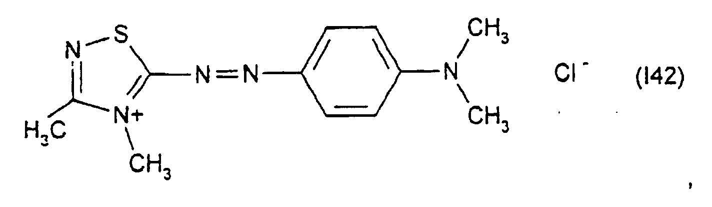 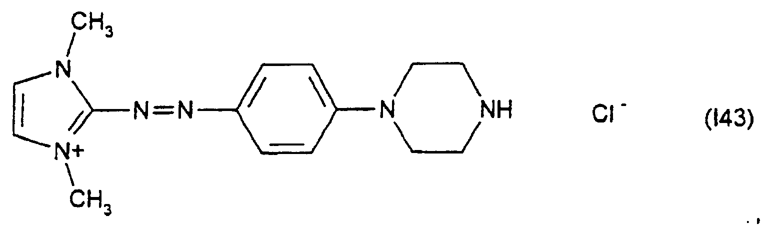 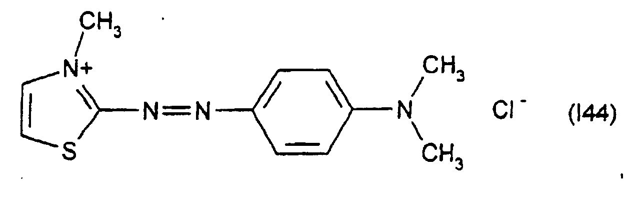 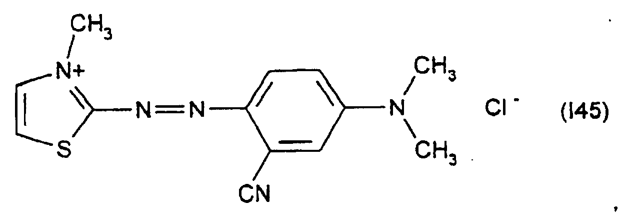 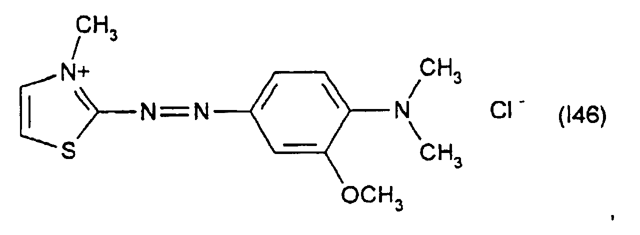 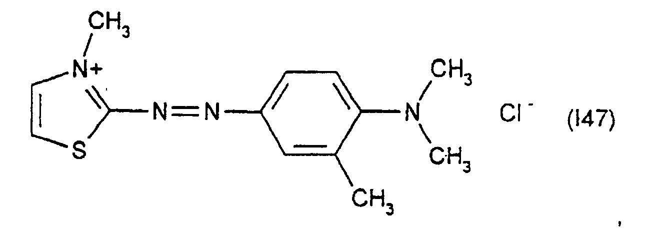 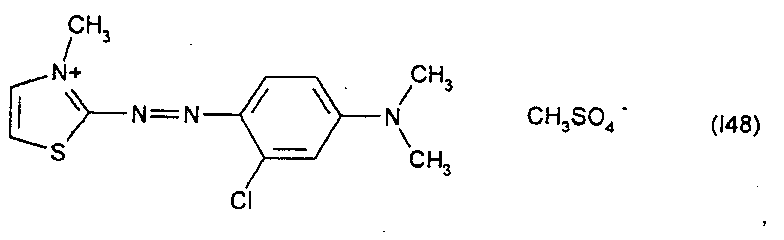 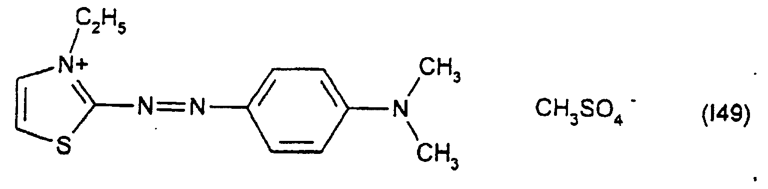 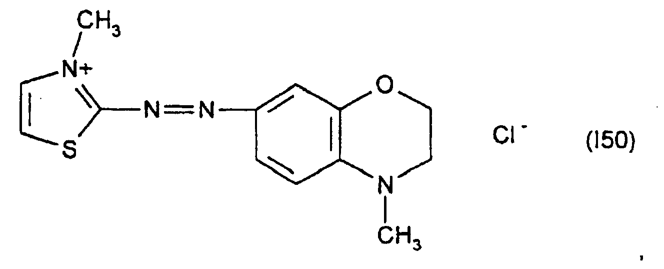 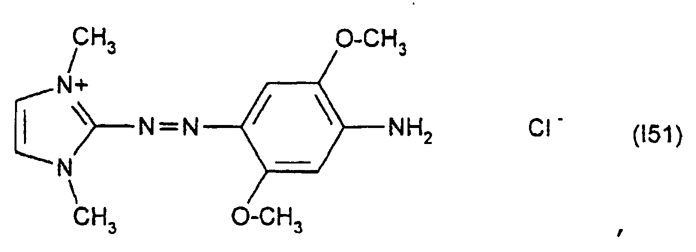 and

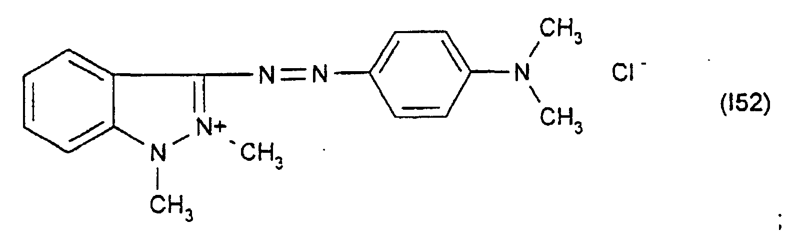

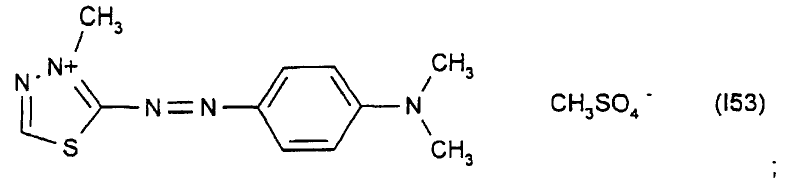

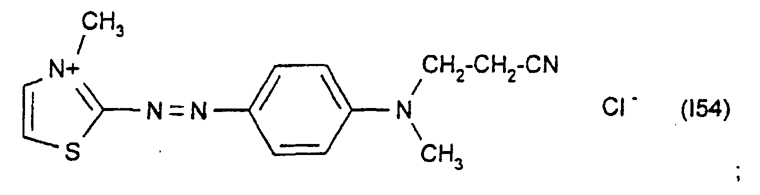

3. Composition according to Claim 2, **characterized in that** the cationic direct dyes correspond to the structures (I1), (I2), (I14), and (I31).

4. Composition according to Claim 1, **characterized in that** the cationic direct dyes of formula (II) are chosen from the compounds corresponding to the following structures (II1) to (II9): 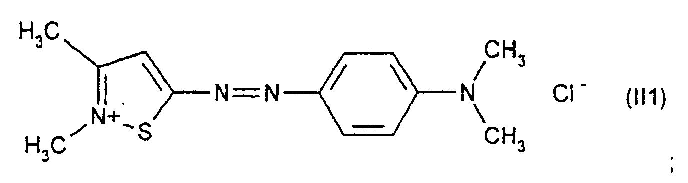 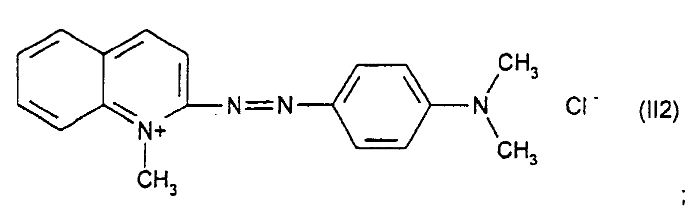 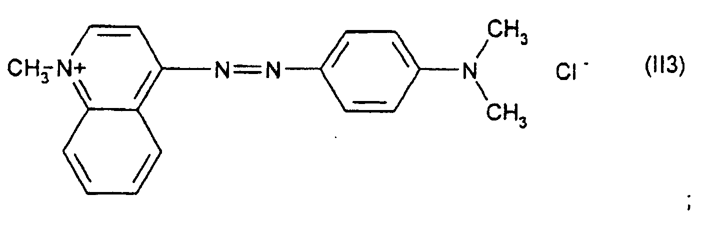 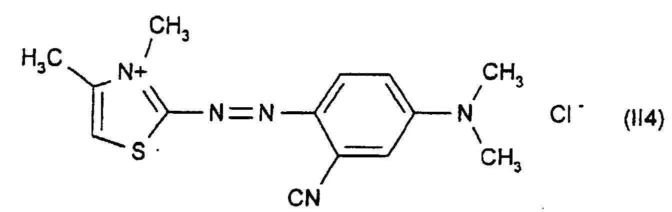 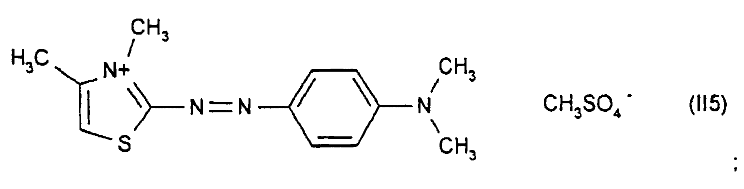 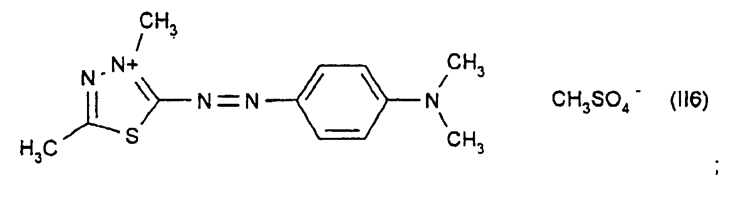 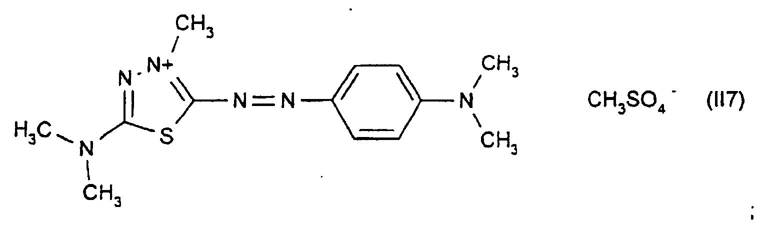 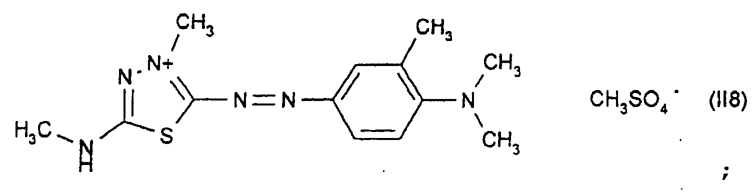 and

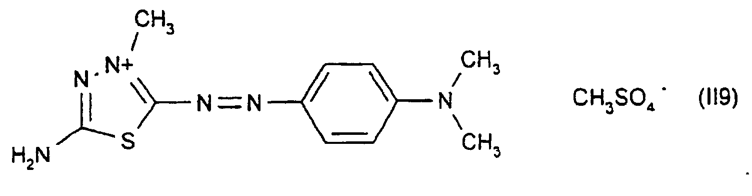

5. Composition according to Claim 1, **characterized in that** the cationic direct dyes of formula (III) are chosen from the compounds corresponding to the following structures (III1) to (III18): 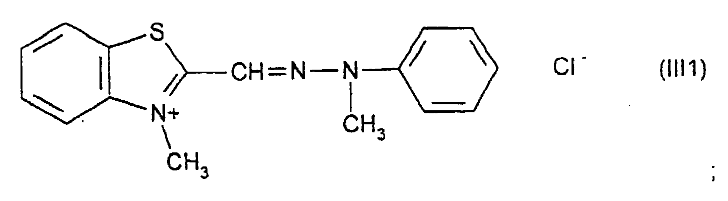 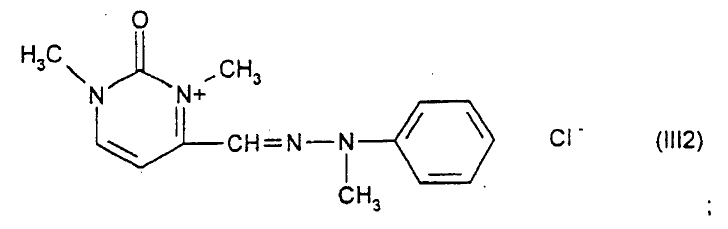 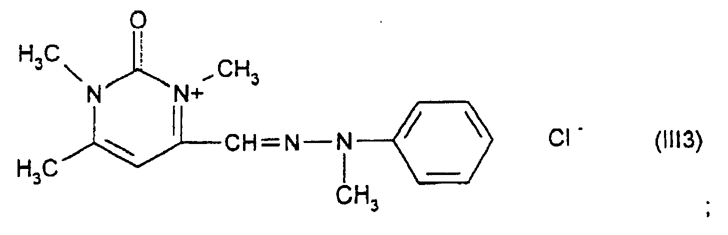 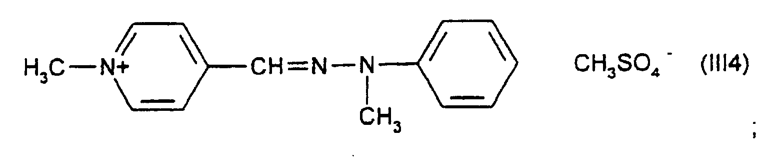 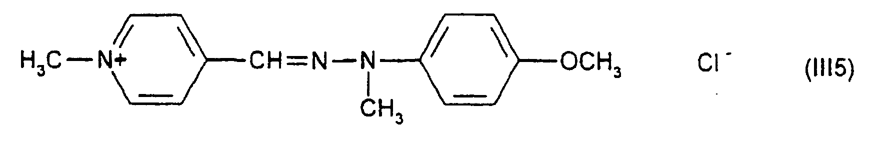 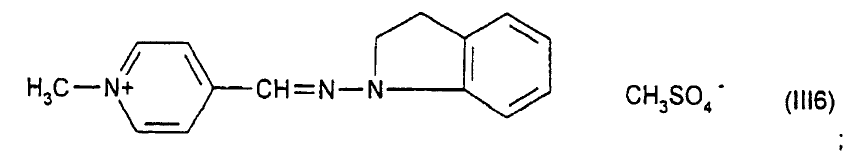 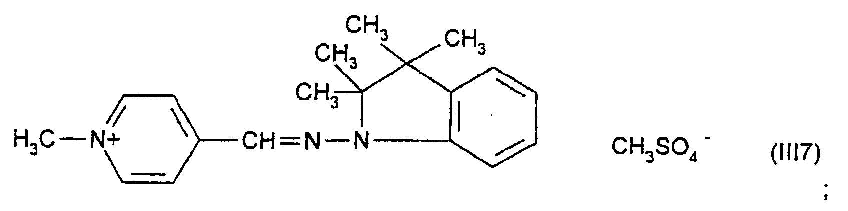 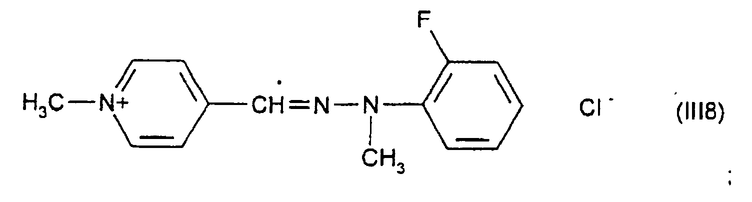 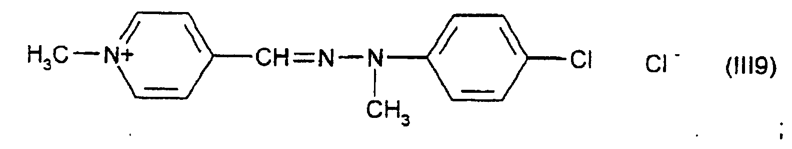 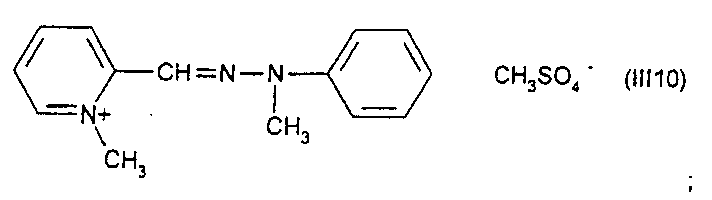 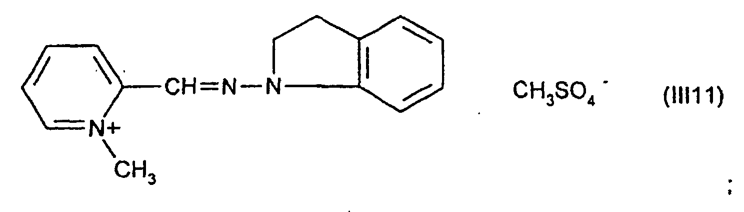 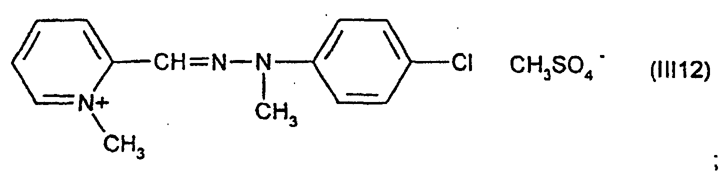 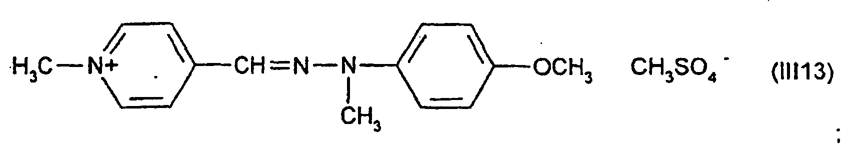 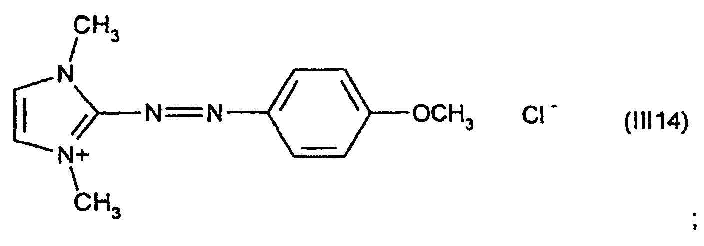 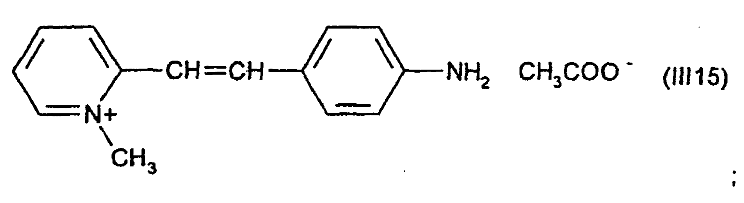 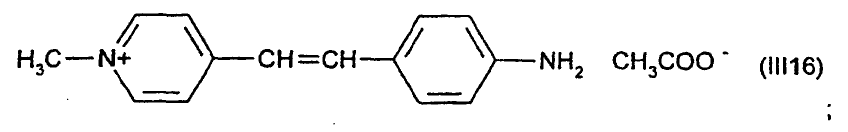 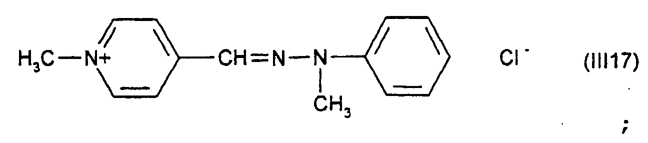 and

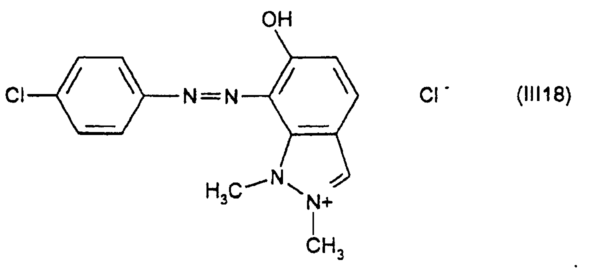

6. Composition according to Claim 5, **characterized in that** the cationic direct dyes of formula (III) are chosen from the compounds corresponding to the structures (III4), (III5) and (III13).

7. Composition according to Claim 1, **characterized in that** the cationic direct dyes of formula (III') are chosen from the compounds corresponding to the following structures (III'1) to (III'3): 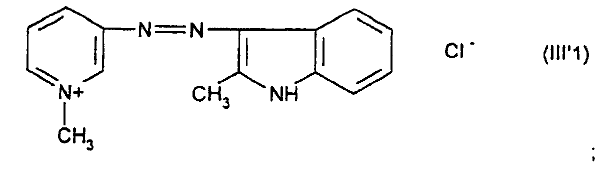 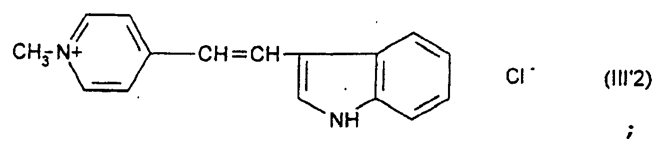 and

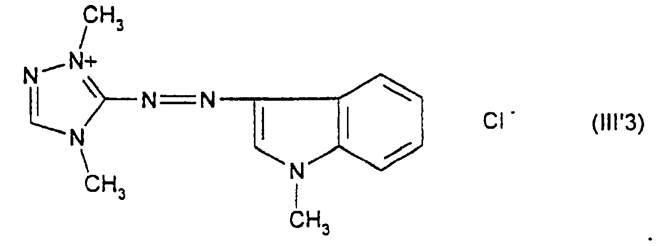

8. Composition according to Claim 1, **characterized in that** the cationic direct dyes of formula (IV) are chosen from the compounds corresponding to the following structures (IV)₁ to (IV)₇₇:

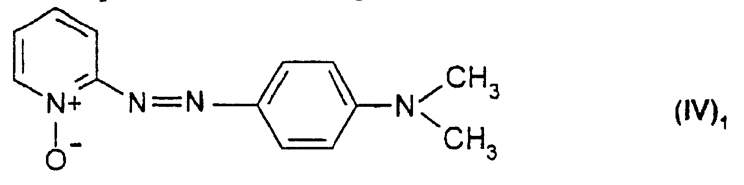

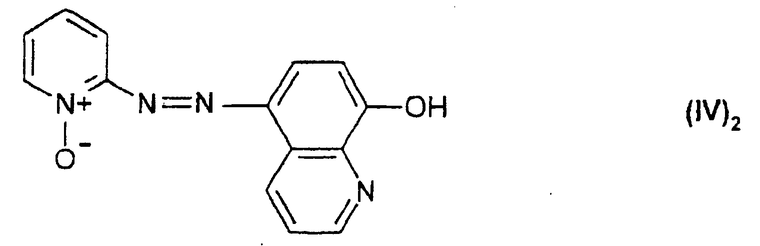

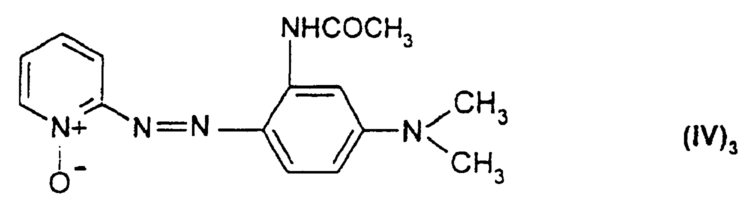

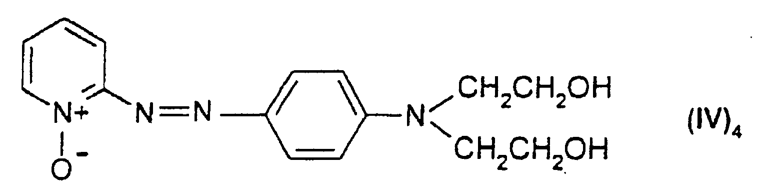

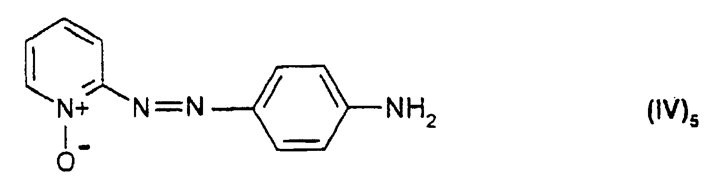

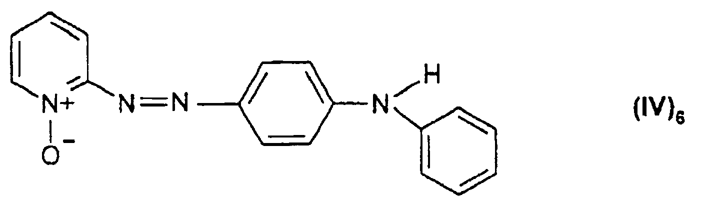

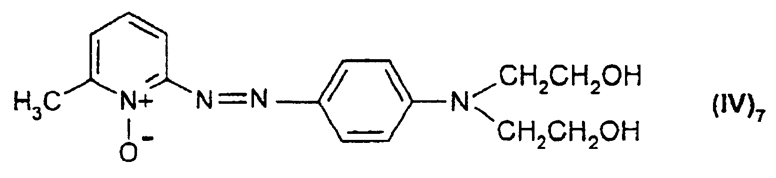

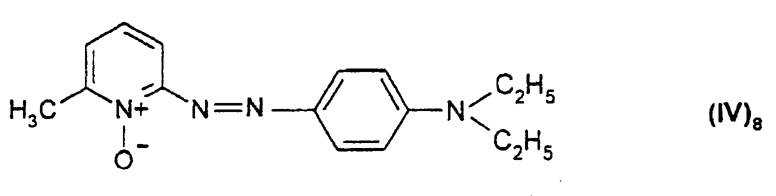

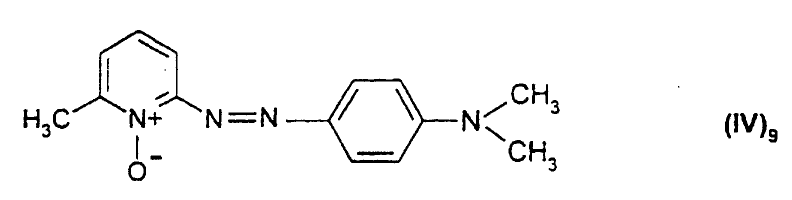

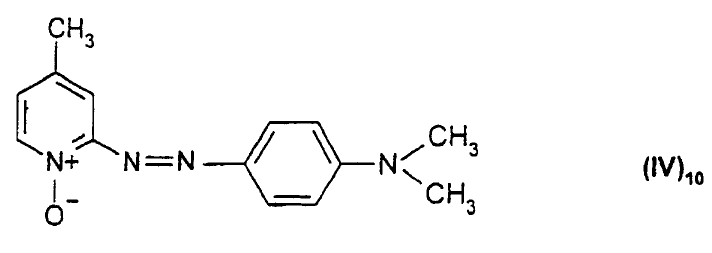

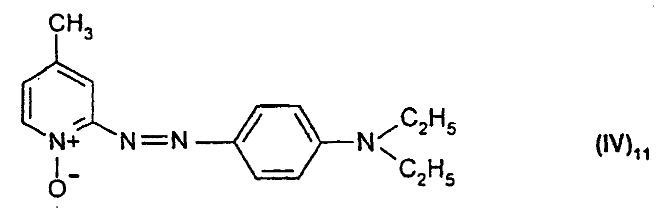

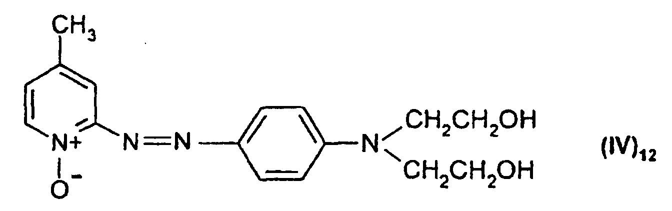

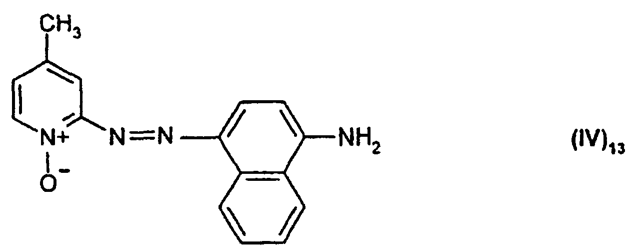

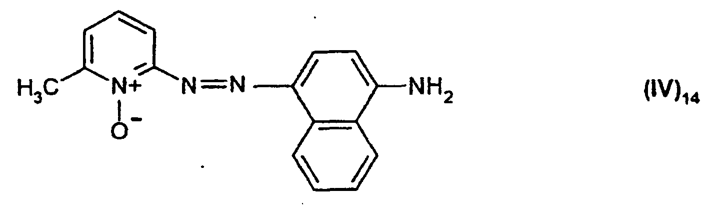

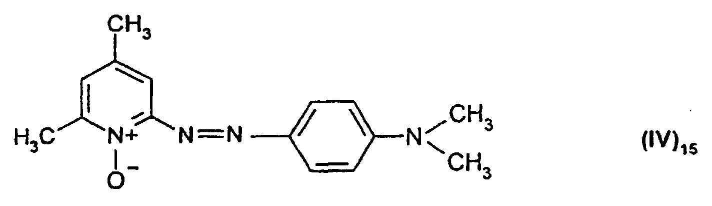

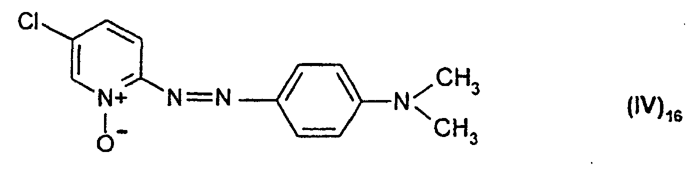

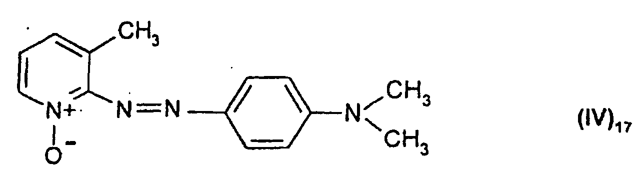

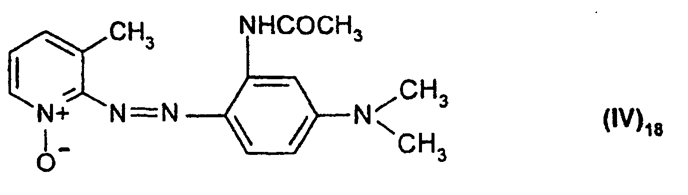

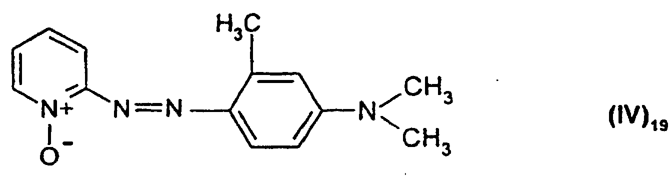

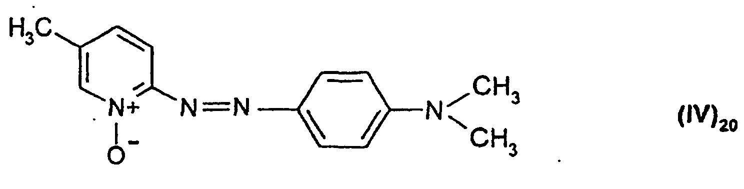

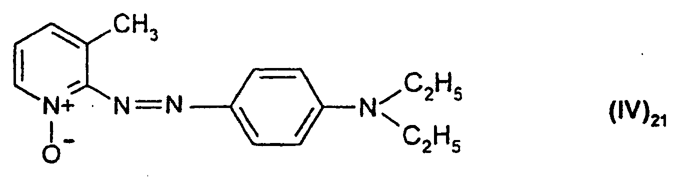

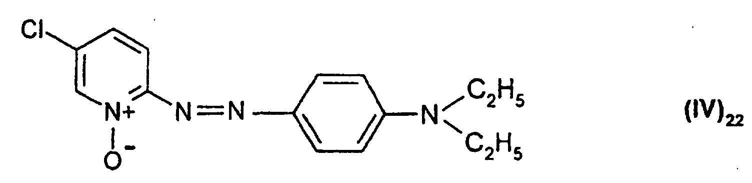

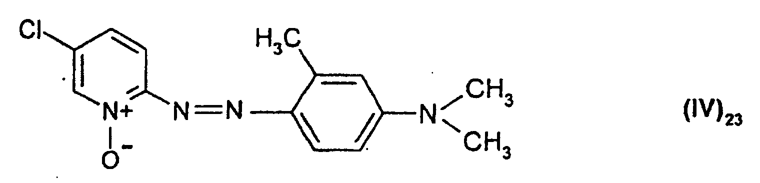

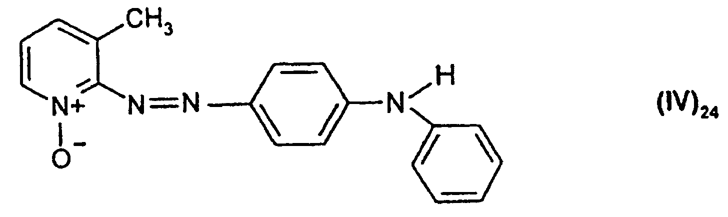

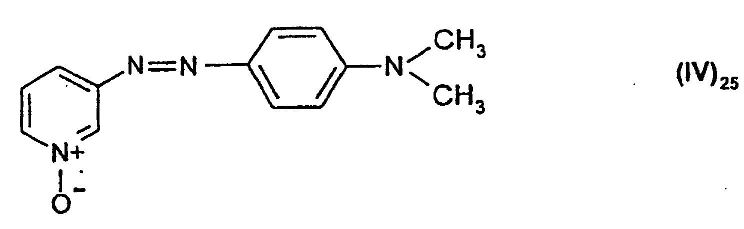

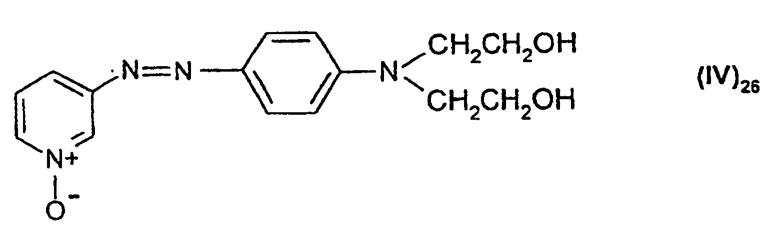

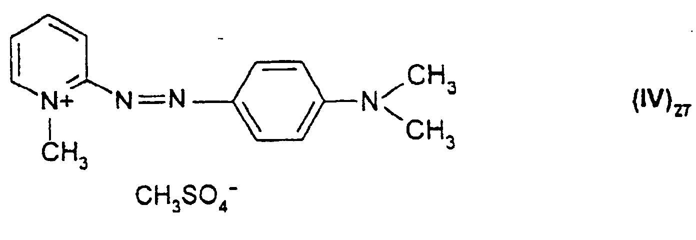

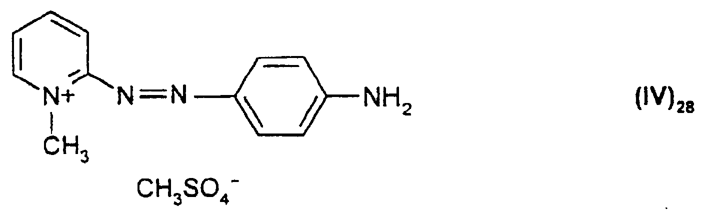

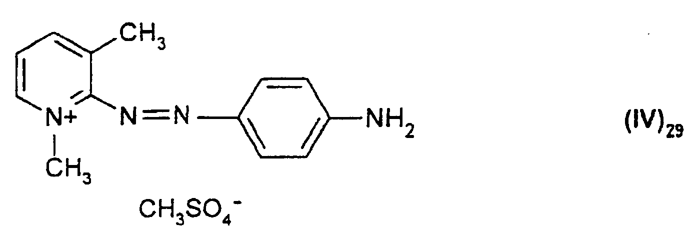

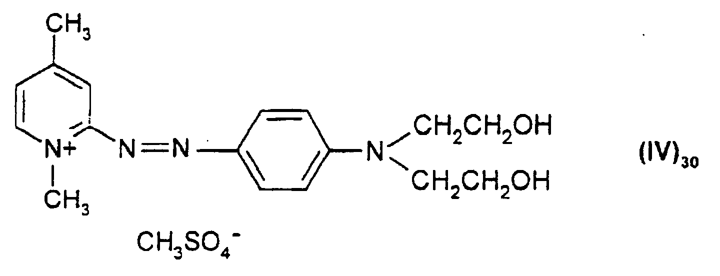

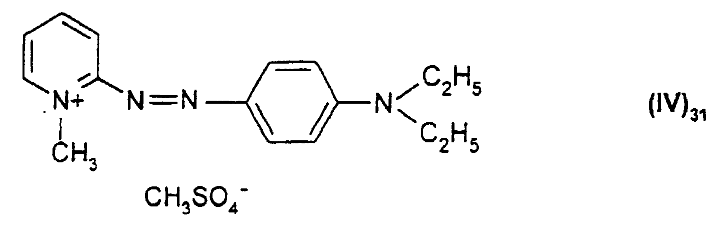

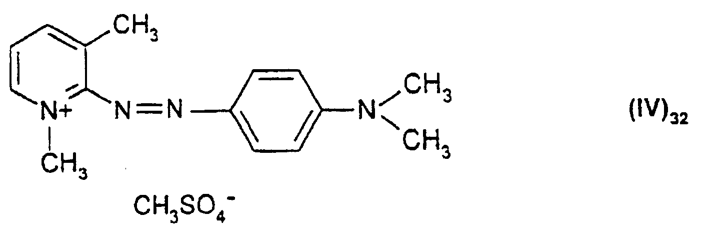

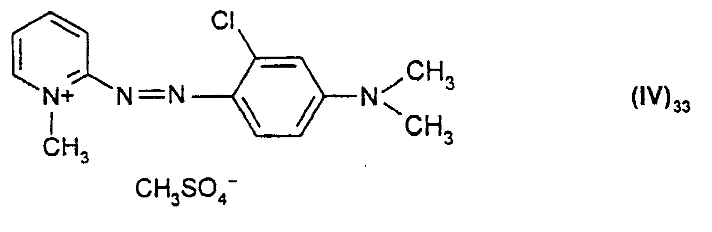

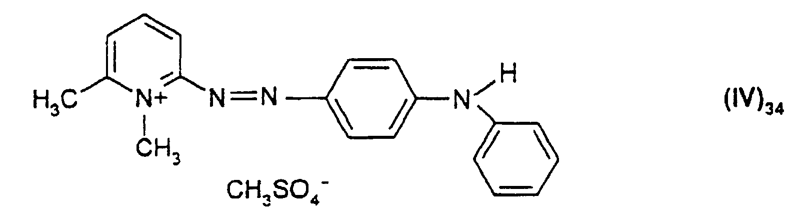

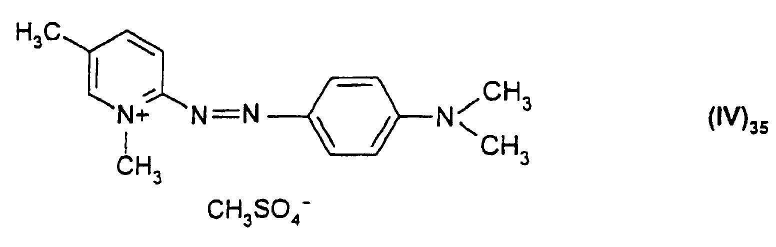

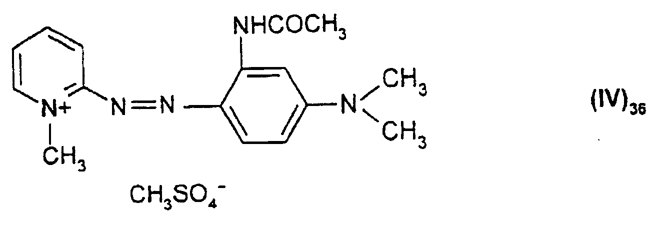

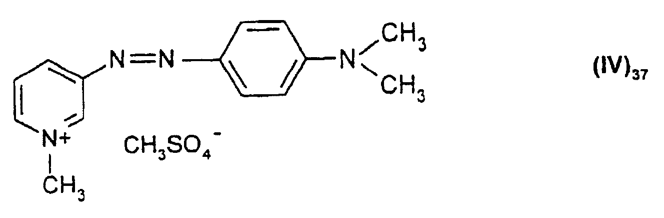

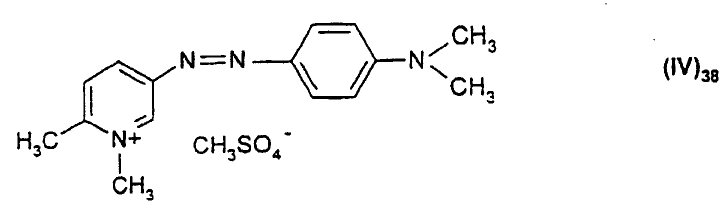

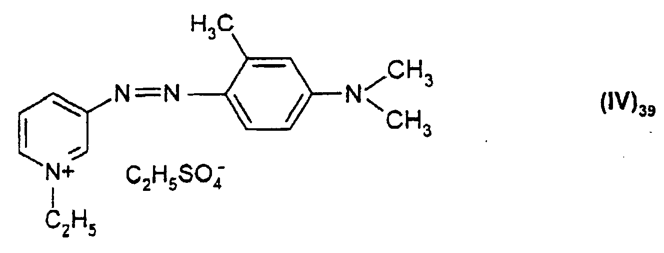

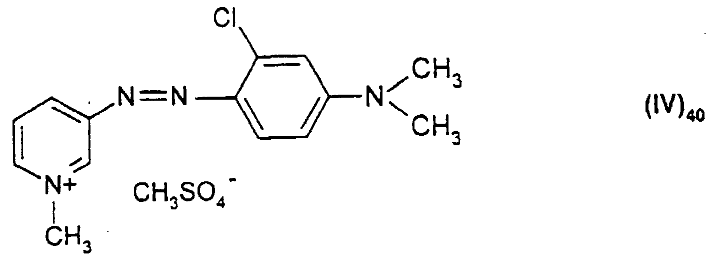

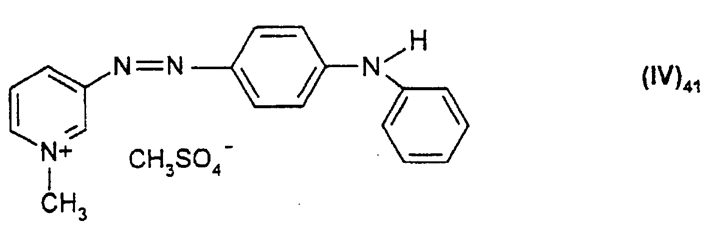

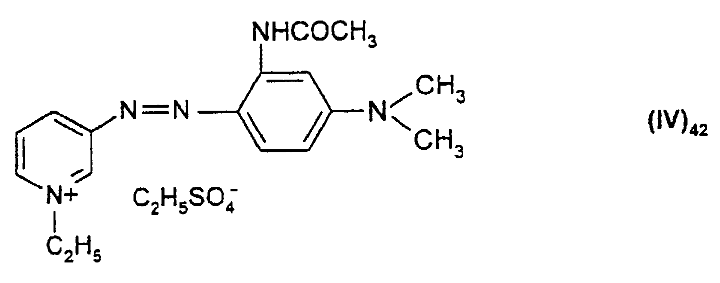

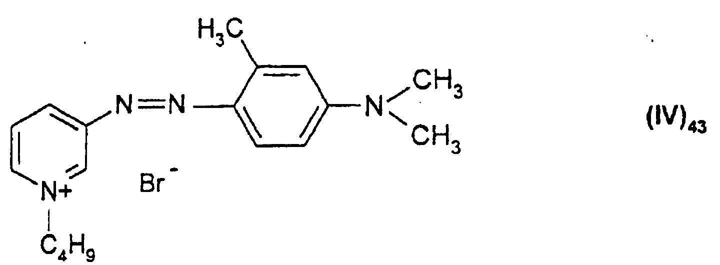

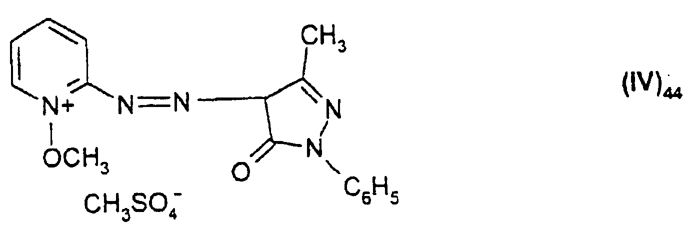

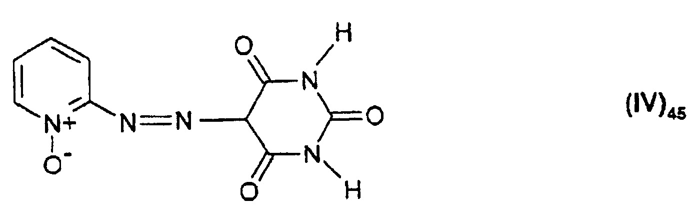

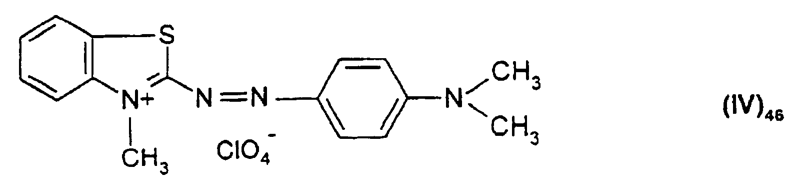

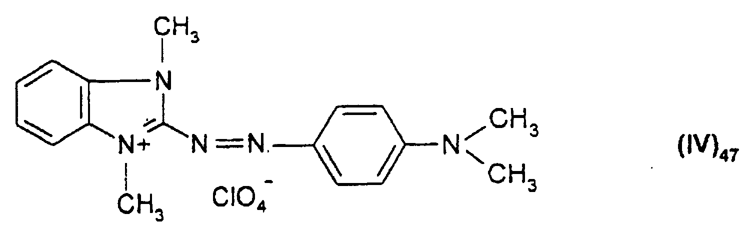

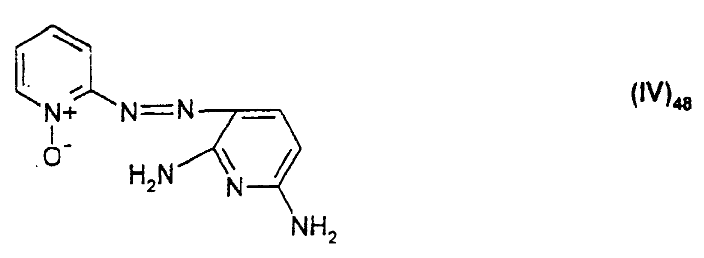

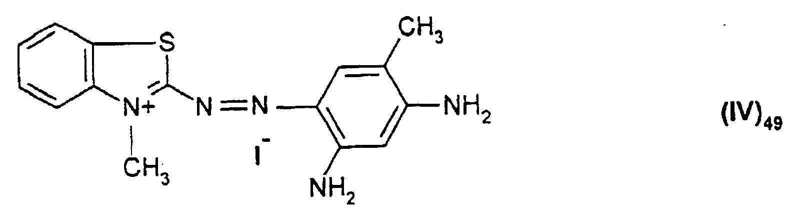

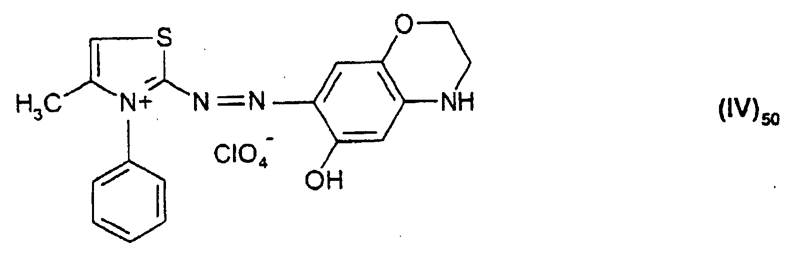

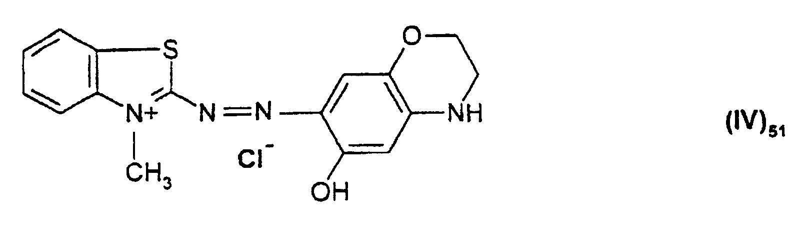

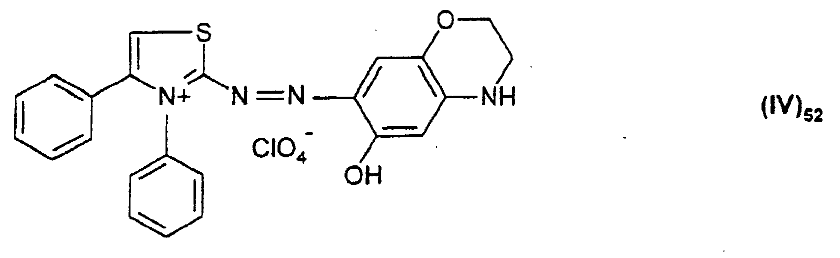

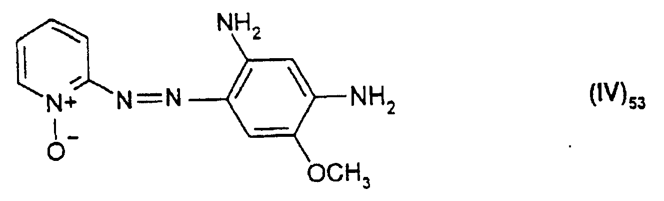

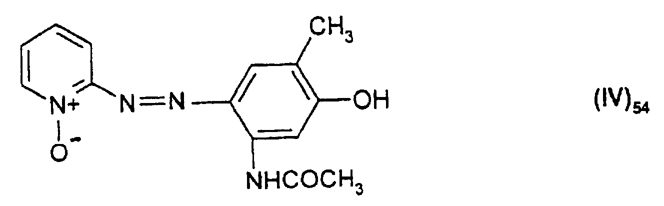

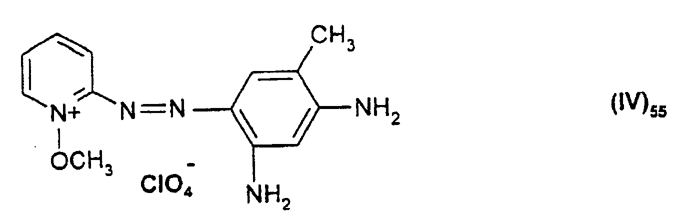

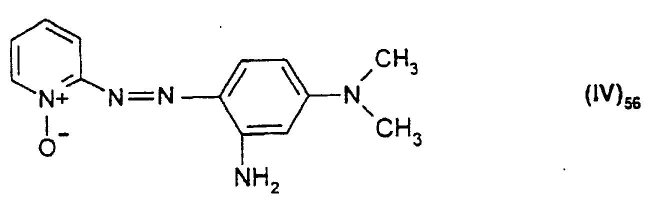

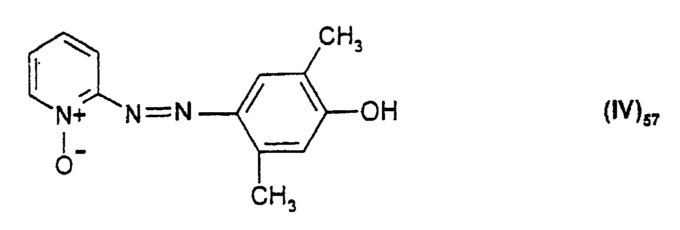

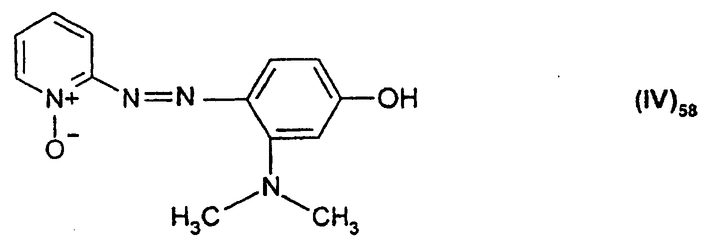

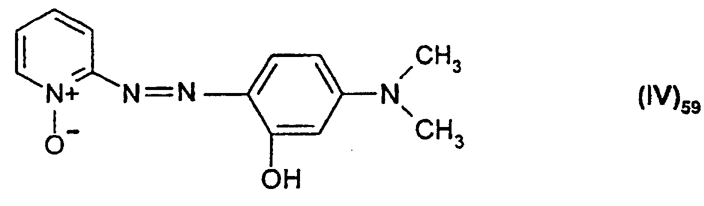

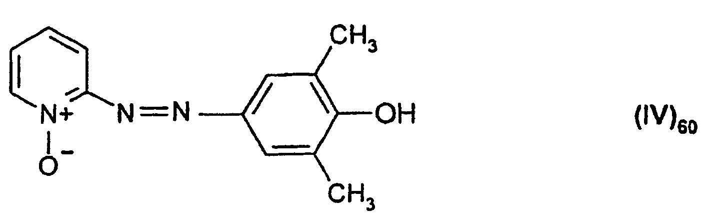

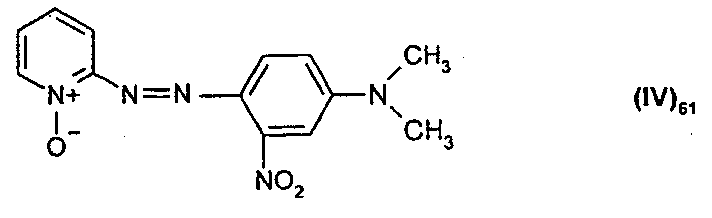

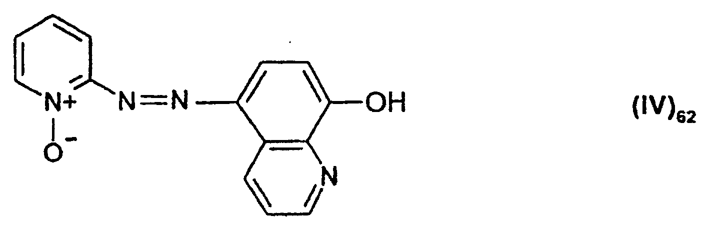

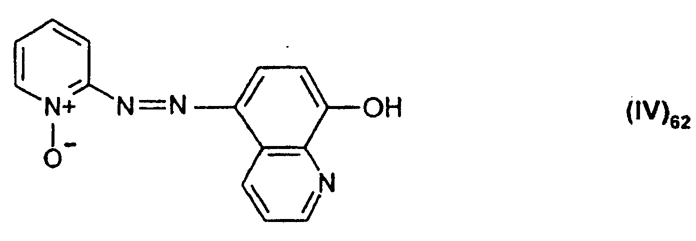

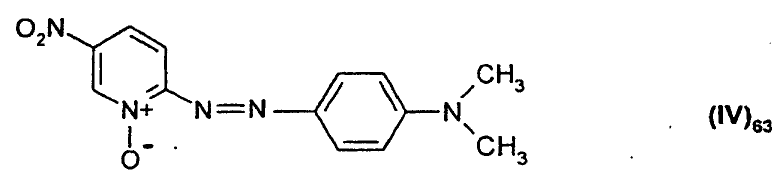

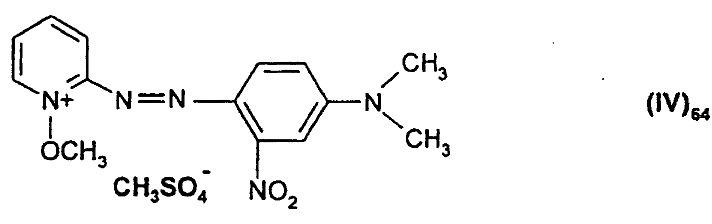

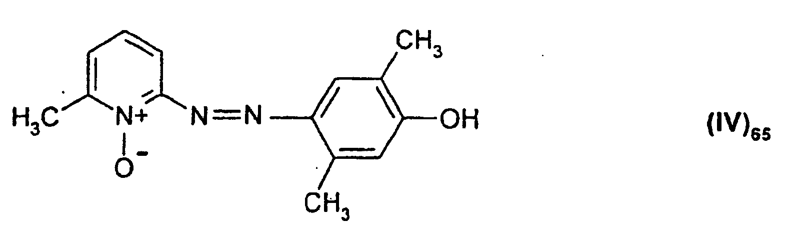

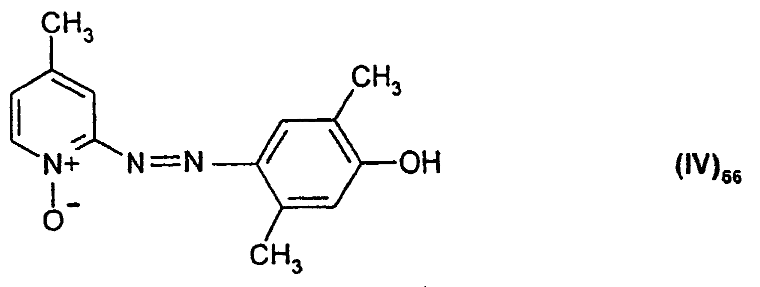

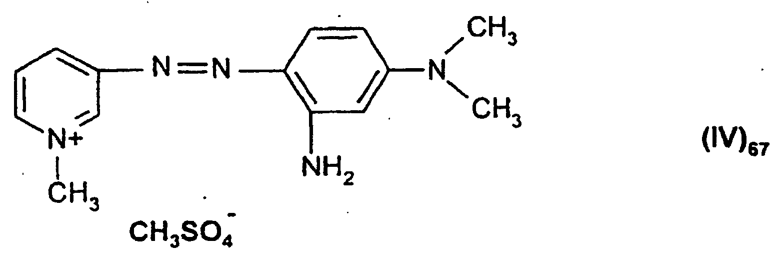

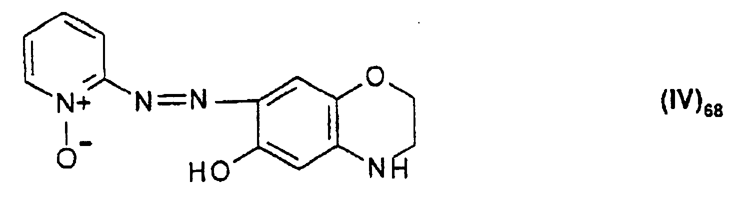

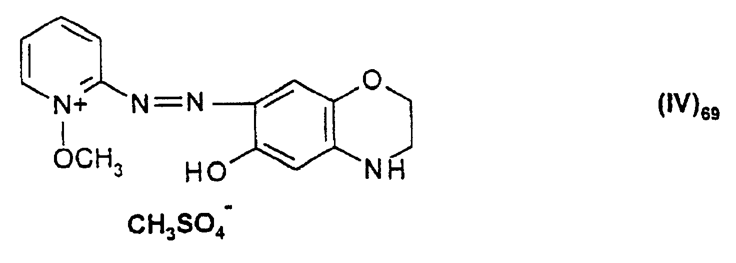

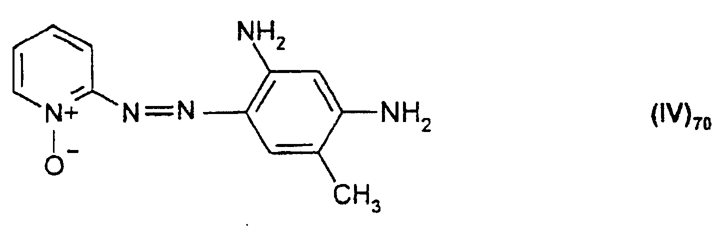

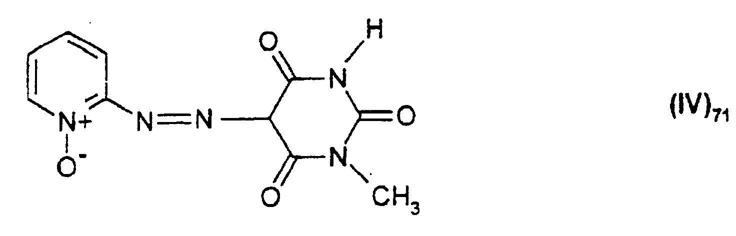

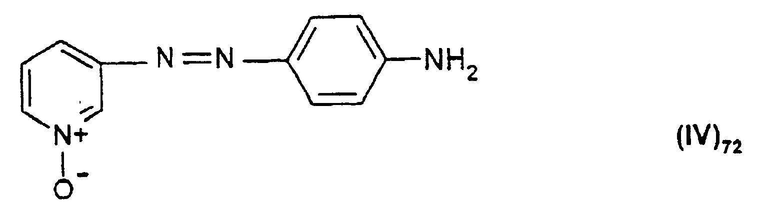

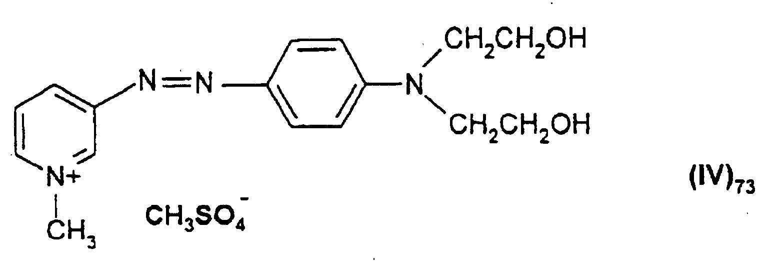

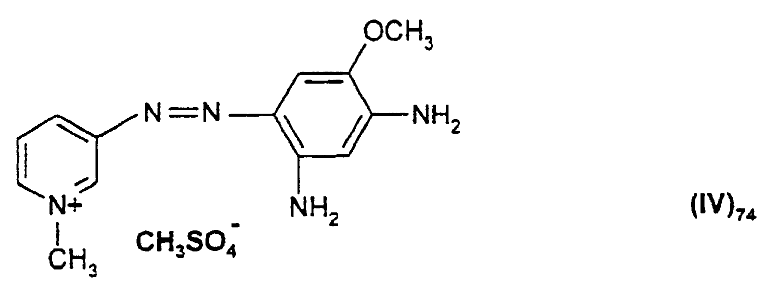

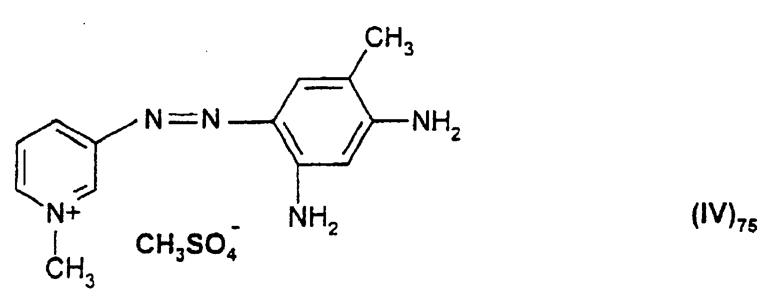

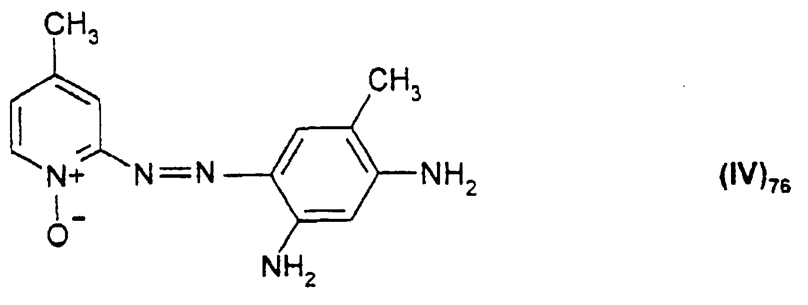

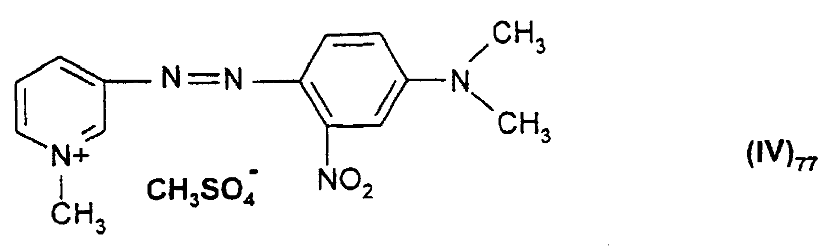

9. Composition according to any one of the preceding claims, **characterized in that** the cationic direct dye(s) of formulae (I), (II), (III), (III') or (IV) represent from 0.001 to 10% by weight of the total weight of the composition.

10. Composition according to Claim 9, **characterized in that** the cationic direct dye(s) of formulae (I), (II), (III), (III') or (IV) represent from 0.005 to 5% by weight of the total weight of the composition.

11. Composition according to any one of the preceding claims, **characterized in that** the quaternary ammonium salt (ii) of formula (V) is a dialkyldimethylammonium or alkyltrimethylammonium salt in which the alkyl radical comprises from 12 to 22 carbon atoms.

12. Composition according to Claim 11, **characterized in that** it is distearyldimethylammonium chloride, cetyltrimethylammonium chloride or behenyltrimethylammonium chloride.

13. Composition according to any one of the preceding claims, **characterized in that** the quaternary ammonium salt (ii) of formula (V) is a di(C₁-C₂ alkyl) (C₁₂-C₂₂ alkyl) hydroxy (C₁-C₂ alkyl) ammonium salt.

14. Composition according to Claim 13, **characterized in that** it is oleocetylhydroxyethylammonium chloride.

15. Composition according to any one of the preceding claims, **characterized in that** the quaternary ammonium salt (ii) of formula (V) is stearamidopropyldimethyl (myristyl acetate) ammonium chloride of formula:

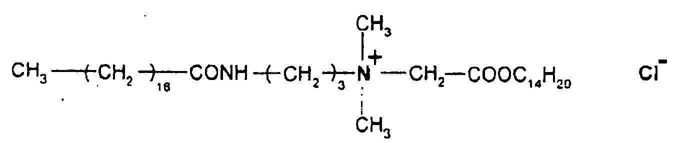

16. Composition according to any one of the preceding claims, **characterized in that** the quaternary ammonium salt (s) (ii) represent from 0.01 to 10% by weight of the total weight of the dyeing composition.

17. Composition according to Claim 16, **characterized in that** the quaternary ammonium salt(s) represent from 0.05 to 5% by weight of the total weight of the dyeing composition.

18. Composition according to any one of the preceding claims, **characterized in that** the appropriate dyeing medium (or carrier) consists of water or of a mixture of water and of at least one organic solvent.

19. Composition according to any one of the preceding claims, **characterized in that** it has a pH of between 2 and 11, and preferably between 5 and 10.

20. Composition according to any one of the preceding claims, **characterized in that** it is intended for oxidation dyeing and **in that** it contains one or more oxidation bases chosen from the para-phenylenediamines, the bis-phenylalkylenediamines, the para-aminophenols, the ortho-aminophenols and the heterocyclic bases.

21. Composition according to Claim 20, **characterized in that** the oxidation base(s) represent 0.0005 to 12% by weight of the total weight of the dyeing composition.

22. Composition according to Claim 21, **characterized in that** the oxidation base(s) represent 0.005 to 6% by weight of the total weight of the dyeing composition.

23. Composition according to any one of Claims 20 to 22, **characterized in that** it contains one or more couplers chosen from the the meta-phenylenediamines, the meta-aminophenols, the meta-diphenols and the heterocyclic couplers.

24. Composition according to Claim 23, **characterized in that** the coupler(s) represent from 0.0001 to 10% by weight of the total weight of the dyeing composition.

25. Composition according to Claim 24, **characterized in that** the coupler(s) represent from 0.005 to 5% by weight of the total weight of the dyeing composition.

26. Composition according to any one of the preceding claims, **characterized in that** it is intended for direct lightening dyeing or oxidation dyeing and **in that** it then contains at least one oxidizing agent.

27. Method of dyeing keratinous fibres and in particular human keratinous fibres such as hair, **characterized in that** at least one dyeing composition as defined in any one of Claims 1 to 26 is applied to the fibres for a sufficient time to develop the desired colour, after which they are rinsed, optionally washed with shampoo, rinsed again and dried.

28. Method of dyeing keratinous fibres and in particular human keratinous fibres such as hair, **characterized in that** at least one dyeing composition as defined in any one of Claims 1 to 26 is applied to the fibres for a sufficient time to develop the desired colour, with no final rinsing.

29. Method of dyeing keratinous fibres and in particular human keratinous fibres such as hair, **characterized in that** it comprises a preliminary stage consisting of storing in a separate form, on the one hand, a composition (A1) comprising, in an appropriate dyeing medium, at least one cationic direct dye (i) as defined in the preceding claims and at least one oxidation base and, on the other hand, a composition (B1) containing, in an appropriate dyeing medium, at least one oxidizing agent, and then mixing them at the time of use before applying this mixture to the keratinous fibres, the composition (A1) or the composition (B1) containing the quaternary ammonium salt (ii) as defined in the preceding claims.

30. Method of dyeing keratinous fibres and in particular human keratinous fibres such as hair, **characterized in that** it comprises a preliminary stage consisting of storing in a separate form, on the one hand, a composition (A2) comprising, in an appropriate dyeing medium, at least one cationic direct dye (i) as defined in the preceding claims and, on the other hand, a composition (B2) containing, in an appropriate dyeing medium, at least one oxidizing agent, and then mixing them at the time of use before applying this mixture to the keratinous fibres, the composition (A2) or the composition (B2) containing the quaternary ammonium salt (ii) as defined in the preceding claims.

31. Multicompartment device or multicompartment dyeing "kit", **characterized in that** a first compartment contains composition (A1) or (A2) as defined in Claim 29 or 30 and a second compartment contains composition (B1) or (B2) as defined in Claim 29 or 30.
